# EUROPEAN PATENT SPECIFICATION

(11) **EP 3 938 340 B1**
(45) Date of publication and mention of the grant of the patent: **09.07.2025**
(21) Application number: 20770967.6
(22) Date of filing: 10.03.2020
(51) Int. Cl.: C07C 211/50, A61K 31/136, A61K 31/395, C07C 211/51

(54) **HETEROAROMATIC AND HETEROBICYCLIC AROMATIC DERIVATIVES FOR THE TREATMENT OF FERROPTOSIS-RELATED DISORDERS**
HETEROAROMATISCHE UND HETEROBICYCLISCHE AROMATISCHE DERIVATE ZUR BEHANDLUNG VON STÖRUNGEN IM ZUSAMMENHANG MIT FERROPTOSE
DÉRIVÉS AROMATIQUES HÉTÉROBICYCLIQUES ET HÉTÉROAROMATIQUES POUR LE TRAITEMENT DE TROUBLES LIÉS À LA FERROPTOSE

(30) Priority: 11.03.2019 US 201962816373 P
(43) Date of publication of application: 19.01.2022
(73) Proprietor: Collaborative Medicinal Development, LLC, Mill Valley, CA 94941 (US)
(72) Inventor: WARNER, John, Mill Valley, CA 94941 (US); BALDINO, Carmen, Mill Valley, CA 94941 (US); MUOLLO, Laura, Mill Valley, CA 94941 (US); LEE, John, Mill Valley, CA 94941 (US); CHERUKU, Srinivasa, Mill Valley, CA 94941 (US); WALKER, Rowan, Mill Valley, CA 94941 (US); SIEDLECKI, James, Mill Valley, CA 94941 (US); PROUDFOOT, John, Mill Valley, CA 94941 (US); JOHNSTONE, Shawn, Mill Valley, CA 94941 (US); NUMA, Mehdi, Mill Valley, CA 94941 (US); ROSENFELD, Craig, Mill Valley, CA 94941 (US)
(74) Representative: Aera A/S
(86) International application number: PCT/US2020/021820
(87) International publication number: WO 2020/185738

(56) References cited:
- WO-A1-2013/152039
- WO-A1-2017/024408
- WO-A1-2019/058393
- WO-A1-2019/106434
- WO-A1-2019/154795
- WO-A1-2019/241746
- WO-A1-2020/113028
- WO-A1-2022/020150
- US-A1- 2007 032 469
- US-A1- 2015 079 035
- US-A1- 2015 366 893
- SHENG XIE-HUANG ET AL: "O -Phenylenediamine: a privileged pharmacophore of ferrostatins for radical-trapping reactivity in blocking ferroptosis", ORGANIC & BIOMOLECULAR CHEMISTRY, vol. 16, no. 21, 1 January 2018 (2018-01-01), pages 3952 - 3960, XP055975425, ISSN: 1477-0520, DOI: 10.1039/C8OB00546J
- SAM HOFMANS ET AL: "Novel Ferroptosis Inhibitors with Improved Potency and ADME Properties", JOURNAL OF MEDICINAL CHEMISTRY, vol. 59, no. 5, 8 February 2016 (2016-02-08), US, pages 2041 - 2053, XP055485611, ISSN: 0022-2623, DOI: 10.1021/acs.jmedchem.5b01641
- RACHID SKOUTA ET AL: "Ferrostatins Inhibit Oxidative Lipid Damage and Cell Death in Diverse Disease Models", JOURNAL OF THE AMERICAN CHEMICAL SOCIETY, vol. 136, no. 12, 14 March 2014 (2014-03-14), pages 4551 - 4556, XP055648750, ISSN: 0002-7863, DOI: 10.1021/ja411006a
- DEVISSCHER ET AL: "Discovery of Novel, Drug-Like Ferroptosis Inhibitors with in Vivo Efficacy", JOURNAL OF MEDICINAL CHEMISTRY, AMERICAN CHEMICAL SOCIETY, US, vol. 61, no. 22, 25 October 2018 (2018-10-25), pages 10126 - 10140, XP002790074, ISSN: 0022-2623, DOI: 10.1021/ACS.JMEDCHEM.8B01299
- CAO JENNIFER YINUO ET AL: "Mechanisms of ferroptosis", CMLS CELLULAR AND MOLECULAR LIFE SCIENCES, BIRKHAUSER VERLAG, HEIDELBERG, DE, vol. 73, no. 11, 5 April 2016 (2016-04-05), pages 2195 - 2209, XP035858596, ISSN: 1420-682X, [retrieved on 20160405], DOI: 10.1007/S00018-016-2194-1

## Description

### TECHNICAL FIELD

Described herein are heterobicyclic aromatic compounds, compositions and the compounds for use in a method of treating of ferroptosis-related disorders including lipid peroxidation-related degenerative diseases, excitotoxic diseases, neurodegenerative diseases, non-apoptotic regulated cell-death diseases, wasting- or necrosis-related diseases, intoxication-related diseases, and infectious diseases.

### BACKGROUND

Presently, there are no known prevention or cure for neurodegenerative diseases or disorders such as Alzheimer's disease (AD) and Parkinson's disease (PD). The present application discloses compounds, compositions and methods for the treatment of such diseases or disorders.

### SUMMARY

Described herein are compounds, compositions and methods for treatment of lipid peroxidation-related degenerative diseases, excitotoxic diseases, neurodegenerative diseases, non-apoptotic regulated cell-death diseases, wasting- or necrosis-related diseases, intoxication-related diseases, and infectious diseases.

Accordingly, described herein a compound of formula II: 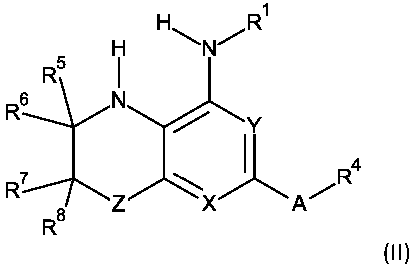 wherein
R¹ is selected from the group consisting of H, substituted or unsubstituted C₁-C₁₀ linear or branched alkyl, substituted or unsubstituted C₂-C₁₀ linear or branched alkenyl, substituted or unsubstituted C₂-C₁₀ linear or branched alkynyl, substituted or unsubstituted C₆-C₁₀ aryl, substituted or unsubstituted C₃-C₁₀ cycloalkyl, substituted or unsubstituted C₃-C₁₀ heterocycloalkyl, substituted or unsubstituted C₅-C₁₀ heteroaryl, substituted or unsubstituted C₆-C₁₀ arylalkyl, substituted or unsubstituted C₅-C₁₀ heteroarylalkyl, substituted or unsubstituted C₁-C₁₀ linear or branched alkylamino and substituted or unsubstituted C₁-C₁₀ linear or branched dialkylamino, or R¹ and its attached N together form a substituted or unsubstituted C₃-C₆ heterocycloalkyl or heteroaryl ring (replacing the H attached to the N);
A is selected from the group consisting of a bond, substituted or unsubstituted C₆-C₁₀ aryl, substituted or unsubstituted C₅-C₁₀ heteroaryl, substituted or unsubstituted C₂-C₁₀ linear or branched alkenyl, substituted or unsubstituted C₂-C₁₀ linear or branched alkynyl, C=O, C=S, -CH₂-, -CH(OH)-, -NH-, -N(CH₃)-, -O-, -S-, and SO₂; and R⁴ is selected from the group consisting of substituted or unsubstituted C₁-C₁₀ linear or branched alkyl, substituted or unsubstituted C₁-C₁₀ linear or branched alkoxy, substituted or unsubstituted C₁-C₁₀ linear or branched alkylamino, substituted or unsubstituted C₁-C₁₀ linear or branched dialkylamino, substituted or unsubstituted C₃-C₁₀ cycloalkyl or heterocycloalkyl, substituted or unsubstituted C₆-C₁₀ aryl, substituted or unsubstituted C₅-C₁₀ heteroaryl, -CN and halo;
R⁵, R⁶, R⁷, R⁸, R⁹ and R¹⁰ are independently selected from the group consisting of H, substituted or unsubstituted C₁-C₁₀ linear or branched alkyl, substituted or unsubstituted C₂-C₁₀ linear or branched alkenyl, substituted or unsubstituted C₂-C₁₀ linear or branched alkynyl, substituted or unsubstituted C₆-C₁₀ aryl, substituted or unsubstituted C₃-C₁₀ cycloalkyl, substituted or unsubstituted C₃-C₁₀ heterocycloalkyl, substituted or unsubstituted C₅-C₁₀ heteroaryl, substituted or unsubstituted C₆-C₁₀ arylalkyl, substituted or unsubstituted C₁-C₁₀ linear or branched alkylamino, and substituted or unsubstituted C₁-C₁₀ linear or branched dialkylamino, or R⁷ and R⁸ together are =O;
X and Y are independently selected from the group consisting of -CH- and -N-; and Z is selected from the group consisting of C=O, -CR⁹R¹⁰-, -NR⁹-, -O-, -S-, -S(O)- and - SO₂-;
and wherein a substituted group has 1, 2 or 3 -H atoms substituted by 1, 2 or 3 substituents selected from halo, trifluoromethyl, trifluoromethoxy, methoxy, -COOH, -CHO, -NH₂, -NO₂, -OH, -SH, -SMe, -NHCH₃, -N(CH₃)₂, -CN, and lower alkyl.

Also described herein is a pharmaceutical composition comprising a therapeutically effective amount of a compound of formula II as described above, and a pharmaceutically acceptable excipient.

Also further described herein is a compound of formula II: 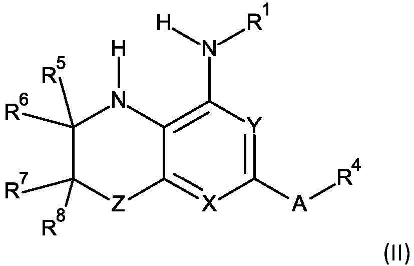 wherein
R¹ is selected from the group consisting of H, substituted or unsubstituted C₁-C₁₀ linear or branched alkyl, substituted or unsubstituted C₂-C₁₀ linear or branched alkenyl, substituted or unsubstituted C₂-C₁₀ linear or branched alkynyl, substituted or unsubstituted C₆-C₁₀ aryl, substituted or unsubstituted C₃-C₁₀ cycloalkyl, substituted or unsubstituted C₃-C₁₀ heterocycloalkyl, substituted or unsubstituted C₅-C₁₀ heteroaryl, substituted or unsubstituted C₆-C₁₀ arylalkyl, substituted or unsubstituted C₅-C₁₀ heteroarylalkyl, substituted or unsubstituted C₁-C₁₀ linear or branched alkylamino and substituted or unsubstituted C₁-C₁₀ linear or branched dialkylamino, or R¹ and its attached N together form a substituted or unsubstituted C₃-C₆ heterocycloalkyl or heteroaryl ring (replacing the H attached to the N);
A is selected from the group consisting of a bond, substituted or unsubstituted C₆-C₁₀ aryl, substituted or unsubstituted C₅-C₁₀ heteroaryl, substituted or unsubstituted C₂-C₁₀ linear or branched alkenyl, substituted or unsubstituted C₂-C₁₀ linear or branched alkynyl, C=O, C=S, -CH₂-, -CH(OH)-, -NH-, -N(CH₃)-, -O-, -S-, and SO₂; and
R⁴ is selected from the group consisting of substituted or unsubstituted C₁-C₁₀ linear or branched alkyl, substituted or unsubstituted C₁-C₁₀ linear or branched alkoxy, substituted or unsubstituted C₁-C₁₀ linear or branched alkylamino, substituted or unsubstituted C₁-C₁₀ linear or branched dialkylamino, substituted or unsubstituted C₃-C₁₀ cycloalkyl or heterocycloalkyl, substituted or unsubstituted C₆-C₁₀ aryl, substituted or unsubstituted C₅-C₁₀ heteroaryl, -CN and halo;
R⁵, R⁶, R⁷, R⁸, R⁹ and R¹⁰ are independently selected from the group consisting of H, substituted or unsubstituted C₁-C₁₀ linear or branched alkyl, substituted or unsubstituted C₂-C₁₀ linear or branched alkenyl, substituted or unsubstituted C₂-C₁₀ linear or branched alkynyl, substituted or unsubstituted C₆-C₁₀ aryl, substituted or unsubstituted C₃-C₁₀ cycloalkyl, substituted or unsubstituted C₃-C₁₀ heterocycloalkyl, substituted or unsubstituted C₅-C₁₀ heteroaryl, substituted or unsubstituted C₆-C₁₀ arylalkyl, substituted or unsubstituted C₁-C₁₀ linear or branched alkylamino, and substituted or unsubstituted C₁-C₁₀ linear or branched dialkylamino, or R⁷ and R⁸ together are =O;
X and Y are independently selected from the group consisting of -CH- and -N-; and Z is selected from the group consisting of C=O, -CR⁹R¹⁰-, -NR⁹-, -O-, -S-, -S(O)- and - SO₂-;
and wherein a substituted group has 1, 2 or 3 -H atoms substituted by 1, 2 or 3 substituents selected from halo, trifluoromethyl, trifluoromethoxy, methoxy, -COOH, -CHO, -NH₂, -NO₂, -OH, -SH, -SMe, -NHCH₃, -N(CH₃)₂, -CN, and lower alkyl for use in a method of treating a ferroptosis-related disease in a patient in need thereof comprising administering to the patient a therapeutically effective amount of the compound of formula II.

The ferroptosis-related disease may be selected from the group consisting of lipid peroxidation-related degenerative diseases, excitotoxic diseases, neurodegenerative diseases, non-apoptotic regulated cell-death diseases, wasting- or necrosis-related diseases, intoxication-related diseases, and infectious diseases.

The ferroptosis-related disease may also be selected from the group consisting of atherosclerosis, ischemia-reperfusion, heart failure, Alzheimer's disease, rheumatic arthritis, thalassemia, chronic obstructive pulmonary disease (COPD), age-related macular degeneration, senescence, cancer, and immunological disorders (including autoimmune diseases (for example, diabetes mellitus, arthritis (including rheumatoid arthritis, juvenile rheumatoid arthritis, osteoarthritis and psoriatic arthritis), multiple sclerosis, encephalomyelitis, myasthenia gravis, systemic lupus erythematosus, autoimmune thyroiditis, dermatitis (including atopic dermatitis and eczematous dermatitis), psoriasis, Sjogren's Syndrome, Crohn's disease, aphthous ulcer, iritis, conjunctivitis, keratoconjunctivitis, ulcerative colitis, asthma, allergic asthma, sepsis and septic shock, inflammatory bowel disorder, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens- Johnson syndrome, glomerulonephritis, idiopathic sprue, lichen planus, Graves' disease, sarcoidosis, primary biliary cirrhosis, uveitis posterior, interstitial lung fibrosis, graft-versus-host disease, transplantation rejection, allergies such as atopic allergy, epilepsy, kidney disease, stroke, myocardial infarction, congestive heart failure, type I diabetes, traumatic brain injury (TBI), periventricular leukomalacia (PVL), Alzheimer's disease, Parkinson's disease, Amyotrophic lateral sclerosis, Friedreich's ataxia, ataxia-telangiectasia, Rett syndrome, X-linked adrenoleukodystrophy, Multiple sclerosis, Huntington's Disease, transmissible spongiform encephalopathy, Charcot-Marie-Tooth disease, Lewy body dementia, Menke's disease, Wilson's disease, Creutzfeldt-Jakob disease, Fahr disease, frontotemporal dementia, amyloidosis, Tay-Sachs disease periventricular leukomalacia, corticobasal degeneration, progressive supranuclear palsy, hereditary spastic paraparesis, a reduction in cell-proliferation, an alteration in cell-differentiation or intracellular signaling, undesirable inflammation, cell death of retinal neuronal cells, cardiac muscle cells, or cells of the immune system or cell death associated with renal failure, neonatal respiratory distress, asphyxia, incarcerated hernia, placental infarct, iron-load complications, endometriosis, congenital disease, head trauma/traumatic brain injury, liver injury, injuries from environmental radiation, burns, cold injuries, mechanical injuries, decompression sickness, priapism, snake, scorpion or spider bites, UV-damage in skin, aging in skin, hair loss, muscle wasting diseases, muscular dystrophies or related diseases (e.g., Becker's muscular dystrophy, Duchenne muscular dystrophy, myotonic dystrophy, limb-girdle muscular dystrophy, Landouzy-Dejerine muscular dystrophy, facioscapulohumeral muscular dystrophy (Steinert's disease), myotonia congenita, Thomsen's disease, and Pompe's disease, ischemia, compartment syndrome, gangrene, pressure sores, sepsis, degenerative arthritis, retinal necrosis, heart disease, liver, gastrointestinal or pancreatic necrotic diseases (such as acute necrotizing pancreatitis), avascular necrosis, diabetes, sickle cell disease, alteration of blood vessels, cancer-chemo/radiation therapy-induced cell-death, and infectious diseases caused by infection by viruses, bacteria, fungi, or other microorganisms.

### DETAILED DESCRIPTION

### DEFINITIONS

Unless specifically noted otherwise herein, the definitions of the terms used are standard definitions used in the art of organic chemistry and pharmaceutical sciences.

Unless defined otherwise, all technical and scientific terms used herein have the same meaning as is commonly understood by one of skill in the art.

As used in the specification and claims, the singular form "a," "an," and "the" include plural references unless the context clearly dictates otherwise.

The term "effective amount" or "therapeutically effective amount" refers to that amount of a compound described herein that is sufficient to effect the intended application including disease treatment, as defined below. The therapeutically effective amount may vary depending upon the intended application (*in vitro* or *in vivo*), or the subject and disease condition being treated, *e.g.,* the weight and age of the subject, the severity of the disease condition, the manner of administration and the like, which can readily be determined by one of ordinary skill in the art. The term also applies to a dose that will induce a particular response in target cells, *e.g.* reduction of platelet adhesion and/or cell migration. The specific dose will vary depending on the particular compounds chosen, the dosing regimen to be followed, whether it is administered in combination with other compounds, timing of administration, the tissue to which it is administered, and the physical delivery system in which it is carried.

The terms "treatment," "treating," "palliating," and "ameliorating" are used interchangeably herein. These terms refer to an approach for obtaining beneficial or desired results including therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient may still be afflicted with the underlying disorder. For prophylactic benefit, the compositions may be administered to a patient at risk of developing a particular disease, or to a patient reporting one or more of the physiological symptoms of a disease, even though a diagnosis of this disease may not have been made.

A "therapeutic effect," as used herein, encompasses a therapeutic benefit and/or a prophylactic benefit as described above. A prophylactic effect includes delaying or eliminating the appearance of a disease or condition, delaying or eliminating the onset of symptoms of a disease or condition, slowing, halting, or reversing the progression of a disease or condition, or any combination thereof.

The term "co-administration," "administered in combination with," and their grammatical equivalents, as used herein, encompass administration of two or more agents to an animal so that both agents and/or their metabolites are present in the animal at the same time. Co-administration includes simultaneous administration in separate compositions, administration at different times in separate compositions, or administration in a composition in which both agents are present.

A "pharmaceutically acceptable salt" means a salt composition that is generally considered to have the desired pharmacological activity, is considered to be safe, non-toxic and is acceptable for veterinary and human pharmaceutical applications. Pharmaceutically acceptable salts may be derived from a variety of organic and inorganic counter ions well known in the art and include, by way of example only, sodium, potassium, calcium, magnesium, ammonium, tetraalkylammonium, and the like; and when the molecule contains a basic functionality, salts of organic or inorganic acids, such as hydrochloride, hydrobromide, tartrate, mesylate, acetate, maleate, oxalate and the like. Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese, aluminum, and the like. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. In some embodiments, the pharmaceutically acceptable base addition salt is chosen from ammonium, potassium, sodium, calcium, and magnesium salts.

"Pharmaceutically acceptable carrier" or "pharmaceutically acceptable excipient" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions described herein is contemplated. Supplementary active ingredients can also be incorporated into the compositions.

The terms "antagonist" and "inhibitor" are used interchangeably, and they refer to a compound having the ability to inhibit a biological function of a target protein, whether by inhibiting the activity or expression of the target protein. Accordingly, the terms "antagonist" and "inhibitors" are defined in the context of the biological role of the target protein. Although antagonists herein generally interact specifically with (*e.g.* specifically bind to) the target, compounds that inhibit a biological activity of the target protein by interacting with other members of the signal transduction pathway of which the target protein is a member are also specifically included within the definition of "antagonist." An exemplary biological activity inhibited by an antagonist is associated with the development, growth, or spread of a tumor, or an undesired immune response as manifested in autoimmune disease.

The term "agonist" as used herein refers to a compound having the ability to initiate or enhance a biological function of a target protein, whether by inhibiting the activity or expression of the target protein. Accordingly, the term "agonist" is defined in the context of the biological role of the target polypeptide. Agonists herein generally interact specifically with (*e.g.* specifically bind to) the target, compounds that initiate or enhance a biological activity of the target polypeptide by interacting with other members of the signal transduction pathway of which the target polypeptide is a member are also specifically included within the definition of "agonist."

As used herein, "agent" or "biologically active agent" refers to a biological, pharmaceutical, or chemical compound or other moiety. Non-limiting examples include simple or complex organic or inorganic molecule, a peptide, a protein, an oligonucleotide, an antibody, an antibody derivative, antibody fragment, a vitamin derivative, a carbohydrate, a toxin, or a chemotherapeutic compound. Various compounds can be synthesized, for example, small molecules and oligomers (*e.g.,* oligopeptides and oligonucleotides), and synthetic organic compounds based on various core structures. In addition, various natural sources can provide compounds for screening, such as plant or animal extracts, and the like. A skilled artisan can readily recognize the limits to the structural nature of the agents described herein.

"Signal transduction" is a process during which stimulatory or inhibitory signals are transmitted into and within a cell to elicit an intracellular response. A modulator of a signal transduction pathway refers to a compound which modulates the activity of one or more cellular proteins mapped to the same specific signal transduction pathway. A modulator may augment (agonist) or suppress (antagonist) the activity of a signaling molecule.

The term "cell proliferation" refers to a phenomenon by which the cell number has changed as a result of division. This term also encompasses cell growth by which the cell morphology has changed (*e.g.,* increased in size) consistent with a proliferative signal.

The term "selective inhibition" or "selectively inhibit" as applied to a biologically active agent refers to the agent's ability to selectively reduce the target signaling activity as compared to off-target signaling activity, via direct or interact interaction with the target.

"Subject" refers to an animal, such as a mammal, for example a human. The methods described herein can be useful in both human therapeutics and veterinary applications. In some embodiments, the patient is a mammal, and in some embodiments, the patient is human.

"Prodrug" is meant to indicate a compound that may be converted under physiological conditions or by solvolysis to a biologically active compound described herein. Thus, the term "prodrug" refers to a precursor of a biologically active compound that is pharmaceutically acceptable. A prodrug may be inactive when administered to a subject, but is converted *in vivo* to an active compound, for example, by hydrolysis. The prodrug compound often offers advantages of solubility, tissue compatibility or delayed release in a mammalian organism (see, *e.g.,* Bundgard, H., Design of Prodrugs (1985), pp. 7-9, 21-24 (Elsevier, Amsterdam). A discussion of prodrugs is provided in Higuchi, T., et al., "Pro-drugs as Novel Delivery Systems," A.C.S. Symposium Series, Vol. 14, and in Bioreversible Carriers in Drug Design, ed. Edward B. Roche, American Pharmaceutical Association and Pergamon Press, 1987, both of which are incorporated in full by reference herein. The term "prodrug" is also meant to include any covalently bonded carriers, which release the active compound *in vivo* when such prodrug is administered to a mammalian subject. Prodrugs of an active compound, as described herein, may be prepared by modifying functional groups present in the active compound in such a way that the modifications are cleaved, either in routine manipulation or *in vivo,* to the parent active compound. Prodrugs include compounds wherein a hydroxy, amino or mercapto group is bonded to any group that, when the prodrug of the active compound is administered to a mammalian subject, cleaves to form a free hydroxy, free amino or free mercapto group, respectively. Examples of prodrugs includeacetate, formate and benzoate derivatives of an alcohol or acetamide, formamide and benzamide derivatives of an amine functional group in the active compound and the like.

The term *"in vivo"* refers to an event that takes place in a subject's body.

The term *"in vitro"* refers to an event that takes places outside of a subject's body. For example, an *in vitro* assay encompasses any assay run outside of a subject assay. *In vitro* assays encompass cell-based assays in which cells alive or dead are employed. *In vitro* assays also encompass a cell-free assay in which no intact cells are employed.

As used herein, the term "lipid peroxidation-related degenerative disease" refers to a degenerative disease, disorder or condition associated with the oxidative degradation of fats, oils, waxes, sterols, triglycerides, and the like.

As used herein, the term "excitotoxic disease" refers to a disease, disorder or condition associated with oxidative cell death and/or increased levels of intracellular reactive oxygen species (ROS), or diseases characterized by oxidative stress or where oxidative stress is likely to play or plays a substantial role.

As used herein, the term "neurodegenerative disease" refers to a disease, disorder or condition associated with degeneration of the central or peripheral nervous system.

As used herein, the term "non-apoptotic regulated cell-death disease" refers to a disease, disorder or condition associated with non-apoptotic regulated cell-death or where non-apoptotic regulated cell-death is likely to play or plays a substantial role.

As used herein, the term "wasting- or necrosis-related disease" refers to a disease, disorder or condition associated with wasting or cell necrosis.

As used herein, the term "intoxication-related disease" refers to a disease, disorder or condition associated with or characterized by cell, tissue, organ or organism intoxication (e.g., nephrotoxicity), including those arising from or associated with drug treatment, drug overdose, acute poisoning, or contrast-agent-induced toxicity.

As used herein, the term "infectious disease" refers to a disease, disorder or condition which are the result of, arise from or are associated with forms of infection of viruses, bacteria, fungi, or other microorganisms.

Unless otherwise stated, structures depicted herein are also meant to include compounds which differ only in the presence of one or more isotopically enriched atoms. For example, compounds as described herein wherein one or more hydrogens are replaced by deuterium or tritium, or the replacement of one or more carbon atoms by the ¹³C- or ¹⁴C-enriched carbon isotope. Further, substitution with heavier isotopes, particularly deuterium (²H or D) may afford certain therapeutic advantages resulting from greater metabolic stability, increased *in vivo* half-life, reduced dosage requirements or an improvement in therapeutic index. It is understood that deuterium in this context is regarded as a substituent of a compound of the formula (I), which is not covered by the subject-matter of the claims.

The compounds described herein may also contain unnatural proportions of atomic isotopes at one or more of atoms that constitute such compounds. For example, the compounds may be radiolabeled with radioactive isotopes, such as for example tritium (³H), iodine-125 (¹²⁵I) or carbon-14 (¹⁴C). All isotopic variations of the compounds described herein, whether radioactive or not, are described.

"Isomers" are different compounds that have the same molecular formula. "Stereoisomers" are isomers that differ only in the way the atoms are arranged in space. "Enantiomers" are a pair of stereoisomers that are non-superimposable mirror images of each other. A 1:1 mixture of a pair of enantiomers is a "racemic" mixture. The term "(..+-..)" is used to designate a racemic mixture where appropriate. "Diastereoisomers" are stereoisomers that have at least two asymmetric atoms, but which are not mirror-images of each other. The absolute stereochemistry is specified according to the Cahn-Ingold-Prelog R--S system. When a compound is a pure enantiomer the stereochemistry at each chiral carbon can be specified by either R or S. Resolved compounds whose absolute configuration is unknown can be designated (+) or (-) depending on the direction (dextro- or levorotatory) which they rotate plane polarized light at the wavelength of the sodium D line. Certain of the compounds described herein contain one or more asymmetric centers and can thus give rise to enantiomers, diastereomers, and other stereoisomeric forms that can be defined, in terms of absolute stereochemistry, as (R)- or (S)-. The present chemical entities, pharmaceutical compositions and methods are meant to include all such possible isomers, including racemic mixtures, optically pure forms and intermediate mixtures. Optically active (R)- and (S)-isomers can be prepared using chiral synthons or chiral reagents, or resolved using conventional techniques. The optical activity of a compound can be analyzed via any suitable method, including chiral chromatography and polarimetry, and the degree of predominance of one stereoisomer over the other isomer can be determined.

When the compounds described herein contain olefinic double bonds or other centers of geometric asymmetry, and unless specified otherwise, it is intended that the compounds include both E and Z geometric isomers.

A "substituted" or "optionally substituted" group, means that a group (such as alkyl, aryl, heterocyclyl, cycloalkyl, hetrocyclylalkyl, arylalkyl, heteroaryl, or heteroarylalkyl) unless specifically noted otherwise, may have 1, 2 or 3 -H groups substituted by 1, 2 or 3 substituents selected from halo, trifluoromethyl, trifluoromethoxy, methoxy, -COOH, -CHO, -NH₂, -NO₂, -OH, -SH, -SMe, -NHCH₃, - N(CH₃)₂, -CN, lower alkyl and the like.

"Tautomers" are structurally distinct isomers that interconvert by tautomerization. "Tautomerization" is a form of isomerization and includes prototropic or proton-shift tautomerization, which is considered a subset of acid-base chemistry. "Prototropic tautomerization" or "proton-shift tautomerization" involves the migration of a proton accompanied by changes in bond order, often the interchange of a single bond with an adjacent double bond. Where tautomerization is possible (*e.g.* in solution), a chemical equilibrium of tautomers can be reached. An example of tautomerization is keto-enol tautomerization. A specific example of keto-enol tautomerization is the interconversion of pentane-2,4-dione and 4-hydroxypent-3-en-2-one tautomers. Another example of tautomerization is phenol-keto tautomerization. A specific example of phenol-keto tautomerization is the interconversion of pyridin-4-ol and pyridin-4(1H)-one tautomers.

Compounds described herein also include crystalline and amorphous forms of those compounds, including, for example, polymorphs, pseudopolymorphs, solvates, hydrates, unsolvated polymorphs (including anhydrates), conformational polymorphs, and amorphous forms of the compounds, as well as mixtures thereof. "Crystalline form," "polymorph," and "novel form" may be used interchangeably herein, and are meant to include all crystalline and amorphous forms of the compound listed above, as well as mixtures thereof, unless a particular crystalline or amorphous form is referred to.

"Solvent," "organic solvent," and "inert solvent" each means a solvent inert under the conditions of the reaction being described in conjunction therewith including, for example, benzene, toluene, acetonitrile, tetrahydrofuran ("THF"), dimethylformamide ("DMF"), chloroform, methylene chloride (or dichloromethane), diethyl ether, methanol, N-methylpyrrolidone ("NMP"), pyridine and the like. Unless specified to the contrary, the solvents used in the reactions described herein are inert organic solvents. Unless specified to the contrary, for each gram of the limiting reagent, one cc (or mL) of solvent constitutes a volume equivalent.

### COMPOSITIONS

Described herein is a compound of formula II: 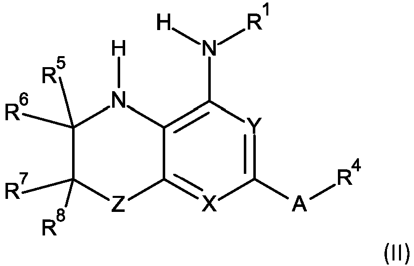 wherein
R¹ is selected from the group consisting of H, substituted or unsubstituted C₁-C₁₀ linear or branched alkyl, substituted or unsubstituted C₂-C₁₀ linear or branched alkenyl, substituted or unsubstituted C₂-C₁₀ linear or branched alkynyl, substituted or unsubstituted C₆-C₁₀ aryl, substituted or unsubstituted C₃-C₁₀ cycloalkyl, substituted or unsubstituted C₃-C₁₀ heterocycloalkyl, substituted or unsubstituted C₅-C₁₀ heteroaryl, substituted or unsubstituted C₆-C₁₀ arylalkyl, substituted or unsubstituted C₅-C₁₀ heteroarylalkyl, substituted or unsubstituted C₁-C₁₀ linear or branched alkylamino and substituted or unsubstituted C₁-C₁₀ linear or branched dialkylamino, or R¹ and its attached N together form a substituted or unsubstituted C₃-C₆ heterocycloalkyl or heteroaryl ring (replacing the H attached to the N);
A is selected from the group consisting of a bond, substituted or unsubstituted C₆-C₁₀ aryl, substituted or unsubstituted C₅-C₁₀ heteroaryl, substituted or unsubstituted C₂-C₁₀ linear or branched alkenyl, substituted or unsubstituted C₂-C₁₀ linear or branched alkynyl, C=O, C=S, -CH₂-, -CH(OH)-, -NH-, -N(CH₃)-, -O-, -S-, and SO₂; and R⁴ is selected from the group consisting of substituted or unsubstituted C₁-C₁₀ linear or branched alkyl, substituted or unsubstituted C₁-C₁₀ linear or branched alkoxy, substituted or unsubstituted C₁-C₁₀ linear or branched alkylamino, substituted or unsubstituted C₁-C₁₀ linear or branched dialkylamino, substituted or unsubstituted C₃-C₁₀ cycloalkyl or heterocycloalkyl, substituted or unsubstituted C₆-C₁₀ aryl, substituted or unsubstituted C₅-C₁₀ heteroaryl, -CN and halo;
R⁵, R⁶, R⁷, R⁸, R⁹ and R¹⁰ are independently selected from the group consisting of H, substituted or unsubstituted C₁-C₁₀ linear or branched alkyl, substituted or unsubstituted C₂-C₁₀ linear or branched alkenyl, substituted or unsubstituted C₂-C₁₀ linear or branched alkynyl, substituted or unsubstituted C₆-C₁₀ aryl, substituted or unsubstituted C₃-C₁₀ cycloalkyl, substituted or unsubstituted C₃-C₁₀ heterocycloalkyl, substituted or unsubstituted C₅-C₁₀ heteroaryl, substituted or unsubstituted C₆-C₁₀ arylalkyl, substituted or unsubstituted C₁-C₁₀ linear or branched alkylamino, and substituted or unsubstituted C₁-C₁₀ linear or branched dialkylamino, or R⁷ and R⁸ together are =O;
X and Y are independently selected from the group consisting of -CH- and -N-; and Z is selected from the group consisting of C=O, -CR⁹R¹⁰-, -NR⁹-, -O-, -S-, -S(O)- and - SO₂-;
and wherein a substituted group has 1, 2 or 3 -H atoms substituted by 1, 2 or 3 substituents selected from halo, trifluoromethyl, trifluoromethoxy, methoxy, -COOH, -CHO, -NH₂, -NO₂, -OH, -SH, -SMe, -NHCH₃, -N(CH₃)₂, -CN, and lower alkyl.

In some embodiments, X=Y= -CH-, and Z is -CH₂-. In some embodiments, X=Y= -CH- and Z=O.

The compound of formula II as defined according to claim 1 1 selected from the group consisting of the compounds listed in in Table 1:

**Table 1**

| **Compound ID** | **Structure** | **IC₅₀ (nM) (RSL3)** |
|---|---|---|
| J-84 | 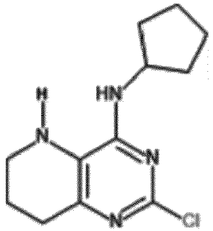 | 273nM |
| C-82 | 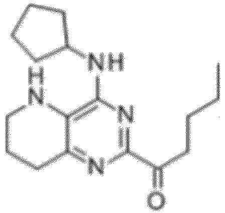 | 84nM |
| C-84 | 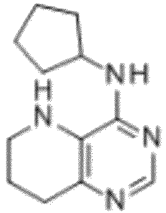 | 646nM |
| C-79 | 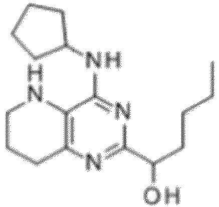 | 112nM |
| C-91 | 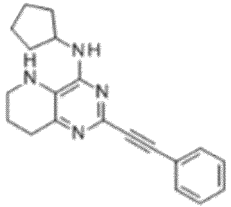 | 35nM |
| C-92 | 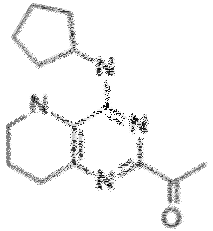 | 2072 |
| A-00 | 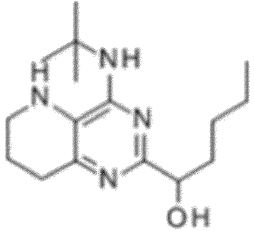 | 139nM |
| A-06 | 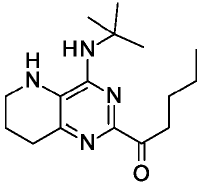 | 76nM |
| A-09 | 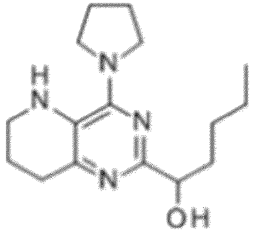 | 492nM |
| A-10 | 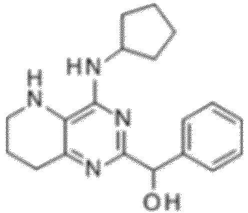 | 71nM |
| A-11 | 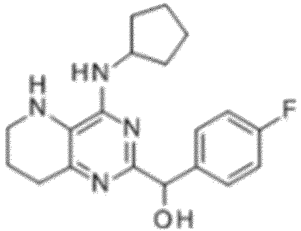 | 36nM |
| A-12 | 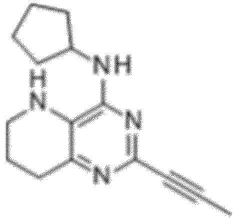 | 109 nM |
| A-16 | 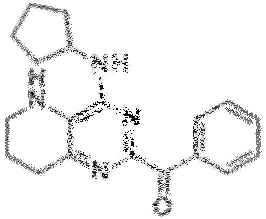 | 285nM |
| A-17 | 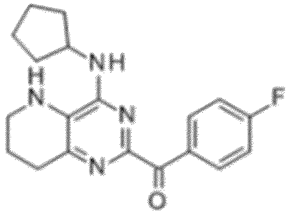 | 123nM |
| F-38 | 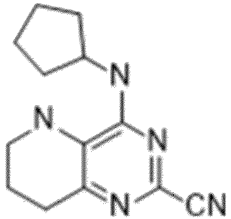 | 812nM |
| A-18 | 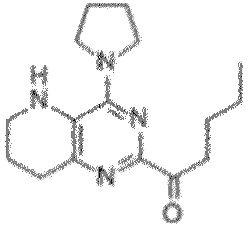 | 217nM |
| B-763 | 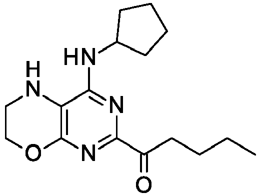 | 391 nM |
| A-27 | 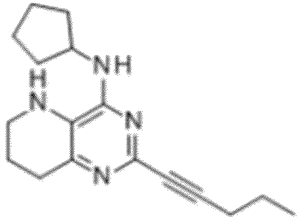 | 34nM |
| A-31 | 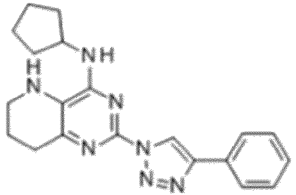 | 21nM |
| A-32 | 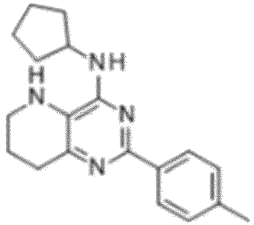 | 6nM |
| A-34 | 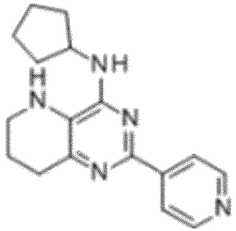 | 57 nM |
| A-35 | 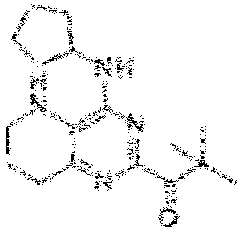 | 248nM |
| G-63 | 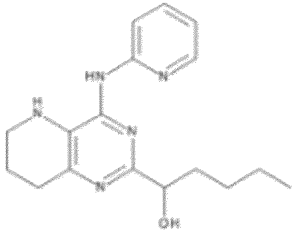 | >10uM |
| G-65 | 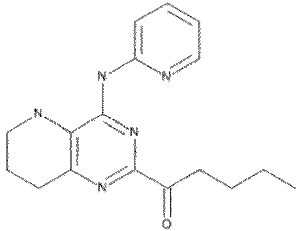 | >10uM |
| H-61 | 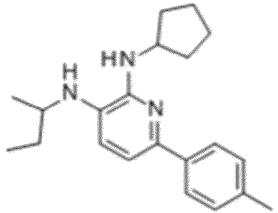 | 7nM |
| F-69 | 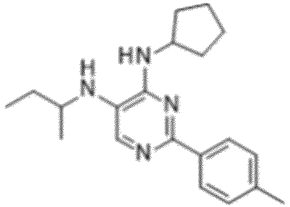 | 28nM |
| A-63 | 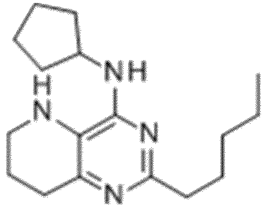 | 313nM |
| F-78 | 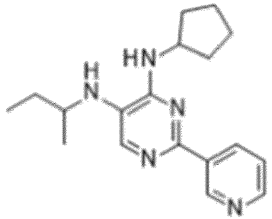 | 89nM |
| H-72 | 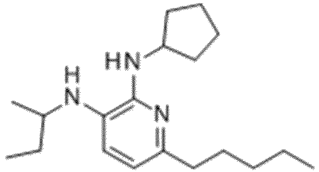 | 6nM |
| H-74 | 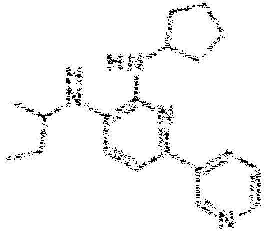 | 5nM |
| F-81 | 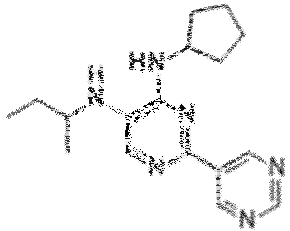 | 121nM |
| K-34 | 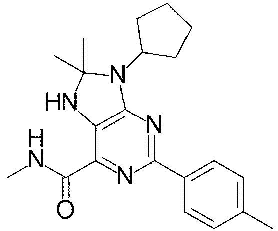 | >10 uM |
| K-36 | 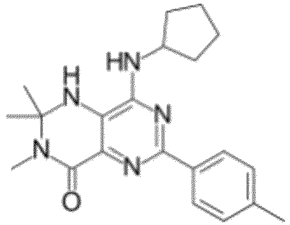 | 525nM |
| F-82 | 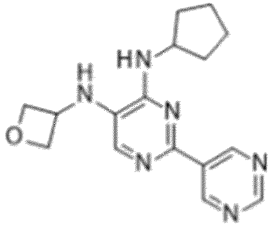 | >625nM |
| H-75 | 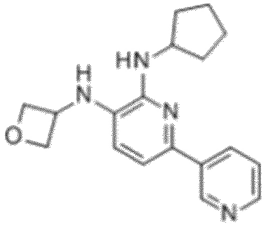 | 44nM |
| H-76 | 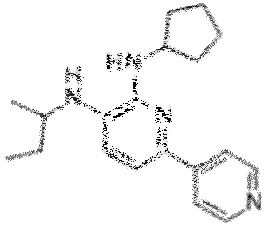 | 6nM |
| H-80 | 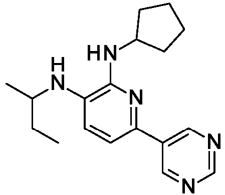 | 7nM |
| H-81 | 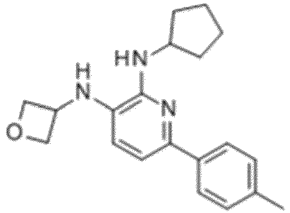 | 5nM |
| F-88 | 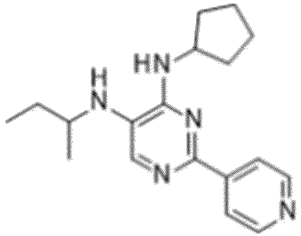 | 106nM |
| H-86 | 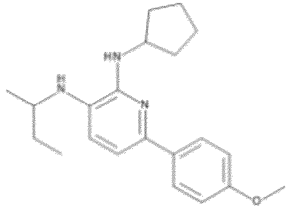 | 8nM |
| H-77 | 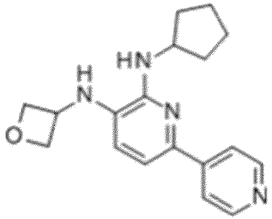 | 13nM |
| H-84 | 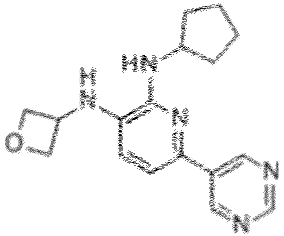 | 32nm |
| H-87 | 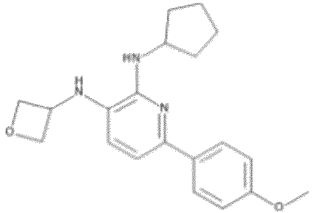 | 3nM |
| F-99 | 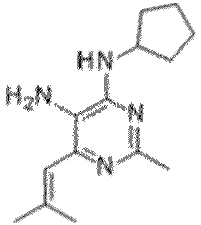 | >625nM |
| A-98 | 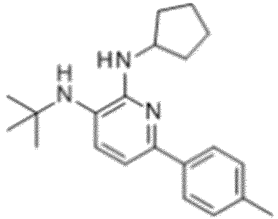 | 7 nM |
| I-85 | 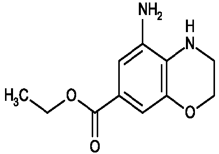 | >1000 |
| B-308 | 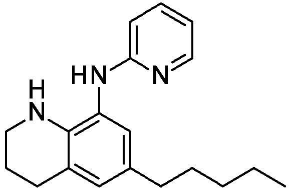 | 18 nM |
| B-397 | 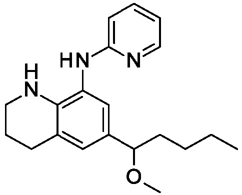 | 70 nM |
| B-250 | 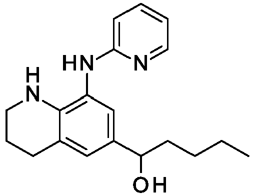 | 139 nM |
| B-249 | 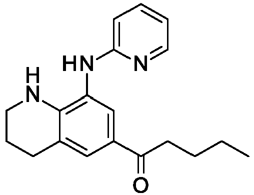 | 343 nM |
| B-273 | 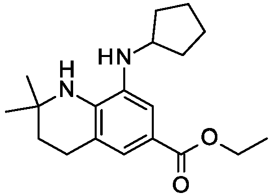 | 1040 nM |
| B-148 | 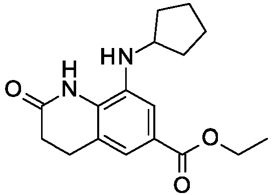 | 1670 nM |
| B-647 | 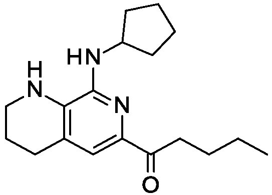 | 8 nM |
| B-601 | 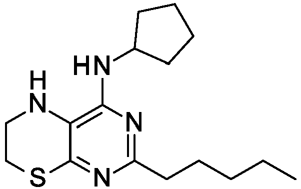 | 3 nM |
| B-710 | 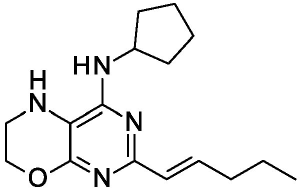 | 22 nM |
| B-388 | 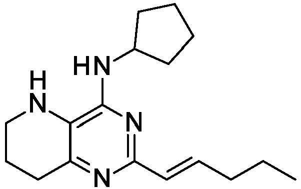 | 40 nM |
| B-711 | 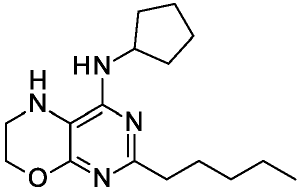 | 41 nM |
| B-323 | 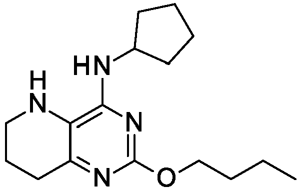 | 43 nM |
| B-059 | 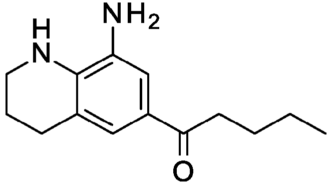 | 149 nM |
| B-456 | 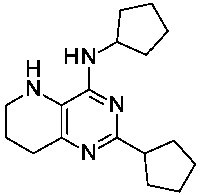 | 153 nM |
| B-495 | 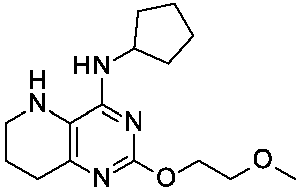 | 160 nM |
| B-349 | 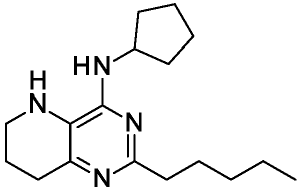 | 170 nM |
| B-322 | 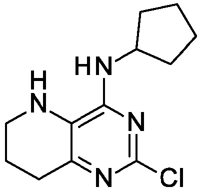 | 323 nM |
| B-604 | 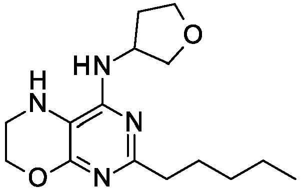 | 388 nM |
| B-434 | 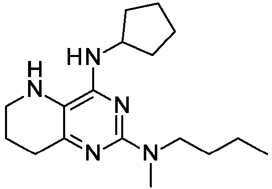 | 397 nM |
| E-09 | 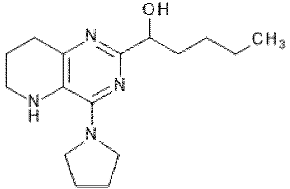 | 492 nM |
| B-433 | 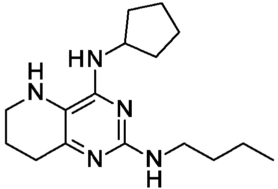 | 726 nM |
| B-603 | 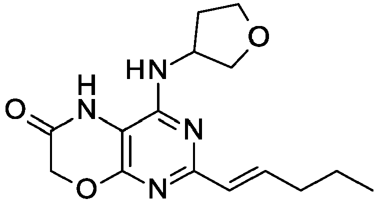 | >3000 nM |
| B-602 | 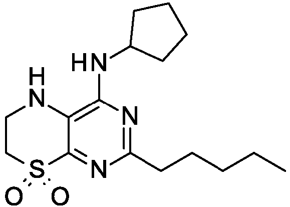 | 3000 nM |
| B-600 | 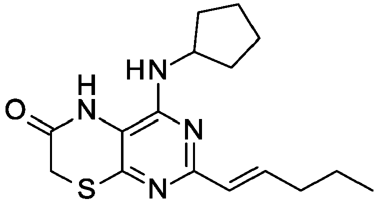 | 3000 nM |
| K-65 | 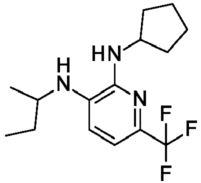 | 45 nM |
| L-02 | 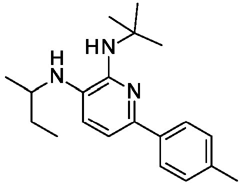 | 5 nM |
| L-03 | 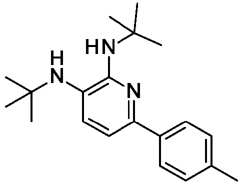 | 14 nM |
| L-04 | 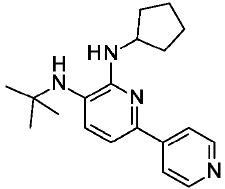 | 5 nM |
| L-22 | 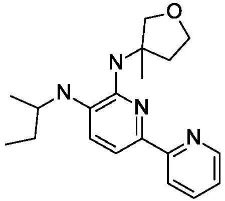 | 14 nM |
| L-23 | 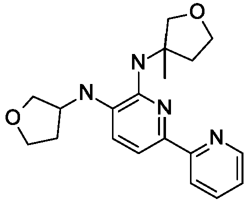 | 141 nM |
| L-34 | 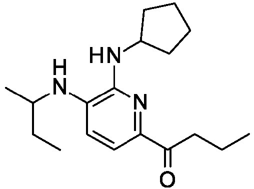 | 21 nM |
| L-42 | 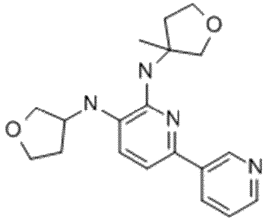 | 285 nM |
| L-45 | 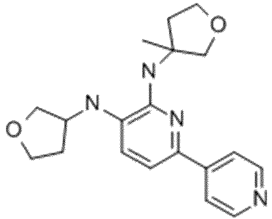 | N/A |
| L-46 | 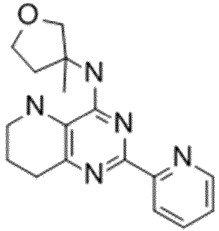 | N/A |
| P-22 | 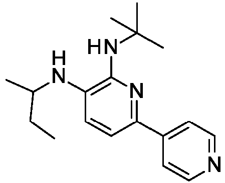 | 1nM |
| K-67 | 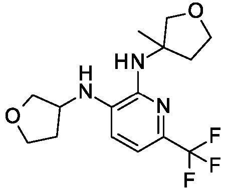 | 355 nM |
| P-48 | 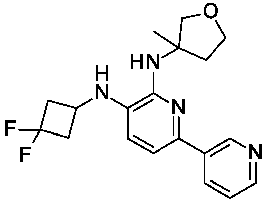 | NA |
| M-09 | 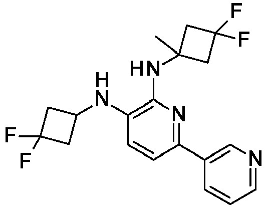 | NA |
| P-51 | 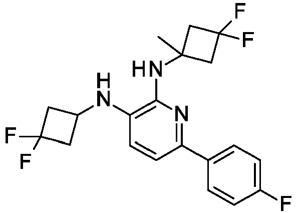 | NA |
| M-10 | 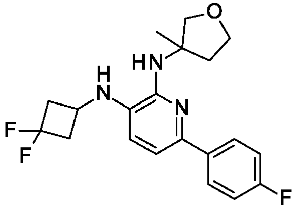 | NA |
| M-14 | 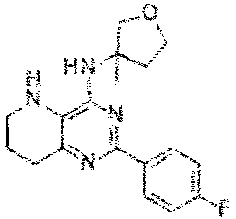 | 29 nM |
| M-23 | 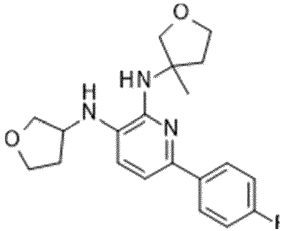 | 3 to 6 nM |
| N-04 | 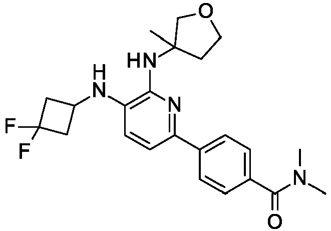 | NA |
| N-53 | 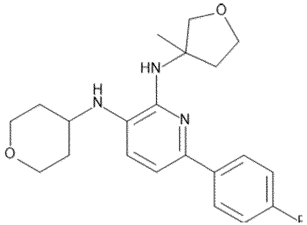 | 1nM |
| P-46 | 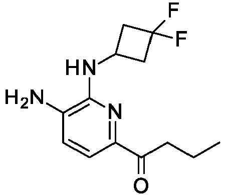 | 498 nM |
| P-47 | 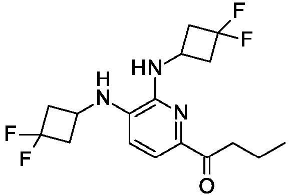 | 201 nM |
| S-101 | 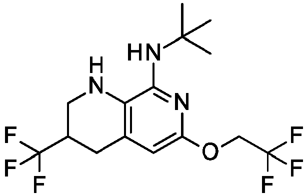 | 1262 nM |
| P-52 | 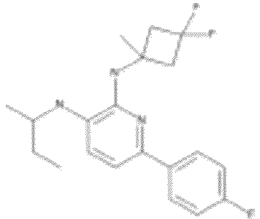 | NA |
| P-53 | 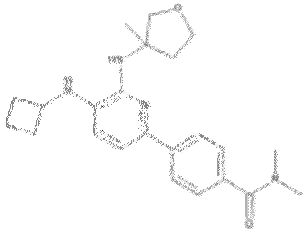 | 24 nM |
| P-54 | 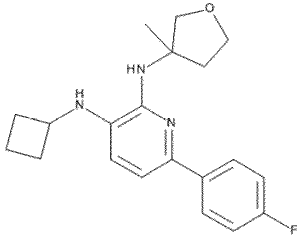 | 3 nM |
| P-71 | 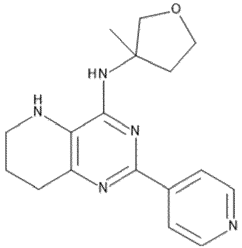 | 463 nM |
| P-72 | 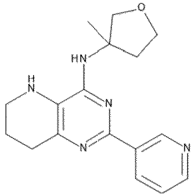 | NA |
| S-168 | 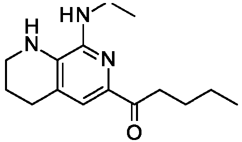 | 13 nM |
| R-830 | 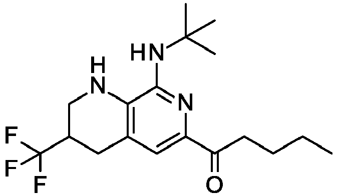 | 36 nM |
| R-812 | 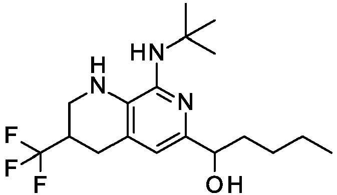 | 40nM |
| B-917 | 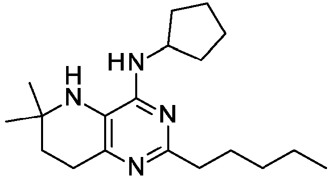 | >3000 nM |
| B-626 | 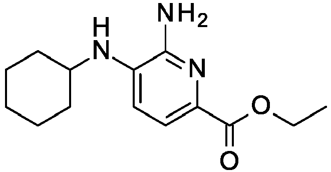 | 108 nM |
| B-256 | 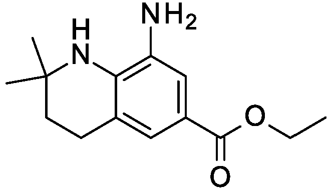 | 3824 nM |
| B-251 | 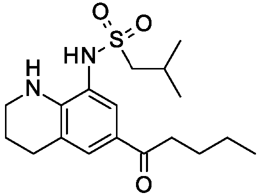 | 9300 nM |
| B-248 | 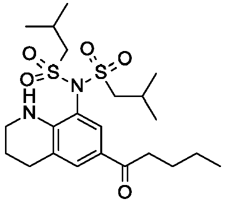 | >3000 nM |
| B-133 | 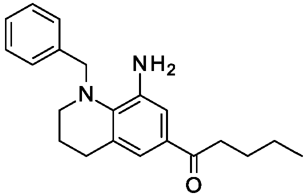 | 2898 nM |
| B-132 | 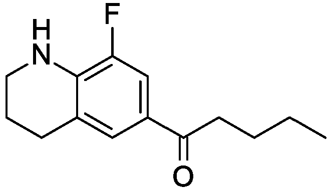 | >3000 nM |
| B-101 | 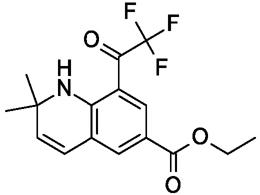 | >3000 nM |
| B-100 | 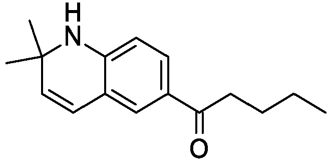 | 7437 nM |
| B-099 | 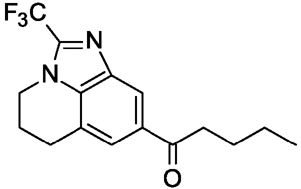 | >3000 nM |
| B-065 | 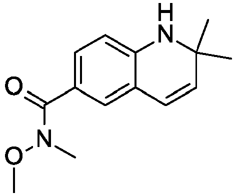 | >3000 nM |
| B-060 | 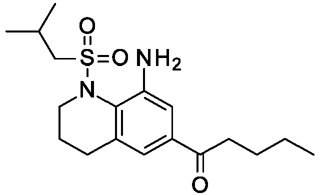 | 4025 nM |
| B-035 | 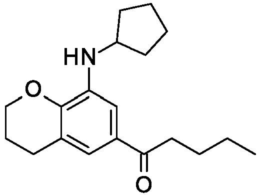 | >3000 nM |
| B-006 | 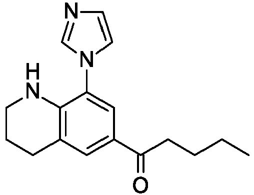 | >3000 nM |
| Q-980 | 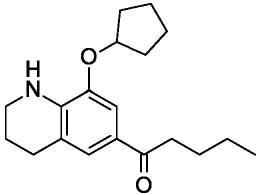 | >3000 nM |
| Q-979 | 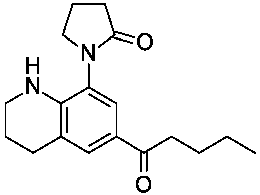 | 1151 nM |
| Q-950 | 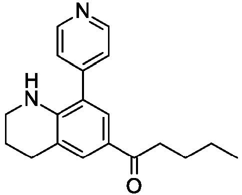 | >3000 nM |
| Q-912 | 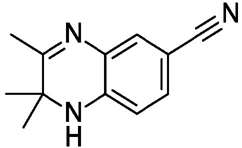 | >3000 nM |
| Q-879 | 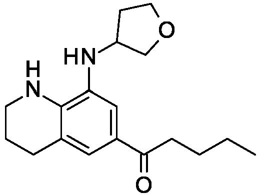 | 16.8 nM |

Isolation and purification of the chemical entities and intermediates described herein can be effected, if desired, by any suitable separation or purification procedure such as, for example, filtration, extraction, crystallization, column chromatography, thin-layer chromatography or thick-layer chromatography, or a combination of these procedures. Specific illustrations of suitable separation and isolation procedures can be had by reference to the examples herein. However, other equivalent separation or isolation procedures can also be used.

When desired, the (R)- and (S)-isomers of the compounds described herein, if present, may be resolved by methods known to those skilled in the art, for example by formation of diastereomeric salts or complexes which may be separated, for example, by crystallization; via formation of diastereomeric derivatives which may be separated, for example, by crystallization, gas-liquid or liquid chromatography; selective reaction of one enantiomer with an enantiomer-specific reagent, for example enzymatic oxidation or reduction, followed by separation of the modified and unmodified enantiomers; or gas-liquid or liquid chromatography in a chiral environment, for example on a chiral support, such as silica with a bound chiral ligand or in the presence of a chiral solvent. Alternatively, a specific enantiomer may be synthesized by asymmetric synthesis using optically active reagents, substrates, catalysts or solvents, or by converting one enantiomer to the other by asymmetric transformation.

The compounds described herein can be optionally contacted with a pharmaceutically acceptable acid to form the corresponding acid addition salts. Pharmaceutically acceptable forms of the compounds recited herein include pharmaceutically acceptable salts, chelates, non-covalent complexes or derivatives, prodrugs, and mixtures thereof. In certain embodiments, the compounds described herein are in the form of pharmaceutically acceptable salts. In addition, if the compound described herein is obtained as an acid addition salt, the free base can be obtained by basifying a solution of the acid salt. Conversely, if the product is a free base, an addition salt, particularly a pharmaceutically acceptable addition salt, may be produced by dissolving the free base in a suitable organic solvent and treating the solution with an acid, in accordance with conventional procedures for preparing acid addition salts from base compounds. Those skilled in the art will recognize various synthetic methodologies that may be used to prepare non-toxic pharmaceutically acceptable addition salts.

When ranges are used herein for physical properties, such as molecular weight, or chemical properties, such as chemical formulae, all combinations and subcombinations of ranges and specific embodiments therein are intended to be included. The term "about" when referring to a number or a numerical range means that the number or numerical range referred to is an approximation within experimental variability (or within statistical experimental error), and thus the number or numerical range may vary from, for example, between 1% and 15% of the stated number or numerical range.

The subject pharmaceutical compositions are typically formulated to provide a therapeutically effective amount of a compound of Formula II as the active ingredient, or a pharmaceutically acceptable salt, ester, prodrug, solvate, hydrate or derivative thereof. Where desired, the pharmaceutical compositions contain pharmaceutically acceptable salt and/or coordination complex thereof, and one or more pharmaceutically acceptable excipients, carriers, including inert solid diluents and fillers, diluents, including sterile aqueous solution and various organic solvents, permeation enhancers, solubilizers and adjuvants.

The subject pharmaceutical compositions can be administered alone or in combination with one or more other agents, which are also typically administered in the form of pharmaceutical compositions. Where desired, a compound of Formula II and other agent(s) may be mixed into a preparation or both components may be formulated into separate preparations to use them in combination separately or at the same time. A compound as described herein may also be used in combination with other agents, e.g., an additional compound that is not of Formula II, for treatment of the diseases listed herein in a subject.

The concentration of one or more of the compounds of Formula II in the pharmaceutical compositions described herein is less than 100%, 90%, 80%, 70%, 60%, 50%, 40%, 30%, 20%, 19%, 18%, 17%, 16%, 15%, 14%, 13%, 12%, 11%, 10%, 9%, 8%, 7%, 6%, 5%, 4%, 3%, 2%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002%, or 0.0001% w/w, w/v or v/v.

In some aspects, the concentration of one or more of the compounds of Formula II is greater than 90%, 80%, 70%, 60%, 50%, 40%, 30%, 20%, 19.75%, 19.50%, 19.25% 19%, 18.75%, 18.50%, 18.25% 18%, 17.75%, 17.50%, 17.25% 17%, 16.75%, 16.50%, 16.25% 16%, 15.75%, 15.50%, 15.25% 15%, 14.75%, 14.50%, 14.25% 14%, 13.75%, 13.50%, 13.25% 13%, 12.75%, 12.50%, 12.25% 12%, 11.75%, 11.50%, 11.25% 11%, 10.75%, 10.50%, 10.25% 10%, 9.75%, 9.50%, 9.25% 9%, 8.75%, 8.50%, 8.25% 8%, 7.75%, 7.50%, 7.25% 7%, 6.75%, 6.50%, 6.25% 6%, 5.75%, 5.50%, 5.25% 5%, 4.75%, 4.50%, 4.25%, 4%, 3.75%, 3.50%, 3.25%, 3%, 2.75%, 2.50%, 2.25%, 2%, 1.75%, 1.50%, 125%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002%, or 0.0001% w/w, w/v, or v/v.

In some aspects, the concentration of one or more of the compounds of Formula II is in the range from approximately 0.0001% to approximately 50%, approximately 0.001% to approximately 40%, approximately 0.01% to approximately 30%, approximately 0.02% to approximately 29%, approximately 0.03% to approximately 28%, approximately 0.04% to approximately 27%, approximately 0.05% to approximately 26%, approximately 0.06% to approximately 25%, approximately 0.07% to approximately 24%, approximately 0.08% to approximately 23%, approximately 0.09% to approximately 22%, approximately 0.1% to approximately 21%, approximately 0.2% to approximately 20%, approximately 0.3% to approximately 19%, approximately 0.4% to approximately 18%, approximately 0.5% to approximately 17%, approximately 0.6% to approximately 16%, approximately 0.7% to approximately 15%, approximately 0.8% to approximately 14%, approximately 0.9% to approximately 12%, approximately 1% to approximately 10% w/w, w/v or v/v.

In some aspects, the concentration of one or more of the compounds of Formula II is in the range from approximately 0.001% to approximately 10%, approximately 0.01% to approximately 5%, approximately 0.02% to approximately 4.5%, approximately 0.03% to approximately 4%, approximately 0.04% to approximately 3.5%, approximately 0.05% to approximately 3%, approximately 0.06% to approximately 2.5%, approximately 0.07% to approximately 2%, approximately 0.08% to approximately 1.5%, approximately 0.09% to approximately 1%, approximately 0.1% to approximately 0.9% w/w, w/v or v/v.

In some aspects, the amount of one or more of the compounds of Formula II is equal to or less than 10 g, 9.5 g, 9.0 g, 8.5 g, 8.0 g, 7.5 g, 7.0 g, 6.5 g, 6.0 g, 5.5 g, 5.0 g, 4.5 g, 4.0 g, 3.5 g, 3.0 g, 2.5 g, 2.0 g, 1.5 g, 1.0 g, 0.95 g, 0.9 g, 0.85 g, 0.8 g, 0.75 g, 0.7 g, 0.65 g, 0.6 g, 0.55 g, 0.5 g, 0.45 g, 0.4 g, 0.35 g, 0.3 g, 0.25 g, 0.2 g, 0.15 g, 0.1 g, 0.09 g, 0.08 g, 0.07 g, 0.06 g, 0.05 g, 0.04 g, 0.03 g, 0.02 g, 0.01 g, 0.009 g, 0.008 g, 0.007 g, 0.006 g, 0.005 g, 0.004 g, 0.003 g, 0.002 g, 0.001 g, 0.0009 g, 0.0008 g, 0.0007 g, 0.0006 g, 0.0005 g, 0.0004 g, 0.0003 g, 0.0002 g, or 0.0001 g.

In some aspects, the amount of one or more of the compounds of Formula II is more than 0.0001 g, 0.0002 g, 0.0003 g, 0.0004 g, 0.0005 g, 0.0006 g, 0.0007 g, 0.0008 g, 0.0009 g, 0.001 g, 0.0015 g, 0.002 g, 0.0025 g, 0.003 g, 0.0035 g, 0.004 g, 0.0045 g, 0.005 g, 0.0055 g, 0.006 g, 0.0065 g, 0.007 g, 0.0075 g, 0.008 g, 0.0085 g, 0.009 g, 0.0095 g, 0.01 g, 0.015 g, 0.02 g, 0.025 g, 0.03 g, 0.035 g, 0.04 g, 0.045 g, 0.05 g, 0.055 g, 0.06 g, 0.065 g, 0.07 g, 0.075 g, 0.08 g, 0.085 g, 0.09 g, 0.095 g, 0.1 g, 0.15 g, 0.2 g, 0.25 g, 0.3 g, 0.35 g, 0.4 g, 0.45 g, 0.5 g, 0.55 g, 0.6 g, 0.65 g, 0.7 g, 0.75 g, 0.8 g, 0.85 g, 0.9 g, 0.95 g, 1 g, 1.5 g, 2 g, 2.5, 3 g, 3.5, 4 g, 4.5 g, 5 g, 5.5 g, 6 g, 6.5 g, 7 g, 7.5 g, 8 g, 8.5 g, 9 g, 9.5 g, or 10 g.

In some aspects, the amount of one or more of the compounds of Formula II is in the range of 0.0001-10 g, 0.0005-9 g, 0.001-8 g, 0.005-7 g, 0.01-6 g, 0.05-5 g, 0.1-4 g, 0.5-4 g, or 1-3 g.

The compound of Formula II described herein are effective over a wide dosage range. For example, in the treatment of adult humans, dosages from 0.01 to 1000 mg, from 0.5 to 100 mg, from 1 to 50 mg per day, and from 5 to 40 mg per day are examples of dosages that may be used. An exemplary dosage is 10 to 30 mg per day. The exact dosage will depend upon the route of administration, the form in which the compound of Formula II is administered, the subject to be treated, the body weight of the subject to be treated, and the preference and experience of the attending physician.

A pharmaceutical composition described herein typically contains an active ingredient (*e.g.,* a compound of Formula II or a pharmaceutically acceptable salt and/or coordination complex thereof), and one or more pharmaceutically acceptable excipients, carriers, including inert solid diluents and fillers, diluents, sterile aqueous solution and various organic solvents, permeation enhancers, solubilizers and adjuvants.

Described below are exemplary pharmaceutical compositions and methods for preparing the same.

### Pharmaceutical Compositions for Oral Administration

Described herein is a pharmaceutical composition for oral administration containing a compound of formula II: 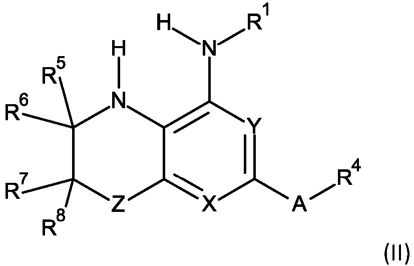 wherein
R¹ is selected from the group consisting of H, substituted or unsubstituted C₁-C₁₀ linear or branched alkyl, substituted or unsubstituted C₂-C₁₀ linear or branched alkenyl, substituted or unsubstituted C₂-C₁₀ linear or branched alkynyl, substituted or unsubstituted C₆-C₁₀ aryl, substituted or unsubstituted C₃-C₁₀ cycloalkyl, substituted or unsubstituted C₃-C₁₀ heterocycloalkyl, substituted or unsubstituted C₅-C₁₀ heteroaryl, substituted or unsubstituted C₆-C₁₀ arylalkyl, substituted or unsubstituted C₅-C₁₀ heteroarylalkyl, substituted or unsubstituted C₁-C₁₀ linear or branched alkylamino and substituted or unsubstituted C₁-C₁₀ linear or branched dialkylamino, or R¹ and its attached N together form a substituted or unsubstituted C₃-C₆ heterocycloalkyl or heteroaryl ring (replacing the H attached to the N);
A is selected from the group consisting of a bond, substituted or unsubstituted C₆-C₁₀ aryl, substituted or unsubstituted C₅-C₁₀ heteroaryl, substituted or unsubstituted C₂-C₁₀ linear or branched alkenyl, substituted or unsubstituted C₂-C₁₀ linear or branched alkynyl, C=O, C=S, -CH₂-, -CH(OH)-, -NH-, -N(CH₃)-, -O-, -S-, and SO₂; and R⁴ is selected from the group consisting of substituted or unsubstituted C₁-C₁₀ linear or branched alkyl, substituted or unsubstituted C₁-C₁₀ linear or branched alkoxy, substituted or unsubstituted C₁-C₁₀ linear or branched alkylamino, substituted or unsubstituted C₁-C₁₀ linear or branched dialkylamino, substituted or unsubstituted C₃-C₁₀ cycloalkyl or heterocycloalkyl, substituted or unsubstituted C₆-C₁₀ aryl, substituted or unsubstituted C₅-C₁₀ heteroaryl, -CN and halo;
R⁵, R⁶, R⁷, R⁸, R⁹ and R¹⁰ are independently selected from the group consisting of H, substituted or unsubstituted C₁-C₁₀ linear or branched alkyl, substituted or unsubstituted C₂-C₁₀ linear or branched alkenyl, substituted or unsubstituted C₂-C₁₀ linear or branched alkynyl, substituted or unsubstituted C₆-C₁₀ aryl, substituted or unsubstituted C₃-C₁₀ cycloalkyl, substituted or unsubstituted C₃-C₁₀ heterocycloalkyl, substituted or unsubstituted C₅-C₁₀ heteroaryl, substituted or unsubstituted C₆-C₁₀ arylalkyl, substituted or unsubstituted C₁-C₁₀ linear or branched alkylamino, and substituted or unsubstituted C₁-C₁₀ linear or branched dialkylamino, or R⁷ and R⁸ together are =O;
X and Y are independently selected from the group consisting of -CH- and -N-; and Z is selected from the group consisting of C=O, -CR⁹R¹⁰-, -NR⁹-, -O-, -S-, -S(O)- and - SO₂-; and wherein a substituted group has 1, 2 or 3 -H atoms substituted by 1, 2 or 3 substituents selected from halo, trifluoromethyl, trifluoromethoxy, methoxy, -COOH, -CHO, -NH₂, -NO₂, -OH, -SH, -SMe, -NHCH₃, -N(CH₃)₂, -CN, and lower alkyl, and a pharmaceutical excipient suitable for oral administration.

Also described herein is a solid pharmaceutical composition for oral administration containing: (i) an effective amount of a compound of Formula II; optionally (ii) an effective amount of a second agent; and (iii) a pharmaceutical excipient suitable for oral administration. In some embodiments, the composition further contains: (iv) an effective amount of a third agent.

In some embodiments, the pharmaceutical composition may be a liquid pharmaceutical composition suitable for oral consumption. Pharmaceutical compositions suitable for oral administration can be presented as discrete dosage forms, such as capsules, cachets, or tablets, or liquids or aerosol sprays each containing a predetermined amount of an active ingredient as a powder or in granules, a solution, or a suspension in an aqueous or non-aqueous liquid, an oil-in-water emulsion, or a water-in-oil liquid emulsion. Such dosage forms can be prepared by any of the methods of pharmacy, but all methods include the step of bringing the active ingredient into association with the carrier, which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet can be prepared by compression or molding, optionally with one or more accessory ingredients. Compressed tablets can be prepared by compressing in a suitable machine the active ingredient in a free-flowing form such as powder or granules, optionally mixed with an excipient such as, a binder, a lubricant, an inert diluent, and/or a surface active or dispersing agent. Molded tablets can be made by molding in a suitable machine a mixture of the powdered compound moistened with an inert liquid diluent.

Also described herein are anhydrous pharmaceutical compositions and dosage forms comprising an active ingredient, since water can facilitate the degradation of some compounds. For example, water may be added (e.g., 5%) in the pharmaceutical arts as a means of simulating long-term storage in order to determine characteristics such as shelf-life or the stability of formulations over time. Anhydrous pharmaceutical compositions and dosage forms can be prepared using anhydrous or low moisture containing ingredients and low moisture or low humidity conditions. Pharmaceutical compositions and dosage forms which contain lactose can be made anhydrous if substantial contact with moisture and/or humidity during manufacturing, packaging, and/or storage is expected. An anhydrous pharmaceutical composition may be prepared and stored such that its anhydrous nature is maintained. Accordingly, anhydrous compositions may be packaged using materials known to prevent exposure to water such that they can be included in suitable formulary kits. Examples of suitable packaging include hermetically sealed foils, plastic or the like, unit dose containers, blister packs, and strip packs.

An active ingredient can be combined in an intimate admixture with a pharmaceutical carrier according to conventional pharmaceutical compounding techniques. The carrier can take a wide variety of forms depending on the form of preparation desired for administration. In preparing the compositions for an oral dosage form, any of the usual pharmaceutical media can be employed as carriers, such as, for example, water, glycols, oils, alcohols, flavoring agents, preservatives, coloring agents, and the like in the case of oral liquid preparations (such as suspensions, solutions, and elixirs) or aerosols; or carriers such as starches, sugars, micro-crystalline cellulose, diluents, granulating agents, lubricants, binders, and disintegrating agents can be used in the case of oral solid preparations, in some embodiments without employing the use of lactose. For example, suitable carriers include powders, capsules, and tablets, with the solid oral preparations. If desired, tablets can be coated by standard aqueous or nonaqueous techniques.

Binders suitable for use in pharmaceutical compositions and dosage forms include corn starch, potato starch, or other starches, gelatin, natural and synthetic gums such as acacia, sodium alginate, alginic acid, other alginates, powdered tragacanth, guar gum, cellulose and its derivatives (e.g., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, microcrystalline cellulose, and mixtures thereof.

Examples of suitable fillers for use in the pharmaceutical compositions and dosage forms disclosed herein include talc, calcium carbonate (e.g., granules or powder), microcrystalline cellulose, powdered cellulose, dextrates, kaolin, mannitol, silicic acid, sorbitol, starch, pre-gelatinized starch, and mixtures thereof.

Disintegrants may be used in the compositions described herein to provide tablets that disintegrate when exposed to an aqueous environment. Too much of a disintegrant may produce tablets which may disintegrate in the bottle. Too little may be insufficient for disintegration to occur and may thus alter the rate and extent of release of the active ingredient(s) from the dosage form. Thus, a sufficient amount of disintegrant that is neither too little nor too much to detrimentally alter the release of the active ingredient(s) may be used to form the dosage forms of the compounds disclosed herein. The amount of disintegrant used may vary based upon the type of formulation and mode of administration, and may be readily discernible to those of ordinary skill in the art. About 0.5 to about 15 weight percent of disintegrant, or about 1 to about 5 weight percent of disintegrant, may be used in the pharmaceutical composition. Disintegrants that can be used to form pharmaceutical compositions and dosage forms include agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums or mixtures thereof.

Lubricants which can be used to form pharmaceutical compositions and dosage forms include calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laureate, agar, or mixtures thereof. Additional lubricants include, for example, a syloid silica gel, a coagulated aerosol of synthetic silica, or mixtures thereof. A lubricant can optionally be added, in an amount of less than about 1 weight percent of the pharmaceutical composition.

When aqueous suspensions and/or elixirs are desired for oral administration, the essential active ingredient therein may be combined with various sweetening or flavoring agents, coloring matter or dyes and, if so desired, emulsifying and/or suspending agents, together with such diluents as water, ethanol, propylene glycol, glycerin and various combinations thereof.

The tablets can be uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

Surfactants which can be used to form pharmaceutical compositions and dosage forms include hydrophilic surfactants, lipophilic surfactants, and mixtures thereof. That is, a mixture of hydrophilic surfactants may be employed, a mixture of lipophilic surfactants may be employed, or a mixture of at least one hydrophilic surfactant and at least one lipophilic surfactant may be employed.

A suitable hydrophilic surfactant may generally have an HLB value of at least 10, while suitable lipophilic surfactants may generally have an HLB value of or less than about 10. An empirical parameter used to characterize the relative hydrophilicity and hydrophobicity of non-ionic amphiphilic compounds is the hydrophilic-lipophilic balance ("HLB" value). Surfactants with lower HLB values are more lipophilic or hydrophobic, and have greater solubility in oils, while surfactants with higher HLB values are more hydrophilic, and have greater solubility in aqueous solutions. Hydrophilic surfactants are generally considered to be those compounds having an HLB value greater than about 10, as well as anionic, cationic, or zwitterionic compounds for which the HLB scale is not generally applicable. Similarly, lipophilic (i.e., hydrophobic) surfactants are compounds having an HLB value equal to or less than about 10. However, HLB value of a surfactant is merely a rough guide generally used to enable formulation of industrial, pharmaceutical and cosmetic emulsions.

Hydrophilic surfactants may be either ionic or non-ionic. Suitable ionic surfactants include alkylammonium salts; fusidic acid salts; fatty acid derivatives of amino acids, oligopeptides, and polypeptides; glyceride derivatives of amino acids, oligopeptides, and polypeptides; lecithins and hydrogenated lecithins; lysolecithins and hydrogenated lysolecithins; phospholipids and derivatives thereof; lysophospholipids and derivatives thereof; carnitine fatty acid ester salts; salts of alkylsulfates; fatty acid salts; sodium docusate; acylactylates; mono- and di-acetylated tartaric acid esters of mono- and di-glycerides; succinylated mono- and di-glycerides; citric acid esters of mono- and di-glycerides; and mixtures thereof.

Within the aforementioned group, ionic surfactants include, by way of example: lecithins, lysolecithin, phospholipids, lysophospholipids and derivatives thereof; carnitine fatty acid ester salts; salts of alkylsulfates; fatty acid salts; sodium docusate; acylactylates; mono- and di-acetylated tartaric acid esters of mono- and di-glycerides; succinylated mono- and di-glycerides; citric acid esters of mono- and di-glycerides; and mixtures thereof.

Ionic surfactants may be the ionized forms of lecithin, lysolecithin, phosphatidylcholine, phosphatidylethanolamine, phosphatidylglycerol, phosphatidic acid, phosphatidylserine, lysophosphatidylcholine, lysophosphatidylethanolamine, lysophosphatidylglycerol, lysophosphatidic acid, lysophosphatidylserine, PEG-phosphatidylethanolamine, PVP-phosphatidylethanolamine, lactylic esters of fatty acids, stearoyl-2-lactylate, stearoyl lactylate, succinylated monoglycerides, mono/diacetylated tartaric acid esters of mono/diglycerides, citric acid esters of mono/diglycerides, cholylsarcosine, caproate, caprylate, caprate, laurate, myristate, palmitate, oleate, ricinoleate, linoleate, linolenate, stearate, lauryl sulfate, teradecyl sulfate, docusate, lauroyl carnitines, palmitoyl carnitines, myristoyl carnitines, and salts and mixtures thereof.

Hydrophilic non-ionic surfactants may include alkylglucosides; alkylmaltosides; alkylthioglucosides; lauryl macrogolglycerides; polyoxyalkylene alkyl ethers such as polyethylene glycol alkyl ethers; polyoxyalkylene alkylphenols such as polyethylene glycol alkyl phenols; polyoxyalkylene alkyl phenol fatty acid esters such as polyethylene glycol fatty acids monoesters and polyethylene glycol fatty acids diesters; polyethylene glycol glycerol fatty acid esters; polyglycerol fatty acid esters; polyoxyalkylene sorbitan fatty acid esters such as polyethylene glycol sorbitan fatty acid esters; hydrophilic transesterification products of a polyol with at least one member of the group consisting of glycerides, vegetable oils, hydrogenated vegetable oils, fatty acids, and sterols; polyoxyethylene sterols, derivatives, and analogues thereof; polyoxyethylated vitamins and derivatives thereof; polyoxyethylene-polyoxypropylene block copolymers; and mixtures thereof; polyethylene glycol sorbitan fatty acid esters and hydrophilic transesterification products of a polyol with at least one member of the group consisting of triglycerides, vegetable oils, and hydrogenated vegetable oils. The polyol may be glycerol, ethylene glycol, polyethylene glycol, sorbitol, propylene glycol, pentaerythritol, or a saccharide.

Other hydrophilic-non-ionic surfactants include PEG-10 laurate, PEG-12 laurate, PEG-20 laurate, PEG-32 laurate, PEG-32 dilaurate, PEG-12 oleate, PEG-15 oleate, PEG-20 oleate, PEG-20 dioleate, PEG-32 oleate, PEG-200 oleate, PEG-400 oleate, PEG-15 stearate, PEG-32 distearate, PEG-40 stearate, PEG-100 stearate, PEG-20 dilaurate, PEG-25 glyceryl trioleate, PEG-32 dioleate, PEG-20 glyceryl laurate, PEG-30 glyceryl laurate, PEG-20 glyceryl stearate, PEG-20 glyceryl oleate, PEG-30 glyceryl oleate, PEG-30 glyceryl laurate, PEG-40 glyceryl laurate, PEG-40 palm kernel oil, PEG-50 hydrogenated castor oil, PEG-40 castor oil, PEG-35 castor oil, PEG-60 castor oil, PEG-40 hydrogenated castor oil, PEG-60 hydrogenated castor oil, PEG-60 corn oil, PEG-6 caprate/caprylate glycerides, PEG-8 caprate/caprylate glycerides, polyglyceryl-10 laurate, PEG-30 cholesterol, PEG-25 phyto sterol, PEG-30 soya sterol, PEG-20 trioleate, PEG-40 sorbitan oleate, PEG-80 sorbitan laurate, polysorbate 20, polysorbate 80, POE-9 lauryl ether, POE-23 lauryl ether, POE-10 oleyl ether, POE-20 oleyl ether, POE-20 stearyl ether, tocopheryl PEG-100 succinate, PEG-24 cholesterol, polyglyceryl-10 oleate, Tween 40, Tween 60, sucrose monostearate, sucrose monolaurate, sucrose monopalmitate, PEG 10-100 nonyl phenol series, PEG 15-100 octyl phenol series, and poloxamers.

Suitable lipophilic surfactants include, by way of example only: fatty alcohols; glycerol fatty acid esters; acetylated glycerol fatty acid esters; lower alcohol fatty acids esters; propylene glycol fatty acid esters; sorbitan fatty acid esters; polyethylene glycol sorbitan fatty acid esters; sterols and sterol derivatives; polyoxyethylated sterols and sterol derivatives; polyethylene glycol alkyl ethers; sugar esters; sugar ethers; lactic acid derivatives of mono- and di-glycerides; hydrophobic transesterification products of a polyol with at least one member of the group consisting of glycerides, vegetable oils, hydrogenated vegetable oils, fatty acids and sterols; oil-soluble vitamins/vitamin derivatives; and mixtures thereof. Within this group, suitable lipophilic surfactants include glycerol fatty acid esters, propylene glycol fatty acid esters, and mixtures thereof, or are hydrophobic transesterification products of a polyol with at least one member of the group consisting of vegetable oils, hydrogenated vegetable oils, and triglycerides.

In one embodiment, the composition may include a solubilizer to ensure good solubilization and/or dissolution of the compound described herein and to minimize precipitation of the compound described herein. This can be especially important for compositions for non-oral use, e.g., compositions for injection. A solubilizer may also be added to increase the solubility of the hydrophilic drug and/or other components, such as surfactants, or to maintain the composition as a stable or homogeneous solution or dispersion.

Examples of suitable solubilizers include the following: alcohols and polyols, such as ethanol, isopropanol, butanol, benzyl alcohol, ethylene glycol, propylene glycol, butanediols and isomers thereof, glycerol, pentaerythritol, sorbitol, mannitol, transcutol, dimethyl isosorbide, polyethylene glycol, polypropylene glycol, polyvinylalcohol, hydroxypropyl methylcellulose and other cellulose derivatives, cyclodextrins and cyclodextrin derivatives; ethers of polyethylene glycols having an average molecular weight of about 200 to about 6000, such as tetrahydrofurfuryl alcohol PEG ether (glycofurol) or methoxy PEG; amides and other nitrogen-containing compounds such as 2-pyrrolidone, 2-piperidone, ε-caprolactam, N-alkylpyrrolidone, N-hydroxyalkylpyrrolidone, N-alkylpiperidone, N-alkylcaprolactam, dimethylacetamide and polyvinylpyrrolidone; esters such as ethyl propionate, tributylcitrate, acetyl triethylcitrate, acetyl tributyl citrate, triethylcitrate, ethyl oleate, ethyl caprylate, ethyl butyrate, triacetin, propylene glycol monoacetate, propylene glycol diacetate, ε-caprolactone and isomers thereof, δ-valerolactone and isomers thereof, β-butyrolactone and isomers thereof; and other solubilizers known in the art, such as dimethyl acetamide, dimethyl isosorbide, N-methyl pyrrolidones, monooctanoin, diethylene glycol monoethyl ether, and water.

Mixtures of solubilizers may also be used. Examples include triacetin, triethylcitrate, ethyl oleate, ethyl caprylate, dimethylacetamide, N-methylpyrrolidone, N-hydroxyethylpyrrolidone, polyvinylpyrrolidone, hydroxypropyl methylcellulose, hydroxypropyl cyclodextrins, ethanol, polyethylene glycol 200-100, glycofurol, transcutol, propylene glycol, and dimethyl isosorbide. Suitable solubilizers include sorbitol, glycerol, triacetin, ethyl alcohol, PEG-400, glycofurol and propylene glycol.

The amount of solubilizer that can be included is not particularly limited. The amount of a given solubilizer may be limited to a bioacceptable amount, which may be readily determined by one of skill in the art. In some circumstances, it may be advantageous to include amounts of solubilizers far in excess of bioacceptable amounts, for example to maximize the concentration of the drug, with excess solubilizer removed prior to providing the composition to a patient using conventional techniques, such as distillation or evaporation. Thus, if present, the solubilizer can be in a weight ratio of 10%, 25%, 50%, 100%, or up to about 200% by weight, based on the combined weight of the drug, and other excipients. If desired, very small amounts of solubilizer may also be used, such as 5%, 2%, 1% or even less. Typically, the solubilizer may be present in an amount of about 1% to about 100%, more typically about 5% to about 25% by weight.

The composition can further include one or more pharmaceutically acceptable additives and excipients. Such additives and excipients include detackifiers, anti-foaming agents, buffering agents, polymers, antioxidants, preservatives, chelating agents, viscomodulators, tonicifiers, flavorants, colorants, odorants, opacifiers, suspending agents, binders, fillers, plasticizers, lubricants, and mixtures thereof.

In addition, an acid or a base may be incorporated into the composition to facilitate processing, to enhance stability, or for other reasons. Examples of pharmaceutically acceptable bases include amino acids, amino acid esters, ammonium hydroxide, potassium hydroxide, sodium hydroxide, sodium hydrogen carbonate, aluminum hydroxide, calcium carbonate, magnesium hydroxide, magnesium aluminum silicate, synthetic aluminum silicate, synthetic hydrocalcite, magnesium aluminum hydroxide, diisopropylethylamine, ethanolamine, ethylenediamine, triethanolamine, triethylamine, triisopropanolamine, trimethylamine, tris(hydroxymethyl)aminomethane (TRIS) and the like. Also suitable are bases that are salts of a pharmaceutically acceptable acid, such as acetic acid, acrylic acid, adipic acid, alginic acid, alkanesulfonic acid, amino acids, ascorbic acid, benzoic acid, boric acid, butyric acid, carbonic acid, citric acid, fatty acids, formic acid, fumaric acid, gluconic acid, hydroquinosulfonic acid, isoascorbic acid, lactic acid, maleic acid, oxalic acid, para-bromophenylsulfonic acid, propionic acid, p-toluenesulfonic acid, salicylic acid, stearic acid, succinic acid, tannic acid, tartaric acid, thioglycolic acid, toluenesulfonic acid, uric acid, and the like. Salts of polyprotic acids, such as sodium phosphate, disodium hydrogen phosphate, and sodium dihydrogen phosphate can also be used. When the base is a salt, the cation can be any convenient and pharmaceutically acceptable cation, such as ammonium, alkali metals, alkaline earth metals, and the like. Examples may include sodium, potassium, lithium, magnesium, calcium and ammonium.

Suitable acids are pharmaceutically acceptable organic or inorganic acids. Examples of suitable inorganic acids include hydrochloric acid, hydrobromic acid, hydriodic acid, sulfuric acid, nitric acid, boric acid, phosphoric acid, and the like. Examples of suitable organic acids include acetic acid, acrylic acid, adipic acid, alginic acid, alkanesulfonic acids, amino acids, ascorbic acid, benzoic acid, boric acid, butyric acid, carbonic acid, citric acid, fatty acids, formic acid, fumaric acid, gluconic acid, hydroquinosulfonic acid, isoascorbic acid, lactic acid, maleic acid, methanesulfonic acid, oxalic acid, para-bromophenylsulfonic acid, propionic acid, p-toluenesulfonic acid, salicylic acid, stearic acid, succinic acid, tannic acid, tartaric acid, thioglycolic acid, toluenesulfonic acid, uric acid and the like.

### Pharmaceutical Compositions for Injection.

Described herein are pharmaceutical compositions for injection containing a compound of formula II: 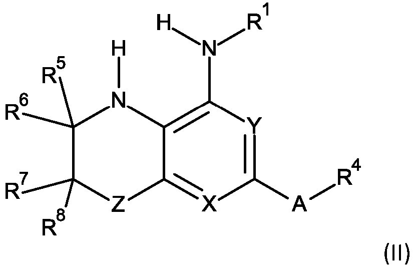 wherein
R¹ is selected from the group consisting of H, substituted or unsubstituted C₁-C₁₀ linear or branched alkyl, substituted or unsubstituted C₂-C₁₀ linear or branched alkenyl, substituted or unsubstituted C₂-C₁₀ linear or branched alkynyl, substituted or unsubstituted C₆-C₁₀ aryl, substituted or unsubstituted C₃-C₁₀ cycloalkyl, substituted or unsubstituted C₃-C₁₀ heterocyloalkyl, substituted or unsubstituted C₅-C₁₀ heteroaryl, substituted or unsubstituted C₆-C₁₀ arylalkyl, substituted or unsubstituted C₅-C₁₀ heteroarylalkyl, substituted or unsubstituted C₁-C₁₀ linear or branched alkylamino and substituted or unsubstituted C₁-C₁₀ linear or branched dialkylamino, or R¹ and its attached N together form a substituted or unsubstituted C₃-C₆ heterocycloalkyl or heteroaryl ring (replacing the H attached to the N);
A is selected from the group consisting of a bond, substituted or unsubstituted C₆-C₁₀ aryl, substituted or unsubstituted C₅-C₁₀ heteroaryl, substituted or unsubstituted C₂-C₁₀ linear or branched alkenyl, substituted or unsubstituted C₂-C₁₀ linear or branched alkynyl, C=O, C=S, -CH₂-, -CH(OH)-, -NH-, -N(CH₃)-, -O-, -S-, and SO₂; and R⁴ is selected from the group consisting of substituted or unsubstituted C₁-C₁₀ linear or branched alkyl, substituted or unsubstituted C₁-C₁₀ linear or branched alkoxy, substituted or unsubstituted C₁-C₁₀ linear or branched alkylamino, substituted or unsubstituted C₁-C₁₀ linear or branched dialkylamino, substituted or unsubstituted C₃-C₁₀ cycloalkyl or heterocycloalkyl, substituted or unsubstituted C₆-C₁₀ aryl, substituted or unsubstituted C₅-C₁₀ heteroaryl, -CN and halo;
R⁵, R⁶, R⁷, R⁸, R⁹ and R¹⁰ are independently selected from the group consisting of H, substituted or unsubstituted C₁-C₁₀ linear or branched alkyl, substituted or unsubstituted C₂-C₁₀ linear or branched alkenyl, substituted or unsubstituted C₂-C₁₀ linear or branched alkynyl, substituted or unsubstituted C₆-C₁₀ aryl, substituted or unsubstituted C₃-C₁₀ cycloalkyl, substituted or unsubstituted C₃-C₁₀ heterocycloalkyl, substituted or unsubstituted C₅-C₁₀ heteroaryl, substituted or unsubstituted C₆-C₁₀ arylalkyl, substituted or unsubstituted C₁-C₁₀ linear or branched alkylamino, and substituted or unsubstituted C₁-C₁₀ linear or branched dialkylamino, or R⁷ and R⁸ together are =O;
X and Y are independently selected from the group consisting of -CH- and -N-; and Z is selected from the group consisting of C=O, -CR⁹R¹⁰-, -NR⁹-, -O-, -S-, -S(O)- and -SO₂-; and wherein a substituted group has 1, 2 or 3 -H atoms substituted by 1, 2 or 3 substituents selected from halo, trifluoromethyl, trifluoromethoxy, methoxy, -COOH, -CHO, -NH₂, -NO₂, - OH, -SH, -SMe, -NHCH₃, -N(CH₃)₂, -CN, and lower alkyl, and a pharmaceutical excipient suitable for injection. Components and amounts of agents in the compositions are as described herein.

The forms in which the novel compositions described herein may be incorporated for administration by injection include aqueous or oil suspensions, or emulsions, with sesame oil, corn oil, cottonseed oil, or peanut oil, as well as elixirs, mannitol, dextrose, or a sterile aqueous solution, and similar pharmaceutical vehicles.

Aqueous solutions in saline are also conventionally used for injection. Ethanol, glycerol, propylene glycol, liquid polyethylene glycol, and the like (and suitable mixtures thereof), cyclodextrin derivatives, and vegetable oils may also be employed. The proper fluidity can be maintained, for example, by the use of a coating, such as lecithin, for the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like.

Sterile injectable solutions are prepared by incorporating a compound of Formula II in the required amount in the appropriate solvent with various other ingredients as enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, certain desirable methods of preparation are vacuum-drying and freeze-drying techniques which yield a powder of the active ingredient plus any additional desired ingredient from a previously sterile-filtered solution thereof.

### Pharmaceutical Compositions for Topical (e.g., Transdermal) Delivery.

Also described herein is a pharmaceutical composition for transdermal delivery containing a compound of formula II: 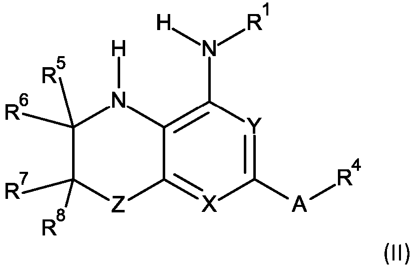 wherein
R¹ is selected from the group consisting of H, substituted or unsubstituted C₁-C₁₀ linear or branched alkyl, substituted or unsubstituted C₂-C₁₀ linear or branched alkenyl, substituted or unsubstituted C₂-C₁₀ linear or branched alkynyl, substituted or unsubstituted C₆-C₁₀ aryl, substituted or unsubstituted C₃-C₁₀ cycloalkyl, substituted or unsubstituted C₃-C₁₀ heterocycloalkyl, substituted or unsubstituted C₅-C₁₀ heteroaryl, substituted or unsubstituted C₆-C₁₀ arylalkyl, substituted or unsubstituted C₅-C₁₀ heteroarylalkyl, substituted or unsubstituted C₁-C₁₀ linear or branched alkylamino and substituted or unsubstituted C₁-C₁₀ linear or branched dialkylamino, or R¹ and its attached N together form a substituted or unsubstituted C₃-C₆ heterocycloalkyl or heteroaryl ring (replacing the H attached to the N);
A is selected from the group consisting of a bond, substituted or unsubstituted C₆-C₁₀ aryl, substituted or unsubstituted C₅-C₁₀ heteroaryl, substituted or unsubstituted C₂-C₁₀ linear or branched alkenyl, substituted or unsubstituted C₂-C₁₀ linear or branched alkynyl, C=O, C=S, -CH₂-, -CH(OH)-, -NH-, -N(CH₃)-, -O-, -S-, and SO₂; and R⁴ is selected from the group consisting of substituted or unsubstituted C₁-C₁₀ linear or branched alkyl, substituted or unsubstituted C₁-C₁₀ linear or branched alkoxy, substituted or unsubstituted C₁-C₁₀ linear or branched alkylamino, substituted or unsubstituted C₁-C₁₀ linear or branched dialkylamino, substituted or unsubstituted C₃-C₁₀ cycloalkyl or heterocycloalkyl, substituted or unsubstituted C₆-C₁₀ aryl, substituted or unsubstituted C₅-C₁₀ heteroaryl, -CN and halo;
R⁵, R⁶, R⁷, R⁸, R⁹ and R¹⁰ are independently selected from the group consisting of H, substituted or unsubstituted C₁-C₁₀ linear or branched alkyl, substituted or unsubstituted C₂-C₁₀ linear or branched alkenyl, substituted or unsubstituted C₂-C₁₀ linear or branched alkynyl, substituted or unsubstituted C₆-C₁₀ aryl, substituted or unsubstituted C₃-C₁₀ cycloalkyl, substituted or unsubstituted C₃-C₁₀ heterocycloalkyl, substituted or unsubstituted C₅-C₁₀ heteroaryl, substituted or unsubstituted C₆-C₁₀ arylalkyl, substituted or unsubstituted C₁-C₁₀ linear or branched alkylamino, and substituted or unsubstituted C₁-C₁₀ linear or branched dialkylamino, or R⁷ and R⁸ together are =O;
X and Y are independently selected from the group consisting of -CH- and -N-; and
Z is selected from the group consisting of C=O, -CR⁹R¹⁰-, -NR⁹-, -O-, -S-, -S(O)- and - SO₂-; and wherein a substituted group has 1, 2 or 3 -H atoms substituted by 1, 2 or 3 substituents selected from halo, trifluoromethyl, trifluoromethoxy, methoxy, -COOH, -CHO, -NH₂, -NO₂, -OH, -SH, -SMe, -NHCH₃, -N(CH₃)₂, -CN, and lower alkyl,
and a pharmaceutical excipient suitable for transdermal delivery.

Compositions described herein can be formulated into preparations in solid, semi-solid, or liquid forms suitable for local or topical administration, such as gels, water soluble jellies, creams, lotions, suspensions, foams, powders, slurries, ointments, solutions, oils, pastes, suppositories, sprays, emulsions, saline solutions, or dimethylsulfoxide (DMSO)-based solutions. In general, carriers with higher densities are capable of providing an area with a prolonged exposure to the active ingredients. In contrast, a solution formulation may provide more immediate exposure of the active ingredient to the chosen area.

The pharmaceutical compositions also may comprise suitable solid or gel phase carriers or excipients, which are compounds that allow increased penetration of, or assist in the delivery of, therapeutic molecules across the stratum corneum permeability barrier of the skin. There are many of these penetration-enhancing molecules known to those trained in the art of topical formulation. Examples of such carriers and excipients include humectants (e.g., urea), glycols (e.g., propylene glycol), alcohols (e.g., ethanol), fatty acids (e.g., oleic acid), surfactants (e.g., isopropyl myristate and sodium lauryl sulfate), pyrrolidones, glycerol monolaurate, sulfoxides, terpenes (e.g., menthol), amines, amides, alkanes, alkanols, water, calcium carbonate, calcium phosphate, various sugars, starches, cellulose derivatives, gelatin, and polymers such as polyethylene glycols.

Another exemplary formulation for use in the methods described herein employs transdermal delivery devices ("patches"). Such transdermal patches may be used to provide continuous or discontinuous infusion of a compound of Formula II in controlled amounts, either with or without another agent.

The construction and use of transdermal patches for the delivery of pharmaceutical agents is well known in the art. See, e.g., U.S. Pat. Nos. 5,023,252, 4,992,445 and 5,001,139. Such patches may be constructed for continuous, pulsatile, or on-demand delivery of pharmaceutical agents.

### Pharmaceutical Compositions for Inhalation.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described supra. The compositions may be administered by the oral or nasal respiratory route, for example, for local or systemic effect. Compositions in pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered in any manner, such as orally or nasally, from devices that deliver the formulation in an appropriate manner.

### Other Pharmaceutical Compositions.

Pharmaceutical compositions may also be prepared from compositions described herein and one or more pharmaceutically acceptable excipients suitable for sublingual, buccal, rectal, intraosseous, intraocular, intranasal, epidural, or intraspinal administration. Preparations for such pharmaceutical compositions are well-known in the art. See, e.g., See, e.g., Anderson, Philip O.; Knoben, James E.; Troutman, William G, eds., Handbook of Clinical Drug Data, Tenth Edition, McGraw-Hill, 2002; Pratt and Taylor, eds., Principles of Drug Action, Third Edition, Churchill Livingston, N.Y., 1990; Katzung, ed., Basic and Clinical Pharmacology, Ninth Edition, McGraw Hill, 2004; Goodman and Gilman, eds., The Pharmacological Basis of Therapeutics, Tenth Edition, McGraw Hill, 2001; Remington's Pharmaceutical Sciences, 20th Ed., Lippincott Williams & Wilkins., 2000; Martindale, The Extra Pharmacopoeia, Thirty-Second Edition (The Pharmaceutical Press, London, 1999).

Administration of the compounds of Formula II or pharmaceutical compositions described herein can be effected by any method that enables delivery of the compounds to the site of action. These methods include oral routes, intraduodenal routes, parenteral injection (including intravenous, intraarterial, subcutaneous, intramuscular, intravascular, intraperitoneal or infusion), topical (e.g. transdermal application), rectal administration, via local delivery by catheter or stent or through inhalation. Compounds can also be administered intraadiposally or intrathecally.

The amount of a compound of Formula II administered will be dependent on the mammal being treated, the severity of the disorder or condition, the rate of administration, the disposition of the compound and the discretion of the prescribing physician. However, an effective dosage is in the range of about 0.001 to about 100 mg per kg body weight per day, such as from about 1 to about 35 mg/kg/day, in single or divided doses. For a 70 kg human, this would amount to about 0.05 to 7 g/day, such as about 0.05 to about 2.5 g/day. In some instances, dosage levels below the lower limit of the aforesaid range may be more than adequate, while in other cases still larger doses may be employed without causing any harmful side effect, e.g. by dividing such larger doses into several small doses for administration throughout the day.

In some embodiments, a compound of Formula II is administered in a single dose. Typically, such administration will be by injection, e.g., intravenous injection, in order to introduce the agent quickly. However, other routes may be used as appropriate.

In some embodiments, a compound of Formula II is administered in multiple doses. Dosing may be about once, twice, three times, four times, five times, six times, or more than six times per day. Dosing may be about once a month, once every two weeks, once a week, or once every other day. In another embodiment a compound and another agent are administered together about once per day to about 6 times per day. In another embodiment the administration of a compound of Formula II and an agent continues for less than about 7 days. In yet another embodiment the administration continues for more than about 6, 10, 14, 28 days, two months, six months, or one year. In some cases, continuous dosing is achieved and maintained as long as necessary.

Administration of the compound(s) of Formula II may continue as long as necessary. In some embodiments, a compound of Formula II is administered for more than 1, 2, 3, 4, 5, 6, 7, 14, or 28 days. In some embodiments, a compound of Formula II is administered for less than 28, 14, 7, 6, 5, 4, 3, 2, or 1 day. In some embodiments, a compound of Formula II is administered chronically on an ongoing basis, e.g., for the treatment of chronic effects.

An effective amount of a compound of Formula II may be administered in either single or multiple doses by any of the accepted modes of administration of agents having similar utilities, including rectal, buccal, intranasal and transdermal routes, by intra-arterial injection, intravenously, intraperitoneally, parenterally, intramuscularly, subcutaneously, orally, topically, or as an inhalant.

The compositions described herein may also be delivered via an impregnated or coated device such as a stent, for example, or an artery-inserted cylindrical polymer. A compound of Formula II may be administered, for example, by local delivery from the struts of a stent, from a stent graft, from grafts, or from the cover or sheath of a stent. In some embodiments, a compound of Formula II is admixed with a matrix. Such a matrix may be a polymeric matrix, and may serve to bond the compound to the stent. Polymeric matrices suitable for such use, include, for example, lactone-based polyesters or copolyesters such as polylactide, polycaprolactonglycolide, polyorthoesters, polyanhydrides, polyaminoacids, polysaccharides, polyphosphazenes, poly (ether-ester) copolymers (e.g. PEO-PLLA); polydimethylsiloxane, poly(ethylene-vinylacetate), acrylate-based polymers or copolymers (e.g. polyhydroxyethyl methylmethacrylate, polyvinyl pyrrolidinone), fluorinated polymers such as polytetrafluoroethylene and cellulose esters. Suitable matrices may be non-degrading or may degrade with time, releasing the compound or compounds. A compound of Formula II may be applied to the surface of the stent by various methods such as dip/spin coating, spray coating, dip-coating, and/or brush-coating. The compounds may be applied in a solvent and the solvent may be allowed to evaporate, thus forming a layer of compound onto the stent. Alternatively, a compound of Formula II may be located in the body of the stent or graft, for example in microchannels or micropores. When implanted, the compound diffuses out of the body of the stent to contact the arterial wall. Such stents may be prepared by dipping a stent manufactured to contain such micropores or microchannels into a solution of a compound of Formula II in a suitable solvent, followed by evaporation of the solvent. Excess drug on the surface of the stent may be removed via an additional brief solvent wash. In yet other embodiments, a compound of Formula II may be covalently linked to a stent or graft. Any bio-labile linkage may be used for such a purpose, such as ester, amide or anhydride linkages. A compound of Formula II may additionally be administered intravascularly from a balloon used during angioplasty. Extravascular administration of a compound of Formula II via the pericardium or via adventitial application of formulations described herein may also be performed to decrease restenosis.

A variety of stent devices which may be used as described are disclosed, for example, in the following references: U.S. Pat. No. 5,451,233; U.S. Pat. No. 5,040,548; U.S. Pat. No. 5,061,273; U.S. Pat. No. 5,496,346; U.S. Pat. No. 5,292,331; U.S. Pat. No. 5,674,278; U.S. Pat. No. 3,657,744; U.S. Pat. No. 4,739,762; U.S. Pat. No. 5,195,984; U.S. Pat. No. 5,292,331; U.S. Pat. No. 5,674,278; U.S. Pat. No. 5,879,382; U.S. Pat. No. 6,344,053.

The compounds of Formula II may be administered in dosages. It is known in the art that due to inter-subject variability in compound pharmacokinetics, individualization of dosing regimen is necessary for optimal therapy. Dosing for a compound of Formula II may be found by routine experimentation in light of the instant disclosure.

When a compound of Formula II is administered in a composition that comprises one or more agents, and the agent has a shorter half-life than the compound of Formula II unit dose forms of the agent and the compound of Formula II may be adjusted accordingly.

The subject pharmaceutical composition may, for example, be in a form suitable for oral administration as a tablet, capsule, pill, powder, sustained release formulations, solution, or suspension, for parenteral injection as a sterile solution, suspension or emulsion, for topical administration as an ointment or cream or for rectal administration as a suppository. The pharmaceutical composition may be in unit dosage forms suitable for single administration of precise dosages. The pharmaceutical composition will include a conventional pharmaceutical carrier or excipient and a compound of Formula II as an active ingredient. In addition, it may include other medicinal or pharmaceutical agents, carriers, adjuvants, etc.

Exemplary parenteral administration forms include solutions or suspensions of active compound in sterile aqueous solutions, for example, aqueous propylene glycol or dextrose solutions. Such dosage forms can be suitably buffered, if desired.

Kits are also described herein. The kits include one or more compounds of Formula II as described herein, in suitable packaging, and written material that can include instructions for use, discussion of clinical studies, listing of side effects, and the like. Such kits may also include information, such as scientific literature references, package insert materials, clinical trial results, and/or summaries of these and the like, which indicate or establish the activities and/or advantages of the composition, and/or which describe dosing, administration, side effects, drug interactions, or other information useful to the health care provider. Such information may be based on the results of various studies, for example, studies using experimental animals involving in vivo models and studies based on human clinical trials. The kit may further contain another agent. In some embodiments, a compound of Formula II and the agent are provided as separate compositions in separate containers within the kit. In some embodiments, the compound described herein and the agent are provided as a single composition within a container in the kit. Suitable packaging and additional articles for use (e.g., measuring cup for liquid preparations, foil wrapping to minimize exposure to air, and the like) are known in the art and may be included in the kit. Kits described herein can be provided, marketed and/or promoted to health providers, including physicians, nurses, pharmacists, formulary officials, and the like. Kits may also, in some embodiments, be marketed directly to the consumer.

### Therapeutic Methods

The compounds and pharmaceutical compositions described herein, in therapeutically effective amounts and as described above, are useful in a method of treating ferroptosis-related diseases such as lipid peroxidation-related degenerative diseases, excitotoxic diseases, neurodegenerative diseases, non-apoptotic regulated cell-death diseases, wasting- or necrosis-related diseases, intoxication-related diseases, and infectious diseases.

The compound of formula II: 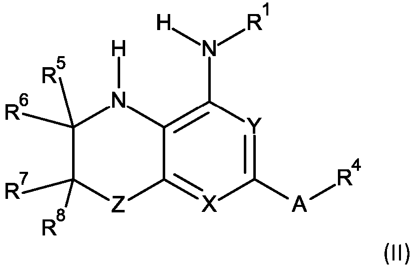 wherein
R¹ is selected from the group consisting of H, substituted or unsubstituted C₁-C₁₀ linear or branched alkyl, substituted or unsubstituted C₂-C₁₀ linear or branched alkenyl, substituted or unsubstituted C₂-C₁₀ linear or branched alkynyl, substituted or unsubstituted C₆-C₁₀ aryl, substituted or unsubstituted C₃-C₁₀ cycloalkyl, substituted or unsubstituted C₃-C₁₀ heterocycloalkyl, substituted or unsubstituted C₅-C₁₀ heteroaryl, substituted or unsubstituted C₆-C₁₀ arylalkyl, substituted or unsubstituted C₅-C₁₀ heteroarylalkyl, substituted or unsubstituted C₁-C₁₀ linear or branched alkylamino and substituted or unsubstituted C₁-C₁₀ linear or branched dialkylamino, or R¹ and its attached N together form a substituted or unsubstituted C₃-C₆ heterocycloalkyl or heteroaryl ring (replacing the H attached to the N);
A is selected from the group consisting of a bond, substituted or unsubstituted C₆-C₁₀ aryl, substituted or unsubstituted C₅-C₁₀ heteroaryl, substituted or unsubstituted C₂-C₁₀ linear or branched alkenyl, substituted or unsubstituted C₂-C₁₀ linear or branched alkynyl, C=O, C=S, -CH₂-, -CH(OH)-, -NH-, -N(CH₃)-, -O-, -S-, and SO₂; and R⁴ is selected from the group consisting of substituted or unsubstituted C₁-C₁₀ linear or branched alkyl, substituted or unsubstituted C₁-C₁₀ linear or branched alkoxy, substituted or unsubstituted C₁-C₁₀ linear or branched alkylamino, substituted or unsubstituted C₁-C₁₀ linear or branched dialkylamino, substituted or unsubstituted C₃-C₁₀ cycloalkyl or heterocycloalkyl, substituted or unsubstituted C₆-C₁₀ aryl, substituted or unsubstituted C₅-C₁₀ heteroaryl, -CN and halo;
R⁵, R⁶, R⁷, R⁸, R⁹ and R¹⁰ are independently selected from the group consisting of H, substituted or unsubstituted C₁-C₁₀ linear or branched alkyl, substituted or unsubstituted C₂-C₁₀ linear or branched alkenyl, substituted or unsubstituted C₂-C₁₀ linear or branched alkynyl, substituted or unsubstituted C₆-C₁₀ aryl, substituted or unsubstituted C₃-C₁₀ cycloalkyl, substituted or unsubstituted C₃-C₁₀ heterocycloalkyl, substituted or unsubstituted C₅-C₁₀ heteroaryl, substituted or unsubstituted C₆-C₁₀ arylalkyl, substituted or unsubstituted C₁-C₁₀ linear or branched alkylamino, and substituted or unsubstituted C₁-C₁₀ linear or branched dialkylamino, or R⁷ and R⁸ together are =O;
X and Y are independently selected from the group consisting of -CH- and -N-; and Z is selected from the group consisting of C=O, -CR⁹R¹⁰-, -NR⁹-, -O-, -S-, -S(O)- and - SO₂-; and wherein a substituted group has 1, 2 or 3 -H atoms substituted by 1, 2 or 3 substituents selected from halo, trifluoromethyl, trifluoromethoxy, methoxy, -COOH, -CHO, -NH₂, -NO₂, -OH, -SH, -SMe, -NHCH₃, -N(CH₃)₂, -CN, and lower alkyl for use in a method of treating a ferroptosis-related disease in a patient in need thereof comprising administering to the patient a therapeutically effective amount of the compound of formula II.

In one embodiment, the therapeutically effective amount of a compound described above is used in methods to treat diseases selected from the group consisting of atherosclerosis, ischemia-reperfusion, heart failure, Alzheimer's disease, rheumatic arthritis, thalassemia, chronic obstructive pulmonary disease (COPD), age-related macular degeneration, senescence, cancer, and immunological disorders.

In one aspect of the above, the disease is an immunological disorder. Exemplary immunological disorders include autoimmune diseases (for example, diabetes mellitus, arthritis (including rheumatoid arthritis, juvenile rheumatoid arthritis, osteoarthritis and psoriatic arthritis), multiple sclerosis, encephalomyelitis, myasthenia gravis, systemic lupus erythematosus, autoimmune thyroiditis, dermatitis (including atopic dermatitis and eczematous dermatitis), psoriasis, Sjogren's Syndrome, Crohn's disease, aphthous ulcer, iritis, conjunctivitis, keratoconjunctivitis, ulcerative colitis, asthma, allergic asthma, sepsis and septic shock, inflammatory bowel disorder, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens- Johnson syndrome, glomerulonephritis, idiopathic sprue, lichen planus, Graves' disease, sarcoidosis, primary biliary cirrhosis, uveitis posterior, interstitial lung fibrosis, graft-versus-host disease, transplantation rejection, and allergies such as atopic allergy.

In another embodiment, the therapeutically effective amount of a compound described above is used in methods to treat diseases selected from the group consisting of epilepsy, kidney disease, stroke, myocardial infarction, congestive heart failure, type I diabetes, traumatic brain injury (TBI), and periventricular leukomalacia (PVL).

In still another embodiment, the therapeutically effective amount of a compound described above is used in methods to treat diseases selected from the group consisting of Alzheimer's disease, Parkinson's disease, Amyotrophic lateral sclerosis, Friedreich's ataxia, ataxia-telangiectasia, Rett syndrome, X-linked adrenoleukodystrophy, Multiple sclerosis, Huntington's Disease, transmissible spongiform encephalopathy, Charcot-Marie-Tooth disease, Lewy body dementia, Menke's disease, Wilson's disease, Creutzfeldt-Jakob disease, Fahr disease, frontotemporal dementia, amyloidosis, Tay-Sachs disease periventricular leukomalacia, corticobasal degeneration, progressive supranuclear palsy, and hereditary spastic paraparesis.

In yet another embodiment, the therapeutically effective amount of a compound described above is used in methods to treat diseases selected from the group consisting of a reduction in cell-proliferation, an alteration in cell-differentiation or intracellular signaling, undesirable inflammation, cell death of retinal neuronal cells, cardiac muscle cells, or cells of the immune system or cell death associated with renal failure, neonatal respiratory distress, asphyxia, incarcerated hernia, placental infarct, iron-load complications, endometriosis, congenital disease, head trauma/traumatic brain injury, liver injury, injuries from environmental radiation, burns, cold injuries, mechanical injuries, decompression sickness, priapism, snake, scorpion or spider bites, UV-damage in skin, aging in skin, and hair loss.

In a further embodiment, the therapeutically effective amount of a compound described above is used in methods to treat diseases selected from the group consisting of muscle wasting diseases, muscular dystrophies or related diseases (e.g., Becker's muscular dystrophy, Duchenne muscular dystrophy, myotonic dystrophy, limb-girdle muscular dystrophy, Landouzy-Dejerine muscular dystrophy, facioscapulohumeral muscular dystrophy (Steinert's disease), myotonia congenita, Thomsen's disease, and Pompe's disease), ischemia, compartment syndrome, gangrene, pressure sores, sepsis, degenerative arthritis, retinal necrosis, heart disease, liver, gastrointestinal or pancreatic necrotic diseases (such as acute necrotizing pancreatitis), avascular necrosis, diabetes, sickle cell disease, alteration of blood vessels, and cancer-chemo/radiation therapy-induced cell-death.

In another embodiment, the therapeutically effective amount of a compound described above is used in methods to treat and infectious diseases, wherein the infectious disease is caused by infection by viruses, bacteria, fungi, or other microorganisms.

In one aspect of the above, the infectious disease is caused by a virus. Exemplary viruses include human immunodeficiency virus (HIV), Epstein- Barr virus (EBV), cytomegalovirus (CMV) (e.g., CMV5), human herpesviruses (HHV) (e.g., HHV6, 7 or 8), herpes simplex viruses (HSV), bovine herpes virus (BHV) (e.g., BHV4), equine herpes virus (EHV) (e.g., EHV2), human T-Cell leukemia viruses (HTLV)5, Varicella-Zoster virus (VZV), measles virus, papovaviruses (JC and BK), hepatitis viruses (E.g., HBV or HCV), myxoma virus, adenovirus, parvoviruses, polyoma virus, influenza viruses, papillomaviruses and poxviruses such as vaccinia virus, molluscum contagiosum virus (MCV), and lyssaviruses. Exemplary diseases caused by viral infection include chicken pox, Cytomegalovirus infections, genital herpes, Hepatitis B and C, influenza, shingles, and rabies.

In another aspect of the above, the infectious disease is caused by bacteria. Exemplary bacteria include *Campylobacter jejuni, Enterobacter* species, *Enterococcus faecium, Enterococcus faecalis, Escherichia coli* (*e.g., E. coli* 0157:H7), Group A *streptococci, Haemophilus influenzae, Helicobacter pylori, listeria, Mycobacterium tuberculosis, Pseudomonas aeruginosa, S. pneumoniae, Salmonella, Shigella, Staphylococcus aureus, Staphylococcus epidermidis, Borrelia* and *Rickettsia.* Exemplary diseases caused by bacterial infection include anthrax, cholera, diphtheria, foodborne illnesses, leprosy, meningitis, peptic ulcer disease, pneumonia, sepsis, septic shock, syphilis, tetanus, tuberculosis, typhoid fever, urinary tract infection, Lyme disease and Rocky Mountain spotted fever.

### EXPERIMENTAL

All reagents were purchased from commercial suppliers and used as supplied unless stated otherwise. Reactions were carried out in air unless stated otherwise. 400 MHz ¹H NMR spectra were obtained on a JEOL AS 400 spectrometer. Low-resolution mass spectra (LRMS) were obtained on a JEOL JMS-T100LC DART/ AccuTOF mass spectrometer. Measurement of reversal of protein aggregation may be carried out using such assays as Bis-ANS Fluorescence as described in, for example, W. T. Chen et al., J. Biol. Chem, 2011, 286 (11), 9646.

### Example 1

### Synthesis of Fused Pyrimidine Ketones

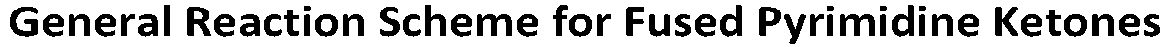

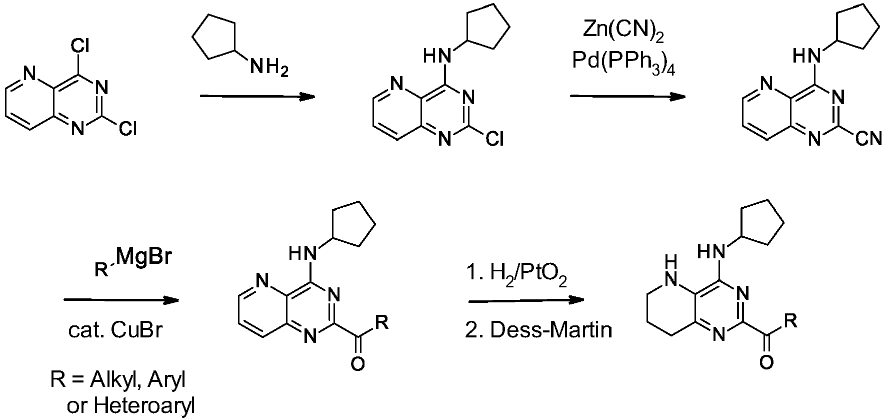

### Step 1. Synthesis of Cl-displacement intermediates

### 2-chloro-N-cyclopentyl-pyrido[3,2-d]pyrimidin-4-amine (K-04).

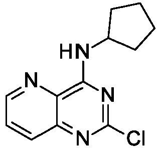

A 250 mL RBF was charged with 2,4-dichloropyrido[3,2-d]pyrimidine (2 g, 10 mmol), a stir bar, THF (20 mL, 0.5 M), DiPEA (1.25 equiv., 2.2 mL, 12.5 mmol), cyclopentylamine (1 equiv., 851 mg, 10 mmol) and was stirred at RT. The reaction mixture immediately became a milky bright yellow color and stirring was continued. After 2 h, the reaction was partitioned between 50 mL of EtOAc and 50 mL of H₂O, the water layer back extracted 1 x 25 mL EtOAc and the combined organic layer was dried over Na₂SO₄ and concentrated under reduced pressure to provide **2-chloro-N-cyclopentyl-pyrido[3,2-d]pyrimidin-4-amine (K-04)** as a viscous yellow oil (2.4 g, 96.5%) and the material was used in the next step without further purification. ¹H NMR (CDCl₃): δ 8.65 (t, 1H), 7.99 (dd, 1H), 7.65 (m, 1H) 7.32 (bs, 1H), 4.63 (m, 1H), 2.20 (m, 2H), 2.72 (m, 6H); ¹³C NMR (CDCl₃): δ 160.2, 158.4, 148.0, 145.4, 134.9, 130.6, 128.1, 52.4, 32.9, 23.7: (APCI) m/e 249.1 (M+H). Note: the reaction can also be run overnight at RT with the same result.

### 2-chloro-4-pyrrolidin-1-yl-pyrido[3,2-d]pyrimidine (K-05).

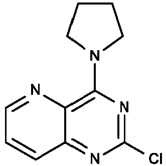

A 40 mL vial was charged with 2,4-dichloropyrido[3,2-d]pyrimidine (400 mg, 2 mmol), a stir bar, THF (4 mL, 0.5 M), DiPEA (1.25 equiv., 323 mg, 2.5 mmol), pyrrolidine (1 equiv., 142 mg, 2 mmol) and was stirred at RT. The reaction mixture immediately became a warm milky yellow color that quickly changed to a thick slurry and stirring was continued. After 24 h, the reaction was partitioned between 25 mL of EtOAc and 25 mL of H₂O, the water layer back extracted 1 x 25 mL EtOAc and the combined organic layer was dried over Na₂SO₄ and concentrated under reduced pressure to provide **2-chloro-4-pyrrolidin-1-yl-pyrido[3,2-d]pyrimidine (K-05)** as a yellow solid (422 mg, 89.9%) and the material was used in the next step without further purification. ¹H NMR (CDCl₃): δ 8.68 (t, 1H), 7.96 (t, 1H), 7.57 (m, 1H), 4.46 (t, 2H), 3.87 (t, 2H), 2.11 (m, 2H), 2.08 (m, 2H); ¹³C NMR (CDCl₃): δ 158.8, 157.4, 148.1, 146.7, 134.3, 133.1, 127.0, 51.7, 50.4, 27.0, 23.6: (APCI) m/e 235.0 (M+H).

### N-tert-butyl-2-chloro-pyrido[3,2-d]pyrimidin-4-amine (K-06).

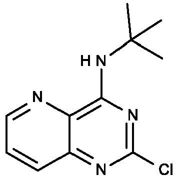

A 40 mL vial was charged with 2,4-dichloropyrido[3,2-d]pyrimidine (400 m g, 2 mmol), a stir bar, THF (4 mL, 0.5 M), DiPEA (1.25 equiv., 323 mg, 2.5 mmol), tert-butyl amine (1.25 equiv., 323 mg, 2.5 mmol) and was stirred at RT. After 24 h, the reaction was partitioned between 25 mL of EtOAc and 25 mL of H₂O, the water layer was back extracted 1 x 25 mL EtOAc and the combined organic layer was dried over Na₂SO₄ and concentrated under reduced pressure to provide a yellow oil. The oil was triturated with diethyl ether to provide **N-tert-butyl-2-chloro-pyrido[3,2-d]pyrimidin-4-amine (K-06)** as a yellow solid (246 mg, 52%) and the material was used in the next step without further purification. ¹H NMR (CDCl₃): δ 8.60 (dd, 1H), 7.95 (dd, 1H), 7.58 (m, 1H) 7.33 (bs, 1H), 1.57 (s, 9H); ¹³C NMR (CDCl₃): δ 160.0, 157.9, 147.8, 145.2, 135.1, 131.0, 128.0, 52.8, 28.4; (APCI) m/e 237.0 (M+H).

### 2-chloro-N-(2-pyridyl)pyrido[3,2-d]pyrimidin-4-amine (K-08).

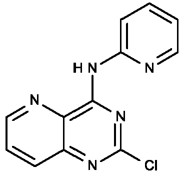

A 250 mL RBF was charged with 2-aminopyridine (1 equiv., 5.0 mmol, 471 mg), tetrahydrofuran (10 mL, 0.5 M), DiPEA (1.5 equiv., 7.5 mmol, 1.31 mL) and then 2,4-dichloropyrido[3,2-d]pyrimidine (1 g, 0.5 mmol). The reaction was stirred at room temperature for 16 h and then partitioned between 50 mL water and 50 mL EtOAc. The water layer was back extracted 2 x 25 mL EtOAc and the combined organic layer was dried over anhydrous sodium sulfate and concentrated under reduced pressure. The resulting residue was purified on SiO₂ (40 g, 5-100% hexanes/EtOAC) to provide **2-chloro-N-(2-pyridyl)pyrido[3,2-d]pyrimidin-4-amine (K-08)** as a pale yellow solid (285 mg, 22%). (APCI) m/e 258.0 (M+H).

### 2-chloro-N-prop-2-ynyl-pyrido[3,2-d]pyrimidin-4-amine (K-13).

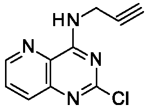

A 40 mL vial was charged with 2,4-dichloropyrido[3,2-d]pyrimidine (400 m g, 2 mmol), a stir bar, THF (4 mL, 0.5 M), DiPEA (1.5 equiv., 0.52 mL, 2.5 mmol), prop-2-yn-1-amine(1 equiv., 110 mg, 2 mmol) and was stirred at RT. The reaction mixture immediately became a warm milky yellow color that quickly changed to a thick slurry and stirring was continued. After 2 h, the reaction was partitioned between 5 mL of EtOAc and 5 mL of H₂O, the water layer back extracted 1 x 5 mL EtOAc and the combined organic layer was dried over Na₂SO₄ and concentrated under reduced pressure to provide 350 mg (80%) desired product that was used directly in the next step without further purification. (APCI) m/e 219.0 (M+H).

### 2-chloro-N-(3-methyltetrahydrofuran-3-yl)pyrido[3,2-d]pyrimidin-4-amine (N-07)

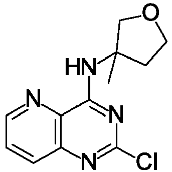

A 100 mL RBF was charged with 2,4-dichloropyrido[3,2-d]pyrimidine (1 g, 5 mmol), a stir bar, THF (10 mL, 0.5 M), DiPEA (2 equiv., 1.75 mL, 10 mmol), 3-methyltetrahydrofuran-3-amine (1 equiv., 506 mg, 5 mmol) and was stirred at RT. After 16 h, the reaction was partitioned between 50 mL of EtOAc and 50 mL of H₂O, the water layer back extracted 1 x 25 mL EtOAc and the combined organic layer was dried over Na₂SO₄ and concentrated under reduced pressure. The residue was purified on silica gel (80 g, 0-60% EtOAc/hexanes) to provide 1.13 g of 2-chloro-N-(3-methyltetrahydrofuran-3-yl)pyrido[3,2-d]pyrimidin-4-amine as a yellow solid (85%). (APCI) m/e 265.0 (M+H).

### Step 2. Synthesis of Cyano Intermediates

### 4-(cyclopentylamino)pyrido[3,2-d]pyrimidine-2-carbonitrile (C-73).

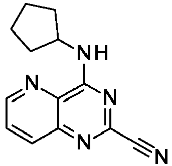

A solution of 2-chloro-N-cyclopentyl-pyrido[3,2-d]pyrimidin-4-amine (1.05g, 4.2mmole) in anhydrous DMF (15.0mL) was degassed 5 x and then successively treated with zinc cyanide (0.993g, 8.4mmol, 2equiv) and then tetrakis(triphenylphosphine)palladium(0) (0.735g, 0.63mmol, 0.15equiv). The reaction mixture was warmed in a microwave to 150°C for 30min. LC/MS analysis of the crude reaction mixture showed conversion to the desired product and full consumption of the starting material. The mixture was filtered and adsorbed onto 10g silica. The product was purified by flash chromatography (40g silica, 0-50% ethyl acetate/hexanes) to afford **4-(cyclopentylamino)pyrido[3,2-d]pyrimidine-2-carbonitrile (C-73)** as a yellow solid (0.784g, 77.6%). ¹H NMR (400 Mz, (CD₃)₂CO) δ 8.74 (1H, dd), 8.26 (1H, dd), 7.69 (1H, dd), 7.23 (1H, bd), 4.68 (1H, sextet), 3.24 (2H, t), 2.22 (2H, m), 1.75, (8H, m), 1.46 (2H, sextet), 0.97 (3H, t); ¹³C NMR (400 Mz, (CD₃)₂CO) δ 160.5, 151.4, 144.8, 142.6, 136.7, 132.7, 129.8, 117.6, 53.5, 32.9, 24.5. MS (APCI) for C₁₃H₁₃N₅; Calculated: 240.1 [M + H⁺], Found: 240.1.

### 4-(tert-butylamino)pyrido[3,2-d]pyrimidine-2-carbonitrile (C-87).

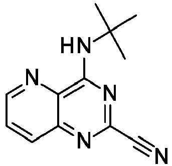

A solution of N-tert-butyl-2-chloro-pyrido[3,2-d]pyrimidin-4-amine (0.21g, 0.88mmole) in anhydrous DMF (3mL) was degassed 5 x and then successively treated with zinc cyanide (0.21g, 1.8mmol, 2equiv) and then tetrakis(triphenylphosphine)palladium(0) (0.153g, 0.13mmol, 0.15equiv). The reaction mixture was warmed in a microwave to 150°C for 30min. LC/MS analysis of the crude reaction mixture showed conversion to the desired product and full consumption of the starting material. The mixture was filtered and adsorbed onto 1g silica. The product was purified by flash chromatography (12g silica, 0-50% ethyl acetate/hexanes) to afford **4-(tert-butylamino)pyrido[3,2-d]pyrimidine-2-carbonitrile (C-87)** as a pale yellow solid (0.167g, 83.2%). MS (APCI) for C₁₂H₁₃N₅; Calculated: 228.1 [M + H⁺], Found: 228.1.

### Step 3. Synthesis of Ketone Intermediates

### 1-[4-(cyclopentylamino)pyrido[3,2-d]pyrimidin-2-yl]pentan-1-one (C-76).

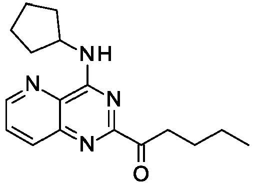

A solution of 4-(cyclopentylamino)pyrido[3,2-d]pyrimidine-2-carbonitrile (0.574g, 2.4mmol) in anhydrous THF (10mL) was cooled to -78°C and then treated with sodium hydride (0.138g, 3.6mmol, 1.5equiv) and the mixture was left stirring for 30min. The mixture was then successively treated with copper (I) bromide (52mg, 0.36mmol, 0.15equiv) and then butylmagnesium bromide (2M in diethyl ether, 1.6mL, 5.3mmol, 2.2equiv). After stirring for 20min, the reaction mixture was slowly warmed to 30°C. LC/MS analysis after four hours showed partial conversion to the desired product. The mixture was then warmed to 0°C. After an additional 4hrs., LC/MS showed clean conversion to the desired product. The reaction mixture was quenched with satd. aq. ammonium chloride (10mL) and poured onto ethyl acetate (50mL). The layers were separated and the aqueous layer was extracted with ethyl acetate (2 x 50mL). The combined organic extracts were dried (Na₂SO₄) and the solvent was removed *in vacuo.* The residual oil was purified by flash chromatography (24g silica, 0-50% ethyl acetate/hexanes) to afford **1-[4-(cyclopentylamino)pyrido[3,2-d]pyrimidin-2-yl]pentan-1-one (C-76)** as a yellow oil (0.630g, 88.0%). ¹H NMR (400 Mz, (CD₃)₂CO) δ 8.74 (1H, dd), 8.02 (1H, dd), 7.78, (1H, dd), 4.52 (1H, pent), 2.03, (2H, m), 1.71 (4H, m), 1.58 (2H, m); ¹³C NMR (400 Mz, (CD₃)₂CO) δ 160.5, 151.4, 144.8, 142.6, 136.7, 132.7, 129.8, 117.6, 53.5, 32.9, 24.5. MS (APCI) for C₁₇H₂₂N₄O; Calculated: 299.2 [M + H⁺], Found: 299.1.

### 1-[4-(cyclopentylamino)pyrido[3,2-d]pyrimidin-2-yl]ethanone (C-89).

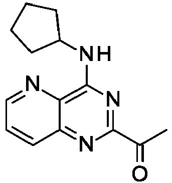

A solution of (0.405g, 1.7mmole) in anhydrous THF (8mL) was treated with copper (I) bromide (37mg, 0.25mmol, 0.15equiv) and then cooled to -78°C. After 10min., the reaction mixture was treated dropwise with methylmagnesium bromide (3M in diethyl ether, 1.3mL, 3.7mol, 2.2equiv) after the addition was complete the reaction was stirred for an additional 10min and then warmed to 0°C. After 2hr., LC/MS analysis showed complete conversion of the starting material to the desired product. The reaction was quenched with satd. aq. ammonium chloride (3mL) and then warmed to room temperature. The biphasic mixture was diluted with ethyl acetate (30mL) and the layers were separated. The aqueous layer was further extracted with ethyl acetate (2 x 30mL). The combined organic layers were dried (Na₂SO₄) and the solvent was removed *in vacuo.* The residual solid was purified by flash chromatography (adsorbed onto 2g silica pre-column, 24g silica, 0-50% ethyl acetate/hexanes) to afford **1-[4-(cyclopentylamino)pyrido[3,2-d]pyrimidin-2-yl]ethanone (C-89)** as an off-white solid (0.138g, 31.3%). MS (APCI) for C₁₄H₁₆N₄O; Calculated: 257.1 [M + H⁺], Found: 257.0.

### 1-[4-(cyclopentylamino)pyrido[3,2-d]pyrimidin-2-yl]propan-1-one (C-90).

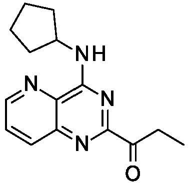

A solution of 4-(cyclopentylamino)pyrido[3,2-d]pyrimidine-2-carbonitrile (0.203g, 0.85mmol) in THF (3mL) was treated with copper (I) bromide (18mg, 0.13mmol, 0.15equiv) and then cooled to -78°C. After 10min., the reaction mixture was treated dropwise with ethylmagnesium bromide (1M in THF, 1.3mL, 3.7mol, 2.2equiv) after the addition was complete the reaction was stirred for an additional 10min and then warmed to 0°C. After 2hr., LC/MS analysis showed complete conversion of the starting material to the desired product. The reaction was quenched with satd. aq. ammonium chloride (3mL) and then warmed to room temperature. The biphasic mixture was diluted with ethyl acetate (20mL) and the layers were separated. The aqueous layer was further extracted with ethyl acetate (2 x 20mL). The combined organic layers were dried (Na₂SO₄) and the solvent was removed *in vacuo.* The residual solid was purified by flash chromatography (adsorbed onto 1g silica pre-column, 24g silica, 0-50% ethyl acetate/hexanes) to afford **1-[4-(cyclopentylamino)pyrido[3,2-d]pyrimidin-2-yl]propan-1-one (C-90)** as an off-white solid (0.145g, 63.2%). MS (APCI) for C₁₅H₁₈N₄O; Calculated: 271.1 [M + H⁺], Found: 271.0.

### [4-(cyclopentylamino)pyrido[3,2-d]pyrimidin-2-yl]-phenyl-methanone (A-02).

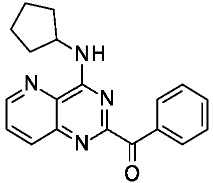

A solution of 4-(cyclopentylamino)pyrido[3,2-d]pyrimidine-2-carbonitrile (0.21g, 0.88mmol) in anhydrous THF (3mL) was treated with copper (I) bromide (19mg, 0.13mmol, 0.15equiv) and then cooled to -78°C. After 10min, the mixture was then treated with phenylmagnesium chloride (2M in THF, 1.1mL, 2.2mmol, 2.5equiv). After stirring for 10min, the reaction mixture was slowly warmed to 0°C. LC/MS analysis after one hour showed clean conversion to the desired product. The reaction mixture was quenched with satd. aq. ammonium chloride (3mL) and poured onto ethyl acetate (10mL). The layers were separated and the aqueous layer was extracted with ethyl acetate (2 x 10mL). The combined organic extracts were dried (Na₂SO₄) and the solvent was removed *in vacuo.* The residual solid was purified by flash chromatography (24g silica, 0-50% ethyl acetate/hexanes) to afford **[4-(cyclopentylamino)pyrido[3,2-d]pyrimidin-2-yl]-phenyl-methanone (A-02)** as a pale yellow solid (0.21 g, 75.2%). MS (APCI) for C₁₉H₁₈N₄O; Calculated: 319.2 [M + H⁺], Found: 319.1.

### [4-(cyclopentylamino)pyrido[3,2-d]pyrimidin-2-yl]-(4-fluorophenyl)methanone (A-03).

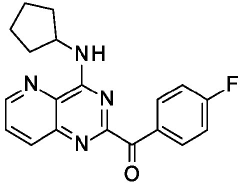

A solution of 4-(cyclopentylamino)pyrido[3,2-d]pyrimidine-2-carbonitrile (0.21g, 0.88mmol) in anhydrous THF (3mL) was treated with copper (I) bromide (19mg, 0.13mmol, 0.15equiv) and then cooled to -78°C. After 10min, the mixture was then treated with 4-fluorophenylmagnesium bromide (2M in diethyl ether, 1.1mL, 2.2mmol, 2.5equiv). After stirring for 10min, the reaction mixture was slowly warmed to 0°C. LC/MS analysis after one hour showed clean conversion to the desired product. The reaction mixture was quenched with satd. aq. ammonium chloride (3mL) and poured onto ethyl acetate (10mL). The layers were separated and the aqueous layer was extracted with ethyl acetate (2 x 10mL). The combined organic extracts were dried (Na₂SO₄) and the solvent was removed *in vacuo.* The residual solid was purified by flash chromatography (24g silica, 0-50% ethyl acetate/hexanes) to afford **[4-(cyclopentylamino)pyrido[3,2-d]pyrimidin-2-yl]-(4-fluorophenyl)methanone (A-03)** as a pale yellow solid (0.287 g, 97.2%). MS (APCI) for C₁₉H₁₇FN₄O; Calculated: 337.1 [M + H⁺], Found: 337.0.

### 1-[4-(cyclopentylamino)pyrido[3,2-d]pyrimidin-2-yl]-2,2-dimethyl-propan-1-one (A-04).

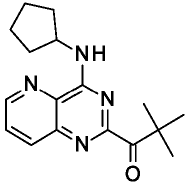

A solution of 4-(cyclopentylamino)pyrido[3,2-d]pyrimidine-2-carbonitrile (0.20g, 0.84mmol) in anhydrous THF (3mL) was treated with copper (I) bromide (18mg, 0.13mmol, 0.15equiv) and then cooled to -78°C. After 10min, the mixture was then treated with tert-butylmagnesium chloride (2M in diethyl ether, 1.1mL, 2.2mmol, 2.6equiv). After stirring for 10min, the reaction mixture was slowly warmed to 0°C. LC/MS analysis after one hour showed conversion to the desired product with some residual starting material. After 2hrs, no further progress was noted and an additional 0.7mL of tert-butylmagnesium chloride solution was added. Two hours after the second addition, the LC/MS analysis showed full consumption of the starting material and formation of the bis tert-butyl addition product. The reaction mixture was quenched with satd. aq. ammonium chloride (3mL) and poured onto ethyl acetate (10mL). The layers were separated and the aqueous layer was extracted with ethyl acetate (2 x 10mL). The combined organic extracts were dried (Na₂SO₄) and the solvent was removed *in vacuo.* The residual solid was purified by flash chromatography (24g silica, 0-50% ethyl acetate/hexanes) to afford **1-[4-(cyclopentylamino)pyrido[3,2-d]pyrimidin-2-yl]-2,2-dimethyl-propan-1-one (A-04)** as a pale yellow film (0.080 g, 32.3%). MS (APCI) for C₁₇H₂₂N₄O; Calculated: 299.2 [M + H⁺], Found: 299.1.

### 1-[4-(tert-butylamino)pyrido[3,2-d]pyrimidin-2-yl]pentan-1-one (C-99).

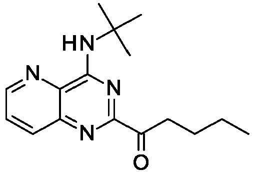

A solution of 4-(tert-butylamino)pyrido[3,2-d]pyrimidine-2-carbonitrile (0.167g, 0.74mmol) in anhydrous THF (3mL) was treated with copper (I) bromide (16mg, 0.11mmol, 0.15equiv) and then cooled to -78°C. After 10min, the mixture was then treated with butylmagnesium chloride (2M in diethyl ether, 1.0mL, 1.8mmol, 2.5equiv). After stirring for 10min, the reaction mixture was slowly warmed to 0°C. LC/MS analysis after one hour showed clean conversion to the desired product. The reaction mixture was quenched with satd. aq. ammonium chloride (3mL) and poured onto ethyl acetate (10mL). The layers were separated and the aqueous layer was extracted with ethyl acetate (2 x 10mL). The combined organic extracts were dried (Na₂SO₄) and the solvent was removed *in vacuo.* The residual solid was purified by flash chromatography (24g silica, 0-50% ethyl acetate/hexanes) to afford **1-[4-(tert-butylamino)pyrido[3,2-d]pyrimidin-2-yl]pentan-1-one (C-99)** as a pale yellow solid (0.167 g, 79.4%). ¹H NMR (400 Mz, CDCl₃) δ 8.70 (1H, dd), 8.25 (1H, dd), 7.65 (1H, dd), 7.30 (1H, bs), 3.20 (2H, t), 1.75, (2H, pent), 1.63 (9H, s), 1.43 (2H, sextet), 0.93 (3H, t); ¹³C NMR (400 Mz, CDCl₃) δ 201.7, 159.5, 156.7, 149.0, 144.1, 137.5, 131.9, 127.8, 52.4, 39.3, 28.5, 26.3, 22.5, 13.9. MS (APCI) for C₁₆H₂₂N₄O; Calculated: 287.2 [M + H⁺], Found: 287.1.

### 1-(4-pyrrolidin-1-ylpyrido[3,2-d]pyrimidin-2-yl)pentan-1-one (A-01).

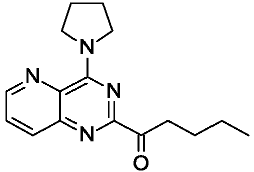

A solution of 4-pyrrolidin-1-ylpyrido[3,2-d]pyrimidine-2-carbonitrile (0.190g, 0.84mmol) in anhydrous THF (3mL) was treated with copper (I) bromide (19mg, 0.13mmol, 0.15equiv) and then cooled to -78°C. After 10min, the mixture was then treated with butylmagnesium chloride (2M in diethyl ether, 1.1mL, 2.1mmol, 2.5equiv). After stirring for 10min, the reaction mixture was slowly warmed to 0°C. LC/MS analysis after one hour showed clean conversion to the desired product. The reaction mixture was quenched with satd. aq. ammonium chloride (3mL) and poured onto ethyl acetate (10mL). The layers were separated and the aqueous layer was extracted with ethyl acetate (2 x 10mL). The combined organic extracts were dried (Na₂SO₄) and the solvent was removed *in vacuo.* The residual solid was purified by flash chromatography (24g silica, 0-50% ethyl acetate/hexanes) to afford **1-[4-(tert-butylamino)pyrido[3,2-d]pyrimidin-2-yl]pentan-1-one (A-01)** as a pale yellow solid (0.073 g, 30.4%). MS (APCI) for C₁₆H₂₀N₄O; Calculated: 285.2 [M + H⁺], Found: 285.0.

### Step 4. Synthesis of Ring Reduced Final Compounds

### 1-[4-(cyclopentylamino)-5,6,7,8-tetrahydropyrido[3,2-d]pyrimidin-2-yl]pentan-1-one (C-82)

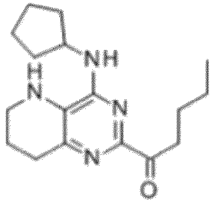

A solution of a mixture of 1-[4-(cyclopentylamino)-5,6,7,8-tetrahydropyrido[3,2-d]pyrimidin-2-yl]pentan-1-ol and 1-[4-(cyclopentylamino)-5,6,7,8-tetrahydropyrido[3,2-d]pyrimidin-2-yl]pentan-1-one (0.203g, 0.67mmol) in methylene chloride (3mL) was treated with Dess-Martin Periodinane (0.34g, 0.80mmol, 1.2 equiv). After stirring for 2hrs., LC/MS analysis showed clean conversion to the desired product. The reaction mixture was dried and the residue was purified by flash chromatography (12g silica, 0-20% acetonitrile/ethyl acetate) to afford 1-[4-(cyclopentylamino)-5,6,7,8-tetrahydropyrido[3,2-d]pyrimidin-2-yl]pentan-1-one, 170mg, as a yellow solid. ¹H NMR (400 Mz, (CD₃)₂CO) δ 4.59 (1H, m), 3.42 (2H, m), 3.12 (2H, t), 2.98 (2H, t), 2.04 (2H, m), 1.95 (2H, m), 1.78 (4H, m), 1.64 (4H, m), 1.37 (2H, m), 0.90 (3H, t); 13C NMR (400 Mz, (CD₃)₂CO) δ 195.7, 151.4, 141.2, 128.5, 116.6, 54.7, 54.6, 40.9, 37.4, 32.9, 26.8, 25.1, 24.7, 23.0, 19.8, 14.1

### 2-chloro-N-cyclopentyl-5,6,7,8-tetrahydropyrido[3,2-d]pyrimidin-4-amine (C-80)

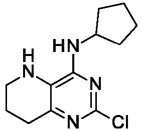

A solution of 2-chloro-N-cyclopentyl-pyrido[3,2-d]pyrimidin-4-amine (0.60g, 2.4mmol) in ethanol (12mL) was treated with TFA (0.18mL, 2.4mmol, 1equiv) and then degassed with nitrogen with 5 cycles. The reaction mixture was then treated with platinum(IV)oxide (0.164g, 0.72mmol, 0.3equiv) and the solution was bubbled with hydrogen gas via balloon for 10min. The needle was removed from the solution and the reaction mixture was stirred overnight under an balloon pressure of hydrogen gas. LC/MS analysis showed complete consumption of the starting material to two products, desired as major and tetrahydropyridine ring with replacement of the chloride for hydrogen as a minor product. The reaction mixture was filtered through Celite and the solvent was removed *in vacuo.* The residue was purified by flash chromatography (12g silica, 0-100% ethyl acetate/hexanes) and then 0-10% methanol/ethyl acetate) to afford 2-chloro-N-cyclopentyl-5,6,7,8-tetahydropyrido[3,2-d]pyrimidin-4-amine (0.346g) as an off-white solid. LCMS: (APCI) m/e 253.1 (M+H).

### N-cyclopentyl-5,6,7,8-tetrahydropyrido[3,2-d]pyrimidin-4-amine (C-84)

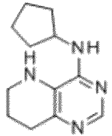

### Isolated by-product from C-80 (80 mg)

1H NMR (400 Mz, (CD₃)₂CO) δ 11.52 (1H, bs), 8.47 (1H, dd), 7.40 (1H, m), 7.19 (1H, m), 7.09 (1H, m), 6.96 (1H, t), 6.73 (1H, d), 6.31 (1H, d), 5.03 (1H, bs), 4.03 (3H, s), 2.07 (2H, m), 1.78 (2H, m), 1.64 (4H, m); 13C NMR (400 Mz, (CD₃)₂CO) δ 152.9, 148.3, 144.3, 125.0, 53.1, 53.0, 47.2, 33.6, 24.3, 22.5.

### 1-[4-(cyclopentylamino)-5,6,7,8-tetrahydropyrido[3,2-d]pyrimidin-2-yl]pentan-1-ol (C-79)

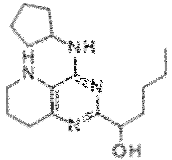

A solution of 1-[4-(cyclopentylamino)pyrido[3,2-d]pyrimidin-2-yl]pentan-1-one (0.20g, 0.67mmol) in ethanol (3mL) was successively treated with nickel (II) chloride (17mg, 0.13mmol, 0.2equiv) and then slowly with sodium borohydride (76mg, 2.0mmol, 3equiv). The reaction mixture slowly released a gas and changed colors to brownish-black. After stirring overnight, LC/MS analysis showed clean conversion to the desired product. The reaction mixture was poured onto satd. aqueous sodium bicarbonate (5mL) and then extracted with ethyl acetate (3 x 25mL). The combined organic extracts were dried (Na₂SO₄) and the solvent was removed *in vacuo.* The residual solid was purified by flash chromatography (12g silica, 0-20% methanol/methylene chloride) to afford 1-[4-(cyclopentylamino)-5,6,7,8-tetrahydropyrido[3,2-d]pyrimidin-2-yl]pentan-1-ol, 0.15g, as a reddish-brown solid. LCMS: (APCI) m/e 305.1 (M+H).

### 1-[4-(cyclopentylamino)-5,6,7,8-tetrahydropyrido[3,2-d]pyrimidin-2-yl]ethanone (C-92)

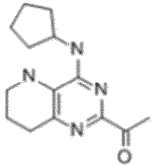

A solution of 1-[4-(cyclopentylamino)pyrido[3,2-d]pyrimidin-2-yl]ethanone (0.138g, 0.54mmol) in ethanol (5mL) was treated with TFA (40uL, 0.54mmo, 1.0equiv) and then degassed by bubbling N₂ through the reaction mixture. After 10min., the reaction mixture was treated with PtO₂ (25mg, 0.11mmol, 0.2equiv) and then the reaction was subjected to bubbling of H₂ gas with a needle exhaust. After 20min., the needle introducing the H₂ gas was raised above the reaction and the mixture was stirred overnight under balloon pressure. LC/MS analysis showed complete consumption of the starting material and 80% conversion to the desired product with additional 20% conversion to the over reduced product where the ketone is also reduced to the alcohol. The reaction mixture was degassed with N₂ gas and then the reaction mixture was filtered through Celite. The solvent was removed *in vacuo* and the residual solid purified by flash chromatography (adsorbed mixture onto 2g silica pre-column, 12g silica, 0-30% methanol/methylene chloride) to afford 1-[4-(cyclopentylamino)-5,6,7,8-tetrahydropyrido[3,2-d]pyrimidin-2-yl]ethanone, 0.123g, as a yellow solid. LCMS: (APCI) m/e 261.1 (M+H).

### 1-[4-(tert-butylamino)-5,6,7,8-tetrahydropyrido[3,2-d]pyrimidin-2-yl]pentan-1-ol (A-00)

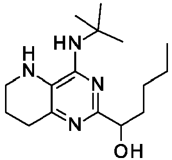

A solution of 1-[4-(tert-butylamino)pyrido[3,2-d]pyrimidin-2-yl]pentan-1-one (0.14g, 0.49mmol) in ethanol (3mL) was treated with TFA (36uL, 0.49mmo, 1.0equiv) and then degassed by bubbling N₂ through the reaction mixture. After 10min., the reaction mixture was treated with PtO₂ (22mg, 0.098mmol, 0.2equiv) and then the reaction was subjected to bubbling of H₂ gas with a needle exhaust. After 20min., the needle introducing the H₂ gas was raised above the reaction and the mixture was stirred overnight balloon pressure. LC/MS analysis showed complete consumption of the starting material and >90% conversion to the over reduced product where the ketone is also reduced to the alcohol. Crude LC/MS does not show a separate peak for the ketone product. The reaction mixture was degassed with N₂ gas and then the reaction mixture was filtered through Celite. The solvent was removed *in vacuo* and the residual solid purified by flash chromatography (12g silica, 0-30% methanol/methylene chloride) to afford 1-[4-(tert-butylamino)-5,6,7,8-tetrahydropyrido[3,2-d]pyrimidin-2-yl]pentan-1-ol, 0.107g, as a viscous yellow oil. In addition, 13mg of the ketone was isolated as a yellow solid (D-06). LCMS: (APCI) m/e 293.1 (M+H).

### 1-[4-(tert-butylamino)-5,6,7,8-tetrahydropyrido[3,2-d]pyrimidin-2-yl]pentan-1-one (A-06)

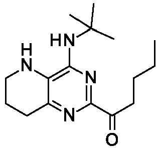

A solution of 1-[4-(tert-butylamino)pyrido[3,2-d]pyrimidin-2-yl]pentan-1-one (0.14g, 0.49mmol) in ethanol (3mL) was treated with TFA (36uL, 0.49mmo, 1.0equiv) and then degassed by bubbling N₂ through the reaction mixture. After 10min., the reaction mixture was treated with PtO₂ (22mg, 0.098mmol, 0.2equiv) and then the reaction was subjected to bubbling of H₂ gas with a needle exhaust. After 20min., the needle introducing the H₂ gas was raised above the reaction and the mixture was stirred overnight under balloon pressure. LC/MS analysis showed complete consumption of the starting material and >90% conversion to the over reduced product where the ketone is also reduced to the alcohol. Crude LC/MS does not show a separate peak for the ketone product. The reaction mixture was degassed with N₂ gas and then the reaction mixture was filtered through Celite. The solvent was removed *in vacuo* and the residual solid purified by flash chromatography (12g silica, 0-30% methanol/methylene chloride) to afford 13mg of the ketone isolated as a yellow solid. In addition, 1-[4-(tert-butylamino)-5,6,7,8-tetrahydropyrido[3,2-d]pyrimidin-2-yl]pentan-1-ol, 0.107g, as a viscous yellow oil. (D-00). LCMS: (APCI) m/e 291.1 (M+H); ¹H NMR (400 Mz, CDCl₃) δ 4.52 (2H, bs), 3.31 (2H, dd), 3.11 (2H, dd), 2.85 (2H, dd), 1.95 (3H, pentet), 1.21 (2H, pentet), 1.51 (9H, s), 1.41 (2H, sextet), 0.93 (3H, t) ; ¹³C NMR (400 Mz, CDCl₃) δ 200.9, 151.7, 131.8, 126.1, 123.8, 51.9, 42.0, 38.6, 29.3, 29.0, 26.8, 22.7, 21.6, 13.9.

### 1-[4-(2-pyridylamino)-5,6,7,8-tetrahydropyrido[3,2-d]pyrimidin-2-yl]pentan-1-ol (G-63)

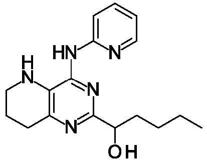

**1-[4-(2-pyridylamino)-5,6,7,8-tetrahydropyrido[3,2-d]pyrimidin-2-yl]pentan-1-ol (G-63)** was prepared following a procedure similar to **A-00** to provide 26 mg (18%). LCMS: (APCI) m/e 314.1 (M+H).

### 1-[4-(2-pyridylamino)-5,6,7,8-tetrahydropyrido[3,2-d]pyrimidin-2-yl]pentan-1-one (G-65)

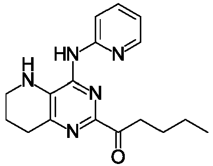

**1-[4-(2-pyridylamino)-5,6,7,8-tetrahydropyrido[3,2-d]pyrimidin-2-yl]pentan-1-one (G-65)** was prepared following a procedure similar to **A-06** to provide 9 mg (6%). LCMS: (APCI) m/e 312.1 (M+H).

### 1-(4-pyrrolidin-1-yl-5,6,7,8-tetrahydropyrido[3,2-d]pyrimidin-2-yl)pentan-1-ol (A-09)

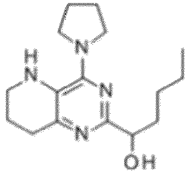

A solution of 1-(4-pyrrolidin-1-ylpyrido[3,2-d]pyrimidin-2-yl)pentan-1-one (73mg, 0.26mmol) in ethanol (1mL) was treated with TFA (19uL, 0.26mmol, 1.0equiv) and then degassed by bubbling N₂ through the reaction mixture. After 10min., the reaction mixture was treated with PtO₂ (6mg, 26umol, 0.1equiv) and then the reaction was subjected to bubbling of H₂ gas with a needle exhaust. After 20min., the needle introducing the H₂ gas was raised above the reaction and the mixture was stirred overnight under balloon pressure. LC/MS analysis showed complete consumption of the starting material and 80% conversion to the desired product with additional 20% conversion to the over reduced product where the ketone is also reduced to the alcohol. The reaction mixture was degassed with N₂ gas and then the reaction mixture was filtered through Celite. The solvent was removed *in vacuo* and the residual solid purified by flash chromatography (adsorbed mixture onto 2g silica pre-column, 12g silica, 0-30% methanol/methylene chloride) to afford 1-(4-pyrrolidin-1-yl-5,6,7,8-tetrahydropyrido[3,2-d]pyrimidin-2-yl)pentan-1-ol, 0.123g, as a yellow solid. LCMS: (APCI) m/e 291.1 (M+H).

### [4-(cyclopentylamino)-5,6,7,8-tetrahydropyrido[3,2-d]pyrimidin-2-yl]-phenyl-methanol (A-10)

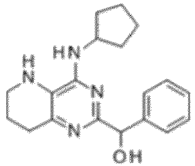

A solution of [4-(cyclopentylamino)pyrido[3,2-d]pyrimidin-2-yl]-phenyl-methanone (0.21g, 0.66mmol) in ethanol (3mL) was treated with TFA (76uL, 0.66mmo, 1.0equiv) and then degassed by bubbling N₂ through the reaction mixture. After 10min., the reaction mixture was treated with PtO₂ (15mg, 0.066mmol, 0.1equiv) and then the reaction was subjected to bubbling of H₂ gas with a needle exhaust. After 20min., the needle introducing the H₂ gas was raised above the reaction and the mixture was stirred overnight under balloon pressure. LC/MS analysis showed complete consumption of the starting material and conversion to the over reduced product. The reaction mixture was degassed with N₂ gas and then the reaction mixture was filtered through Celite. The solvent was removed *in vacuo* and the residual solid purified by flash chromatography (adsorbed mixture onto 2g silica pre-column, 12g silica, 0-30% methanol/methylene chloride) to afford [4-(cyclopentylamino)-5,6,7,8-tetrahydropyrido[3,2-d]pyrimidin-2-yl]-phenyl-methanol, 0.185g, as a pale yellow solid. LCMS: (APCI) m/e 325.1 (M+H).

### [4-(cyclopentylamino)-5,6,7,8-tetrahydropyrido[3,2-d]pyrimidin-2-yl]-(4-fluorophenyl)methanol (A-11)

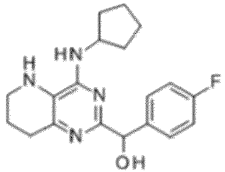

A solution of [4-(cyclopentylamino)pyrido[3,2-d]pyrimidin-2-yl]-(4-fluorophenyl)methanone (0.287g, 0.85mmol) in ethanol (3mL) was treated with TFA (98uL, 0.85mmo, 1.0equiv) and then degassed by bubbling N₂ through the reaction mixture. After 10min., the reaction mixture was treated with PtO₂ (20mg, 0.085mmol, 0.1equiv) and then the reaction was subjected to bubbling of H₂ gas with a needle exhaust. After 20min., the needle introducing the H₂ gas was raised above the reaction and the mixture was stirred overnight under balloon pressure. LC/MS analysis showed complete consumption of the starting material and conversion to the alcohol. The reaction mixture was degassed with N₂ gas and then the reaction mixture was filtered through Celite. The solvent was removed *in vacuo* and the residual solid purified by flash chromatography (adsorbed mixture onto 2g silica pre-column, 12g silica, 0-30% methanol/methylene chloride) to afford [4-(cyclopentylamino)-5,6,7,8-tetrahydropyrido[3,2-d]pyrimidin-2-yl]-(4-fluorophenyl)methanol, 0.245g, as a pale yellow solid. LCMS: (APCI) m/e 343.1 (M+H).

### [4-(cyclopentylamino)-5,6,7,8-tetrahydropyrido[3,2-d]pyrimidin-2-yl]-phenyl-methanone (A-16)

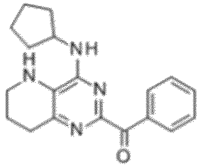

A solution of a mixture of 1-[4-(cyclopentylamino)-5,6,7,8-tetrahydropyrido[3,2-d]pyrimidin-2-yl]phenyl-methanol (0.069g, 24mmol) in methylene chloride (1mL) was treated with Dess-Martin Periodinane (0.12g, 0.28mmol, 1.2 equiv). After stirring for 2hrs., LC/MS analysis showed clean conversion to the desired product. The reaction mixture was dried and the residue was purified by flash chromatography (12g silica, 0-20% acetonitrile/ethyl acetate) to afford 1-[4-(cyclopentylamino)-5,6,7,8-tetrahydropyrido[3,2-d]pyrimidin-2-yl]phenyl-methanone, 170mg, as a yellow solid. ¹H NMR (400 Mz, (CD₃)₂CO) δ 4.59 (1H, m), 3.42 (2H, m), 3.12 (2H, t), 2.98 (2H, t), 2.04 (2H, m), 1.95 (2H, m), 1.78 (4H, m), 1.64 (4H, m), 1.37 (2H, m), 0.90 (3H, t); 13C NMR (400 Mz, (CD₃)₂CO) δ 195.7, 151.4, 141.2, 128.5, 116.6, 54.7, 54.6, 40.9, 37.4, 32.9, 26.8, 25.1, 24.7, 23.0, 19.8, 14.1

### [4-(cyclopentylamino)-5,6,7,8-tetrahydropyrido[3,2-d]pyrimidin-2-yl]-(4-fluorophenyl)methanone (A-17)

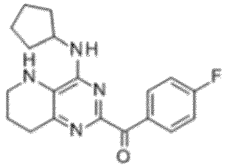

A solution of a mixture of [4-(cyclopentylamino)-5,6,7,8-tetrahydropyrido[3,2-d]pyrimidin-2-yl]-(4-fluorophenyl)methanol (0.069g, 24mmol) in methylene chloride (1mL) was treated with Dess-Martin Periodinane (0.12g, 0.28mmol, 1.2 equiv). After stirring for 2hrs., LC/MS analysis showed clean conversion to the desired product. The reaction mixture was dried and the residue was purified by flash chromatography (12g silica, 0-20% acetonitrile/ethyl acetate) to afford [4-(cyclopentylamino)-5,6,7,8-tetrahydropyrido[3,2-d]pyrimidin-2-yl]-(4-fluorophenyl)methanone, 170mg, as a yellow solid. ¹H NMR (400 Mz, (CD₃)₂CO) δ 4.59 (1H, m), 3.42 (2H, m), 3.12 (2H, t), 2.98 (2H, t), 2.04 (2H, m), 1.95 (2H, m), 1.78 (4H, m), 1.64 (4H, m), 1.37 (2H, m), 0.90 (3H, t); 13C NMR (400 Mz, (CD₃)₂CO) δ 195.7, 151.4, 141.2, 128.5, 116.6, 54.7, 54.6, 40.9, 37.4, 32.9, 26.8, 25.1, 24.7, 23.0, 19.8, 14.1

### 1-(4-pyrrolidin-1-yl-5,6,7,8-tetrahydropyrido[3,2-d]pyrimidin-2-yl)pentan-1-ol (A-18)

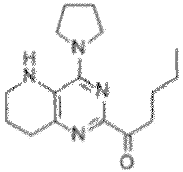

A solution of 1-(4-pyrrolidin-1-yl-5,6,7,8-tetrahydropyrido[3,2-d]pyrimidin-2-yl)pentan-1-ol (43mg, 0.15mmol) in acetone (1mL) was successively treated with Dess-Martin reagent (63mg, 0.15mmol, 1.0equiv). After stirring for 2hrs., LC/MS analysis showed complete and clean conversion to the desired ketone. The solvent was removed in vacuo and the residual solid was purified by flash chromatography (12g silica, 0-10% methanol/methylene chloride) to afford 1-(4-pyrrolidin-1-yl-5,6,7,8-tetrahydropyrido[3,2-d]pyrimidin-2-yl)pentan-1-one, mg, as a yellow gum. LCMS: (APCI) m/e 289.1 (M+H); ¹H NMR (d6-DMSO): δ 5.07 (bs, 2H), 3.56 (m, 3H), 3.28 (m, 2H), 3.00 (m, 2H), 2.72 (m, 2H), 1.85 (m, 6H), 1.32 (m, 4H), 0.86 (t. 3H).

### 1-[4-(cyclopentylamino)-5,6,7,8-tetrahydropyrido[3,2-d]pyrimidin-2-yl]-2,2-dimethyl-propan-1-one (A-35)

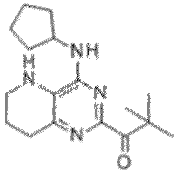

A solution of 1-[4-(cyclopentylamino)-5,6,7,8-tetrahydropyrido[3,2-d]pyrimidin-2-yl]-2,2-dimethyl-propan-1-ol (33mg, 0.11mmol) in acetone (1mL) was treated with Dess-Martin periodinane (51mg, 0.12mmol, 1.1equiv) and the reaction was stirred at RT. After 16 h, the reaction was complete by crude LCMS. The reaction mixture was partitioned between 20mL DCM and 20mL 1M NaOH (aq); and stirred for 10 minutes. The aqueous layer was extracted extract with DCM (3 x 20 mL). The combined organic layer was dried over Na₂SO₄ and concentrated under reduced pressure. The residue was purified on silica gel (40 g, 0-30% EtOAc/hexanes) to provide 30 mg of 1-[4-(cyclopentylamino)-5,6,7,8-tetrahydropyrido[3,2-d]pyrimidin-2-yl]-2,2-dimethyl-propan-1-one (91 %). LCMS: (APCI) m/e 303.1 (M+H).

### N-cyclopentyl-2-pentyl-5,6,7,8-tetrahydropyrido[3,2-d]pyrimidin-4-amine (A-63)

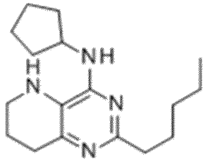

A solution of 1-[4-(tert-butylamino)pyrido[3,2-d]pyrimidin-2-yl]pentan-1-one (0.14g, 0.49mmol) in ethanol (3mL) was treated with TFA (36uL, 0.49mmo, 1.0equiv) and then degassed by bubbling N₂ through the reaction mixture. After 10min., the reaction mixture was treated with PtO₂ (22mg, 0.098mmol, 0.2equiv) and then the reaction was subjected to bubbling of H₂ gas with a needle exhaust. After 20min., the needle introducing the H₂ gas was raised above the reaction and the mixture was stirred overnight under balloon pressure. LC/MS analysis showed complete consumption of the starting material and >90% conversion to the over reduced product where the ketone is also reduced to the alcohol. Crude LC/MS does not show a separate peak for the ketone product. The reaction mixture was degassed with N₂ gas and then the reaction mixture was filtered through Celite. The solvent was removed *in vacuo* and the residual solid purified by flash chromatography (12g silica, 0-30% methanol/methylene chloride) to afford N-cyclopentyl-2-pentyl-5,6,7,8-tetrahydropyrido[3,2-d]pyrimidin-4-amine (A-63), 0.107g, as a viscous yellow oil. LCMS: (APCI) m/e 289.1 (M+H).

### 4-(cyclopentylamino)-5,6,7,8-tetrahydropyrido[3,2-d]pyrimidine-2-carbonitrile (F-38)

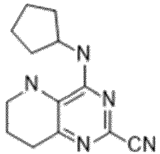

In a 25mL microwave vial, a solution of 2-chloro-N-cyclopentyl-5,6,7,8-tetrahydropyrido[3,2-d]pyrimidin-4-amine (0.75g, 2.97mmole), zinc cyanide (0.7g, 5.93mmol, 2eq), and benzaldehyde (0.332mL, 3.26mmol, 1.2eq) in anhydrous DMF (10mL) was degassed 4x (until no more bubbling) and then treated with *tetrakis*(triphenylphosphine)palladium(0) (0.686g, 0.593mmol, 0.2equiv). The reaction mixture was warmed in a microwave to 150°C for 45min. LC/MS analysis of the crude reaction mixture showed conversion to the desired product and full consumption of the starting material. The mixture was filtered and adsorbed onto silica. The product was purified by flash chromatography to afford 4-(cyclopentylamino)-5,6,7,8-tetrahydropyrido[3,2-d]pyrimidine-2-carbonitrile, 0.62g, as a yellow/beige solid. LCMS: (APCI) m/e 244.1 (M+H).

### Example 2

### Synthesis of Fused Pyrimidine Alkynes

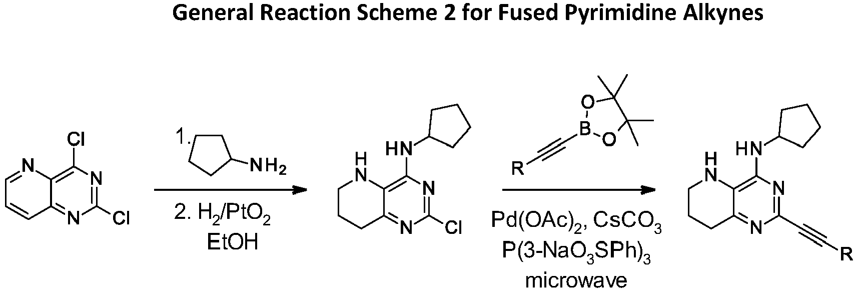

### Synthesis of Final Compounds

### N-cyclopentyl-2-(2-phenylethynyl)-5,6,7,8-tetrahydropyrido[3,2-d]pyrimidin-4-amine (C-91)

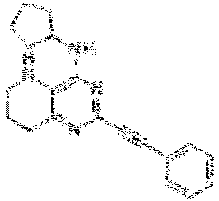

A slurry of 2-chloro-N-cyclopentyl-5,6,7,8-tetrahydropyrido[3,2-d]pyrimidin-4-amine (0.15g, 0.59mmol) in triethylamine (2.0mL) was treated with phenylacetylene (0.1mL, 0.89mmol, 1.5equiv) and then degassed with bubbling nitrogen. After 10min., the reaction mixture was successively treated with palladium (II) acetate (35mg, 0.15mmol, 0.25equiv) and then triphenylphosphine (82mg, 0.31mmol, 0.52equiv). The reaction mixture was then microwaved at 100°C for 1hr. LC/MS analysis showed approx. 10% of the desired product had formed. The reaction mixture was diluted with methylene chloride, filtered through Celite^{®} and the solvent was removed *in vacuo.* The residue was purified by flash chromatography (12g silica, 0-100% ethyl acetate/hexanes) to afford N-cyclopentyl-2-(2-phenylethynyl)-5,6,7,8-tetrahydropyrido[3,2-d]pyrimidin-4-amine, 6.2mg, as a yellowish-red film. LCMS: (APCI) m/e 319.1 (M+H).

### N-cyclopentyl-2-prop-1-ynyl-5,6,7,8-tetrahydropyrido[3,2-d]pyrimidin-4-amine (A-12)

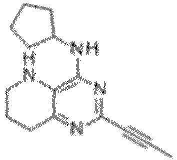

A solution of 2-chloro-N-cyclopentyl-5,6,7,8-tetrahydropyrido[3,2-d]pyrimidin-4-amine (0.1g, 0.48mmol) in acetonitrile (0.75mL) and water (1.5mL) was successively treated with 4,4,5,5-tetramethyl-2-prop-1-ynyl-1,3,2-dioxaborolane (0.085mL, 0.48mmol, 1.2equiv) and cesium carbonate (0.387g, 1.2mmole, 3.0equiv) and then degassed with bubbling nitrogen. After 10min., the reaction mixture was successively treated with palladium(II) acetate (9mg, 40umol, 0.1equiv) and Triphenylphosphine-3,3',3"-trisulfonic acid trisodium salt (90mg, 1.6mmol, 0.4equiv). The reaction mixture was then microwaved at 160°C for 1hr. LC/MS analysis showed 50% product formation. The reaction mixture was diluted with methylene chloride, filtered through Celite^{®} and the solvent was removed *in vacuo.* The residue was purified by flash chromatography (12g silica, 0-10% methanol/methylene chloride) to afford N-cyclopentyl-2-prop-1-ynyl-5,6,7,8-tetrahydropyrido[3,2-d]pyrimidin-4-amine, 14mg, as a yellow film. LCMS: (APCI) m/e 257.1 (M+H).

### N-cyclopentyl-2-pent-1-ynyl-5,6,7,8-tetrahydropyrido[3,2-d]pyrimidin-4-amine (A-27)

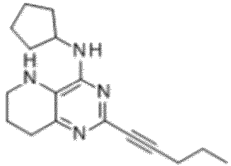

A solution of 2-chloro-N-cyclopentyl-5,6,7,8-tetrahydropyrido[3,2-d]pyrimidin-4-amine (0.12g, 0.48mmol) in 1,4-dioxane (2.0mL) was successively treated with 4,4,5,5-tetramethyl-2-prop-1-ynyl-1,3,2-dioxaborolane (0.8mL, 0.48mmol, 1.0equiv) and sodium carbonate (0.13g, 1.2mmole, 2.5equiv) and then degassed with bubbling nitrogen. After 10min., the reaction mixture was successively treated with *tetrakis*(triphenylphosphine)palladium (0.11g, 95umol, 0.2equiv). The reaction mixture was then microwaved at 160°C for 1hr. LC/MS analysis showed approx. 10% of the desired product had formed. The reaction mixture was diluted with methylene chloride, filtered through Celite^{®} and the solvent was removed *in vacuo.* The residue was purified by flash chromatography (12g silica, 0-100% ethyl acetate/hexanes) to afford N-cyclopentyl-2-(2-phenylethynyl)-5,6,7,8-tetrahydropyrido[3,2-d]pyrimidin-4-amine, 6.2mg, as a yellowish-red film. LCMS: (APCI) m/e 285.1 (M+H).

### Example 3

### Synthesis of Fused Pyrimidine Aromatics

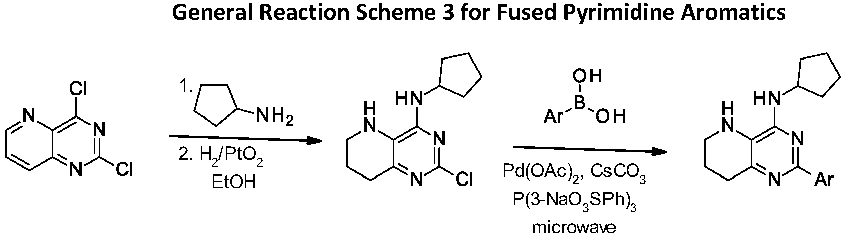

### Syntheses of Final Compounds

### N-cyclopentyl-2-(4-phenyltriazol-1-yl)-5,6,7,8-tetrahydropyrido[3,2-d]pyrimidin-4-amine (A-31)

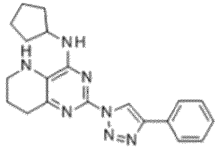

A solution of 2-azido-N-cyclopentyl-5,6,7,8-tetrahydropyrido[3,2-d]pyrimidin-4-amine (75mg, 2.9mmol) in DMSO (1mL) was degassed with bubbling N₂ via balloon for 20min. The reaction mixture was then treated with phenylacetylene (48uL, 4.3mmol, 1.5equiv) and then copper (I) iodide (12mg, 58umol, 0.2equiv) and then the reaction mixture was warmed to 60°C. After 1hr., LC/MS analysis showed clean conversion to the desired product. The reaction mixture was diluted with water 10mL and the mixture was extracted with ethyl acetate (4 x 10mL). The combined organic extracts were dried (Na₂SO₄) and solvent was removed *in vacuo.* The residual solid was purified by flash chromatography (12g silica, 0-10% methylene chloride/methanol) to afford N-cyclopentyl-2-(4-phenyltriazol-1-yl)-5,6,7,8-tetrahydropyrido[3,2-d]pyrimidin-4-amine, 44mg, as a yellow solid. LCMS: (APCI) m/e 362.1 (M+H).

### N-cyclopentyl-2-(p-tolyl)-5,6,7,8-tetrahydropyrido[3,2-d]pyrimidin-4-amine (A-32)

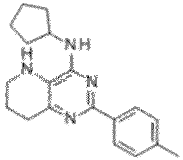

A microwave tube containing 2-chloro-N-cyclopentyl-5,6,7,8-tetrahydropyrido[3,2-d]pyrimidin-4-amine (0.2g, 0.79mmol), cesium carbonate (1.0g, 3.2mmol, 4equiv), p-tolylboronic acid (0.27g, 2.0mmol, 2.5equiv), palladium (II) acetate (18mg, 79umol, 0.1equiv) and triphenylphosphine-3,3',3"-trisulfonic acid trisodium salt (0.18g,3.2mmol, 0.4equiv) was purged with N₂ gas for 2min and then sealed. The mixture was then diluted with water (1.5mL) and acetonitrile (0.75mL). The reaction mixture was then microwaved at 175°C for 2hr. LC/MS analysis showed approx. 50% of the desired product had formed. The reaction mixture was diluted with methylene chloride (5mL) and the layers were separated. The aqueous phase was extracted with methylene chloride (2 x 10mL) and the combined organic extracts were dried (Na₂SO₄) and the solvent was removed *in vacuo.* The residue was purified by flash chromatography (12g silica, 0-10% methanol/methylene chloride) to afford N-cyclopentyl-2-(p-tolyl)-5,6,7,8-tetrahydropyrido[3,2-d]pyrimidin-4-amine, 19.6mg, as a yellowish solid. LCMS: (APCI) m/e 309.1 (M+H); ¹H NMR (d6-DMSO): δ 8.15 (d, 2H), 7.08 (d, 2H), 5.56 (bs, 1H), 4.45 (bs, 1H), 3.17 (m, 2H), 2.67 (m, 1H), 2.23 (s, 3H), 1.94 (m, 6H), 1.92 (m, 6H).

### N-cyclopentyl-2-(4-pyridyl)-5,6,7,8-tetrahydropyrido[3,2-d]pyrimidin-4-amine (A-34)

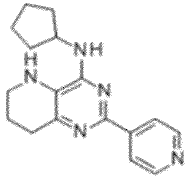

A microwave tube containing 2-chloro-N-cyclopentyl-5,6,7,8-tetrahydropyrido[3,2-d]pyrimidin-4-amine (0.2g, 0.79mmol), cesium carbonate (1.0g, 3.2mmol, 4equiv), 4-pyridylboronic acid (0.24g, 2.0mmol, 2.5equiv), palladium (II) acetate (18mg, 79umol, 0.1equiv) and triphenylphosphine-3,3',3"-trisulfonic acid trisodium salt (0.18g,3.2mmol, 0.4equiv) was purged with N₂ gas for 2min and then sealed. The mixture was then diluted with water (1.5mL) and acetonitrile (0.75mL). The reaction mixture was then microwaved at 150°C for 2hr. LC/MS analysis showed approx. 50% of the desired product had formed. The reaction mixture was diluted with methylene chloride (5mL) and the layers were separated. The aqueous phase was extracted with methylene chloride (2 x 10mL) and the combined organic extracts were dried (Na₂SO₄) and the solvent was removed *in vacuo.* The residue was purified by flash chromatography (12g silica, 0-10% methanol/methylene chloride) to afford N-cyclopentyl-2-(4-pyridyl)-5,6,7,8-tetrahydropyrido[3,2-d]pyrimidin-4-amine, 47.5mg, as a yellowish solid. LCMS: (APCI) m/e 296.1 (M+H); ¹H NMR (CDCl₃): δ 8.71 (d, 1H), 8.69 (bs, 1H), 8.17 (bs, 1H), 7.73 (d, 1H), 5.86 (bs, 1H), 4.56 (bs, 1H), 3.32 (m, 1H), 2.76 (m, 3H), 2.73 (m, 3H), 2.03 (m, 3H), 1.94 (m, 2H), 1.26 (m, 2H).

### Example 4

### Synthesis of Pyrimidine Aromatics

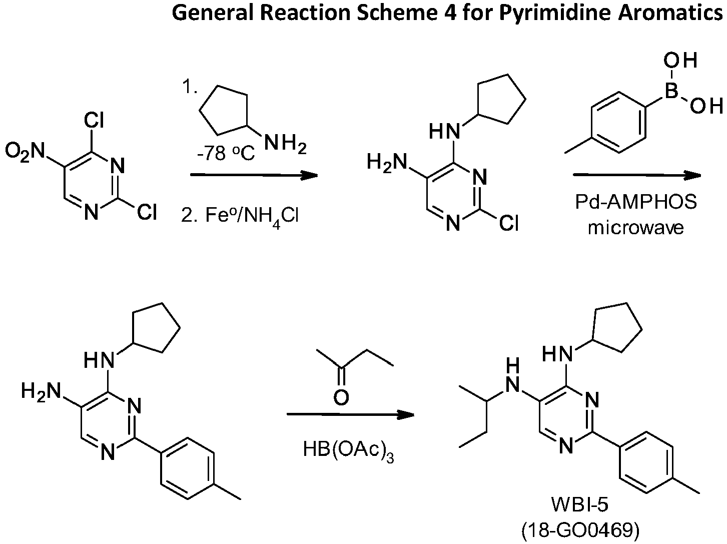

### Step 1. Synthesis of Cl-displacement intermediates

### N-benzyl-2-chloro-N-cyclopentyl-5-nitro-pyrimidin-4-amine (K-39).

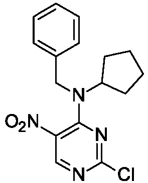

A 250 mL RBF was charged with 2,4-dichloro-5-nitro-pyrimidine (500 mg, 2.58 mmol), THF (25 mL, 0.1 M) and cooled to -78 °C in a dry ice bath. The cooled reaction mixture was then treated carefully with DiPEA (3 eq., 7.74 mmol, 1.4 mL). The reaction mixture was then treated with N-benzylcyclopentanamine;hydrochloride (1 eq., 2.58 mmol, 546 mg) as a solid. The reaction was purged with nitrogen and allowed to gradually warm to RT. After 16 h, the reaction was partitioned between water (50 mL) and EtOAc (50 mL), the water layer was back extracted 3 x 50 mL EtOAc and the combined organic layer was dried over Na₂SO₄ and concentrated under reduced pressure to provide a red oil (850 mg, 99%) and used directly in the next step. (APCI) m/e 333.0 (M+H).

### Step 2. Synthesis of Final Analogs

### N⁴-cyclopentyl-2-(p-tolyl)-N⁵-sec-butyl-pyrimidine-4,5-diamine (F-69)

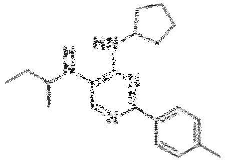

A 40mL vial fitted with a stirbar was charged with N⁴-cyclopentyl-2-(p-tolyl)pyrimidine-4,5-diamine (F-68, 0.065g, 0.242mmol), MEK (1.3 eq., 0.028mL, 0.315mmol), TFA (2 eq., 0.036mL, 2.47 mmol), and isopropyl acetate (3.25mL). The reaction was stirred at RT for 15min, and treated carefully with sodium triacetoxyborohydride (0.0565g, 0.266mmol), purged with N₂ and allowed to stir at RT for 3 days. The reaction mixture was partitioned between sat. NaHCO₄ (10 mL) and EtOAc (10 mL). The aqueous layer was back extracted 3 x 10 mL EtOAc and the combined organic later was dried over Na₂SO₄, concentrated under reduced pressure and the residue was purified on silica gel (24 g, Hexane/Ethyl Acetate). LCMS: (APCI) m/e 325.1 (M+H); ¹H NMR (CDCl₃): δ 8.17 (d, 2H), 7.63 (s, 1H), 7.17 (t, 2H), 4.43 (bs, 1H), 3.13 (bs, 1H), 2.32 (s, 3H), 2.10 (m, 2H), 1.62 (m, 10H), 0.91 (m, 3H), 0.88 (t, 3H).

### N⁴-cyclopentyl-2-(3-pyridyl)-N⁵-sec-butyl-pyrimidine-4,5-diamine (F-78)

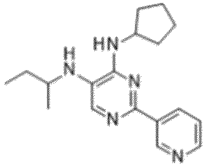

A 40mL vial fitted with a stirbar was charged with N⁴-cyclopentyl-2-(3-pyridyl)pyrimidine-4,5-diamine (F-76, 0.150g, 0.588mmol), MEK (3 eq., 0.158mL, 1.76mmol), TFA (2 eq., 0.0873mL, 1.18mmol), and isopropyl acetate (7.5mL).The reaction was stirred at RT for 15min and treated carefully with sodium triacetoxyborohydride (1.1eq, 0.138g, 0.646mmol), purged with N₂ and allowed to stir at RT for 3 days. The reaction mixture was partitioned between sat. NaHCO₄ (10 mL) and EtOAc (10 mL). The aqueous layer was back extracted 3 x 10 mL EtOAc and the combined organic later was dried over Na₂SO₄, concentrated under reduced pressure and the residue was purified on silica gel (24 g, Hexane/Ethyl Acetate). LCMS: (APCI) m/e 312.1 (M+H); ¹H NMR (CDCl₃): δ 9.35 (bs, 1H), 8.46 (m, 2H), 7.39 (bs, 1H), 6.63 (d, 2H), 4.96 (bs, 1H), 4.53 (m, 1H), 3.42 (m, 1H), 2.09 (m, 2H), 1.60 (m, 8H), 1.17 (m, 3H), 0.93 (t, 3H).

### N⁴-cyclopentyl-2-pyrimidin-5-yl-N⁵-sec-butyl-pyrimidine-4,5-diamine (F-81)

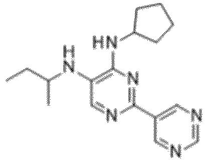

A 40mL vial fitted with a stirbar was charged with N⁴-cyclopentyl-2-pyrimidin-5-yl-pyrimidine-4,5-diamine (F-79, 0.300g, 1.17mmol), MEK (3 eq., 0.315mL, 3.51mmol), TFA (2 eq., 0.174mL, 2.34mmol), and isopropyl acetate (15mL). The reaction was stirred at RT for 15min and treated carefully with sodium triacetoxyborohydride (1.1eq, 0.273g, 1.29mmol), purged with N₂ and allowed to stir at RT for 3 days. The reaction mixture was partitioned between sat. NaHCO₄ (10 mL) and EtOAc (10 mL). The aqueous layer was back extracted 3 x 10 mL EtOAc and the combined organic later was dried over Na₂SO₄, concentrated under reduced pressure and the residue was purified on silica gel (24 g, Hexane/Ethyl Acetate). LCMS: (APCI) m/e 313.1 (M+H); ¹H NMR (CDCl₃): δ 9.41 (bs, 2H), 9.12 (bs, 1H), 7.64 (s, 1H), 6.84 (d, 1H), 5.08 (m, 1H), 4.46 (m, 1H), 2.02 (m, 2H), 1.65 (m, 9H), 1.16 (m, 3H), 0.91 (t, 3H).

### N⁴-cyclopentyl-N⁵-(oxetan-3-yl)-2-pyrimidin-5-yl-pyrimidine-4,5-diamine (F-82)

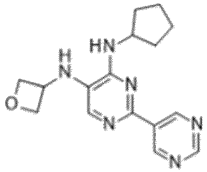

A 40mL vial fitted with a stirbar was charged with N⁴-cyclopentyl-2-pyrimidin-5-yl-pyrimidine-4,5-diamine (F-79, 0.300g, 1.17mmol), oxetanone (3 eq., 0.206mL, 3.51mmol), TFA (2 eq., 0.174mL, 2.34mmol), and isopropyl acetate (15mL). The reaction was stirred at RT for 15min and treated carefully with sodium triacetoxyborohydride (1.1eq, 0.273g, 1.29mmol), purged with N₂ and allowed to stir at RT for 3 days. The reaction mixture was partitioned between sat. NaHCO₄ (10 mL) and EtOAc (10 mL). The aqueous layer was back extracted 3 x 10 mL EtOAc and the combined organic later was dried over Na₂SO₄, concentrated under reduced pressure and the residue was purified on silica gel (24 g, DCM/Methanol). LCMS: (APCI) m/e 313.1 (M+H).

### N⁴-cyclopentyl-2-(4-pyridyl)-N⁵-sec-butyl-pyrimidine-4,5-diamine (F-88)

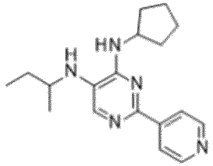

A 40mL vial fitted with a stirbar was charged with N⁴-cyclopentyl-2-(4-pyridyl)pyrimidine-4,5-diamine (F-84, 0.123g, 0.482mmol), MEK (3 eq., 0.13mL, 1.45mmol), TFA (2 eq., 0.072mL, 0.964mmol), and isopropyl acetate (6.5mL). The reaction was stirred at RT for 15min and treated carefully with sodium triacetoxyborohydride (1.1eq, 0.112g, 0.53mmol), purged with N₂ and allowed to stir at RT for 24 hours. The reaction mixture was partitioned between sat. NaHCO₄ (10 mL) and EtOAc (10 mL). The aqueous layer was back extracted 3 x 10 mL EtOAc and the combined organic later was dried over Na₂SO₄, concentrated under reduced pressure and the residue was purified on silica gel (24 g, Hexane/Ethyl Acetate). LCMS: (APCI) m/e 312.1 (M+H); ¹H NMR (CDCl₃): δ 8.55 (t, 2H), 8.06 (t, 2H), 7.54 (s, 1H), 6.63 (d, 1H), 5.11 (d, 1H), 4.43 (m, 1H), 3.42 (m, 2H), 2.08 (m, 1H), 1.60 (m, 8H), 1.13 (m, 3H), 0.91 (t, 3H).

### N⁴-cyclopentyl-2-methyl-6-(2-methylprop-1-enyl)pyrimidine-4,5-diamine (F-99)

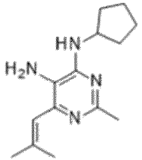

A 20mL microwave vial fitted with a stirbar was charged with the 6-chloro-N⁴-cyclopentyl-2-methyl-pyrimidine-4,5-diamine (F-98, 1g, 4.41mmol), n-butanol (12mL), water (1.2mL), 2,2-dimethylethenylboronic acid (2.5 eq., 1.1g, 11 mmol) and potassium acetate (3.5 eq., 1.52g, 15.4 mmol). The vial was then evacuated and backfilled with nitrogen (2x) and treated with tetrakis(triphenylphosphine)palladium(0) (0.01 eq; 35mg, 0.0441mmol), the vial sealed and then heated in the microwave at 110°C for 15 minutes. LC indicates primarily the desired product with trace starting material. The reaction mixture was filtered through a PTFE 0.45um syringe filter into a 250 ml RBF and concentrated under reduced pressure. The residue was dissolved in 3 mL DCM and absorbed on silica gel, concentrated under reduced pressure and the solid material was heated at 100°C overnight. The solid was purified directly on silica gel (50 g, Hexane/Ethyl Acetate) to provide the desired product (F-99). LCMS: (APCI) m/e 247.1 (M+H).

### Example 5 - not encompassed by the wording of the claims but are considered as useful for understanding the invention

### Synthesis of Pyridine Aromatics

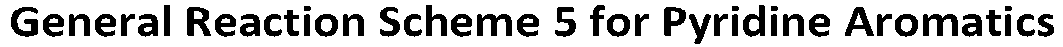

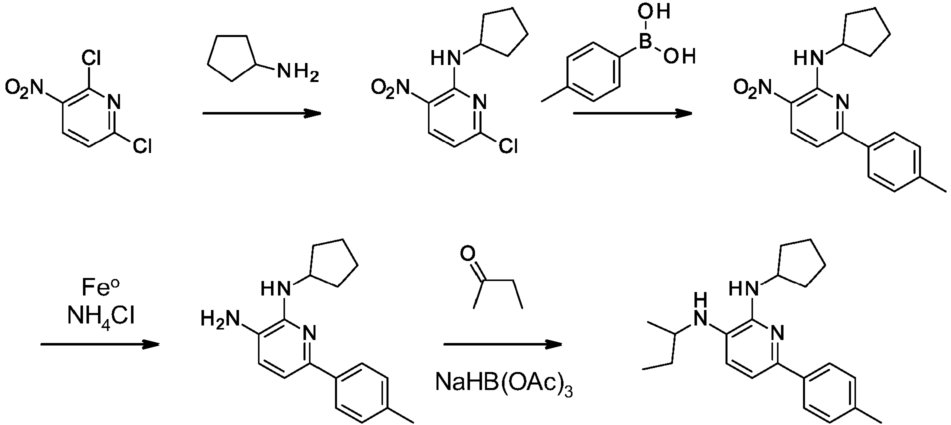

### Step 1. Synthesis of Cl-displacement intermediates

### 6-chloro-N-cyclopentyl-3-nitro-pyridin-2-amine (H-40)

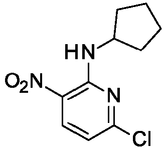

In a 40-mL vial equipped with stir bar, 2,6-dichloro-3-nitro-pyridine (0.500 g, 2.59 mmol) was dissolved in THF (5 mL). To this was added DIEA (0.554 mL, 3.24 mmol, 1.25 equiv) followed by cyclopentylamine (0.256 mL, 2.59 mmol, 1 equiv). The reaction was allowed to stir at room temperature for 2 hours, at which time LCMS analysis suggested formation of desired product. The reaction mixture was poured into water (^{~}20 mL) and extracted with ethyl acetate (3 x ^{~}25 mL). The organic extracts were combined, dried over anhydrous magnesium sulfate, filtered, and rotavapped down. The resulting orange oil was purified via flash chromatography (hexanes/EtOAc). Desired product fractions were combined, rotavapped down, and dried overnight at 40 °C under vacuum to yield 6-chloro-N-cyclopentyl-3-nitropyridin-2-amine as an orange oil (375 mg, 60.0%). ¹H-NMR (400 MHz, CDCl₃): δ 8.31 (d, 1H), 6.56 (d, 1H), 4.53 (m, 1H), 2.13 (m, 2H), 1.76 (m, 2H), 1.67 (m, 2H), 1.54 (m, 2H). LCMS: (APCI) m/e 242 (M+H).

### N-tert-butyl-6-chloro-3-nitro-pyridin-2-amine (K-57).

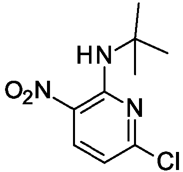

A 250 mL RBF was charged with 2,6-dichloro-3-nitro-pyridine (1.0 g, 5.18 mmol), a stir bar, THF (10 mL, 0.5M), DiEA (2 eq., 1.8 mL, 10.4 mmol), 2-methylpropan-2-amine (1 eq., 5.18 mmol, 380 mg) in 4 mL of THF (1 eq., 5.18 mmol, 380 mg) and the reaction was stirred at RT overnight. The reaction was then partitioned between 75 mL of water and 75 mL EtOAc. The water layer was extracted 3 x 50 mL EtOAc and the combined organic layer was dried over Na₂SO₄ and concentrated under reduced pressure to provide 1.15 of an oil that was >70% pure by LCMS and was purified on silica gel (40 g, 0-50% EtOAc/hexanes) to provide 550 mg as a yellow oil (46%). LCMS: (APCI) m/e 230.1 (M+H).

### 6-chloro-N-(3-methyloxetan-3-yl)-3-nitro-pyridin-2-amine (K-58).

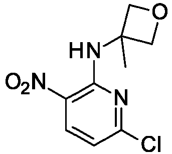

A 250 mL RBF was charged with 2,6-dichloro-3-nitro-pyridine (1.0 g, 5.18 mmol), a stir bar, THF (8 mL, 0.5M), DiEA (2 eq., 1.8 mL, 10.4 mmol), 3-methyloxetan-3-amine in 2 mL of THF (1 eq., 5.18 mmol, 451 mg) and the reaction was stirred at RT overnight. The reaction was then partitioned between 75 mL of water and 75 mL EtOAc. The water layer was extracted 3 x 50 mL EtOAc and the combined organic layer was dried over Na₂SO₄ and concentrated under reduced pressure to provide 1.5 of an oil that was >70% pure by LCMS and was purified on silica gel (80 g, 0-50% EtOAc/hexanes) to provide 740 mg as a yellow solid (58%). LCMS: (APCI) m/e 244.0 (M+H).

### N-benzyl-6-chloro-N-cyclopentyl-3-nitro-pyridin-2-amine (K-64).

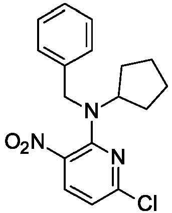

A 40 mL vial was charged with 2-chloro-6-methyl-3-nitro-pyridine (1.0 g, 5.79 mmol), a stir bar, DMF (5 mL, 1 M), DiEA (3 eq., 3.1 mL, 17.4 mmol), N-benzylcyclopentanamine;hydrochloride (1.1 eq., 6.37 mmol, 1.35 g), 80 °C overnight. After 16 h, the starting material had been consumed and the desired product was confirmed in the crude LCMS. The reaction mixture was partitioned between 75 mL of water and 75 mL EtOAc. The water layer was back extracted 3 x 50 mL EtOAc and the combined organic layer was dried over Na₂SO₄. The residue was purified on silica gel (80 g, 0-30% EtOAc/hexanes) to provide 1.2 g (85%) as a yellow solid. LCMS: (APCI) m/e 312.1 (M+H).

### 6-chloro-N-(3,3-difluorocyclobutyl)-3-nitro-pyridin-2-amine (K-60).

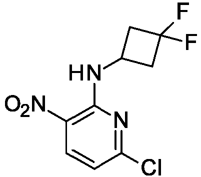

A 40 mL vial was charged with 2-chloro-6-methyl-3-nitro-pyridine (1.0 g, 5.79 mmol), a stir bar, DMF (5 mL, 1 M), DiEA (3 eq., 3.1 mL, 17.4 mmol), 3,3-difluorocyclobutanamine;hydrochloride (1 eq., 5.79 mmol, 937 mg) and the reaction was stirred at 80 °C overnight. The reaction was then heated for 24 h at 75 °C and the THF evaporated under reduced pressure. The residue was directly purified on silica gel (80 g, 0-30% EtOAc/hexanes) to provide 1.2 g (85%) as a yellow solid. LCMS: (APCI) m/e 244.1 (M+H).

### 6-chloro-N-(3,3-difluoro-1-methyl-cyclobutyl)-3-nitro-pyridin-2-amine (K-89).

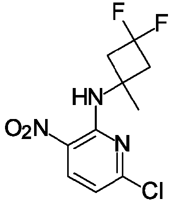

A 250 mL RBF was charged with 2,6-dichloro-3-nitro-pyridine (1.0 g, 5.18 mmol), a stir bar, DMF (8 mL, 0.5M), DiEA (3 eq., 2.7 mL, 15.5 mmol), 3,3-difluoro-1-methylcyclobutanamine;hydrochloride (1 eq., 5.18 mmol, 817 mg) and the reaction was stirred at RT for 3 d. The reaction was then partitioned between 75 mL of water and 75 mL EtOAc. The water layer was extracted 3 x 50 mL EtOAc and the combined organic layer was dried over Na₂SO₄ and concentrated under reduced pressure to provide an oil that was >70% pure by LCMS and was purified on silica gel (80 g, 0-40% EtOAc/hexanes) to provide 1.07 g of 6-chloro-N-(3,3-difluoro-1-methylcyclobutyl)-3-nitro-pyridin-2-amine as a yellow solid (74%). LCMS: (APCI) m/e 278.1 (M+H).

### 6-chloro-N-(3-methyltetrahydrofuran-3-yl)-3-nitro-pyridin-2-amine (K-86).

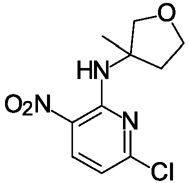

A 250 mL RBF was charged with 2,6-dichloro-3-nitro-pyridine (1.0 g, 5.18 mmol), a stir bar, THF (8 mL, 0.5M), DiEA (2 eq., 1.8 mL, 10.4 mmol), 3-methyltetrahydrofuran-3-amine in 2 mL of THF (1 eq., 5.18 mmol, 524 mg) and the reaction was stirred at RT for 3 d. The reaction was then partitioned between 75 mL of water and 75 mL EtOAc. The water layer was extracted 3 x 50 mL EtOAc and the combined organic layer was dried over Na₂SO₄ and concentrated under reduced pressure to provide an oil that was >70% pure by LCMS and was purified on silica gel (80 g, 0-40% EtOAc/hexanes) to provide 770 mg as a yellow solid (48%). LCMS: (APCI) m/e 358.0 (M+H).

### Step 2. Synthesis of Suzuki coupling intermediates

### N-cyclopentyl-3-nitro-6-(p-tolyl)pyridin-2-amine (H-54) (Represents general procedure followed for all boronic acid couplings in this series)

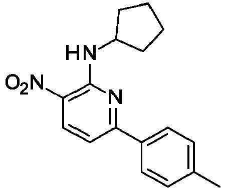

In a 2.0-5.0 mL microwave vial, 6-chloro-N-cyclopentyl-3-nitro-pyridin-2-amine (0.750 g, 3.10 mmol) was dissolved in DMF (5 mL). To this were added cesium carbonate (2.53 g, 7.76 mmol, 2.5 equiv) and p-tolylboronic acid (0.844 g, 6.20 mmol, 2 equiv). The mixture was then purged with nitrogen. tetrakis(triphenylphosphine)palladium(0) (0.538 g, 0.466 mmol, 0.15 equiv) was then added. The vial was sealed and heated in the microwave reactor for 20 min at 120 °C. The reaction mix was then filtered, loaded onto silica and purified by flash chromatography (hexanes/EtOAc). Desired product fractions 13-21 combined, rotavapped down and dried at 40 °C overnight to yield N-cyclopentyl-3-nitro-6-(p-tolyl)pyridin-2-amine as a yellow-orange solid (354 mg, 38.3%). LCMS: (APCI) m/e 298 (M+H).

### N-(3,3-difluoro-1-methyl-cyclobutyl)-3-nitro-6-(3-pyridyl)pyridin-2-amine (M-03).

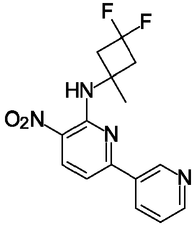

In a 40-mL vial, 6-chloro-N-(3,3-difluoro-1-methyl-cyclobutyl)-3-nitro-pyridin-2-amine (0.400 g, 1.44 mmol), 3-pyridylboronic acid (0.354 g, 2.88 mmol, 2 equiv) and potassium carbonate (0.597 g, 4.32 mmol, 3 equiv) were stirred in THF (4 mL) and water (2 mL). *tetrakis*(triphenylphosphine)palladium(0) (0.166 g, 0.144 mmol, 0.1 equiv) was added, and the vial capped and stirred at 60 °C.

After overnight reaction, LCMS analysis of crude reaction mixture suggests predominant formation of desired product. Reaction mixture was poured onto water (^{~}25 mL), and extracted with EtOAc (4 x ^{~}30 mL). Organic extracts were combined, dried over anhydrous Mg sulfate, and rotavapped down to a deep red oil. This was subsequently dried under vacuum for ^{~}1 hr at 40 °C. Resulting mass is greater than expected yield, which is presumably due to the presence of tetrakis byproduct(s) (also suggested by LCMS). This material was used in the next step (H-71) without further purification, assuming quantitative yield. LCMS: (APCI) m/e 321.0 (M+H).

### 6-(4-fluorophenyl)-N-(3-methyltetrahydrofuran-3-yl)-3-nitro-pyridin-2-amine (K-99).

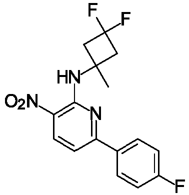

A 40 mL vial was charged with 6-chloro-N-(3-methyltetrahydrofuran-3-yl)-3-nitro-pyridin-2-amine(518 mg, 2.01 mmol), THF (4 mL), water (2 mL), (4-fluorophenyl)boronic acid (2 eq., 563 mg, 4.02 mmol), sodium carbonate (4 eq., 852 mg, 8.04 mmol) and then fitted with a stir bar, and septa. The solution was degassed using a stream of nitrogen directly in the solution and an exit needle for 10 min. The reaction mixture was then treated with tetrakis(triphenylphosphine)palladium(0) (0.1 eq., 232 mg, 0.201 mmol) and fitted with a nitrogen balloon and stirred at 60 °C. After 2 h, crude LCMS confirmed complete consumption of the starting material and the major product exhibited the correct MS for the desired product. The reaction mixture was allowed to cool to RT and then partitioned between 20 mL of EtOAC and 20 mL water. The aqueous layer was back extracted 2 x 20 mL EtOAc and the combined organic layer dried over Na₂SO₄. The solvent was removed under reduced pressure and the resulting residue was purified on silica gel (80 g, 0-30% EtOAc/hexanes) to provide 6-(4-fluorophenyl)-N-(3-methyltetrahydrofuran-3-yl)-3-nitro-pyridin-2-amine as a yellow solid confirmed (500 mg, 78%). LCMS: (APCI) m/e 318.1 (M+H).

### N,N-dimethyl-4-[6-[(3-methyltetrahydrofuran-3-yl)amino]-5-nitro-2-pyridyl]benzamide (N-02).

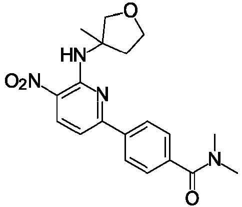

A 40 mL vial was charged with 6-chloro-N-(3-methyltetrahydrofuran-3-yl)-3-nitro-pyridin-2-amine (550 mg, 2.13 mmol), THF (4 mL), water (2 mL), [4-(dimethylcarbamoyl)phenyl]boronic acid (2 eq., 824 mg, 4.27 mmol), sodium carbonate (4 eq., 905 mg, 8.54 mmol) and then fitted with a stir bar, and septa. The solution was degassed using a stream of nitrogen directly in the solution and an exit needle for 10 min. The reaction mixture was then treated with tetrakis(triphenylphosphine)-palladium(0) (0.1 eq., 247 mg, 0.213 mmol) and fitted with a nitrogen balloon and stirred at 60 °C. After 4 h, crude LCMS confirmed complete consumption of the starting material and the major product exhibited the correct MS for the desired product. The reaction mixture was allowed to cool to RT and then partitioned between 20 mL of EtOAC and 20 mL water. The aqueous layer was back extracted 2 x 20 mL EtOAc and the combined organic layer dried over Na₂SO₄. The solvent was removed under reduced pressure and the resulting residue was purified on silica gel (80 g, 0-30% EtOAc/hexanes) to provide N,N-dimethyl-4-[6-[(3-methyltetrahydrofuran-3-yl)amino]-5-nitro-2-pyridyl]benzamide as a yellow solid (700 mg, 85%). LCMS: (APCI) m/e 371.1 (M+H).

### 4-[6-[(3,3-difluoro-1-methyl-cyclobutyl)amino]-5-nitro-2-pyridyl]-N,N-dimethyl-benzamide (N-06)

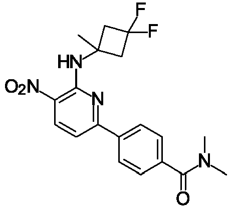

A 40 mL vial was charged with 6-chloro-N-(3,3-difluoro-1-methyl-cyclobutyl)-3-nitro-pyridin-2-amine (550 mg, 2.33 mmol), THF (4 mL), water (2 mL), [4-(dimethylcarbamoyl)phenyl]boronic acid (2 eq., 898 mg, 4.65 mmol), sodium carbonate (4 eq., 986 mg, 9.31 mmol) and then fitted with a stir bar, and septa. The solution was degassed using a stream of nitrogen directly in the solution and an exit needle for 10 min. The reaction mixture was then treated with tetrakis(triphenylphosphine)-palladium(0) (0.1 eq., 269 mg, 0.233 mmol) and fitted with a nitrogen balloon and stirred at 60 °C. After 16 h, crude LCMS confirmed complete consumption of the starting material and the major product exhibited the correct MS for the desired product. The reaction mixture was allowed to cool to RT and then partitioned between 50 mL of EtOAC and 50 mL water. The aqueous layer was back extracted 2 x 50 mL EtOAc and the combined organic layer dried over Na₂SO₄. The solvent was removed under reduced pressure and the resulting residue was purified on silica gel (80 g, 0-40% EtOAc/hexanes) to provide 4-[6-[(3,3-difluoro-1-methyl-cyclobutyl)amino]-5-nitro-2-pyridyl]-N,N-dimethyl-benzamide as a yellow solid (830 mg, 91%). LCMS: (APCI) m/e 391.1 (M+H).

### Step 3. Synthesis of Nitro reduction intermediates

### N²-cyclopentyl-6-(p-tolyl)pyridine-2,3-diamine (H-59) (Represents general procedure for all nitro reduction reactions in this series)

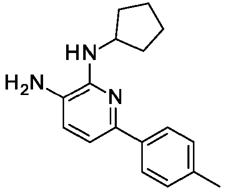

In a 40-mL vial equipped with stir bar, N-cyclopentyl-3-nitro-6-(p-tolyl)pyridin-2-amine (0.354 g, 1.19 mmol), ammonium chloride (0.0636 g, 1.19 mmol) and iron filings (0.332 g, 5.95 mmol) were stirred in 5 mL ethanol:water 4:1. The vial was sealed and the mixture stirred at 80 °C in a reaction block. After 2 hours, LCMS showed clean conversion to desired product. The reaction was cooled to room temperature and the iron filtered off. The filtrate was poured into water and extracted with ethyl acetate (x3). Combined organic extracts were dried over magnesium sulfate, filtered, and rotavapped down and dried under vacuum at 40 °C overnight to yield N²-cyclopentyl-6-(p-tolyl)pyridine-2,3-diamine as a dark brown solid (0.3032 g, 95.3%). LCMS: (APCI) m/e 268 (M+H).

### N²-(3,3-difluoro-1-methyl-cyclobutyl)-6-(3-pyridyl)pyridine-2,3-diamine (M-05).

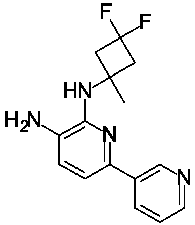

In a 40-mL vial equipped with stir bar, N-(3,3-difluoro-1-methyl-cyclobutyl)-3-nitro-6-(3-pyridyl)pyridin-2-amine (M-03, 0.299 g, 0.933 mmol), ammonium chloride (0.0499 g, 0.933 mmol) and iron filings (0.260 g, 4.66 mmol) were stirred in 5 mL ethanol:water 4:1. The vial was sealed and the mixture stirred at 80 °C in a reaction block for 8 hours. LC-MS suggests reaction has gone to completion. Reaction was cooled to room temperature, diluted with methanol and filtered through a plug of Celite^{®}. Filtrate was rotavapped down and dried under vacuum at 40 °C overnight to provide a quantitative yield. The material was used directly in the next step without further purification. LCMS: (APCI) m/e 291.1 (M+H).

### 4-[5-amino-6-[(3-methyltetrahydrofuran-3-yl)amino]-2-pyridyl]-N,N-dimethyl-benzamide (N-03)

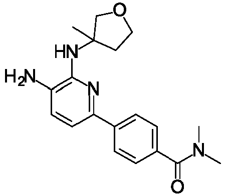

A 20 mL microwave vial was charged with N,N-dimethyl-4-[6-[(3-methyltetrahydrofuran-3-yl)amino]-5-nitro-2-pyridyl]benzamide (700 mg, 1.89 mmol), EtOH (5 mL), water (1.25 mL), ammonium chloride (1 eq., 1.89 mmol, 102 mg), iron shavings (5 eq., 9.45 mmol, 528 mg), fitted with a stir bar, was purged with nitrogen, sealed and stirred at 80 °C. After 16 h, the reaction was cooled to RT and filtered using an ISCO sample cartridge with wet Celite^{®} (MeOH) and washed several times with MeOH. The yellow solution dried over Na₂SO₄, filtered and was concentrated under reduced pressure to provide 850 mg. The residue was dissolved in 50 ml 0.1 M HCl and 50 mL EtOAC. The aq. layer was extracted 2 x 50 mL EtOAc and the combined organic layer was discarded. The acidic layer was made pH 12 with the addition of 5 N NaOH and then extracted 4 x 50 mL DCM, dried over Na₂SO₄ and concentrated under reduced pressure to provide 590 mg of 4-[5-amino-6-[(3-methyltetrahydrofuran-3-yl)amino]-2-pyridyl]-N,N-dimethyl-benzamide (91%) as a pale green solid. The material was pure by LCMS and was used directly in the next step. LCMS: (APCI) m/e 341.1 (M+H).

### 6-(4-fluorophenyl)-N²-(3-methyltetrahydrofuran-3-yl)pyridine-2,3-diamine (N-01)

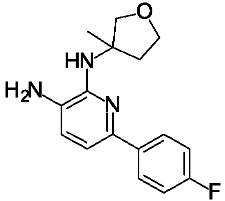

A 20 mL microwave vial was charged with 6-(4-fluorophenyl)-N-(3-methyltetrahydrofuran-3-yl)-3-nitro-pyridin-2-amine (500 mg, 1.58 mmol), EtOH (4 mL), water (1 mL), ammonium chloride (1 eq., 1.58 mmol, 86 mg), iron shavings (5 eq., 7.88 mmol, 440 mg), fitted with a stir bar, was purged with nitrogen, sealed and stirred at 80 °C. After 3 h, the reaction was cooled to RT and filtered using an ISCO sample cartridge with wet Celite^{®} (MeOH) and washed several times with MeOH. The yellow solution dried over Na₂SO₄, filtered and was concentrated under reduced pressure to provide 950 mg. The residue was dissolved in 50 ml 0.1 M HCl and 50 mL EtOAC. The aq. layer was extracted 2 x 50 mL EtOAc and the combined organic layer was discarded. The acidic layer was made pH 12 with the addition of 5 N NaOH and then extracted 4 x 50 mL DCM, dried over Na₂SO₄ and concentrated under reduced pressure to provide 420 mg of 6-(4-fluorophenyl)-N²-(3-methyltetrahydrofuran-3-yl)pyridine-2,3-diamine (92%) as a grey solid. The material was pure by LCMS and was used directly in the next step. LCMS: (APCI) m/e 288.1 (M+H).

### Step 4. Synthesis of Final Compounds

### N²-cyclopentyl-6-(p-tolyl)-N³-sec-butyl-pyridine-2,3-diamine (H-61)

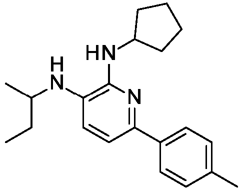

To a vial containing N²-cyclopentyl-6-(p-tolyl)pyridine-2,3-diamine (0.3032 g, 1.13 mmol) and a stir bar, 2-butanone (0.112 mL, 1.25 mmol, 1.1 equiv), TFA (0.168 mL, 2 equiv) and isopropyl acetate (4 mL) were added. To this was added sodium triacetoxyborohydride (0.288 g, 1.36 mmol, 1.2 equiv) over ^{~}5 min. An additional 1 mL isopropyl acetate was added to facilitate mixing. The reaction was then allowed to stir at room temperature for 1.5 hours. The reaction mixture was then filtered, the filtrate poured onto water and extracted with EtOAc (x3). Combined organic extracts were dried over anhydrous magnesium sulfate, filtered and concentrated by rotavap. Material was then loaded onto silica and purified by flash chromatography (24 g column, hexanes/EtOAc). Desired product fractions were combined and dried down to provide N²-cyclopentyl-6-(p-tolyl)-N³-sec-butyl-pyridine-2,3-diamine as a red-brown oil (42.2 mg, 11.5%). ¹H-NMR (400 MHz, DMSO-d6): δ 7.80 (d, 2H), 7.15 (d, 2H), 6.97 (d, 1H), 6.55 (d, 1H), 5.71 (d, 1H (NH)), 4.74 (d, 1H), 4.36 (m, 1H), 2.29 (s, 3H), 2.07 (m, 2H), 1.71 (m, 2H), 1.58 (m, 2H), 1.52 (m, 2H), 1.43 (m, 2H), 1.14 (d, 3H), 0.91 (t, 3H). ¹³C-NMR (400 MHz, DMSO-d6): 146.53, 139.92, 137.60, 135.21, 129.30, 128.89 (2C), 124.64 (2C), 113.28, 107.91, 52.62, 48.87, 32.73 (2C), 28.51, 23.91 (2C), 20.75, 19.74, 10.62. LCMS: (APCI) m/e 324 (M+H).

### N³-tert-butyl-N²-cyclopentyl-6-(p-tolyl)pyridine-2,3-diamine (A-98)

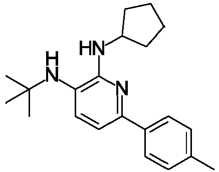

A solution of N²-cyclopentyl-6-(p-tolyl)pyridine-2,3-diamine (0.238g, 0.89mmol) in dichloromethane (2.5mL) was treated with tert-butyl 2,2,2-trichloroethanimidate (0.39g, 1.8mmol, 2equiv) and then borontrifluoride etherate (22uL, 0.18mmol, 0.2equiv). After stirring for 3hrs., LC/MS analysis showed partial conversion to the desired product and a significant amount of starting material. The reaction mixture was treated with an additional amount of tert-butyl 2,2,2-trichloroethanimidate (0.39g, 1.8mmol, 2equiv) and borontrifluoride etherate (22uL, 0.18mmol, 0.2equiv). After stirring overnight, LC/MS analysis showed 50% conversion to the desired product and 50% starting material. Purification on silica gel provided 23 mg (8%) of **N³-tert-butyl-N²-cyclopentyl-6-(p-tolyl)pyridine-2,3-diamine (A-98).** LCMS: (APCI) m/e 324.1 (M+H).

### N²-cyclopentyl-6-pentyl-N³-sec-butyl-pyridine-2,3-diamine (H-72)

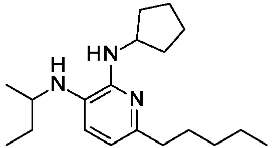

To a vial containing N²-cyclopentyl-6-pentyl-pyridine-2,3-diamine (0.268 g, 1.08 mmol) and a stir bar, 2-butanone (0.107 mL, 1.19 mmol, 1.1 equiv), TFA (0.161 mL, 2.17 mmol, 2 equiv) and isopropyl acetate (5 mL) were added. To this was added sodium triacetoxyborohydride (0.276 g, 1.30 mmol, 1.2 equiv) over ^{~}5 min. The reaction was then allowed to stir at room temperature. After 45 min reaction time, LCMS suggests conversion to desired product. Reaction was filtered, the filtrate poured onto water and extracted with EtOAc (x3). Combined organic extracts were dried over anhydrous magnesium sulfate, rotavapped down, loaded onto silica and purified by column chromatography (40 g column, hexanes/EtOAc). Desired product fractions were combined and dried down to provide N²-cyclopentyl-6-pentyl-N³-sec-butyl-pyridine-2,3-diamine (40.3 mg, 12.3%). ¹H-NMR (400 MHz, DMSO-d6): δ 6.40 (d, 1H), 6.20 (d, 1H), 5.44 (d, 1H (NH)), 4.30 (d, 1H (NH)), 4.23 (m, 1H), 4.21 (m, 1H), 2.39 (t, 2H), 1.97 (m, 4H), 1.67 (m, 2H), 1.55 (m, 4H), 1.41 (m, 2H), 1.26 (m, 4H), 1.09 (d, 3H), 0.89 (t, 3H), 0.85 (t, 3H). LCMS: (APCI) m/e 304 (M+H).

### N²-cyclopentyl-6-(3-pyridyl)-N³-sec-butyl-pyridine-2,3-diamine (H-74)

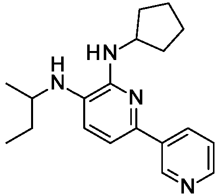

To a vial containing N²-cyclopentyl-6-(3-pyridyl)pyridine-2,3-diamine (0.316 g, 1.24 mmol) and a stir bar, 2-butanone (0.122 mL, 1.37 mmol, 1.1 equiv), TFA (0.185 mL, 2.48 mmol, 2 equiv) and isopropyl acetate (5 mL) were added. To this was added sodium triacetoxyborohydride (0.316 g, 1.49 mmol, 1.2 equiv) over ^{~}5 min. The reaction was then allowed to stir at room temperature. After 45 min, LCMS suggested conversion to desired product. Reaction was filtered, the filtrate poured onto water and extracted with EtOAc (x3). Combined organic extracts were dried over anhydrous magnesium sulfate, rotavapped down, and loaded onto silica. The product was purified by column chromatography (hexanes/ethyl acetate). Desired product fractions were combined and dried down to afford N²-cyclopentyl-6-pentyl-N³-sec-butyl-pyridine-2,3-diamine (0.1443 g, 37.4%) as a light brown solid. ¹H-NMR (400 MHz, DMSO-d6): δ 9.13 (d, 1H), 8.39 (dd, 1H), 8.23 (m, 1H), 7.36 (m, 1H), 7.11 (d, 1H), 6.58 (d, 1H), 5.86 (d, 1H (NH)), 4.93 (d, 1H), 4.37 (m, 1H), 2.08 (m, 2H), 1.71 (m, 2H), 1.60 (m, 2H), 1.53 (m, 2H), 1.45 (m, 2H), 1.15 (d, 3H), 0.93 (t, 3H). ¹³C-NMR (400 MHz, DMSO-d6): δ 147.00, 146.70, 146.33, 136.86, 135.48, 131.68, 130.27, 123.47, 112.71, 109.02, 52.69, 48.84, 32.67 (2C), 28.47, 23.91 (2C), 19.71, 10.64. LCMS: (APCI) m/e 311 (M+H).

### N²-cyclopentyl-N³-(oxetan-3-yl)-6-(3-pyridyl)pyridine-2,3-diamine (H-75)

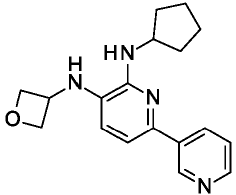

To a vial containing N²-cyclopentyl-6-(3-pyridyl)pyridine-2,3-diamine (0.316 g, 1.24 mmol) and a stir bar, oxetan-3-one (0.087 mL, 1.37 mmol, 1.1 equiv), TFA (0.185 mL, 2.48 mmol, 2 equiv) and isopropyl acetate (5 mL) were added. To this was added sodium triacetoxyborohydride (0.316 g, 1.49 mmol, 1.2 equiv) over ^{~}5 min. The reaction was then allowed to stir at room temperature. After 45 min reaction time, LCMS suggested conversion to desired product. The reaction mixture was poured onto water and extracted with EtOAc (x3). Combined organic extracts were dried over anhydrous magnesium sulfate, rotavapped down, loaded onto silica, and purified by column chromatography (hexanes/ethyl acetate). Desired product fractions were combined and dried down to afford N²-cyclopentyl-N³-(oxetan-3-yl)-6-(3-pyridyl)pyridine-2,3-diamine as an off-white solid (89 mg, 23.1%). ¹H-NMR (400 MHz, DMSO-d6): δ 9.13 (d, 1H), 8.42 (dd, 1H), 8.24 (m, 1H), 7.37 (m, 1H), 7.08 (d, 1H), 6.34 (d, 1H), 5.85 (m, 2H (NHs)), 4.91 (t, 2H), 4.54 (m, 1H), 4.46 (t, 2H), 4.38 (m, 1H), 2.09 (m, 2H), 1.73 (m, 2H), 1.61 (m, 2H), 1.54 (m, 2H). ¹³C-NMR (400 MHz, DMSO-d6): 147.48, 147.22, 146.59, 138.95, 135.26, 131.99, 129.07, 123.50, 113.62, 108.77, 77.44 (2C), 52.59, 47.60, 32.74 (2C), 23.86 (2C).LCMS: (APCI) m/e 311 (M+H).

### N²-cyclopentyl-6-(4-pyridyl)-N³-sec-butyl-pyridine-2,3-diamine (H-76)

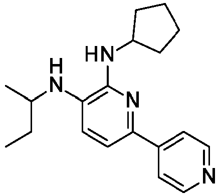

To a vial containing N²-cyclopentyl-6-(4-pyridyl)pyridine-2,3-diamine (0.1391 g, 0.547 mmol) and a stir bar, 2-butanone (0.108 mL, 1.204 mmol), TFA (0.081 mL, 1.09 mmol), and isopropyl acetate (5 mL) were added. To this was added sodium triacetoxyborohydride (0.139 g, 0.656 mmol) over ^{~}2 min. The reaction was then allowed to stir at room temperature overnight. The resulting reaction mixture was then poured onto water and extracted with ethyl acetate (x3). Combined organic extracts were dried over anhydrous magnesium sulfate, filtered, rotavapped down, loaded onto silica and purified by column chromatography (24 g column, hexanes/EtOAc). Desired product fractions were combined and dried down to yield N²-cyclopentyl-6-(4-pyridyl)-N³-sec-butyl-pyridine-2,3-diamine as a brown solid (39.6 mg, 23.3%). ¹H-NMR (400 MHz, DMSO-d6): δ 8.48 (d, 2H), 7.86 (d, 2H), 7.23 (d, 1H), 6.58 (d, 1H), 5.91 (d, 1H (NH)), 5.10 (d, 1H (NH)), 4.37 (m, 1H), 3.40 (m, 1H), 2.09 (m, 2H), 1.72 (m, 2H), 1.59 (m, 2H), 1.52 (m, 2H), 1.45 (m, 2H), 1.15 (d, 3H), 0.92 (t, 3H). ¹³C-NMR (400 MHz, DMSO-d6): δ 149.73 (2C), 147.00, 146.42, 136.20, 131.40, 118.88 (2C), 112.10, 110.22, 52.71, 48.85, 32.66, 28.46, 23.96 (2C), 19.68, 10.65. LCMS: (APCI) m/e 311 (M+H).

### N²-cyclopentyl-N³-(oxetan-3-yl)-6-(4-pyridyl)pyridine-2,3-diamine (H-77)

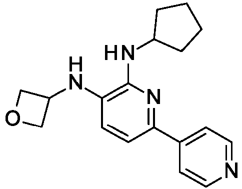

To a vial containing N²-cyclopentyl-6-(4-pyridyl)pyridine-2,3-diamine (0.1375 g, 0.541 mmol) and a stir bar, oxetan-3-one (0.076 mL, 1.19 mmol), TFA (0.080 mL, 1.08 mmol), and isopropyl acetate (5 mL) were added. To this was added sodium triacetoxyborohydride (0.137 g, 0.649 mmol) over ^{~}2 min. The reaction was then allowed to stir at room temperature overnight. The resulting reaction mixture was poured onto water and extracted with ethyl acetate (x3). Combined organic extracts were dried over anhydrous magnesium sulfate, filtered, rotavapped down and loaded onto silica. The material was purified by column chromatography (24 g column, DCM/MeOH). Desired product fractions were combined and dried down to afford N²-cyclopentyl-N³-(oxetan-3-yl)-6-(4-pyridyl)pyridine-2,3-diamine as a pale yellow solid (8.4 mg, 5.01%). ¹H-NMR (400 MHz, DMSO-d6): δ 8.49 (d, 2H), 7.86 (d, 2H), 7.18 (d, 1H), 6.32 (d, 1H), 6.01 (d, 1H (NH)), 5.88 (d, 1H (NH)), 4.89 (t, 2H), 4.54 (m, 1H), 4.45 (m, 2H), 4.37 (m, 1H), 2.09 (m, 2H), 1.71 (m, 2H), 1.60 (m, 2H), 1.52 (m, 2H). ¹³C-NMR (400 MHz, DMSO-d6): 149.81 (2C), 146.96, 146.79, 138.25, 130.19, 119.15 (2C), 113.04, 109.87, 77.34 (2C), 52.59, 47.55, 32.72 (2C), 23.89 (2C).LCMS: (APCI) m/e 311 (M+H).

### N²-cyclopentyl-6-pyrimidin-5-yl-N³-sec-butyl-pyridine-2,3-diamine (H-80)

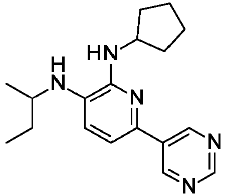

In a 2.0 - 5.0 mL capacity microwave vial equipped with stir bar, 6-chloro-N²-cyclopentyl-N³-sec-butyl-pyridine-2,3-diamine (byproduct recovered from H-72, 0.1559 g), potassium acetate (0.171 g, 3 equiv), and pyrimidin-5-ylboronic acid (0.159 g, 2.2 equiv) were combined in n-butanol (3 mL) and water (0.3 mL). The reaction mixture was flushed with nitrogen. Dichlorobis{[4-(N,N-dimethylamino)phenyl]di-t-butylphenylphosphino}palladium(II) (8.2 mg, 0.02 equiv) was then added and the vial sealed. The vial was then placed in the microwave reactor for 20 min at 110 °C. The resulting mixture was poured onto water and extracted with ethyl acetate (x3). Organic extracts were combined and dried over anhydrous magnesium sulfate. Material was then filtered, concentrated, loaded onto silica and purified via flash chromatography (hexanes/ethyl acetate). Desired product fractions were combined and dried down to yield N²-cyclopentyl-6-pyrimidin-5-yl-N³-sec-butyl-pyridine-2,3-diamine as a light brown solid (73.8 mg, 40.7%). 1H-NMR (400 MHz, DMSO-d6): δ 9.24 (s, 2H), 8.98 (s, 1H), 7.19 (d, 1H), 6.57 (d, 1H), 5.94 (d, 1H (NH)), 5.05 (d, 1H (NH)), 4.35 (m, 1H), 3.38 (m, 1H), 2.07 (m, 2H), 1.69 (m, 2H), 1.57 (m, 2H), 1.49 (m, 2H), 1.44 (m, 2H), 1.13 (d, 3H), 0.90 (t, 3H). 13C-NMR (400 MHz, DMSO-d6): δ 155.88, 152.76 (2C), 146.82, 133.80, 132.97, 130.98, 112.31, 109.62, 52.70, 48.82, 32.59 (2C), 28.43, 23.88 (2C), 19.65, 10.59. LCMS: (APCI) m/e 312 (M+H).LCMS: (APCI) m/e 312 (M+H).

### N²-cyclopentyl-N³-(oxetan-3-yl)-6-(p-tolyl)pyridine-2,3-diamine (H-81)

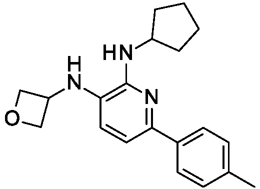

To a vial containing N²-cyclopentyl-6-(p-tolyl)pyridine-2,3-diamine (0.278 g, 1.04 mmol) and a stir bar, oxetan-3-one (0.100 mL, 1.56 mmol, 1.5 equiv), TFA (0.154 mL, 2.08 mmol, 2 equiv) and isopropyl acetate (5 mL) were added. To this was added sodium triacetoxyborohydride (0.331 g, 1.56 mmol, 1.5 equiv) over ^{~}2 min. The reaction was then allowed to stir at room temperature. After 2 hours, the reaction mixture was poured onto water and extracted with EtOAc (x3). Combined organic extracts were dried over anhydrous magnesium sulfate, filtered and concentrated by rotavap. Material was then loaded onto silica and purified by flash chromatography (24 g column, hexanes/EtOAc). Desired product fractions were combined and dried down to yield N²-cyclopentyl-N³-(oxetan-3-yl)-6-(p-tolyl)pyridine-2,3-diamine as a pale purple solid (59.6 mg, 17.7%). ¹H-NMR (400 MHz, DMSO-d6): δ 7.81 (d, 2H), 7.16 (d, 2H), 6.94 (d, 1H), 6.30 (d, 1H), 5.70 (m, 2H (NHs)), 4.90 (t, 2H), 4.50 (m, 1H), 4.45 (t, 2H), 4.38 (m, 1H), 2.29 (s, 3H), 2.08 (m, 2H), 1.72 (m, 2H), 1.60 (m, 2H), 1.53 (m, 2H). ¹³C-NMR (400 MHz, DMSO-d6): δ 147.03, 141.89, 137.36, 135.68, 128.92 (2C), 128.10, 124.90 (2C), 114.06, 107.69, 77.51 (2C), 52.51, 47.70, 32.79 (2C), 23.84 (2C), 20.76.LCMS: (APCI) m/e 324 (M+H).

### N²-cyclopentyl-N³-(oxetan-3-yl)-6-pyrimidin-5-yl-pyridine-2,3-diamine (H-84)

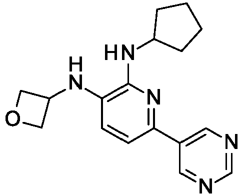

To a vial containing N²-cyclopentyl-6-pyrimidin-5-yl-pyridine-2,3-diamine (0.213 g, 0.834 mmol) and a stir bar, oxetan-3-one (0.081 mL, 1.25 mmol, 1.5 equiv), TFA (0.124 mL, 1.67 mmol, 2 equiv) and isopropyl acetate (5 mL) were added. To this was added sodium triacetoxyborohydride (0.212 g, 1.00 mmol, 1.2 equiv) over ^{~}2 min. The reaction was then allowed to stir at room temperature overnight. The reaction was stopped, poured onto water, and extracted with ethyl acetate (x4). Combined organic extracts were dried over anhydrous magnesium sulfate, filtered, concentrated by rotavap and loaded onto silica. Material was purified by column chromatography (hexanes/ethyl acetate). Desired product fractions were combined and dried down to afford N²-cyclopentyl-N³-(oxetan-3-yl)-6-pyrimidin-5-yl-pyridine-2,3-diamine as a yellow oil (21.4 mg, 8.24%). ¹H-NMR (400 MHz, DMSO-d6): δ 9.26 (s, 2H), 9.01 (s, 1H), 7.17 (d, 1H), 6.33 (d, 1H), 5.99 (d, 1H (NH)), 5.93 (d, 1H (NH)), 4.89 (t, 2H), 4.53 (m, 1H), 4.45 (t, 2H), 4.36 (m, 1H), 2.07 (m, 2H), 1.71 (m, 2H), 1.60 (m, 2H), 1.52 (m, 2H). ¹³C-NMR (400 MHz, DMSO-d6): 156.28, 153.09 (2C), 147.34, 135.88, 132.79, 129.81, 113.22, 109.36, 77.34 (2C), 52.61, 47.54, 32.66 (2C), 23.84 (2C).LCMS: (APCI) m/e 312 (M+H).

### N²-cyclopentyl-6-(4-methoxyphenyl)-N³-sec-butyl-pyridine-2,3-diamine (H-86)

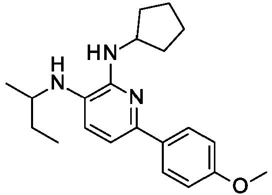

To a vial containing N²-cyclopentyl-6-(4-methoxyphenyl)pyridine-2,3-diamine (0.250 g, 0.882 mmol) and a stir bar, isopropyl acetate (5 mL), TFA (0.131 mL, 1.76 mmol), and 2-butanone (0.119 mL, 1.32 mmol) were added. To the stirring mixture was added sodium triacetoxyborohydride (0.224 g, 1.06 mmol) over ^{~}2 min. The reaction was then allowed to stir at room temperature. After 45 min, saturated sodium bicarbonate (aq) was added, and the organic layer isolated and loaded onto silica. The material was then purified by column chromatography (hexanes/EtOAc). Desired product fractions were combined and rotavapped down to afford N²-cyclopentyl-6-(4-methoxyphenyl)-N³-sec-butyl-pyridine-2,3-diamine as a viscous brown oil (0.2594 g, 86.6%). ¹H-NMR (400 MHz, DMSO-d6): δ 7.83 (d, 2H), 6.91 (d, 2H), 6.90 (d, 1H), 6.53 (d, 1H), 5.68 (d, 1H (NH)), 4.66 (d, 1H), 4.33 (m, 1H), 3.74 (s, 3H), 2.07 (m, 2H), 1.69 (m, 2H), 1.56 (m, 2H), 1.50 (m, 2H), 1.41 (m, 2H), 1.12 (d, 3H), 0.90 (t, 3H). ¹³C-NMR (400 MHz, DMSO-d6): 158.10, 146.59, 139.95, 133.08, 128.88, 125.88 (2C), 113.69, 113.55, 107.37, 55.02, 52.63, 48.89, 32.74 (2C), 28.53, 23.90 (2C), 19.75, 10.62. LCMS: (APCI) m/e 340 (M+H).

### N²-cyclopentyl-6-(4-methoxyphenyl)-N³-(oxetan-3-yl)pyridine-2,3-diamine (H-87)

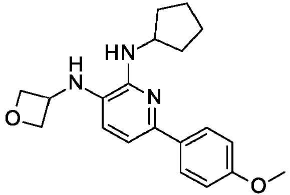

To a vial containing N²-cyclopentyl-6-(4-methoxyphenyl)pyridine-2,3-diamine (0.250 g, 0.882 mmol) and a stir bar, isopropyl acetate (5 mL), TFA (0.131 mL, 1.76 mmol), and oxetanone (0.0851 mL, 1.32 mmol) were added. To the stirring mixture was added sodium triacetoxyborohydride (0.224 g, 1.06 mmol) over ^{~}2 min. The reaction was then allowed to stir at room temperature for 3 hours. At this time, saturated sodium bicarbonate (aq) was added, and the organic layer isolated and loaded onto silica. The material was purified by column chromatography (hexanes/EtOAc). Desired product fractions were combined, rotavapped down, and dried under vacuum at 40 °C to afford N²-cyclopentyl-6-(4-methoxyphenyl)-N³-(oxetan-3-yl)pyridine-2,3-diamine as a fluffy tan solid (169.3 mg, 56.5%). ¹H-NMR (400 MHz, DMSO-d6): δ 7.85 (d, 2H), 6.93 (d, 2H), 6.89 (d, 1H), 6.29 (d, 1H), 5.65 (m, 2H (NH)), 4.89 (t, 2H), 4.49 (m, 1H), 4.45 (m, 2H), 4.37 (m, 1H), 3.76 (s, 3H), 2.07 (m, 2H), 1.72 (m, 2H), 1.60 (m, 2H), 1.52 (m, 2H). ¹³C-NMR (400 MHz, DMSO-d6): 158.38, 147.07, 141.87, 132.80, 127.69, 126.17 (2C), 114.26, 113.74 (2C), 107.16, 77.55 (2C), 55.06, 52.53, 47.73, 32.81 (2C), 23.85 (2C). LCMS: (APCI) m/e 340 (M+H).

### N²-tert-butyl-6-(p-tolyl)-N³-sec-butyl-pyridine-2,3-diamine (L-02)

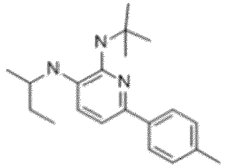

A solution of N²-tert-butyl-6-(p-tolyl)pyridine-2,3-diamine (0.147g, 0.58mmol) in isopropylacetate (3.0mL) was successively treated with 2-butanone (63mg, 0.86mmol, 1.5equiv) and then TFA (85ul, 1.1mmol, 2.0equiv). After 30min, the reaction was then treated with sodium triacetoxyborohydride (0.147g, 0.68mmol, 1.2euiv). After 1hr., LC/MS analysis showed clean conversion to the desired product. The reaction mixture was quenched with satd. aq. NaCl (5mL) and extracted with ethyl acetate (3 x 10mL). The combined extracts were dried (Na₂SO₄) and the solvent removed in vacuo. The residue was purified by flash chromatography (12g silica, 0-100% ethyl acetate/hexanes) to afford N²-tert-butyl-6-(p-tolyl)-N³-sec-butyl-pyridine-2,3-diamine (0.154g, 86%) as a blue oil. LCMS: (APCI) m/e 312.2 (M+H).

### N²,N³-di-tert-butyl-6-(p-tolyl)pyridine-2,3-diamine (L-03)

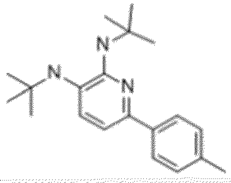

A solution of N²-cyclopentyl-6-(p-tolyl)pyridine-2,3-diamine (0.238g, 0.89mmol) in dichloromethane (2.5mL) was treated with tert-butyl 2,2,2-trichloroethanimidate (0.39g, 1.8mmol, 2equiv) and then borontrifluoride etherate (22uL, 0.18mmol, 0.2equiv). After stirring for 3hrs., LC/MS analysis showed partial conversion to the desired product and a significant amount of starting material. The reaction mixture was treated with an additional amount of tert-butyl 2,2,2-trichloroethanimidate (0.39g, 1.8mmol, 2equiv) and borontrifluoride etherate (22uL, 0.18mmol, 0.2equiv). After stirring overnight, LC/MS analysis showed 50% conversion to the desired product and 50% starting material. After stirring overnight, LC/MS showed only slight increase in conversion to the desired product. The reaction mixture was quenched with satd. aq. ammonium chloride (5mL) and the mixture was extracted with methylene chloride (3 x 15mL). The combined organic extracts were dried (Na₂SO₄) and the solvent was removed in vacuo. The residue was purified by flash chromatography (12g silica, 0-100% ethyl acetate/hexanes) to afford N²,N³-di-tert-butyl-6-(p-tolyl)pyridine-2,3-diamine (0.229g, 56%) as a blue solid. LCMS: (APCI) m/e 312.2 (M+H); ¹H NMR (CDCl₃): δ 7.92 (d, 2H), 7.22 (m, 2H), 7.05 (bs, 1H), 6.92 (t, 1H), 2.40 (s, 3H), 1.27 (s, 9H), 1.12 (s, 9H).

### N³-tert-butyl-N²-cyclopentyl-6-(4-pyridyl)pyridine-2,3-diamine (L-04)

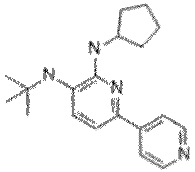

A solution of N²-cyclopentyl-6-(p-tolyl)pyridine-2,3-diamine (0.238g, 0.89mmol) in dichloromethane (2.5mL) was treated with tert-butyl 2,2,2-trichloroethanimidate (0.39g, 1.8mmol, 2equiv) and then borontrifluoride etherate (22uL, 0.18mmol, 0.2equiv). After stirring for 3hrs., LC/MS analysis showed partial conversion to the desired product and a significant amount of starting material. The reaction mixture was treated with an additional amount of tert-butyl 2,2,2-trichloroethanimidate (0.39g, 1.8mmol, 2equiv) and borontrifluoride etherate (22uL, 0.18mmol, 0.2equiv). After stirring overnight, LC/MS analysis showed 50% conversion to the desired product and 50% starting material. The reaction was quenched with satd. aq. ammonium chloride (5mL) and the mixture was extracted with ethyl acetate (3 x 15mL methylene chloride). The combined organic extracts were dried (Na₂SO₄) and the solvent removed in vacuo. The residue was purified by flash chromatography (0-100% ethyl acetate/hexanes). The product co-eluted with an impurity from an unknown source. The product was re-purified by RP-HPLC to afford N³-tert-butyl-N²-cyclopentyl-6-(4-pyridyl)pyridine-2,3-diamine (7mg, 4%) as a red solid. LCMS: (APCI) m/e 311.1 (M+H).

### 6-(2-pyridyl)-N³-sec-butyl-N²-tetrahydrofuran-3-yl-pyridine-2,3-diamine (L-19)

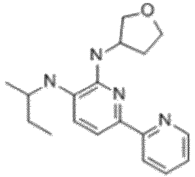

A solution of 6-(2-pyridyl)-N²-tetrahydrofuran-3-yl-pyridine-2,3-diamine (87mg, 034mmol) in methanol (1mL) was successively treated with 2-butanone (38mg, 0.51mmol, 1.5equiv) and then acetic acid (40uL, 0.68mmol, 2.0equiv). After stirring for 30min, the reaction mixture was then treated with sodium cyanoborohydride (33mg, 0.51mmol, 1.5equiv). After stirring overnight, LC/MS analysis showed partial conversion to the desired product. Additional 1.5 equiv of 2-butanone and sodium cyanoborohydride was added to drive the reaction to product. LC/MS analysis showed clean conversion to the desired product. The reaction mixture was adsorbed onto a 12g cartridge and purified by flash chromatography (12g silica, 0-100% ethyl acetate/hexanes) to afford 6-(2-pyridyl)-N³-sec-butyl-N²-tetrahydrofuran-3-yl-pyridine-2,3-diamine (0.101g, 95%) as an orangish-yellow solid. LCMS: (APCI) m/e 313.1 (M+H).

### 6-(2-pyridyl)-N²,N³-di(tetrahydrofuran-3-yl)pyridine-2,3-diamine (L-21)

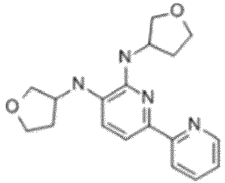

A solution of 6-(2-pyridyl)-N²-tetrahydrofuran-3-yl-pyridine-2,3-diamine (77mg, 0.30mmol) in methanol (1mL) was successively treated with tetrahydrofuran-3-one (39mg, 0.45mmol, 1.5equiv) and then acetic acid (35uL, 0.60mmol, 2.0equiv). After stirring for 30min, the reaction mixture was then treated with sodium cyanoborohydride (29mg, 0.45mmol, 1.5equiv). After stirring overnight, LC/MS analysis showed partial conversion to the desired product. Additional 1.5 equiv of tetrahydrofuran-3-one and sodium cyanoborohydride was added to drive the reaction to product. LC/MS analysis showed clean conversion to the desired product. The reaction mixture was adsorbed onto a 12g cartridge and purified by flash chromatography (12g silica, 0-100% ethyl acetate/hexanes) to afford 6-(2-pyridyl)-N²,N³-di(tetrahydrofuran-3-yl)pyridine-2,3-diamine (0.047g, 48%) as a brown solid. LCMS: (APCI) m/e 327.1 (M+H); ¹H NMR (CDCl₃): δ 8.50 (d, 1H), 8.31 (d, 1H), 7.76 (t, 2H), 7.17 (d, 1H), 6.82 (d, 1H), 5.55 (bs, 1H), 4.82 (bs, 1H), 3.76 (m, 8H), 2.03 (m, 6H).

### N²-(3-methyltetrahydrofuran-3-yl)-6-(2-pyridyl)-N³-sec-butyl-pyridine-2,3-diamine (L-22)

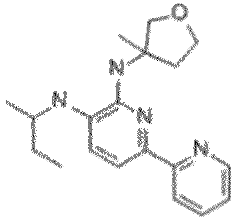

A solution of N²-(3-methyltetrahydrofuran-3-yl)-6-(2-pyridyl)pyridine-2,3-diamine (79mg, 0.29mmol) in methanol (1mL) was successively treated with 2-buantone (32mg, 0.44mmol, 1.5equiv) and then acetic acid (33uL, 0.58mmol, 2.0equiv). After stirring for 30min, the reaction mixture was then treated with sodium cyanoborohydride (28mg, 0.44mmol, 1.5equiv). After stirring overnight, LC/MS analysis showed partial conversion to the desired product. Additional 1.5 equiv of 2-butanone and sodium cyanoborohydride was added to drive the reaction to product. LC/MS analysis showed clean conversion to the desired product. The reaction mixture was adsorbed onto a 12g cartridge and purified by flash chromatography (12g silica, 0-100% ethyl acetate/hexanes) to afford N²-(3-methyltetrahydrofuran-3-yl)-6-(2-pyridyl)-N³-sec-butyl-pyridine-2,3-diamine (0.079g, 83%) as an orangish-yellow solid. LCMS: (APCI) m/e 327.2 (M+H); ¹H NMR (CDCl₃): δ 8.35 (bs, 1H), 8.22 (bs, 1H), 7.77 (d, 2H), 7.15 (m, 1H), 6.82 (d, 1H), 3.87 (m, 4H), 2.82 (m, 3H), 2.02 (m, 4H), 1.67 (s, 3H), 1.07 (m, 4H).

### N²-(3-methyltetrahydrofuran-3-yl)-6-(2-pyridyl)-N³-tetrahydrofuran-3-yl-pyridine-2,3-diamine (L-23)

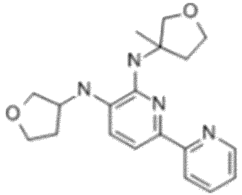

A solution of N²-(3-methyltetrahydrofuran-3-yl)-6-(2-pyridyl)pyridine-2,3-diamine (83mg, 0.31mmol) in methanol (1mL) was successively treated with tetrahydrofuran-3-one (40mg, 0.46mmol, 1.5equiv) and then acetic acid (35uL, 0.61mmol, 2.0equiv). After stirring for 30min, the reaction mixture was then treated with sodium cyanoborohydride (29mg, 0.46mmol, 1.5equiv). After stirring overnight, LC/MS analysis showed partial conversion to the desired product. Additional 1.5 equiv of tetrahydrofuran-3-one and sodium cyanoborohydride was added to drive the reaction to product. LC/MS analysis showed clean conversion to the desired product. The reaction mixture was adsorbed onto a 12g cartridge and purified by flash chromatography (12g silica, 0-100% ethyl acetate/hexanes) to afford N²-(3-methyltetrahydrofuran-3-yl)-6-(2-pyridyl)-N³-tetrahydrofuran-3-yl-pyridine-2,3-diamine (0.082g, 79%) as a brown solid. LCMS: (APCI) m/e 341.1 (M+H); ¹H NMR (CDCl₃): δ 8.54 (d, 1H), 8.27 (d, 1H), 7.76 (m, 2H), 7.25 (m, 1H), 6.85 (d, 1H), 3.69 (m, 8H), 2.02 (m, 5H), 1.66 (s, 3H).

### N³-(3,3-difluorocyclobutyl)-N²-(3,3-difluoro-1-methyl-cyclobutyl)-6-(3-pyridyl)pyridine-2,3-diamine (M-09)

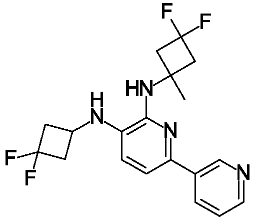

A 40 mL vial was charged with N²-(3,3-difluoro-1-methyl-cyclobutyl)-6-(3-pyridyl) pyridine-2,3-diamine (0.271 g, 0.933 mmol). A stir bar, 3,3-difluorocyclobutanone (1.6 eq., 0.158 g, 1.49 mmol), TFA (1.2 eq., 0.083 mL, 1.12 mmol), and isopropyl acetate (6 mL) were added. To this was added sodium triacetoxyborohydride (1.5 eq., 0.297 g, 1.40 mmol). The reaction was stirred at 25 °C overnight, after which LCMS analysis suggested bulk of material had converted to desired product. The reaction was partitioned between water and ethyl acetate. The organic layer was isolated, and the water layer extracted three times with ethyl acetate. Organic extracts were combined and dried over anhydrous magnesium sulfate, filtered, and concentrated via rotavap. The resulting concentrate was loaded onto silica and purified by column chromatography (hexanes/ethyl acetate), to afford N³-(3,3-difluorocyclobutyl)-N²-(3,3-difluoro-1-methyl-cyclobutyl)-6-(3-pyridyl)pyridine-2,3-diamine (58.7 mg, 16.5%) as a pale peach-colored solid. LCMS: (APCI) m/e 381 (M+H); ¹H NMR (DMSO-d₆): δ 8.65 (m, 1H), 8.42 (m, 1H), 8.21 (m, 1H), 7.38 (m, 1H), 7.19 (d, 1H), 6.60 (d, 1H), 6.13 (bs, 1H (NH)), 5.53 (d, 1H (NH)), 3.81 (m, 1H), 3.12 (m, 2H), 2.96 (m, 2H), 2.83 (m, 2H), 2.53 (m, 2H), 1.65 (s, 3H).

### N³-(3,3-difluorocyclobutyl)-6-(4-fluorophenyl)-N²-(3-methyltetrahydrofuran-3-yl)pyridine-2,3-diamine (M-10)

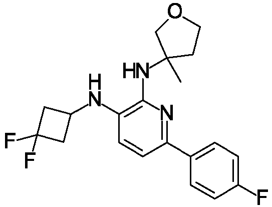

A 40 mL vial was charged with 6-(4-fluorophenyl)-N²-(3-methyltetrahydrofuran-3-yl) pyridine-2,3-diamine (0.200 g, 0.696 mmol). A stir bar, 3,3-difluorocyclobutanone (1.2 eq., 0.089 g, 0.835 mmol), TFA (1.2 eq., 0.062 mL, 0.835 mmol), and isopropyl acetate (6 mL) were added. To this was added sodium triacetoxyborohydride (1.5 eq., 0.221 g, 1.04 mmol). The reaction was stirred at 25 °C overnight. LCMS after overnight reaction suggested conversion to desired product. The reaction was partitioned between water and ethyl acetate. The organic layer was isolated, and water layer extracted three times with ethyl acetate. Combined organic extracts were dried over anhydrous magnesium sulfate, filtered, and concentrated via rotavap. The resulting concentrate was loaded onto silica and purified by column chromatography (hexanes/ethyl acetate), to afford N³-(3,3-difluorocyclobutyl)-6-(4-fluorophenyl)-N²-(3-methyltetrahydrofuran-3-yl) pyridine-2,3-diamine (102 mg, 38.8%) as a pale tan solid. LCMS: (APCI) m/e 378 (M+H); ¹H NMR (DMSO-d₆): δ 7.90 (m, 2H), 7.19 (m, 2H), 7.03 (d, 1H), 6.55 (d, 1H), 5.67 (bs, 1H (NH)), 5.54 (d, 1H (NH)), 4.00 (d, 1H), 3.91 (d, 1H), 3.82 (m, 2H), 3.77 (m, 1H), 3.10 (m, 2H), 2.54 (m, 1H), 2.41 (m, 1H), 2.01 (m, 1H), 1.58 (s, 3H).

### 4-[5-[(3,3-difluorocyclobutyl)amino]-6-[(3-methyltetrahydrofuran-3-yl)amino]-2-pyridyl]-N,N-dimethyl-benzamide (N-04)

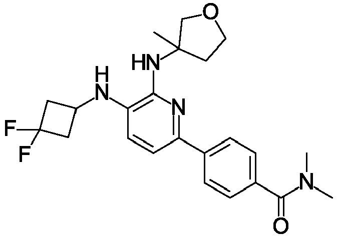

A 40 mL vial was charged with 4-[5-amino-6-[(3-methyltetrahydrofuran-3-yl)amino]-2-pyridyl]-N,N-dimethyl-benzamide (279 g, 0.820 mmol) and a stir bar, 3,3-difluorocyclobutanone (1.2 eq., 104 mg, 0.983 mmol), TFA (1.2 eq., 0.74 mL, 0.983 mmol), and isopropyl acetate (5 mL. 0.2 M) were added. To this was added sodium triacetoxyborohydride (1.5 eq., 261 mg, 1.23 mmol) over ^{~}2 min. The reaction was then allowed to stir at room temperature. After 16 h, the reaction was complete by LCMS and was partitioned between 25 mL of water and 25 mL of EtOAc. The water layer was extracted 3 x 25 mL EtOAc, dried over Na₂SO₄, filtered and concentrated under reduced pressure. The residue was purified on silica gel (40 g, 0-50% EtOAc/hexanes) to provide 110 mg of 4-[5-[(3,3-difluorocyclobutyl)amino]-6-[(3-methyltetrahydrofuran-3-yl)amino]-2-pyridyl]-N,N-dimethyl-benzamide (31%) as a yellow film. LCMS (APCI) m/e 431.1 (M+H); 1H NMR (CDCl₃): δ 7.92 (d, 2H), 7.41 (d, 2H), 7.06 (d, 1H), 6.58 (d, 1H), 4.56 (bs, 1H), 4.00 (m, 2H), 3.95 (m, 2H), 3.92 (bs, 1H), 3.00 (m, 6H), 2.47 (m, 3H), 2.02 (m, 2H), 1.66 (m, 3H), 1.22 (t, 2H).

### Example 6 - not encompassed by the wording of the claims but are considered as useful for understanding the invention

### Synthesis of Gem-dimethyl Pyrimidine Compounds

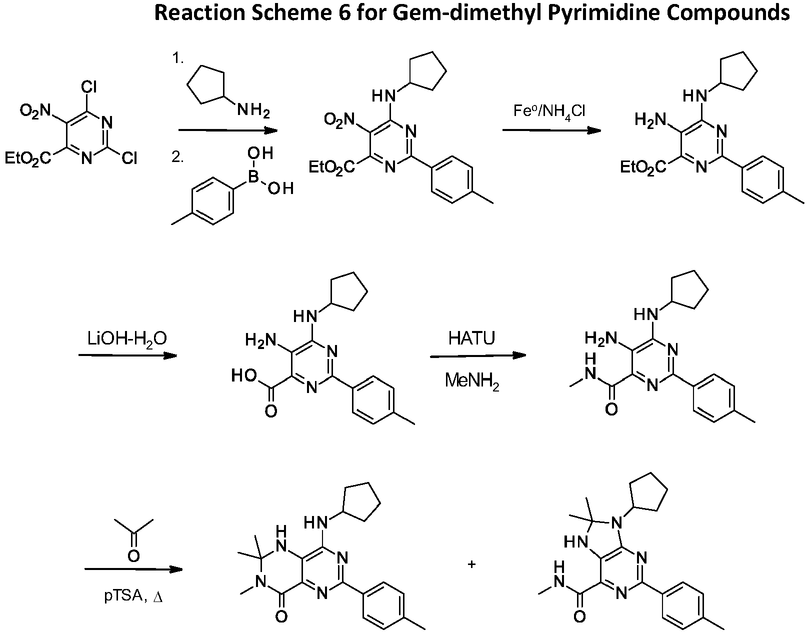

### ethyl 2-chloro-6-(cyclopentylamino)-5-nitro-pyrimidine-4-carboxylate (K-19).

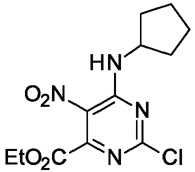

A 100 mL 14/22 RBF was charged with ethyl 2,6-dichloro-5-nitro-pyrimidine-4-carboxylate (500 mg, 1.88 mmol), THF (4 mL), fitted with a balloon of nitrogen and cooled to -78 °C. The reaction was then treated with DiPEA (1.5 eq., 2.8 mmol, 0.5 mL) and then treated dropwise with a solution of cyclopentanamine (1.0 eq., 1.88 mmol, 160 mg) in THF (3 mL) over a 15 min period. The reaction mixture was allowed to gradually warn to RT overnight. After 16 h, the reaction was partitioned between 25 mL of EtOAc and 25 mL of H₂O, the water layer back extracted 2 x 25 mL EtOAc and the combined organic layer was dried over Na₂SO₄ and concentrated under reduced pressure to provide **ethyl 2-chloro-6-(cyclopentylamino)-5-nitro-pyrimidine-4-carboxylate (K-19)** as a viscous yellow oil (450 mg, 76%) and the material was used in the next step without further purification. ¹H NMR (CDCI₃): δ 8.50 (bs, 1H), 4.50 (m, 1H), 4.46 (q, 2H) 2.18 (m, 2H), 1.72 (m, 3H), 1.56 (m, 3H), 1.40 (t, 3H); LCMS (APCI) m/e 315.0 (M+H).

### ethyl 6-(cyclopentylamino)-5-nitro-2-(p-tolyl)pyrimidine-4-carboxylate (K-20).

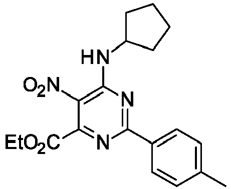

A 40 mL vial was charged with the chloropyrimidine (500 mg, 1.6 mmol), THF (3 mL), water (1.5 mL), p-tolylboronic acid (2 eq., 432 mg, 3.2 mmol), sodium carbonate (4 eq., 674 mg, 6.4 mmol) and then fitted with a stir bar, and septa. The solution was degassed using a stream of nitrogen directly in the solution and an exit needle for 20 min. The reaction mixture was then treated with tetrakis(triphenylphosphine)palladium(0) (0.1 eq., 184 mg, 0.159 mmol) and fitted with a nitrogen balloon and stirred at 60 °C. After 0.5 h, LCMS confirmed complete consumption of the starting material and the major product exhibited the correct MS for the desired product. The reaction mixture was allowed to cool to RT and then partitioned between 20 mL of EtOAc and 20 mL water. The aqueous layer was back extracted 2 x 20 mL EtOAc and the combined organic layer dried over Na₂SO₄. The solvent was removed under reduced pressure and the resulting residue was purified on silica gel (40 g, 0-30% EtOAc/hexanes) to provide **ethyl 6-(cyclopentylamino)-5-nitro-2-(p-tolyl)pyrimidine-4-carboxylate (K-20)** as a yellow solid (400 mg, 68%). ¹H NMR (CDCl₃): δ 8.35 (bs, 1H), 8.23 (d, 2H), 7.18 (d, 2H) 4.65 (m, 1H), 4.41 (q, 2H), 2.32 (s, 3H), 2.21 (m, 2H), 1.65 (m, 4H), 1.47 (m, 2H), 1.32 (t, 3H); LCMS (APCI) m/e 371.1 (M+H).

### ethyl 5-amino-6-(cyclopentylamino)-2-(p-tolyl)pyrimidine-4-carboxylate (K-33).

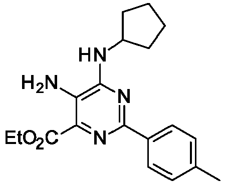

A 40 mL vial was charged with, ethyl 6-(cyclopentylamino)-5-nitro-2-(p-tolyl)pyrimidine-4-carboxylate (520 mg, 1.4 mmol), EtOH (8 mL), water (2 mL), ammonium chloride (1 eq., 1.4 mmol, 75 mg), iron powder (5 eq., 7 mmol, 392 mg), fitted with a stir bar, purged with nitrogen, sealed and stirred at 80 °C. After 16 h, the reaction was cooled to RT and filtered using a syringe filter. The reaction residue was washed 3 x 5 mL of EtOH allowed to settle and filtered. The yellow solution was concentrated under reduced pressure to provide **ethyl 5-amino-6-(cyclopentylamino)-2-(p-tolyl)pyrimidine-4-carboxylate (K-33)** (270 mg, 55%) as a brown powder. The material was pure by LCMS and was used directly in the hydrolysis step. LCMS (APCI) m/e 341.1 (M+H).

### 5-amino-6-(cyclopentylamino)-2-(p-tolyl)pyrimidine-4-carboxylic acid (K-35).

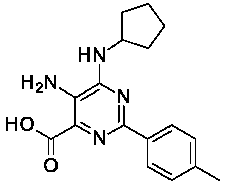

A 20 mL vial was charged with ethyl 5-amino-6-(cyclopentylamino)-2-(p-tolyl)pyrimidine-4-carboxylate (118 mg, 0.9347 mmol), THF (1 mL), methanol (0.5 mL), H₂O (0.5 mL), LiOH-H₂O (1.5 eq., 0.52 mmol, 22 mg), fitted with a stir bar and stirred at RT. After 3 d, crude LCMS confirmed complete consumption of the starting ethyl ester. The reaction mixture was partitioned between 25 mL of water and 25 mL of EtOAc. The water layer was back extracted 2 x 25 mL of EtOAc but the water layer remained yellow with a pH = 8. The water layer was treated with 1 mL of 1 N HCI and a precipitate formed and the pH = 4. The acidic aqueous layer was extracted 3 x 20 mL DCM and the combined organic layer was dried over Na₂SO₄ and concentrated under reduced pressure to provide **5-amino-6-(cyclopentylamino)-2-(p-tolyl)pyrimidine-4-carboxylic acid (K-35)** as a reddish solid (40 mg, 37%). The material was pure by LCMS and was used directly in the amide coupling step. LCMS (APCI) m/e 313.1 (M+H).

### 5-amino-6-(cyclopentylamino)-N-methyl-2-(p-tolyl)pyrimidine-4-carboxamide (K-31).

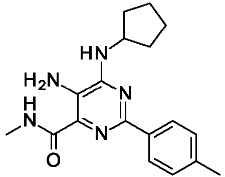

A 4 mL vial was charged with 5-amino-6-(cyclopentylamino)-2-(p-tolyl)pyrimidine-4-carboxylic acid (40 mg, 0.128 mmol), DMF 1 mL), DiPEA (3 eq., 0.384 mmol, 50 mg), 1-[Bis(dimethylamino)methylene]-1H-1,2,3-triazolo[4,5-b]pyridinium 3-oxide hexafluorophosphate (HATU, 1.5 eq., 0.192 mmol, 73 mg) and the reaction was stirred for 20 minutes at RT. The reaction was then treated with methylamine hydrochloride (1.5 eq., 0.192 mmol, 13 mg) and the reaction was stirred at RT. After 16 h, crude LCMS complete consumption of the starting carboxylic acid. The reaction mixture was treated with 2 mL water the afforded **5-amino-6-(cyclopentylamino)-N-methyl-2-(p-tolyl)pyrimidine-4-carboxamide (K-31)** as an offwhite precipitate that was isolated by filtration (40.0 mg, 96%). The material was pure by LCMS and was used directly in the cyclization step. LCMS (APCI) m/e 326.1 (M+H).

### Step 2. Synthesis of Final Compounds

### 9-cyclopentyl-N,8,8-trimethyl-2-(p-tolyl)-5,7-dihydro-4H-purine-6-carboxamide (K-34).

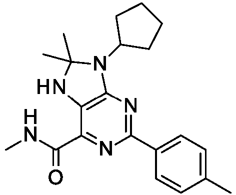

A 20 ml microwave vial was charged with a solution of 5-amino-6-(cyclopentylamino)-N-methyl-2-(p-tolyl)pyrimidine-4-carboxamide in acetone, p-Toluenesulfonic acid monohydrate (0.25 eq., 190.22 MW, 0.031 mmol, 69 mg), glacial acetic acid (1 mL) sealed and heated at 70 °C. After 16 h, the starting material was consumed and a major and minor product with the correct M+H+ was observed in the crude LCMS. The reaction mixture was cooled to RT and partitioned between 15 mL of water and 15 mL of EtOAc. There was a precipitate in the EtOAc layer. The aqueous later was back extracted 2 x 10 mL of EtOAC and the combined organic layer was concentrated under reduced pressure without drying over Na₂SO₄ and concentrated under reduced pressure to provide 45 mg of a yellow solid. The solid was triturated 5 x 3 mL of ether to provide a yellow powder that was dried under reduced pressure to afford **9-cyclopentyl-N,8,8-trimethyl-2-(p-tolyl)-5,7-dihydro-4H-purine-6-carboxamide (K-34)** (23.0 mg, 50.9%). LCMS (APCI) m/e 366.1 (M+H). The ether layer was further purified as described for K-36.

### 8-(cyclopentylamino)-2,2,3-trimethyl-6-(p-tolyl)-1H-pyrimido[5,4-d]pyrimidin-4-one (K-36).

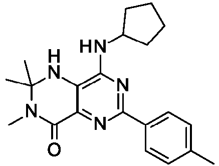

The combined ether layer from K-34 was concentrated under reduced pressure to provide 12 mg of a 1:1 mixture of the major and minor products from K-34. A 5-inch pipette was plugged with cotton and filled 3/4 with silica gel. The silica gel was washed with 3 column volumes of 10% EtOAc/hexanes. The crude residue was dissolved in the smallest amount of DMC possible and loaded onto the column. The material was eluted using 12 column volumes of 10% EtOAc/hexanes via a pipette bulb collecting 2 fractions per column volume, then 8 column volumes of 50% EtOAc/hexanes was flushed through the column, which resulted in the elution of **8-(cyclopentylamino)-2,2,3-trimethyl-6-(p-tolyl)-1H-pyrimido[5,4-d]pyrimidin-4-one (K-36)** as a residue (2 mg, 3.7%). LCMS (APCI) m/e 366.1 (M+H).

### Example 7 - not encompassed by the wording of the claims but are considered as useful for understanding the invention

### Synthesis of Pyridine Ketones

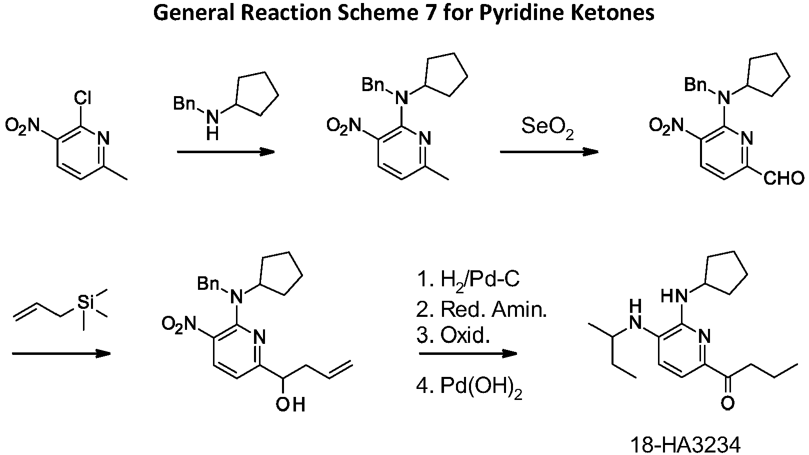

### Synthesis of Pyridine Ketone Analog 1

### N-benzyl-N-cyclopentyl-6-methyl-3-nitro-pyridin-2-amine (K-64).

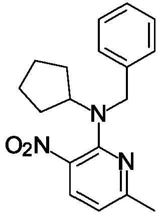

A 40 mL vial was charged with 2-chloro-6-methyl-3-nitro-pyridine (1.0 g, 5.79 mmol), a stir bar, DMF (5 mL, 1 M), DiEA (3 eq., 3.1 mL, 17.4 mmol), N-benzylcyclopentanamine: hydrochloride (1.1 eq., 6.37 mmol, 1.35 g), 80 °C overnight. After 16 h, the starting material had been consumed and the desired product was confirmed in the crude LCMS. The reaction mixture was partitioned between 75 mL of water and 75 mL EtOAc. The water layer was back extracted 3 x 50 mL EtOAc and the combined organic layer was dried over Na₂SO₄. The residue was purified on silica gel (80 g, 0-30% EtOAc/hexanes) to provide 1.2 g of **N-benzyl-N-cyclopentyl-6-methyl-3-nitro-pyridin-2-amine** (85%) as a yellow solid. LCMS (APCI) m/e 312.1 (M+H).

### 6-[benzyl(cyclopentyl)amino]-5-nitro-pyridine-2-carbaldehyde (L-20).

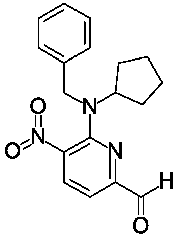

A solution of N-benzyl-N-cyclopentyl-6-methyl-3-nitro-pyridin-2-amine (0.69g, 2.2mmol) in 1,4-dioxane (10mL) was treated with selenium dioxide (0.370g, 3.3mmol, 1.5equiv) and then warmed to 100C. After stirring overnight, LC/MS analysis showed partial conversion to the desired product. Additional selenium dioxide was added and the reaction was progressed an additional 8hrs. LC/MS analysis showed no further progress of the reaction. The mixture was dried onto silica (10g) and purified by flash chromatography (24g silica, 0-50% methylene chloride/hexanes) to afford 6-[benzyl(cyclopentyl)amino]-5-nitro-pyridine-2-carbaldehyde (0.498g, 69%) as a orangish-yellow solid. LCMS (APCI) m/e 326.1 (M+H).

### 1-[6-[benzyl(cyclopentyl)amino]-5-nitro-2-pyridyl]but-3-en-1-ol (L-24).

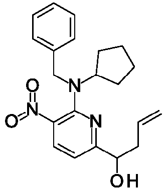

A solution of 6-[benzyl(cyclopentyl)amino]-5-nitro-pyridine-2-carbaldehyde (0.243g, 0.75mmol) in anhydrous dichloromethane (3mL) was cooled to -78 °C and then successively treated with allyltrimethylsilane (0.142mL, 90mmol, 1.2equiv) and then dropwise titanium tetrachloride (40uL, 0.37mmol, 0.5equiv). After 1hr., LC/MS analysis showed complete and clean conversion to the desired product as two peaks, consistent with one being the Ti-complexed and the other as the non-complexed product. The reaction mixture was quenched with satd. aq. ammonium chloride (10mL) and then diluted with methylene chloride (10mL). The layers were separated and the aqueous layer was further extracted with methylene chloride (2 x 15mL). The combined organic extracts were dried (Na₂SO₄) and the solvent removed in vacuo. The residue was purified by flash chromatography (12g silica, 0-100% ethyl acetate/hexanes) to afford the 1-[6-[benzyl(cyclopentyl)amino]-5-nitro-2-pyridyl]but-3-en-1-ol (0.20g, 73%) in two different peaks as a yellow solid. LCMS (APCI) m/e 368.1 (M+H).

### 1-[5-amino-6-[benzyl(cyclopentyl)amino]-2-pyridyl]butan-1-ol (L-26).

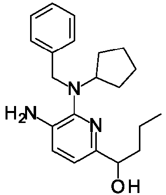

A solution of 1-[6-[benzyl(cyclopentyl)amino]-5-nitro-2-pyridyl]but-3-en-1-ol (0.20g, 0.54mmol) in methanol (2mL) was degassed with nitrogen balloon for 15 min. The reaction mixture was then treated with Pd/C (58mg, 54umol, 0.1equiv) and then charged with hydrogen via balloon. After stirring overnight, LC/MS analysis showed only reduction of the nitro and olefin with the benzyl moiety being retained. The sample was filtered through Celite^{®} and the 1-[5-amino-6-[benzyl(cyclopentyl)amino]-2-pyridyl]butan-1-ol (0.16g, 87%) was carried forward without any further purification. LCMS (APCI) m/e 340.1 (M+H).

### 1-[6-[benzyl(cyclopentyl)amino]-5-(sec-butylamino)-2-pyridyl]butan-1-ol (L-29).

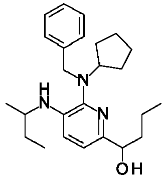

A solution of 1-[5-amino-6-[benzyl(cyclopentyl)amino]-2-pyridyl]butan-1-ol (0.16g, 0.47mmol,) in anhydrous methanol (2mL) was treated with 2-butanone (68mg, 0.94mmol, 2.0equiv) and then acetic acid (57uL, 0.94mmol, 2.0equiv). After 1hr., the reaction was treated with sodium cyanoborohydride (45mg, 0.71mmol, 1.5equiv). After stirring overnight, LC/MS analysis showed conversion to the desired product. In addition, the mixture had two peaks consistent with the formation of the product from acetone and acetaldehyde. The mixture was dissolved onto silica and purified by flash chromatography (12g silica, 0-100% ethyl acetate/hexanes) to afford 1-[6-[benzyl(cyclopentyl)amino]-5-(sec-butylamino)-2-pyridyl]butan-1-ol (44mg, 24%) as a red oil. LCMS (APCI) m/e 396.1 (M+H).

### 1-[6-[benzyl(cyclopentyl)amino]-5-(sec-butylamino)-2-pyridyl]butan-1-one (L-32).

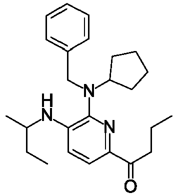

A solution of 1-[6-[benzyl(cyclopentyl)amino]-5-(sec-butylamino)-2-pyridyl]butan-1-ol (45mg, 0.11mmol) in acetone (0.5mL) was treated with Dess-Martin reagent (58mg, 0.14mmol, 1.2equiv). After stirring overnight, LC/MS analysis showed clean conversion to the desired ketone. The reaction mixture was filtered through a plug of silica (1g, ethyl acetate) and the filtrated was dried *in vacuo.* The residue was carried forward without any further purification. LCMS (APCI) m/e 394.1 (M+H).

### 1-[6-(cyclopentylamino)-5-(sec-butylamino)-2-pyridyl]butan-1-one (L-34).

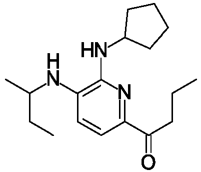

A solution of 1-[6-[benzyl(cyclopentyl)amino]-5-(sec-butylamino)-2-pyridyl]butan-1-ol (44mg, 0.11mmol), in anhydrous methanol (0.5mL) was degassed with N₂ balloon. After 15min., the reaction was treated with 20% palladium hydroxide on carbon (50% wetted, 32mg, 23umol, 0.2equiv). The reaction mixture was then subjected to bubbling H2 via balloon and then left to react. After stirring overnight, LC/MS analysis showed conversion to the desired product and the formation of some bi-products. The sample was filtered through Celite and dried *in vacuo.* The sample was then purified by RP-HPLC to provide 1-[6-(cyclopentylamino)-5-(sec-butylamino)-2-pyridyl]butan-1-one (5.5mg, 16%) as a yellow film. LCMS (APCI) m/e 304.1 (M+H).

### Synthesis of Pyridine Ketone Analog 2

### N-benzyl-N-(3,3-difluorocyclobutyl)-6-methyl-3-nitro-pyridin-2-amine (K-87).

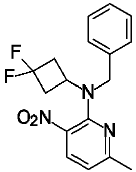

A 40 mL vial was charged with 2-chloro-6-methyl-3-nitro-pyridine (400 mg, 2.32 mmol), a stir bar, DMF (5 mL, 0.5 M), DiPEA (2 eq., 0.8 mL, 4.64 mmol), N-benzyl-3,3-difluoro-cyclobutanamine (2 eq., 4.64 mmol, 0.914 g), and stirred at 80 °C for 72h. Crude LCMS confirmed the reaction was complete. The reaction mixture was partitioned between 50 mL of water and 50 mL of EtOAc. The water layer was back extracted 3 x 50 mL EtOAC and the combined organic layer was dried over Na₂SO₄ and concentrated under reduced pressure. The residue was purified on silica gel (80 g, 0-30% EtOAc/hexanes) to provide 700 mg of N-benzyl-N-(3,3-difluorocyclobutyl)-6-methyl-3-nitro-pyridin-2-amine (90%) as a yellow solid. LCMS (APCI) m/e 334.1 (M+H).

### 6-[benzyl-(3,3-difluorocyclobutyl)amino]-5-nitro-pyridine-2-carbaldehyde (K-91).

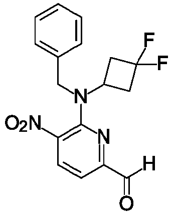

A 40 mL vial was charged with N-benzyl-N-(3,3-difluorocyclobutyl)-6-methyl-3-nitro-pyridin-2-amine(700 mg, 2.10 mmol), dioxane (0.4 M, 5 mL), SeO2 (2 eq., 4.2 mmol, 466mg), purged with nitrogen and stirred at 100 °C. After 16 h, the reaction was complete by crude LCMS and was directly purified on silica gel (40 g, 0-50% EtOAc/hexanes) to provide 540 mg of 6-[benzyl-(3,3-difluorocyclobutyl)amino]-5-nitro-pyridine-2-carbaldehyde (74%) as a yellow oil. LCMS (APCI) m/e 348.1 (M+H).

### 1-[6-[benzyl-(3,3-difluorocyclobutyl)amino]-5-nitro-2-pyridyl]but-3-en-1-ol (K-92).

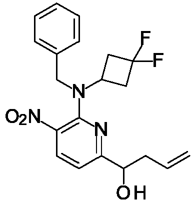

A solution of 6-[benzyl-(3,3-difluorocyclobutyl)amino]-5-nitro-pyridine-2-carbaldehyde (540 mg, 1.55 mmol) in anhydrous dichloromethane (6 mL, 0.25 M) was cooled to -78 °C and then successively treated with allyltrimethylsilane (0.25 mL, 1.86 mmol, 1.2 equiv.) and then dropwise titanium tetrachloride (85 µL, 0.78 mmol, 0.5 equiv). After 2 hr., LC/MS analysis showed complete and clean conversion to the desired product as two peaks, consistent with one being the Ti-complexed and the other as the non-complexed product. The reaction mixture was quenched with satd. aq. ammonium chloride (20mL) and then diluted with methylene chloride (20mL). The layers were separated and the aqueous layer was further extracted with methylene chloride (2 x 30mL). The combined organic extracts were dried (Na₂SO₄) and the solvent removed in vacuo. The residue was purified by flash chromatography (80 g silica, 0-40% ethyl acetate/hexanes) to afford the 11-[6-[benzyl-(3,3-difluorocyclobutyl)amino]-5-nitro-2-pyridyl]but-3-en-1-ol (0.320g, 52%) as a yellow oil. LCMS (APCI) m/e 390.1 (M+H).

### Synthesis of Final Compounds

### 1-[6-[benzyl-(3,3-difluorocyclobutyl)amino]-5-nitro-2-pyridyl]but-3-en-1-one (K-96).

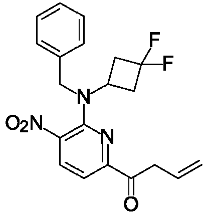

A 40 mL vial was charged with 1-[6-[benzyl-(3,3-difluorocyclobutyl)amino]-5-nitro-2-pyridyl]but-3-en-1-ol (320 mg, 0.822 mmol), DCM (8 mL, 0.1 M), sodium bicarbonate (10eq., 8.22 mmol, 690 mg) and stirred for 5 min. The reaction mixture was then treated with Dess-Martin Periodinane (1.5 eq., 1.23 mmol, 523 mg) and stirred at RT. After 3 h. the reaction was 50% complete. The reaction was treated with Dess-Martin Periodinane (1.5 eq., 1.23 mmol, 523 mg) and stirred at RT overnight. After 16 h, the reaction was complete by crude LCMS. The reaction mixture was partitioned between 20mL DCM and 20mL 1M NaOH (aq); stir for 10 minutes. The aqueous layer was extracted extract with DCM (3 x 20 mL). The combined organic layer was dried over Na₂SO₄ and concentrated under reduced pressure. The residue was purified on silica gel (40 g, 0-30% EtOAc/hexanes) to provide 156 mg of 1-[6-[benzyl-(3,3-difluorocyclobutyl)amino]-5-nitro-2-pyridyl]but-3-en-1-one (318 mg, 49 %) as a yellow solid. LCMS (APCI) m/e 388.1 (M+H).

### 1-[5-amino-6-[(3,3-difluorocyclobutyl)amino]-2-pyridyl]butan-1-one (P-46).

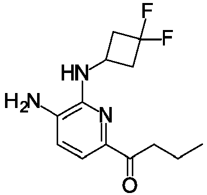

156 mgs of 1-[6-[benzyl-(3,3-difluorocyclobutyl)amino]-5-nitro-2-pyridyl]but-3-en-1-one, (K-96) was dissolved in 10 ml of MeOH. The solution was degassed and flushed with nitrogen. The solution was charged with 50 mg of 20% Pd(OH)2 on carbon followed by a hydrogen balloon. The reaction was stirred at RT for 18 h. LC-MS showed one peak with the mass of the desired product. There was no evidence of any starting material. The reaction was worked up by filtration. The MeOH was evaporated to give 97 mgs (90%) of a brown solid. LC-MS and NMR confirms the structure and purity. LCMS (APCI) m/e 270.1 (M+H); ¹H NMR (d6-DMSO): δ 7.20 (d, 1H), 6.73 (d, 1H), 6.30 (d, 1H), 5.65 (bs, 2H), 4.18 (bs, 1H), 3.03 (m, 2H), 2.91 (t, 2H), 2.48 (m, 2H), 1.59 (q, 2H), 0.951 (t, 3H).

### 1-[5,6-bis[(3,3-difluorocyclobutyl)amino]-2-pyridyl]butan-1-one (P-47).

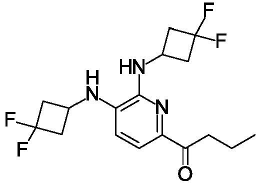

1-[5,6-bis[(3,3-difluorocyclobutyl)amino]-2-pyridyl]butan-1-one (P-47) was prepared using the standard reductive amination conditions (similar to **L-29).** LCMS (APCI) m/e 360.1 (M+H); ¹H NMR (d6-DMSO): δ 7.37 (d, 1H), 6.61 (d, 1H), 6.37 (d, 1H), 6.05 (d, 1H), 4.23 (bs, 1H), 3.86 (bs, 1H), 3.35 (m, 2H), 3.11 (m, 4H), 2.93 (m, 2H), 2.43 (m, 2H), 1.46 (m, 2H),0.960 (t, 3H).

### Example 8

### Synthesis of Heterocycloalkyl Aromatic Compounds

### Intermediate 1: 2-chloro-N-cyclopentyl-6,7-dihydro-5H-pyrimido[4,5-b][1,4]oxazin-4-amine

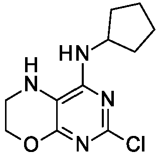

2-Chloro-4-(cyclopentylamino)-5H-pyrimido[4,5-b][1,4]oxazin-6-one (550 mg, 2.05 mmol) was dissolved in dry THF under argon. A 1 M solution of BH₃-THF complex (10.0 equiv) was slowly added. The mixture was stirred for 1h. The mixture was diluted with water. The aqueous phase was extracted with ethyl acetate. The organic layer was dried over Na₂SO₄, filtered, and concentrated under reduced pressure. The product was purified by silica gel chromatography (hexane / ethyl acetate as eluent) to provide the title compound as a solid (350 mg, 67.1%). LC-MS m/z: ES+ [M+H]⁺:255.1; t_{R} = 2.23 min.

### Intermediate 2, step 1: ethyl 8-chloro-1,7-naphthyridine-6-carboxylate

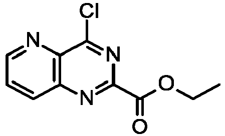

A mixture of ethyl 8-hydroxy-1,7-naphthyridine-6-carboxylate (300 mg) in POCl₃ (7 ml) was stirred for 30 mins at 110°C. When all starting material was converted to the product, the mixture was cooled down, concentrated, then the residue obtained was poured onto crushed ice and stirred for 15 mins. The pH of the aqueous mixture was basified to pH 8 at 0°C by careful addition of aq. sat. sodium carbonate. The product was extracted three times with DCM, the organic phases were combined, washed with brine, dried, filtered then concentrated. The residue obtained was purified by silica-gel column chromatography (12 g) using a gradient 0-50% Ethyl acetate in hexanes. The desired product was isolated in 58% yield (189 mg). LC-MS m/z: ES+ [M+H]⁺:237.1; (B05) t_{R} = 1.99 mins.

### Intermediate 2, step 2: 2,4-dichloropyrido[3,2-d]pyrimidine

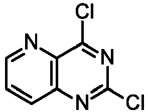

A mixture of pyrido[3,2-d]pyrimidine-2,4-diol (1 g), POCl₃ (10 ml) and PCI₅ (5.11 g) was heated at 120 °C for 12 h under argon. The reaction mixture was cooled down to rt, POCl₃ was evaporated under reduced pressure, and the residue obtained was taken up in DCM. Ice and water was added, the mixture was cooled down to 0 °C, and the pH was adjusted to 8 by slow addition of aq saturated NaHCO₃. The aqueous phase was extracted three times with DCM, the organic phases were combined then washed successively with water and brine. The organic phase was filtered, concentrated, and the residue obtained was purified by silica-gel column chromatography (40 g) using a gradient 0-20% EtOAc in hexanes providing 2,4-dichloropyrido[3,2-d]pyrimidine in 42% yield (510 mg). ¹H NMR (500 MHz, CDCl3) δ 9.15 (dd, J = 4.1, 1.4 Hz, 1H), 8.33 (dd, J = 8.6, 1.4 Hz, 1H), 7.92 (dd, J = 8.6, 4.2 Hz, 1H); LC-MS m/z: ES+ [M+H]⁺:200.1; (B05) t_{R} = 2.0 m.

### Synthesis of Final Compounds

### 4-[(oxolan-3-yl)amino]-2-[(1E)-pent-1-en-1-yl]-5H,6H,7H-pyrimido[4,5-b][1,4]oxazin-6-one (B-603)

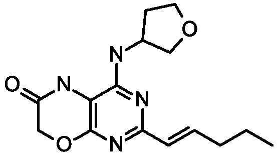

A mixture composed of 2-chloro-4-(tetrahydrofuran-3-ylamino)-5H-pyrimido[4,5-b][1,4]oxazin-6-one (45.0 mg, 0.166 mmol), [(E)-pent-1-enyl]boronic acid (56.8 mg, 0.498 mmol), and Potassium carbonate (68.9 mg, 0.499 mmol) in Toluene (0.800 mL), Ethanol (0.20 ml), and water (0.20 ml) was degassed for 10 mins by bubbling argon. Tetrakis(triphenylphosphine)palladium(0) (38.4 mg, 0.0332 mmol) was added, the vial was sealed then stirred at 100 °C for 16 h. The mixture was cooled down to rt, diluted with ethyl acetate and aq. Sat. NaHCO₃. The organic phase was separated and the aqueous phase was further extracted twice with EtOAc. The organic phases were combined, washed with brine, dried over sodium sulfate, filtered, and concentrated. The residue obtained was purified by silica-gel column chromatography using a gradient 0-10% MeOH in DCM to afford the title compound (23.0 mg, 46 %). LC-MS m/z: ES+ [M+H]+:305.2, LCMS; t_{R} = 4.14 mins (10 mins run).

### N-cyclopentyl-2-pentyl-5H,6H,7H-pyrimido[4,5-b][1,4]thiazin-4-amine (B-601)

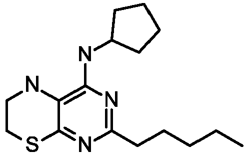

To a solution of 4-(cyclopentylamino)-2-[(E)-pent-1-enyl]-5H-pyrimido[4,5-b][1,4]thiazin-6-one (150 mg, 0.471 mmol) in dry Tetrahydrofuran (10.0 mL) under argon was added BH₃.THF (0.405 g, 4.71 mmol) dropwise. Then the mixture was stirred for 1 h at rt, diluted with water and ethyl acetate, and the organic phase was separated. The organic layer was washed with brine, dried over Na₂SO₄, filtered, concentrated and the residue obtained was purified by silica-gel column chromatography using agradient 0-100% ethyl acetate in hexanes as eluent to afford the title compound (102 mg, 71%). ¹H NMR (500 MHz, CD₃OD) δ 4.38 (p, J = 6.8 Hz, 1H), 3.52 - 3.47 (m, 2H), 3.10 - 3.05 (m, 2H), 2.51 (t, J = 7.5 Hz, 2H), 2.04 (dt, J = 14.1, 6.5 Hz, 2H), 1.74 (d, J = 6.5 Hz, 2H), 1.70 - 1.59 (m, 4H), 1.49 (td, J = 13.7, 7.1 Hz, 2H), 1.38 - 1.25 (m, 4H), 0.89 (t, J = 6.9 Hz, 3H). LC-MS m/z: ES+ [M+H]+:307.2; t_{R} = 3.70 min.

### 4-(cyclopentylamino)-2-[(1E)-pent-1-en-1-yl]-5H,6H,7H-pyrimido[4,5-b][1,4]thiazin-6-one (B-600)

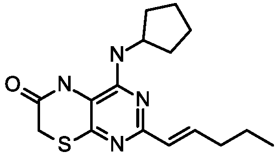

A mixture of 2-chloro-4-(cyclopentylamino)-5H-pyrimido[4,5-b][1,4]thiazin-6-one (250 mg), 1-Pentenylboronic acid (100 mg), and potassium carbonate (364 mg) in Toluene (1.5 ml), Ethanol (0.7 ml), and water (0.7 ml) was degassed for 10 mins by bubbling argon. Pd(PPh₃)₄ was added, the vial was sealed and the mixture was stirred at 100 °C for 12 h. The mixture was cooled down to rt and the product was partitioned between aq. sat. NaHCO₃ and EtOAc. The separated organic layer was separated, washed with brine, dried over Na₂SO₄, filtered, concentrated and the residue obtained was purified by silica-gel column chromatography using a gradient 0-100% EtOAc in Hexane as an eluent to afford the title compound (155 mg, 56%). ¹H NMR (500 MHz,CD₃OD ) δ 7.02 - 6.92 (m, 1H), 6.22 (d, J = 15.4 Hz, 1H), 4.44 (p, *J* = 6.7 Hz, 1H), 3.53 (s, 2H), 2.21 (q, *J* = 7.2 Hz, 2H), 2.08 (dt, J = 12.3, 6.1 Hz, 2H), 1.82 - 1.71 (m, 2H), 1.66 (dd, *J* = 14.9, 7.9 Hz, 2H), 1.53 (tq, *J* = 14.6, 7.2 Hz, 4H), 0.96 (t, *J* = 7.4 Hz, 3H). LC-MS m/z: ES+ [M+H]+:319.2; tR = 4.82 mins.

### 1-{8-[(pyridin-2-yl)amino]-1,2,3,4-tetrahydroquinolin-6-yl}pentan-1-one (B-249)

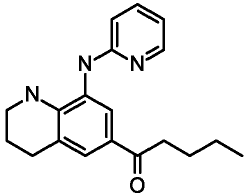

To a solution of 1-[1-benzyl-8-(2-pyridylamino)-3,4-dihydro-2H-quinolin-6-yl]pentan-1-one in anhydrous EtOAc (5 mL) under argon atmosphere and Pd-C was added carefully. The flask, was connected a H2 balloon. The resulting suspension was stirred at room temperature for 6 h and after this time the reaction was stopped filtering the mixture through Celite^{®}. The solvent was evaporated under reduced pressure obtaining a reaction crude that was purified by flash chromatography (0-50% EtOAc/hexane). 1H NMR (500 MHz, CD₃OD) δ 7.95 (d, J = 4.3 Hz, 1H), 7.60 (d, J = 1.8 Hz, 1H), 7.52 (s, 1H), 7.51 - 7.46 (m, 1H), 6.69 - 6.65 (m, 1H), 6.49 (d, J = 8.5 Hz, 1H), 3.37 - 3.33 (m, 2H), 3.29 (dt, J = 2.9, 1.5 Hz, 2H), 2.85 - 2.79 (m, 4H), 1.90 (dt, J = 11.9, 6.1 Hz, 2H), 1.62 (dt, J = 20.8, 7.6 Hz, 2H), 1.41 - 1.32 (m, 2H), 0.92 (t, J = 7.4 Hz, 3H). LC-MS m/z: ES+ [M+H]+:310.2, tR: 3.29 min

### Additional Synthetic Schema for Heterocycloalkyl Aromatics

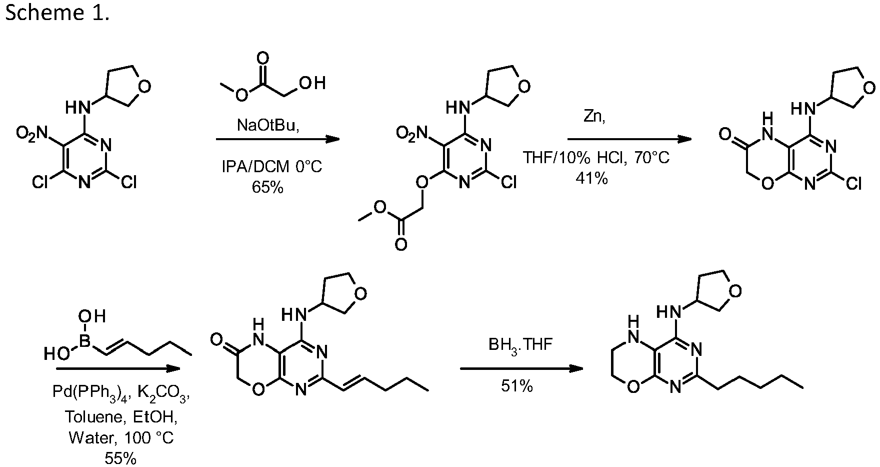

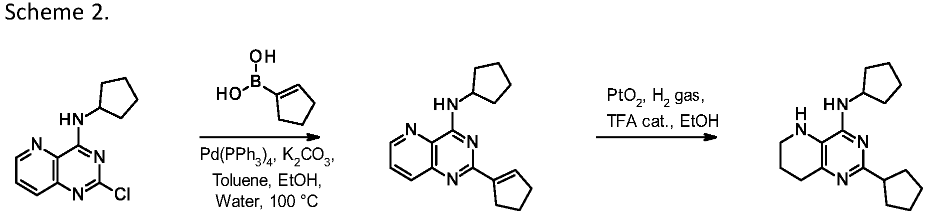

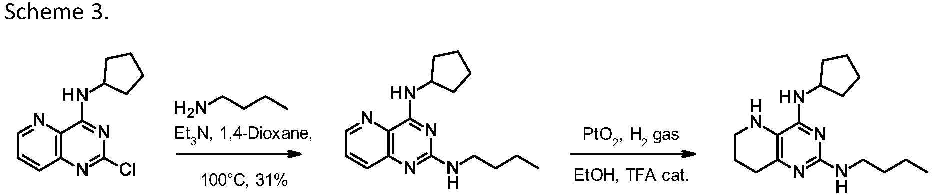

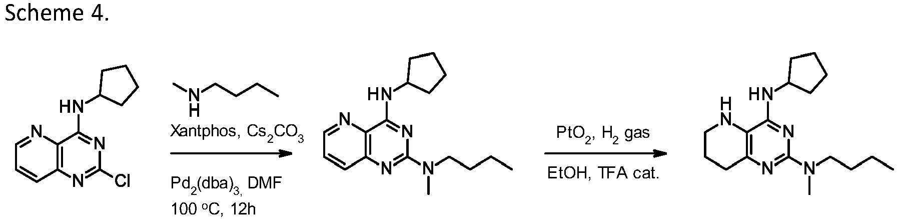

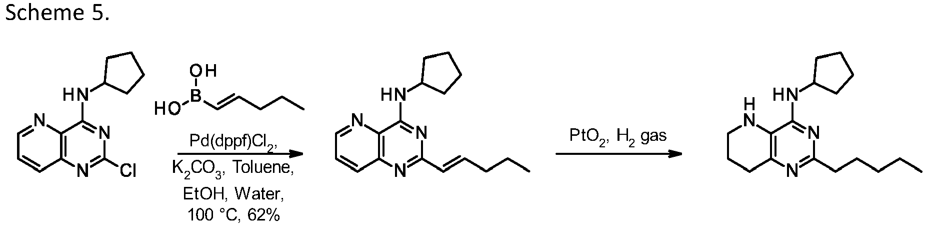

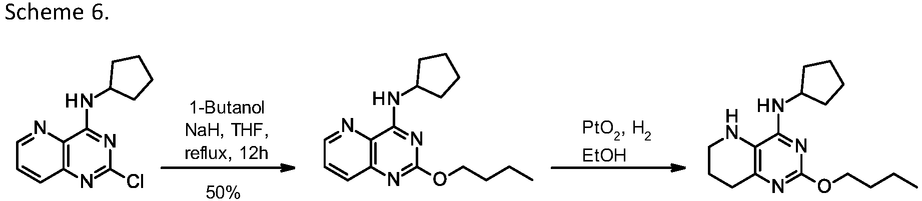

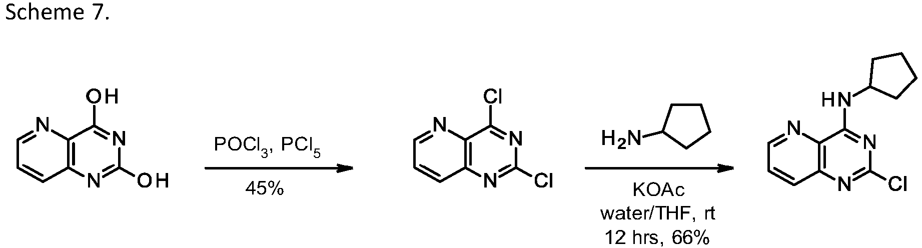

### Example 9 - not encompassed by the wording of the claims but are considered as useful for understanding the invention

### Synthesis of Pyridine Aromatics

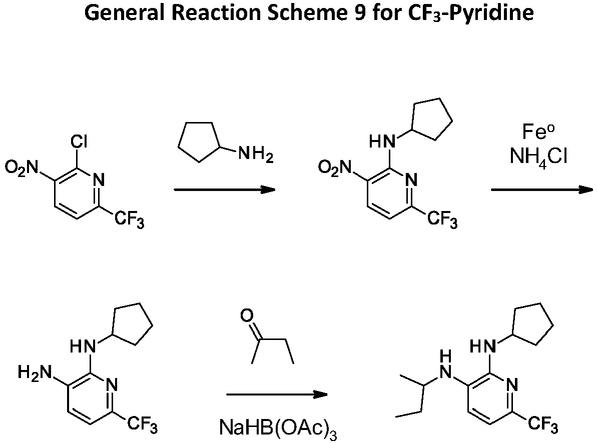

### N-cyclopentyl-3-nitro-6-(trifluoromethyl)pyridin-2-amine (K-61).

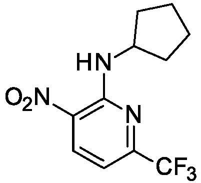

A 40 mL vial was charged with 2-chloro-3-nitro-6-(trifluoromethyl)pyridine (0.5 g, 2.21 mmol), a stir bar, THF (3 mL, 0.5 M), DiEA (2 eq., 0.8 mL, 4.41 mmol), cyclopentanamine in 2 mL of THF (1 eq., 2.21 mmol, 188 mg) and the reaction was stirred at RT. After 2 h, the reaction was complete by LCMS and the reaction was then partitioned between 50 mL of water and 50 mL EtOAc. The water layer was extracted 3 x 30 mL EtOAc and the combined organic layer was dried over Na₂SO₄ and concentrated under reduced pressure to provide 370 mg of an oil (60%) that was >90% pure by LCMS and was used in the next step without further purification. LCMS: (APCI) m/e 276.0 (M+H).

### N-(3-methyltetrahydrofuran-3-yl)-3-nitro-6-(trifluoromethyl)pyridin-2-amine (K-63).

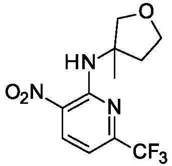

A 40 mL vial was charged with 2-chloro-3-nitro-6-(trifluoromethyl)pyridine (0.5 g, 2.21 mmol), a stir bar, THF (3 mL, 0.5 M), DiEA (2 eq., 0.8 mL, 4.41 mmol), 3-methyltetrahydrofuran-3-amine in 2 mL of THF (1.1 eq., 2.43 mmol, 246 mg) and the reaction was stirred at RT. After 24h, the reaction was ^{~}60% complete an additional 0.5 eq. of the amine was added (1.22 mmol, 123 mg). After 48 h, the reaction was complete by LCMS and the reaction was then partitioned between 50 mL of water and 50 mL EtOAc. The water layer was extracted 3 x 30 mL EtOAc and the combined organic layer was dried over Na₂SO₄ and concentrated under reduced pressure to provide a yellow residue that was purified on silica gel (80 g, 0-30% EtOAc/hexanes) to afford 410 mg of N-(3-methyltetrahydrofuran-3-yl)-3-nitro-6-(trifluoromethyl)pyridin-2-amine as a yellow oil (63%). LCMS: (APCI) m/e 292.0 (M+H).

### N²-cyclopentyl-6-(trifluoromethyl)pyridine-2,3-diamine (K-62).

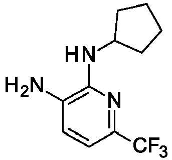

A 20 mL microwave vial was charged with N-cyclopentyl-3-nitro-6-(trifluoromethyl)pyridin-2-amine (370 mg, 1.34 mmol), EtOH (8 mL), water (2 mL), ammonium chloride (1 eq., 1.34 mmol, 72 mg), iron shavings (5 eq., 6.72 mmol, 375 mg), fitted with a stir bar, was bubbled with nitrogen for 10 min, sealed and stirred at 80 °C. After 4 h, the reaction was cooled to RT and filtered using a ISCO sample cartridge with wet Celite^{®} (MeOH) and washed several times with MeOH. The yellow solution dried over Na₂SO₄ and was concentrated under reduced pressure to provide 320 mg (97%) as a yellow oil. The material was pure by LCMS and was used directly in the next step. LCMS: (APCI) m/e 246.1 (M+H).

### N²-(3-methyltetrahydrofuran-3-yl)-6-(trifluoromethyl)pyridine-2,3-diamine (K-66).

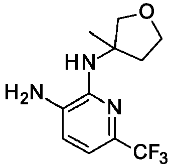

A 20 mL microwave vial was charged with N-(3-methyltetrahydrofuran-3-yl)-3-nitro-6-(trifluoromethyl)pyridin-2-amine (410 mg, 1.41 mmol), EtOH (8 mL), water (2 mL), ammonium chloride (1 eq., 1.41 mmol, 75 mg), iron shavings (5 eq., 7.042 mmol, 393 mg), fitted with a stir bar, was purged with nitrogen, sealed and stirred at 80 °C. After 3 h, the reaction was cooled to RT and filtered using an ISCO sample cartridge with wet Celite^{®} (MeOH) and washed several times with MeOH. The yellow solution dried over Na₂SO₄, filtered and was concentrated under reduced pressure to provide 350 mg (95%) of N²-(3-methyltetrahydrofuran-3-yl)-6-(trifluoromethyl)pyridine-2,3-diamine as an orange film. The material was pure by LCMS and was used directly in the next step. LCMS: (APCI) m/e 262.1 (M+H).

### N²-cyclopentyl-N³-sec-butyl-6-(trifluoromethyl)pyridine-2,3-diamine (K-65).

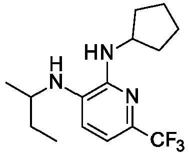

A 40 mL vial was charged with N²-cyclopentyl-6-(trifluoromethyl)pyridine-2,3-diamine (320 g, 1.30 mmol) and a stir bar, 2-butanone (1.1 eq., 103 mg, 1.44mmol), TFA (2 eq., 0.194 mL, 2.61 mmol), and isopropyl acetate(4 mL, 0.3 M) were added. To this was added sodium triacetoxyborohydride (1.2 eq., 332 mg, 1.57 mmol) over ^{~}2 min. The reaction was then allowed to stir at room temperature. After 2 h, the reaction was complete by LCMS and was partitioned between 25 mL of water and 25 mL of EtOAc. The water layer was extracted 3 x 25 mL EtOAc, dried over Na₂SO₄, filtered and concentrated under reduced pressure. The residue was purified on silica gel (40 g, 0-50% EtOAc/hexanes) to provide 276 mg (70%) as a yellow oil. ¹H NMR (CDCl₃): δ 6.97 (d, 1H), 6.67 (d, 1H), 4.31 (t, 1H) 3.96 (bs, 1H), 3.34 (q, 1H), 2.16 (t, 2H), 1.64 (m, 6H), 1.52 (m, 3H), 1.21 (m, 3H), 0.99 (m, 3H); LCMS (APCI) m/e 302.1 (M+H).

### N²-(3-methyltetrahydrofuran-3-yl)-N³-tetrahydrofuran-3-yl-6-(trifluoromethyl)pyridine-2,3-diamine (K-67).

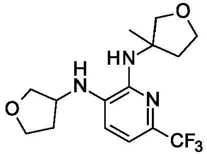

A 40 mL vial was charged with N²-(3-methyltetrahydrofuran-3-yl)-6-(trifluoromethyl)pyridine-2,3-diamine (350 mg, 1.34 mmol) and a stir bar, tetrahydrofuran-3-one (1.1 eq., 127 mg, 1.47 mmol), TFA (2 eq., 0.306 mL, 2.68 mmol), and isopropyl acetate(4 mL, 0.3 M) were added. To this was added sodium triacetoxyborohydride (1.2 eq., 341 mg, 1.61 mmol). The reaction was then allowed to stir at room temperature. After 1 h, the reaction was complete by LCMS and was partitioned between 25 mL of water and 25 mL of EtOAc. The water layer was extracted 3 x 25 mL EtOAc, dried over Na₂SO₄, filtered and concentrated under reduced pressure. The residue was purified on silica gel (40 g, 0-100% EtOAc/hexanes) to provide 270 mg (61%) of N²-(3-methyltetrahydrofuran-3-yl)-N³-tetrahydrofuran-3-yl-6-(trifluoromethyl)pyridine-2,3-diamine as an orange foam. LCMS: (APCI) m/e 232.1 (M+H); ¹H NMR (CDCI3): δ 6.96 (d, 1H), 6.68 (d, 1H), 3.94 (m, 10H) 2.46 (m, 1H), 2.28 (m, 1H), 2.04 (m, 1H), 2.01 (m, 1H), 1.60 (s, 3H), 1.09 (bs, 1H).

### Example 10

### Intermediate Syntheses

### Intermediate 1: 2-chloro-N-cyclopentyl-6,7-dihydro-5H-pyrimido[4,5-b][1,4]oxazin-4-amine

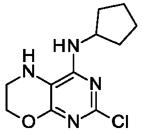

To a solution of 2-chloro-4-(cyclopentylamino)-5H-pyrimido[4,5-b][1,4]oxazin-6-one (550 mg, 2.05 mmol) in dry THF (10 mL) under argon, was slowly added a 1 M solution of BH₃.THF (20.5 mL, 20.5 mmol,) and the reaction mixture was stirred for 1 h at rt. The mixture was diluted with water and the aqueous layer was extracted with EtOAc. The organic layer was dried (Na₂SO₄), filtered then concentrated under reduced pressure. The material was purified by column chromatography on silica gel using a gradient of 0-100% EtOAc in hexane to afford title compound (350 mg, 67%) as a solid. LCMS m/z: ES+ [M+H]⁺ = 255.1; t_{R} = 2.23 min.

### Intermediate 2, Step 1: ethyl 8-chloro-1,7-naphthyridine-6-carboxylate

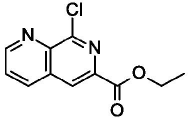

A mixture of ethyl 8-hydroxy-1,7-naphthyridine-6-carboxylate (300 mg, 1.37 mmol) in POCl₃ (7 mL) was stirred for 30 min at 110 °C. The mixture was cooled to rt and concentrated under reduced pressure. The residue was poured onto crushed ice and stirred for 15 min. The pH was adjusted to 8 at 0 °C by careful addition of aqueous saturated aqueous sodium carbonate. The aqueous layer was extracted with DCM, and the combined organic layers were washed with brine, then dried (Na₂SO₄), filtered and concentrated under reduced pressure. The residue obtained was purified by column chromatography on silica gel (12 g) using a gradient of 0-50% EtOAc in hexane to afford title compound (189 mg, 58%) as a solid. ¹H NMR (500 MHz, CDCl₃) δ 9.22 (dd, J = 4.2, 1.6 Hz, 1H), 8.51 (s, 1H), 8.34 (dd, J = 8.3, 1.6 Hz, 1H), 7.77 (dd, J = 8.3, 4.2 Hz, 1H), 4.52 (q, J = 7.1 Hz, 2H), 1.46 (t, J = 7.1 Hz, 3H). LCMS m/z: ES+ [M+H]⁺ = 237.1; (B05) t_{R} = 1.99 min.

### Intermediate 3, step 1: 2,4-dichloropyrido[3,2-d]pyrimidine

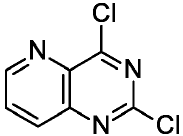

A mixture of pyrido[3,2-d]pyrimidine-2,4-diol (1.0 g, 6.13 mmol), POCl₃ (10.1 mL, 110 mmol) and PCI₅ (5.11 g, 24.5 mmol) was heated at 120 °C for 12 h under argon. The mixture was cooled to rt, and the volatiles were evaporated under reduced pressure. The residue was diluted with DCM, ice and water were added, and the mixture was cooled to 0 °C. The pH was adjusted to 8 by slow addition of aqueous saturated aqueous NaHCO₃. The aqueous layer was extracted with DCM, and the combined organic layers were washed with water and brine. The organic layer was dried (Na₂SO₄), filtered, concentrated under reduced pressure. The material was purified by column chromatography on silica gel (40 g) using a gradient of 0-20% EtOAc in hexane to afford title compound (510 mg, 42%) as a solid. ¹H NMR (500 MHz, CDCl₃) δ 9.15 (dd, J = 4.1, 1.4 Hz, 1H), 8.33 (dd, J = 8.6, 1.4 Hz, 1H), 7.92 (dd, J = 8.6, 4.2 Hz, 1H); LCMS m/z: ES+ [M+H]⁺ = 200.1; t_{R} = 2.00 min.

### Example 11

### Synthesis of B-647

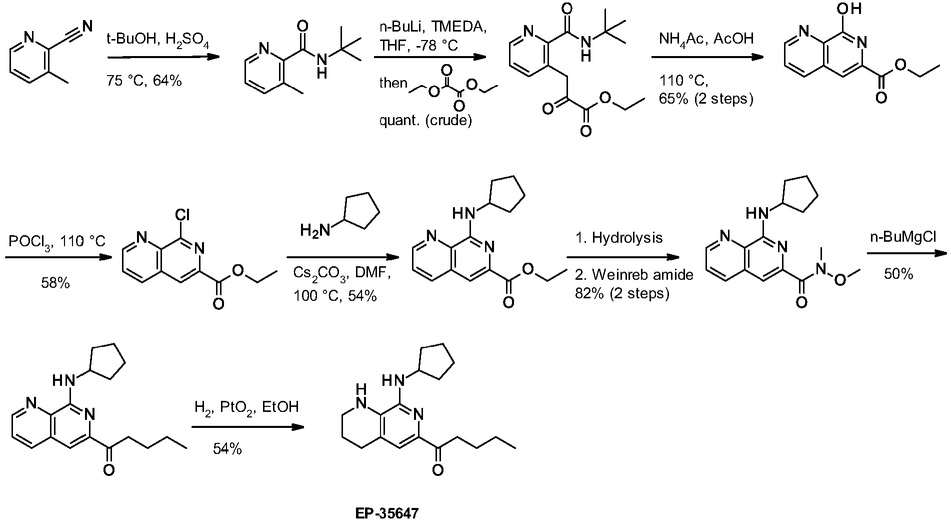

### Step 1: Synthesis of N-tert-butyl-3-methyl-pyridine-2-carboxamide

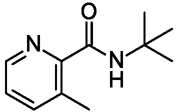

To a suspension of 3-methylpyridine-2-carbonitrile (10 g, 84.6 mmol) in tert-butanol (30 mL) at 70 °C, was added dropwise sulfuric acid (10 mL, 186 mmol). The mixture was stirred for 30 min at 75 °C, diluted with water (150 mL) then cooled to rt. The volatiles were evaporated, and the aqueous layer was extracted with EtOAc (3 x 50 mL). The combined organic layers were dried (Na₂SO₄), filtered and concentrated under reduced pressure. The material was purified by column chromatography on silica gel (120 g) using 0.5% EtOAc in hexanes to afford title compound (10.44 g, 64%) as a solid. ¹H NMR (500 MHz, CDCl₃) δ 8.34 (d, J = 2.2 Hz, 1H), 8.03 (s, 1H), 7.55 (d, J = 7.6 Hz, 1H), 7.26 (dd, J = 7.4, 4.5 Hz, 1H), 2.72 (s, 3H), 1.47 (d, J = 1.9 Hz, 9H). LCMS m/z: ES+ [M+H]⁺ = 193.2; t_{R} = 2.00 min.

### Step 2: Synthesis of Ethyl 3-[2-(tert-butylcarbamoyl)-3-pyridyl]-2-oxo-propanoate

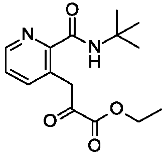

A solution of n-BuLi in hexane (1.6 M in hexane, 23.6 mL, 37.8 mmol) was added dropwise to a stirred solution of N-tert-butyl-3-methylpyridine-2-carboxamide (3.3 g, 17.2 mmol) in THF (48 mL) at -78 °C under argon. N,N,N',N'-tetramethylethylenediamine (2.57 mL, 17.2 mmol) was then added dropwise and the resulting solution was stirred for 30 min at -78 °C. A solution of diethyl oxalate (4.65 mL, 34.3 mmol) in THF (48 mL) was added dropwise to the reaction mixture and the resulting solution was stirred for 1 h at -78 °C. The reaction was diluted with saturated aqueous NH₄Cl and the aqueous layer was extracted with EtOAc (3 x 20 mL). The combined organic layers were dried (Na₂SO₄), filtered, and concentrated under reduced pressure to afford title compound (5.5 g) as a solid, which was used in the next step without further purification. LCMS m/z: ES+ [M+H]⁺ = 293.2; t_{R} = 2.45 min.

### Step 3: Synthesis of Ethyl 8-hydroxy-1,7-naphthyridine-6-carboxylate

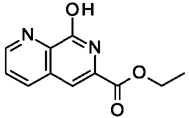

A mixture of ethyl 3-[2-(tert-butylcarbamoyl)-3-pyridyl]-2-oxo-propanoate (5.30 g, 18.1 mmol) and ammonium acetate (2.88 g, 36.3 mmol) in acetic acid (50 mL) was stirred at 110 °C. The mixture was concentrated under vacuum and the material was purified by column chromatography on silica gel using a gradient of 0-4% MeOH in DCM to afford title compound (2.17 g, 55% over 2 steps) as a solid. ¹H NMR (500 MHz, CDCI₃) δ 10.30 (s, 1H), 8.86 (d, J = 3.7 Hz, 1H), 7.98 (d, J = 8.0 Hz, 1H), 7.57 (dd, J = 8.0, 4.4 Hz, 1H), 7.26 (d, J = 5.1 Hz, 1H), 4.36 (q, J = 7.1 Hz, 2H), 1.33 (t, J = 7.1 Hz, 3H). LC-MS m/z: ES+ [M+H]⁺ = 219.1; t_{R} = 1.65 min.

### Step 4: Synthesis of Ethyl 8-chloro-1,7-naphthyridine-6-carboxylate

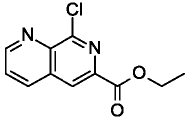

A mixture of ethyl 8-hydroxy-1,7-naphthyridine-6-carboxylate (300 mg, 1.37 mmol) in POCl₃ (7 mL) was stirred for 30 min at 110 °C. The mixture was cooled to rt, concentrated, and then was poured onto crushed ice and stirred for 15 min. The pH of the aqueous mixture was basified to pH 8 at 0 °C by careful addition of saturated aqueous NaHCO₃. The aqueous layer was extracted with DCM (3 x 15 mL), and the combined organic layers were washed with brine, then dried (Na₂SO₄), filtered and concentrated under reduced pressure. The material was purified by column chromatography on silica gel (12 g) using a gradient of 0-50% EtOAc in hexane to afford title compound (189 mg, 58%) as a solid. LCMS m/z: ES+ [M+H]⁺ = 237.1; t_{R} = 1.99 min.

### Step 5: Synthesis of Ethyl 8-(cyclopentylamino)-1,7-naphthyridine-6-carboxylate

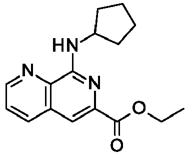

A mixture of ethyl 8-chloro-1,7-naphthyridine-6-carboxylate (350 mg, 1.48 mmol), cyclopentylamine (126 mg, 1.48 mmol) and Cs₂CO₃ (482 mg, 1.48 mmol) in anhydrous DMF (3 mL) under argon, was sealed and the resulting mixture was heated at 100 °C for 12 h. The mixture was cooled to rt, diluted with water (10 mL) and the aqueous layer was extracted with EtOAc (3 x 15 mL). The combined organic layers were dried (Na₂SO₄), filtered, and concentrated under reduced pressure. The material was purified by column chromatography on silica gel using a gradient of 0-100% EtOAc in hexane to afford title compound (255 mg, 54%) as a solid. LCMS m/z: ES+ [M+H]⁺ = 286.2; t_{R} = 2.24 min.

### Step 6: Synthesis of 8-(cyclopenylamino)- N-methoxy-N-methyl-1,7-naphthyridine-6-carboxamide

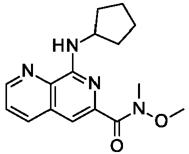

**A)** A solution of ethyl 8-(cyclopentylamino)-1,7-naphthyridine-6-carboxylate (350 mg, 1.22 mmol) in a mixture composed of THF: MeOH: H₂O (15 mL, 3:1:1), was added LiOH (59 mg, 2.45 mmol) and the mixture was stirred at rt for 4 h. The volatiles were evaporated, and the aqueous layer was washed once with EtOAc and then the pH was adjusted to 2 by adding of 1 N HCI. The aqueous layer was extracted with EtOAc (3 x 15 mL), and the combined organic layers were dried (Na₂SO₄), filtered and concentrated under reduced pressure to afford 8-(cyclopentylamino)-1,7-naphthyridine-6-carboxylic acid as a solid, which was used in the next step without further purification. LCMS m/z: ES+ [M+H]⁺ = 258.1; t_{R} = 1.61 min.
**B)** To a solution of above material (200 mg, 0.77 mmol) in anhydrous DMF (10 mL) was successively added N,O-dimethylhydroxylamine, HCI (91 mg, 0.933 mmol), HATU (355 mg, 0.933 mmol) and DIPEA (0.314 mL, 2.31 mmol) and the resulting mixture was stirred for 8 h at rt. The mixture was diluted with EtOAc (15 mL) and 0.1 N HCI (3 mL). The layers were separated, and the aqueous layer was extracted with EtOAc (2 x 15 mL). The combined organic layers were washed with brine, then dried (Na₂SO₄), filtered and concentrated under reduced pressure. The material was purified by column chromatography on silica gel using a gradient 0-60% EtOAc in hexane to afford title compound (190 mg, 82%) as a solid. LCMS m/z: ES+ [M+H]+ = 301.2; t_{R} = 1.95 min.

### Step 7: Synthesis of 1-[8-(cyclopentylamino)-1,7-naphthyridin-6-yl]pentan-1-one

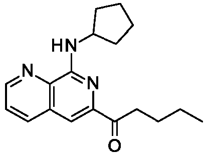

To a solution of 8-(cyclopentylamino)-N-methoxy-N-methyl-1,7-naphthyridine-6-carboxamide (145 mg, 0.483 mmol) in THF (10 mL) was added n-BuMgCl (2 M in THF, 0.3 mL, 0.579 mmol) at 0 °C and the reaction mixture was warmed up to rt and stirred for 2 h. The mixture was quenched with saturated aqueous NH₄CI and then the aqueous layer was extracted with EtOAc (3 x 10 mL). The combined organic layers were dried (Na₂SO₄), filtered and concentrated under reduced pressure. The material was purified by column chromatography on silica gel using a gradient of 0-20% EtOAc in hexane to afford title compound (70 mg, 50%) as an oil. LCMS m/z: ES+ [M+H]⁺ = 298.2, t_{R} = 2.83 min.

### Step 8: Synthesis of 1-[8-(cyclopentylamino)-1,2,3,4-tetrahydro-1,7-naphthyridin-6-yl]pentan-1-one

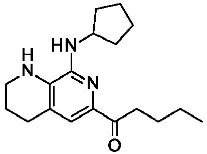

A mixture of 1-[8-(cyclopentylamino)-1,7-naphthyridin-6-yl]pentan-1-one (40 mg, 0.135 mmol) and PtO₂ (15 mg, 0.068 mmol) in anhydrous EtOH (10 mL) and TFA (1 drop) was hydrogenated under hydrogen atmosphere at rt for 6 h. The mixture was filtered through Celite, washed with EtOH (2 × 20 mL) and the filtrate was concentrated under reduced pressure. The material was purified by column chromatography on silica gel using a gradient of 0-100% EtOAc in hexane to afford title compound (22 mg, 54%) as a solid. ¹H NMR (500 MHz,CD₃OD) δ 7.54 (s, 1H), 4.29 (dd, J = 11.9, 5.9 Hz, 1H), 3.54 - 3.46 (m, 2H), 2.94 (t, J = 7.3 Hz, 2H), 2.85 (t, J = 5.8 Hz, 2H), 2.28 - 2.19 (m, 2H), 1.99 - 1.93 (m, 2H), 1.88 - 1.81 (m, 2H), 1.72 (qd, J = 15.1, 7.3 Hz, 6H), 1.40 (dt, J = 13.3, 6.7 Hz, 2H), 0.95 (t, J = 7.3 Hz, 3H). LCMS m/z: ES+ [M+H]+ = 302.3, t_{R} = 3.63 min.

### Example 12

### Synthesis of S-168

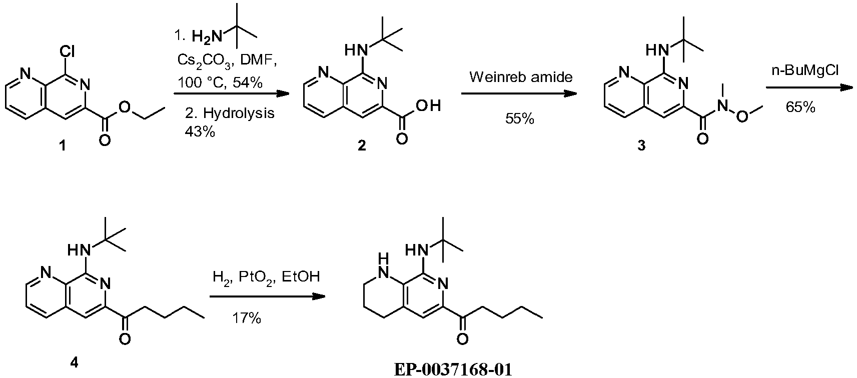

### Step 1: Synthesis of 8-(tert-butylamino)-1,7-naphthyridine-6-carboxylic acid

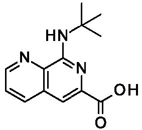

A solution of ethyl 8-chloro-1,7-naphthyridine-6-carboxylate (900 mg, 3.80 mmol), DIPEA (2 mL, 11.68 mmol) and 2-methylpropan-2-amine (3.2 mL, 30.4 mmol) in dry DMF (4.0 mL) and were heated in a microwave at 170 °C for 2 h. Note: the reaction was performed 5 times for a total of 4.5 g. The vials were combined, and the volatiles were evaporated under reduced procedure and then used in next step without further purification. To the above material in a mixture of THF/MeOH/Water (125 mL, 3:1:1) at rt, was added LiOH.H₂O (1.6 g, 38 mmol) and the reaction mixture was stirred at rt for 18 h. The volatiles were evaporated under reduced pressure and then water (250 mL) was added. The mixture was acidified to pH 1 using 1 N aqueous HCI and then the aqueous layer was extracted with CHCI₃ (3 x 150 mL). The combined organic layers were dried (Na₂SO₄), filtered and concentrated under reduced pressure. The material was triturated with ether (25 mL) and the resulting precipitate was filtered, then dried to afford title compound (2 g, 43%) as a solid. LCMS m/z: ES+ [M+H]⁺ = 246.1. t_{R} = 1.63 min.

### Step 2: Synthesis of 8-(tert-butylamino)-N-methoxy-N-methyl-1,7-naphthyridine-6-carboxamide

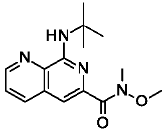

To a solution of 8-(tert-butylamino)-1,7-naphthyridine-6-carboxylic acid (1.00 g, 4.08 mmol) and HATU (1.66 g, 4.36 mmol) in acetonitrile (10 mL) at rt, was added DIPEA (1.40 mL, 8.15 mmol) and then N-methoxymethanamine;hydrochloride (0.437 g, 4.48 mmol) was added and the resulting mixture was stirred for 1 h. The mixture was diluted with EtOAc (50 mL) and 0.1N aqueous HCI (10 mL). The layers were separated, and the aqueous layer was extracted with EtOAc (2 x 25 mL). The combined organic phases were washed with brine, then dried (MgSO₄), filtered and concentrated under reduced pressure. The material was purified by column chromatography on silica gel (40 g) using a gradient 0-60% EtOAc in hexane to afford title compound (652 mg, 56%) as a solid. LCMS m/z: ES+ [M+H]⁺ = 289.5. t_{R} = 2.31 min.

### Step 3: Synthesis of 1-[8-(tert-butylamino)-1,7-naphthyridin-6-yl]pentan-1-one

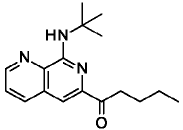

To a solution of 8-(tert-butylamino)-N-methoxy-N-methyl-1,7-naphthyridine-6-carboxamide 3 (452 mg, 1.57 mmol) in THF (10.0 mL) at 0 °C, was added n-BuMgCl (2N in THF, 3.14 mL, 6.27 mmol) and the reaction mixture was stirred at rt for 3 h. The mixture was diluted with water (20 mL) and the pH was adjusted to 3 using 1N aqueous HCI. The aqueous layer was extracted with Et₂O (2 x 25 mL) and the combined organic layers were dried (MgSO₄), filtered and concentrated under reduced pressure to afford title compound 4 (290 mg, 65%) as a solid. ¹H NMR (500 MHz, CDCI₃) δ 8.78 (dd, J = 4.3, 1.4 Hz, 1H), 8.15 (d, J = 5.7 Hz, 1H), 7.68 (s, 1H), 7.57 (dd, J = 8.0, 4.2 Hz, 1H), 7.13 (s, 1H), 3.30 - 3.14 (m, 2H), 1.79 - 1.70 (m, 2H), 1.65 (s, 9H), 1.49 - 1.42 (m, 2H), 0.96 (t, J = 7.4 Hz, 3H). LCMS m/z: ES+ [M+H]⁺ = 287.8. t_{R} = 2.94 min.

### Step 4: Synthesis of 1-[8-(tert-butylamino)-1,2,3,4-tetrahydro-1,7-naphthyridin-6-yl]pentan-1-one

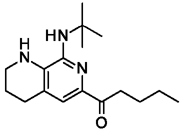

A solution of 1-[8-(tert-butylamino)-1,7-naphthyridin-6-yl]pentan-1-one (180 mg, 0.63 mmol), PtO₂ (14.3 mg, 0.06 mmol) and TFA (0.23 mL, 3.15 mmol) in EtOH (7.00 mL) was hydrogenated under hydrogen atmosphere for 3 h at rt. The mixture was filtered on Celite, washed and concentrated under reduced pressure. The material was purified by column chromatography on silica gel (12 g) using a gradient of 0-100% EtOAc in hexane and was further purified by preparative HPLC (BEH C18 30x100; using 66-86% 10 mM ammonium formate in water and MeCN) to afford title compound (31.0 mg, 17%) as a solid. ¹H NMR (500 MHz, CDCI₃) δ 7.19 (s, 1H), 3.47 (br, 2H), 3.29 (s, 2H), 3.01 (t, J = 7.0 Hz, 2H), 2.64 (t, J = 6.1 Hz, 2H), 1.85 - 1.78 (m, 2H), 1.61 (dd, J = 15.1, 7.7 Hz, 2H), 1.45 (s, 9H), 1.34 (dq, J = 14.7, 7.4 Hz, 2H), 0.86 (t, J = 7.3 Hz, 3H). LCMS m/z: ES+ [M+H]⁺ = 290.3. t_{R} = 1.89 min.

### Example 13

### Synthesis of R-830

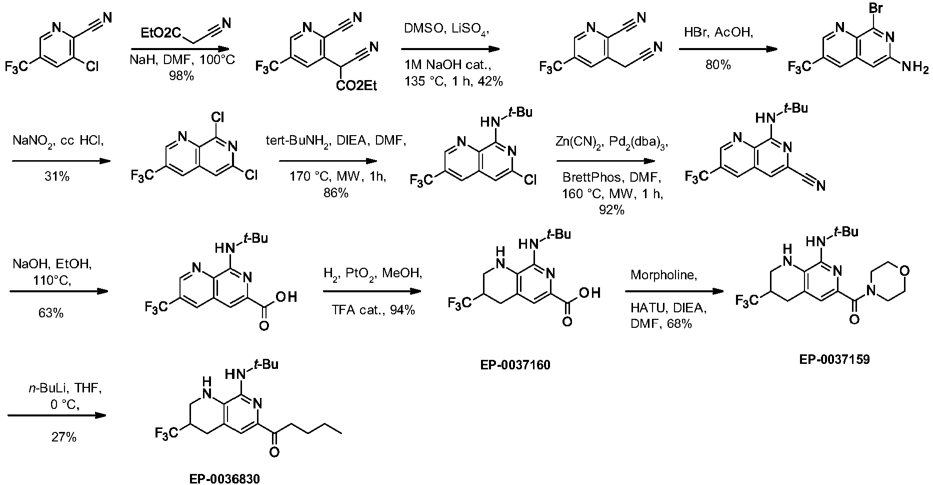

### Step 1: Synthesis of Ethyl 2-cyano-2-[2-cyano-5-(trifluoromethyl)-3-pyridyl]acetate

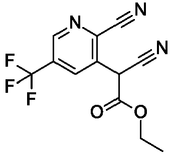

To a mixture of NaH (60.0 %, 9.28 g, 242 mmol) in DMF (130.0 mL) at 0 °C, was added slowly a solution of ethyl 2-cyanoacetate (17.4 mL, 163 mmol) in DMF (20.0 mL) and the mixture was stirred for 15 min. A solution 3-chloro-5-(trifluoromethyl)pyridine-2-carbonitrile (25.0 g, 121 mmol) in DMF (20.0 mL) was slowly added and the reaction mixture was then heated to 70 °C and stirred for 2 h. The mixture was cooled to rt and diluted with EtOAc and 1N aqueous HCI. The layers were separated, and the aqueous layer was extracted with EtOAc. The combined organic layers were washed with brine, then dried (Na₂SO₄), filtered and concentrated under reduced pressure. The material was purified by column chromatography on silica gel (330 g) using a gradient 0-100% EtOAc in hexane to afford title compound (31.0 g, 91 %) as an oil. LCMS m/z: ES- [M-H]- = 282.6; t_{R} = 2.38 min.

### Step 2: Synthesis of 3-(cyanomethyl)-5-(trifluoromethyl)pyridine-2-carbonitrile

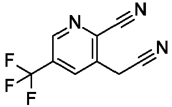

To a solution of ethyl 2-cyano-2-[2-cyano-5-(trifluoromethyl)-3-pyridyl]acetate (31.0 g, 109 mmol) in DMSO (100.0 mL), was added a solution of lithium Sulfate (20.1 g, 183 mmol) and NaOH (0.438 g, 10.9 mmol) in water (28.0 mL) and the resulting mixture was stirred at 135 °C for 1 h. The mixture was cooled to rt diluted with water (100.0 mL). The aqueous layer was extracted with EtOAc (3 x 350.0 mL), and the combined organic layers were washed with brine, then dried (Na₂SO₄), filtered and concentrated under reduced pressure. The material was purified by column chromatography on silica gel using a mixture of DCM/Ethyl acetate/hexane (1:1:6) to afford title compound (9.60 g, 42%) as an oil. ¹H NMR (500 MHz, CDCI₃) δ 8.98 (d, J = 0.8 Hz, 1H), 8.27 (d, J = 1.0 Hz, 1H), 4.14 (s, 2H). LCMS: m/z: ES- [M-H]- = 210.1; t_{R} = 2.21 min.

### Step 3: Synthesis of 8-bromo-3-(trifluoromethyl)-1,7-naphthyridin-6-amine

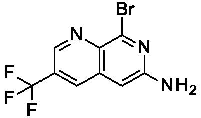

To a solution of 3-(cyanomethyl)-5-(trifluoromethyl)pyridine-2-carbonitrile (4.00 g, 18.9 mmol) in DCM (100.0 mL) at 0 °C, was added dropwise HBr (5.00 M, 11.4 mL, 56.8 mmol, 30% in AcOH) and the reaction mixture was warned to rt and stirred for 30 min. The mixture was diluted with water and stirred vigorously for 15 min. The layers were separated, and the aqueous layer was extracted with DCM (75.0 mL). The combined organics layers were washed with saturated aqueous NaHCO₃ (2 × 60.0 mL), then dried (Na₂SO₄), filtered and concentrated to afford title compound (4.50 g, 82%) as a solid. LCMS m/z: ES+ [M+H]+ = 292.0; t_{R} = 2.41 min.

### Step 4: Synthesis of 6,8-dichloro-3-(trifluoromethyl)-1,7-naphthyridine

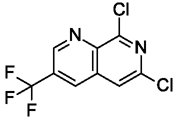

To 8-bromo-3-(trifluoromethyl)-1,7-naphthyridin-6-amine (360 mg, 1.23 mmol) at 0 °C, was slowly added concentrated HCI (12.0 M, 3.39 mL, 40.7 mmol) and the resulting mixture was stirred for 30 min at 0 °C. NaNO₂ (0.170 g, 2.47 mmol) was then added slowly and the mixture was stirred for another 10 min at 0 °C and then for 1.5 h at rt. The mixture was diluted with DCM and water at 0 °C. Saturated aqueous Na₂CO₃ was slowly added and the layers were separated. The aqueous layer was extracted DCM (2 x), and the organic combined layers were washed with saturated aqueous NaHCO₃, then dried (Na₂SO₄), filtered and concentrated under reduced pressure. The material was purified by column chromatography on silica gel using a gradient 0-20% EtOAc in hexane to afford title compound (105 mg, 32%) as a solid. ¹H NMR (500 MHz, CDCI₃) δ 9.26 (d, J = 2.1 Hz, 1H), 8.43 (d, J = 0.8 Hz, 1H), 7.80 (s, 1H). LCMS m/z: ES+ [M+H]+ = 267.0, LCMS; t_{R} = 2.61 min.

### Step 5: Synthesis of N-tert-butyl-6-chloro-3-(trifluoromethyl)-1,7-naphthyridin-8-amine

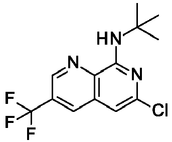

A solution of 6,8-dichloro-3-(trifluoromethyl)-1,7-naphthyridine (2.10 g, 7.86 mmol), tert-butylamine (0.690 g, 9.44 mmol) and DIPEA (1.62 mL, 9.44 mmol) in anhydrous DMF (10.2 mL) was heated at 170 °C in a microwave for 1 h. The mixture was diluted with EtOAc (150.0 mL) and the organic layer was washed with saturated aqueous NaHCO₃ (50.0 mL) and brine (50.0 mL), then dried (Na₂SO₄), filtered and concentrated under reduced pressure. The material was purified by column chromatography on silica gel using a gradient 0-100% DCM in hexane to afford title compound (2.05, 86%) as a solid. ¹H NMR (500 MHz, CDCI₃) δ 8.79 (d, J = 2.0 Hz, 1H), 8.08 (d, J = 1.0 Hz, 1H), 7.06 (bs, 1H), 6.81 (s, 1H), 1.59 (s, 9H). LCMS m/z: ES+ [M+H]+ = 304.1; t_{R} = 3.36 min.

### Step 6: Synthesis of 8-(tert-butylamino)-3-(trifluoromethyl)-1,7-naphthyridine-6-carboxamide

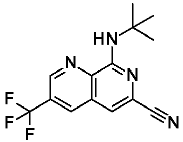

A mixture of N-tert-butyl-6-chloro-3-(trifluoromethyl)-1,7-naphthyridin-8-amine (1.75 g, 5.76 mmol), Zn(CN)₂ (1.27 g, 10.8 mmol), and BrettPhos (0.579 g, 1.08 mmol) in DMF (23.1 mL) was degassed by bubbling argon for 10 min. (Note: the mixture was transferred under argon into 3 microwave vials. Each vial was processed as follows: Pd₂(dba)₃ (0.166 g, 0.180 mmol) was added, the mixture was degassed for 5 min after and then the vial was sealed and heated at 160 °C in a microwave for 1 h). The mixtures were combined, diluted with EtOAc (100.0 mL) and saturated aqueous NaHCO₃ (50.0 mL). The layers were separated, and the organic layer was washed with brine, then dried (Na₂SO₄), filtered and concentrated under reduced pressure. The material was purified by column chromatography on silica gel (40 g) using a gradient 0-100% DCM in hexane to afford title compound (1.65 g, 92%) as a solid. ¹H NMR (500 MHz, CDCI₃) δ 8.97 (d, J = 2.0 Hz, 1H), 8.23 (s, 1H), 7.28 (s, 1H), 7.17 (s, 1H), 1.59 (s, 9H). LCMS m/z: ES+ [M+H]+ = 314.1, LCMS; t_{R} = 2.56 min.

### Step 7: Synthesis of 8-(tert-butylamino)-3-(trifluoromethyl)-1,7-naphthyridine-6-carboxamide

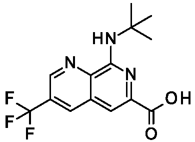

To a solution of 8-(tert-butylamino)-3-(trifluoromethyl)-1,7-naphthyridine-6-carbonitrile (1.54 g, 5.23 mmol) in ethanol (120.0 mL), was added aqueous NaOH (5.00 M, 41.9 mL, 209 mmol) and the reaction mixture was heated at 100 °C for 2 h then cooled to rt. The volatiles were evaporated under reduced pressure and the residue diluted with water and then the aqueous layer was washed with EtOAc. The aqueous layer was acidified to pH 2^{~}4 by slow addition of 1N aqueous HCI (approx. 250 mL). The aqueous layer was extracted with EtOAc (3 x 150.0 mL). The combined organic layers were dried (Na₂SO₄), filtered and concentrated under reduced pressure to afford title compound (1.03 g, 63%) as a solid. ¹H NMR (500 MHz, MeOD) δ 9.07 (d, J = 2.1 Hz, 1H), 8.65 (d, J = 1.0 Hz, 1H), 7.81 (s, 1H), 1.63 (s, 9H). LCMS m/z: ES+ [M+H]+ = 314.1, LCMS; t_{R} = 2.56 min.

### Step 8: Synthesis of 8-(tert-butylamino)-3-(trifluoromethyl)-1,2,3,4-tetrahydro-1,7-naphthyridine-6-carboxylic acid

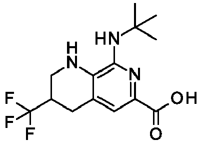

To a solution of 8-(tert-butylamino)-3-(trifluoromethyl)-1,7-naphthyridine-6-carboxylic acid (1030 mg, 3.29 mmol) in ethanol (41.0 mL), was added TFA (0.122 mL, 1.64 mmol). platinum(IV)oxide (0.224 g, 0.986 mmol) was added and the resulting mixture was hydrogenated under hydrogen atmosphere for 10 h. The mixture was filtered on Celite, washed and the filtrate was concentrated under reduced pressure to afford title compound (976 mg, 94%) as a solid, which was used in the next step without further purification. ¹H NMR (500 MHz, MeOD) δ 7.25 (s, 1H), 3.68 (ddd, J = 12.3, 3.5, 2.3 Hz, 1H), 3.35 - 3.27 (m, 1H), 3.03 (ddd, J = 16.9, 5.0, 1.8 Hz, 1H), 2.91 (dd, J = 16.8, 10.3 Hz, 1H), 2.84 - 2.71 (m, 1H), 1.56 (s, 9H). LCMS m/z: ES+ [M+H]+ = 318.2, LCMS; t_{R} = 1.91 min.

### Step 9: Synthesis of 8-(tert-butylamino)-3-(trifluoromethyl)-1,2,3,4-tetrahydro-1,7-naphthyridine-6-carboxylic acid

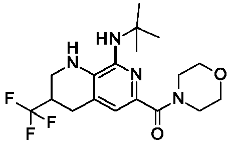

To a solution of 8-(tert-butylamino)-3-(trifluoromethyl)-1,2,3,4-tetrahydro-1,7-naphthyridine-6-carboxylic acid (1.03 g, 3.25 mmol) in DMF (17.6 mL) was added morpholine (0.341 mL, 3.90 mmol), followed by bis(dimethylamino)methylene-(triazolo[4,5-b]pyridin-3-yl) oxonium;hexafluorophosphate (1.48 g, 3.90 mmol) and DIPEA (1.67 mL, 9.74 mmol). The reaction mixture was stirred for 3 h at rt. The mixture was diluted with brine (10 mL), and the aqueous layer was extracted with EtOAc (3 x 50.0 mL). The combined organic layers were washed with saturated aqueous NaHCO₃ (10 mL) and brine (10.0 mL), then dried (Na₂SO₄), filtered, concentrated under reduced pressure. The material was purified by column chromatography on silica gel using a gradient 0-100% EtOAc in hexane to afford title compound (850 mg, 68%) as a solid. ¹H NMR (500 MHz, CDCI₃) δ 6.84 (s, 1H), 4.06 (s, 1H), 3.80 (m, 6H), 3.72 - 3.64 (m, 2H), 3.64 - 3.56 (m, 1H), 3.20 - 3.05 (m, 2H), 2.88 (ddd, J = 16.7, 5.5, 1.7 Hz, 1H), 2.80 (dd, J = 16.7, 10.8 Hz, 1H), 2.62 - 2.43 (m, 1H), 1.44 (s, 9H). LCMS m/z: ES+ [M+H]+ = 387.2, LCMS; t_{R} = 2.48 min.

### Step 10: Synthesis of 3-[8-(tert-butylamino)-3-(trifluoromethyl)-1,2,3,4-tetrahydro-1,7-naphthyridin-6-yl]pentan-1-one

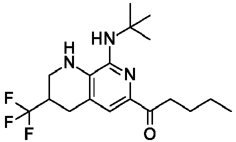

To a solution of [8-(tert-butylamino)-3-(trifluoromethyl)-1,2,3,4-tetrahydro-1,7-naphthyridin-6-yl]-morpholino-methanone (39.0 mg, 0.101 mmol) in anhydrous THF (0.767 mL) at 0 °C, was added n-BuLi (2.50 M in hexane, 0.121 mL, 0.303 mmol). The mixture was stirred for 15 min at 0 °C and then warmed to rt and stirred for 1 h. The mixture was cooled to 0 °C then diluted with water (0.3 mL), EtOAc (1.0 mL) and 1M aqueous HCI (0.2 mL). The layers were separated, and the organic layer was dried (Na₂SO₄), filtered and concentrated reduced pressure. The material was purified by reverse phase chromatography on C18 (5.5 g) using a gradient 10-100% acetonitrile in water (contains 0.1% formic acid) and was further purified by column chromatography on silica gel using a gradient 0-100% EtOAc in hexane to afford title compound (9.5 mg, 27%) as a solid. ¹H NMR (500 MHz, CDCI₃) δ 7.28 (s, 1H), 3.86 (bs, 1H), 3.65 (d, J = 12.0 Hz, 1H), 3.43 (bs, 1H), 3.22 (t, J = 11.4 Hz, 1H), 3.12 - 3.05 (m, 2H), 2.92 (ddd, J = 16.6, 5.2, 1.7 Hz, 1H), 2.84 (dd, J = 16.6, 11.0 Hz, 1H), 2.62 - 2.51 (m, 1H), 1.73 - 1.65 (m, 2H), 1.52 (s, 9H), 1.45 - 1.36 (m, 2H), 0.93 (t, J = 7.4 Hz, 3H). LCMS m/z: ES+ [M+H]+ = 358.2, LCMS; t_{R} = 6.20 mins (10 mins run).

### Example 14

### Synthesis of R-812

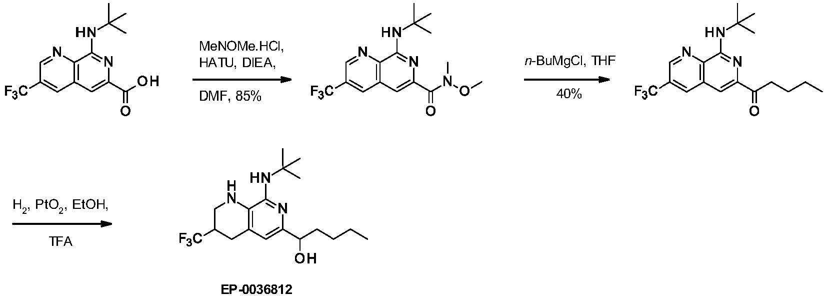

### Step 1: 8-(tert-butylamino)-N-methoxy-N-methyl-3-(trifluoromethyl)-1,7-naphthyridine-6-carboxamide

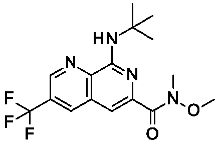

To a solution of 8-(tert-butylamino)-3-(trifluoromethyl)-1,7-naphthyridine-6-carboxylic acid (0.880 g, 2.81 mmol) in anhydrous DMF (15.0 mL) was successively added N,O-dimethylhydroxylamine hydrochloride (0.329 g, 3.37 mmol), HATU (674 mg, 1.77 mmol) and DIPEA (0.77 mL, 4.43 mmol). The mixture was stirred for 8 h at rt then diluted with EtOAc (100 mL) and 0.1N aqueous HCI (6 mL). The layers were separated, and the aqueous layer was extracted with EtOAc (2 x 50 mL). The combined organic layers were washed with brine, then dried (Na₂SO₄), filtered and concentrated under reduced pressure. The material was purified by column chromatography on silica gel using a gradient 0-60% EtOAc in hexane to afford title compound (850 mg, 85%) as a solid. LCMS m/z: ES+ [M+H]+ = 357.2; t_{R} = 2.69 min.

### Step 2: Synthesis of 1-[8-(tert-butylamino)-3-(trifluoromethyl)-1,7-aphthyridin-6-yl]pentan-1-one

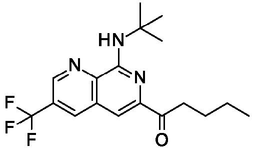

To a solution of 8-(tert-butylamino)-N-methoxy-N-methyl-3-(trifluoromethyl)-1,7-naphthyridine-6-carboxamide (850 mg, 2.39 mmol) in THF (15.0 mL) at -78 °C, was added n-butyl magnesium chloride (2.00 M, 4.77 mL, 9.54 mmol) and the reaction mixture was stirred -78 °C for 5 min, and then warmed to rt and stirred for 1 h. The reaction was diluted with saturated aqueous NH₄CI (50 mL) at -78°C, warmed to rt and the aqueous layer was extracted with EtOAc (3 x 50 mL). The combined organic layers were dried (Na₂SO₄), filtered and concentrated under reduced pressure. The material was purified by column chromatography on silica gel using a gradient of 0-100% DCM in hexane to afford title compound (350 mg, 42%) as a solid. LCMS m/z: ES+ [M+H]+ = 354.2; t_{R} = 3.43 min.

### Step 3: Synthesis of 1-[8-(tert-butylamino)-3-(trifluoromethyl)-1,2,3,4-tetrahydro-1,7-naphthyridin-6-yl]pentan-1-ol

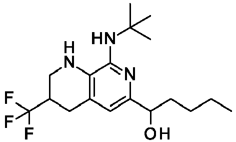

A solution of 1-[8-(tert-butylamino)-3-(trifluoromethyl)-1,7-naphthyridin-6-yl]pentan-1-one (60.0 mg, 0.170 mmol) in ethanol (3.0 mL) was added platinum(IV)oxide (0.0416 g, 0.170 mmol) and 3 drops of TFA and the reaction mixture was hydrogenated under hydrogen atmosphere for 50 min. The mixture was diluted with EtOAc and filtered through Celite. The volatiles were evaporated under reduced pressure and the material was purified by reverse phase chromatography on C18 using 10-100% MeCN in water to afford title compound (61 mg, 25%) as a solid. ¹H NMR (300 MHz, MeOD) δ 6.39 (s, 1H), 4.48 (dd, J = 7.1, 5.4 Hz, 1H), 3.59 (ddd, J = 12.3, 3.3, 1.9 Hz, 1H), 3.11 (ddd, J = 12.2, 10.3, 1.8 Hz, 1H), 2.88 (ddd, J = 17.1, 6.1, 1.9 Hz, 1H), 2.80 (dd, J = 16.6, 10.4 Hz, 1H), 2.72 - 2.52 (m, 1H), 1.97 - 1.80 (m, 1H), 1.77 - 1.59 (m, 1H), 1.50 (d, J = 2.2 Hz, 9H), 1.44 - 1.25 (m, 5H), 0.99 - 0.84 (m, 3H). LCMS: m/z: ES+ [M+H]+ = 360.3; t_{R} = 2.37 min.

### Example 15

### Synthesis of B-917

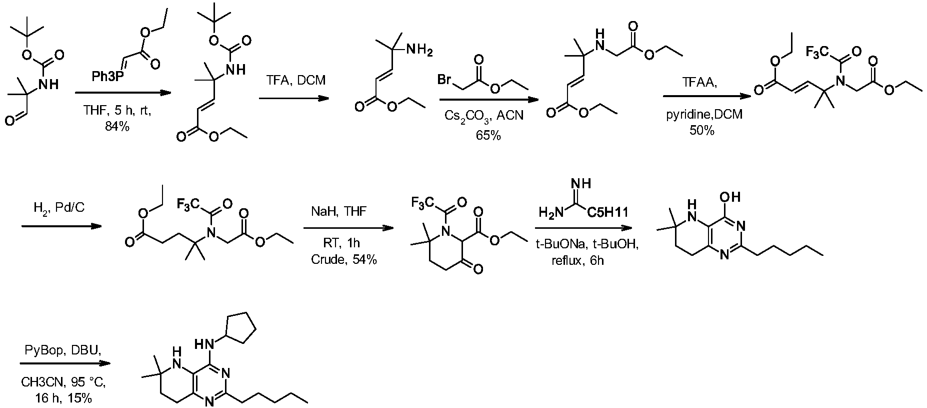

### Step 1: Synthesis of Ethyl (E)-4-(tert-butoxycarbonylamino)-4-methyl-pent-2-enoate

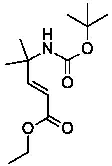

To a solution of *tert*-butyl N-(1,1-dimethyl-2-oxo-ethyl)carbamate (150 mg, 0.80 mmol) in anhydrous THF (2.5 mL) under argon at rt, was added triphenylcarbethoxy methylenephosphorane (558 mg, 1.60 mmol) in one portion and the reaction mixture was stirred for 5 h at rt. The mixture was diluted with saturated aqueous NH₄CI and the aqueous layer was extracted with EtOAc (3 x). The combined organic layers were washed with brine, then dried (Na₂SO₄), filtered, and concentrated under reduced pressure. The material was purified by reverse phase chromatography on C18 using a gradient 10-100% MeCN in water (contains 0.1% formic acid) to afford title compound (173 mg, 84%) as an oil. ¹H NMR (500 MHz, CDCI₃) δ 6.92 (d, J = 15.9 Hz, 1H), 5.75 (d, J = 15.9 Hz, 1H), 4.80 (s, 1H), 4.10 (dd, J = 5.0, 10.0 Hz, 2H), 1.33 (s, 9H), 1.32 (s, 6H), 1.19 (t, J = 7.1 Hz, 3H; LCMS m/z: ES+ [M+Na]⁺ 280.1; t_{R} = 2.42 min.

### Step 2: Synthesis of Ethyl (E)-4-amino-4-methyl-pent-2-enoate;2,2,2-trifluoroacetic acid

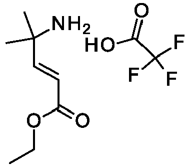

To a solution of ethyl (E)-4-(tert-butoxycarbonylamino)-4-methyl-pent-2-enoate (510 mg, 1.98 mmol) in DCM (5.0 mL) was added TFA (637 µlL, 9.91 mmol), and the mixture was stirred for 3 h at rt. The volatiles were concentrated under reduced pressure to afford title compound (538 mg) as a solid, which was used in the next step without further purification. ¹H NMR (500 MHz, CDCI₃) δ 7.95 (s, 2H), 6.99 (d, J = 16.1 Hz, 1H), 6.06 (d, J = 16.1 Hz, 1H), 4.23 (q, J = 7.2 Hz, 2H), 1.57 (s, 6H), 1.31 (dd, J = 11.7, 4.5 Hz, 3H). LCMS (ES+): m/z [M+H]⁺ 158.5; t_{R} = 0.86 min.

### Step 3: Synthesis of Ethyl (E)-4-[(2-ethoxy-2-oxo-ethyl)amino]-4-methyl-pent-2-enoate

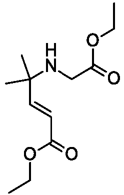

To a solution of ethyl (E)-4-amino-4-methyl-pent-2-enoate;2,2,2-trifluoroacetic acid (4.14 g; 8.06 mmol) in anhydrous acetonitrile (25.0 mL) under argon at rt, was added Cs₂CO₃ (9.19 g, 1.6 mmol, 28.2mmol) followed by ethyl 2-bromoacetate (1.34 mL, 12.1 mmol) and the resulting mixture was stirred for 16 h at rt. The mixture was filtered, and the filtrate was concentrated under reduced pressure. The material was purified by column chromatography on silica gel using a gradient 0-100% EtOAc in hexane to afford title compound (0.874 g, 45%) as an oil. LCMS (ES+): m/z [M+H]⁺ 244.7; t_{R} = 1.44 min.

### Step 4: Synthesis of Ethyl (E)-4-[(2-ethoxy-2-oxo-ethyl)-(2,2,2-trifluoroacetyl)amino]-4-methyl-pent-2-enoate

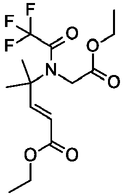

To a solution of ethyl (E)-4-[(2-ethoxy-2-oxo-ethyl)amino]-4-methyl-pent-2-enoate (0.878 g, 3.61 mmol)) in anhydrous DCM (3.0 mL) under argon at 0 °C, was added anhydrous pyridine (3.81 mL, 72.2 mmol) followed by Trifluoroacetic anhydride (0.752 mL, 5.41 mmol) and the reaction mixture was stirred for 30 min at 0 °C. The mixture was diluted with water, and the aqueous layer was extracted with EtOAc (3 x 150 mL). The organic combined layers were washed with 1M aqueous HCI and brine, then dried (Na₂SO₄), filtered, and concentrated under reduced pressure. The material was purified by column chromatography on silica gel using a gradient of 0-50% EtOAc in hexane to afford title compound (60%, 733 mg) as an oil. ¹H NMR (500 MHz, CDCI₃) δ 7.10 (d, J = 16.0 Hz, 1H), 5.89 (d, J = 16.0 Hz, 1H), 4.29 - 4.16 (m, 6H), 1.54 (s, 6H), 1.33 - 1.26 (m, 6H). LCMS (ES+): m/z [M+H]⁺ 339.7; t_{R} = 2.62 min.

### Step 5: Synthesis of Ethyl 4-[(2-ethoxy-2-oxo-ethyl)-(2,2,2-trifluoroacetyl)amino]-4-methyl-pentanoate

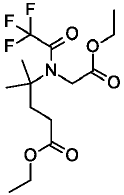

A mixture of ethyl (E)-4-[(2-ethoxy-2-oxo-ethyl)-(2,2,2-trifluoroacetyl)amino]-4-methyl-pent-2-enoate (0.743 g, 2.19 mmol) and Pd/C (0.233 g, 0.219 mmol) in EtOAc (5.0 mL) was hydrogenated under hydrogen atmosphere for 2h at rt. The mixture was filtered through Celite, was washed with EtOAc and the filtrate was concentrated under reduced pressure to afford title compound (0.787 g, 100%) as an oil, which was used in the next without further purification. ¹H NMR (500 MHz, CDCI₃) δ 4.23 (q, J = 7.2 Hz, 4H), 4.14 - 4.08 (m, 4H), 2.25 (s, 4H), 1.31 - 1.22 (m, 12H). LCMS (ES+): m/z [M+H]⁺ 342.8; t_{R} = 2.70 min.

### Step 6: Synthesis of Ethyl 6,6-dimethyl-3-oxo-1-(2,2,2-trifluoroacetyl)piperidine-2-carboxylate

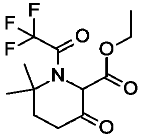

To a solution of ethyl 4-[(2-ethoxy-2-oxo-ethyl)-(2,2,2-trifluoroacetyl)amino]-4-methyl-pentanoate (0.454 g, 1.33 mmol) in anhydrous THF (5.0 mL) at 0 °C, was added NaH (61.2 mg, 1.60 mmol) and the reaction mixture was warmed to rt and stirred for 1 h. The mixture was diluted with water (5.0 mL), and the aqueous layer was extracted with EtOAc (3 x 15.0 mL). The combined organic layers were washed with water and brine, then dried (Na₂SO₄), filtered, and concentrated under reduced pressure. The material was purified by column chromatography in silica gel using a gradient of 0-50% EtOAc in hexane to afford title compound (0.115 g, 29%) as a solid. LCMS (ES+): m/z [M+H]⁺ 296.1; t_{R} = 2.71 min.

### Step 7: Synthesis of 6,6-dimethyl-2-pentyl-7,8-dihydro-5H-pyrido[3,2-d]pyrimidin-4-ol

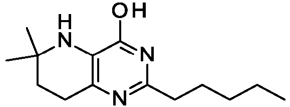

To a solution of ethyl 6,6-dimethyl-3-oxo-1-(2,2,2-trifluoroacetyl)piperidine-2-carboxylate (255 mg, 0.86 mmol) in MeOH (2.0 mL) was added, hexanamidine;hydrochloride (195 mg, 1.3 mmol) and the reaction mixture was heated at 110 °C for 48 h. The mixture was concentrated, and the material was purified by column chromatography on silica gel using a gradient 0-20% MeOH in DCM to afford title compound (23 mg, 11%) as a solid. ¹H NMR (500 MHz, CDCl₃) δ 3.83 (s, 2H), 2.60 (t, J = 6.5 Hz, 2H), 2.41 (s, 2H), 1.77 - 1.70 (m, 2H), 1.25 (s, 6H), 1.20 (s, 6H), 0.90 (t, J = 6.9 Hz, 3H). LCMS (ES+): m/z [M+H]⁺ 250.2; t_{R} = 1.67 min.

### Step 8: Synthesis of N-cyclopentyl-6,6-dimethyl-2-pentyl-5H,6H,7H,8H-pyrido[3,2-d]pyrimidin-4-amine

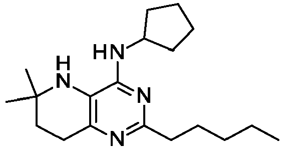

To a solution of 4-chloro-6,6-dimethyl-2-pentyl-7,8-dihydro-5H-pyrido[3,2-d]pyrimidine (11.0 mg, 0.041 mmol) and cyclopentanamine (16.0 µL, 0.16 mmol) in anhydrous n-butanol (1.0 mL), was added DIPEA (28.0 µL, 0.16 mmol) and the reaction mixture was heated to 95 °C for 16 h. The mixture was concentrated under reduced pressure, and the material was purified by reverse phase chromatography on C18 using a gradient 10-60% acetonitrile in water (contains 0.1% formic acid) to afford title compound (1.8 mg, 14%) as a solid. ¹H NMR (500 MHz, CD₃OD) δ 4.51 (dd, J = 14.8, 7.4 Hz, 1H), 4.01 (s, 2H), 2.66 (t, J = 7.6 Hz, 2H), 2.52 (s, 2H), 2.11 - 2.02 (m, 2H), 1.83 - 1.75 (m, 4H), 1.69 - 1.61 (m, 2H), 1.58 - 1.51 (m, 2H), 1.40 (s, 6H), 1.38 - 1.34 (m, 4H), 0.92 (t, J = 6.7 Hz, 3H). LCMS (ES+): m/z [M+H]⁺ 317.3; t_{R} = 3.31 min.

### Example B-647, Step x: 1-[8-(cyclopentylamino)-1,2,3,4-tetrahydro-1,7-naphthyridin-6-yl]pentan-1-one

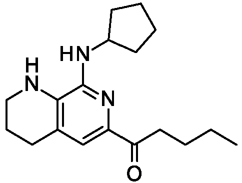

To a suspension of 1-[8-(cyclopentylamino)-1,7-naphthyridin-6-yl]pentan-1-one (40.0 mg, 0.135 mmol) in anhydrous EtOH (10.0 mL) under argon, was added platinum oxide (0.0189 g, 0.161 mmol) and 1 drop of TFA, was hydrogenated under hydrogen atmosphere for 6 h at rt. The mixture was filtered on Celite, and the filtrate was evaporated under reduced pressure. The material was purified by column chromatography on silica gel using a gradient of 0-50% EtOAc in hexane to afford title compound (22 mg, 54%) as a solid. ¹H NMR (300 MHz, MeOD) δ 7.55 (s, 1H), 4.35 - 4.25 (m, 1H), 3.54 - 3.48 (m, 2H), 2.96 (t, J = 7.4 Hz, 2H), 2.87 (t, J = 6.2 Hz, 2H), 2.32 - 2.18 (m, 2H), 2.02 - 1.93 (m, 2H), 1.91 - 1.65 (m, 7H), 1.43 (ddt, J = 14.6, 9.5, 6.4 Hz, 2H), 0.97 (t, J = 7.3 Hz, 3H). LCMS m/z: ES+ [M+H]+ = 302.3, LCMS; t_{R} = 3.66 min.

### Example B-626, Step x: 1-[8-(cyclopentylamino)-1,2,3,4-tetrahydro-1,7-naphthyridin-6-yl]pentan-1-one

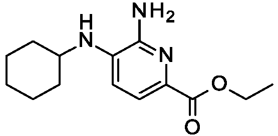

B-626, FER-1, was purchased from Combi Blocks, San Diego, WZ9339.

### Example 16

### Synthesis of B-604

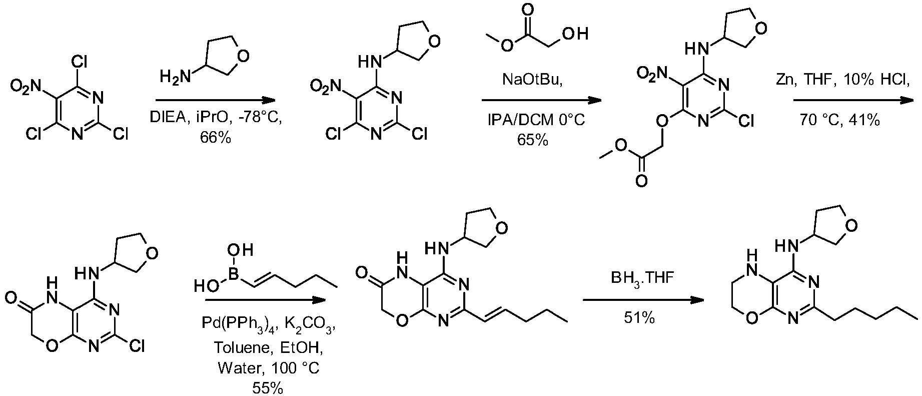

### Step 1: Synthesis of 2,6-dichloro-5-nitro-N-tetrahydrofuran-3-yl-pyrimidin-4-amine

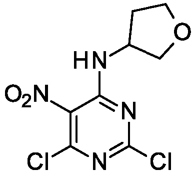

To a solution of 2,4,6-trichloro-5-nitro-pyrimidine (100 mg, 0.438 mmol) in iPrOH (2.0 mL) at -78 °C under argon, was added a solution of tetrahydrofuran-3-amine (38.1 mg, 0.438 mmol) in iPrOH (1.0 mL) over 15 min and the reaction mixture was stirred at 30 min at -78 °C and then warmed to rt and stirred for 1 h. DIPEA (0.150 mL, 0.876 mmol) was then added and the resulting mixture was stirred for 2 h at rt. The volatiles were evaporated under reduced pressure and the material was purified by column chromatography on silica gel using a gradient 0-100% EtOAc in hexane to afford title compound (80 mg, 66%) as a solid. ¹H NMR (500 MHz, CDCl₃) δ 7.87 (s, 1H), 4.95 - 4.77 (m, 1H), 4.02 (dd, J = 15.6, 7.7 Hz, 1H), 3.96 (dd, J = 9.8, 5.4 Hz, 1H), 3.86 (td, J = 8.6, 6.0 Hz, 1H), 3.79 (dd, J = 9.8, 2.3 Hz, 1H), 2.43 (td, J = 14.6, 7.5 Hz, 1H), 1.98 - 1.89 (m, 1H). LCMS m/z: ES+ [M+H]+ = 279.5; t_{R} = 2.27 min.

### Step 2: Synthesis of Methyl 2-[2-chloro-5-nitro-6-(tetrahydrofuran-3-ylamino)pyrimidin-4-yl]oxyacetate

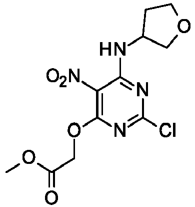

To a solution of 2,6-dichloro-5-nitro-N-tetrahydrofuran-3-yl-pyrimidin-4-amine (0.205 g, 0.734 mmol) and methyl 2-hydroxyacetate (99 mg, 1.10 mmol) in iPrOH (8.0 mL) and DCM (2.0 mL) under argon at 0 °C, was added sodium tert-butoxide (2.00 M, 0.404 mL, 8.08 mmol) in THF (0.50 mL) and the reaction mixture was stirred for 1 h at rt. The mixture was diluted with water and the aqueous layer extracted with DCM (3 x 20.0 mL). The combined organic layers were dried (Na₂SO₄), filtered, and concentrated under reduced pressure. The material was purified by column chromatography on silica gel using a gradient of 0-100% EtOAc in hexane to afford title compound (158 mg, 64%) as a solid. LCMS m/z: ES+ [M+H]+ = 331.1, t_{R}: 2.27 min.

### Step 3: Synthesis of 2-Chloro-4-(tetrahydrofuran-3-ylamino)-5H-pyrimido[4,5-b][1,4]oxazin-6-one

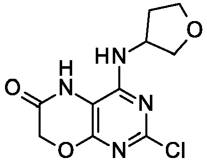

To a solution of methyl 2-[2-chloro-5-nitro-6-(tetrahydrofuran-3-ylamino)pyrimidin-4-yl]oxyacetate (150 mg, 0.451 mmol) in THF (6.0 mL) and 10% aqueous HCl (3.0 mL), was added Zn (88.5 mg, 1.35 mmol) and the reaction mixture was heated to 70 °C for 30 min. The mixture was diluted with saturated aqueous NaHCO₃ and the aqueous layer was extracted with EtOAc (3 x 20.0 mL). The combined organics were dried (Na₂SO₄), filtered, and concentrated under reduced pressure. The material was purified by column chromatography on silica gel using a gradient of 0-100% EtOAc in hexane to afford title compound (50.0 mg, 41%) as a solid. LCMS m/z: ES+ [M+H]+ = 271.1, t_{R}: 1.80 min.

### Step 4: Synthesis of 4-[(oxolan-3-yl)amino]-2-[(1E)-pent-1-en-1-yl]-5H,6H,7H-pyrimido[4,5-b][1,4]oxazin-6-one

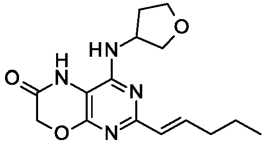

A mixture of 2-chloro-4-(tetrahydrofuran-3-ylamino)-5H-pyrimido[4,5-b][1,4]oxazin-6-one (45.0 mg, 0.166 mmol), [(E)-pent-1-enyl]boronic acid (56.8 mg, 0.498 mmol), and potassium carbonate (68.9 mg, 0.500 mmol) in toluene (0.80 mL), ethanol (0.20 mL), and water (0.20 mL) was degassed for 10 min by bubbling argon. Tetrakis(triphenylphosphine)palladium(0) (38.4 mg, 0.0332 mmol) was added, the vial was sealed then stirred at 100 °C for 16 h. The mixture was cooled to rt, diluted with EtOAc and saturated aqueous NaHCO₃. The layers were separated, and the aqueous layer was extracted with EtOAc (2x). The combined organic layers were washed with brine, then dried (Na₂SO₄), filtered, and concentrated under reduced pressure. The material was purified by column chromatography on silica gel using a gradient 0-10% MeOH in DCM to afford title compound (23.0 mg, 46%) as a solid. LCMS m/z: ES+ [M+H]+ = 305.2, LCMS; t_{R} = 4.14 mins (10 mins run).

### Step 5: Synthesis of 2-pentyl-N-tetrahydrofuran-3-yl-6,7-dihydro-5H-pyrimido[4,5-b][1,4]oxazin-4-amine

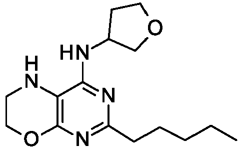

To a solution of 2-[(E)-pent-1-enyl]-4-(tetrahydrofuran-3-ylamino)-5H-pyrimido[4,5-b][1,4]oxazin-6-one (19.0 mg, 0.0624 mmol) in THF (0.25 mL) at 0 °C, was added BH₃.THF (1.00 M, 0.624 mL, 0.624 mmol) and the reaction mixture was warmed and stirred at rt for 2 h. The mixture was diluted with saturated aqueous NaHCO₃ and the aqueous layer was extracted with EtOAc (3 x 2.0 mL). The combined organic layers were dried (Na₂SO₄), filtered, and concentrated under reduced pressure. The material was purified by column chromatography on silica gel using a gradient of 0-10% MeOH in DCM to afford title compound (8.0 mg, 44%) as a solid. ¹H NMR (500 MHz, CD₃OD) δ 4.53 (d, J = 6.9 Hz, 1H), 3.45 (dt, J = 8.1, 4.1 Hz, 2H), 2.67 (t, J = 7.4 Hz, 2H), 2.13 - 2.02 (m, 2H), 1.84 - 1.72 (m, 4H), 1.66 (dd, J = 14.3, 10.1 Hz, 2H), 1.62 - 1.52 (m, 2H), 1.38-1.29 (m, 4H), 0.91 (t, J = 6.5 Hz, 3H). LCMS m/z: ES+ [M+H]+ = 293.2, LCMS; t_{R} = 2.96 min.

### Example 17

### Synthesis of B-322

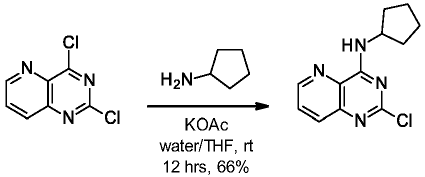

### Step 1: Synthesis of 2-chloro-N-cyclopentyl-pyrido[3,2-d]pyrimidin-4-amine

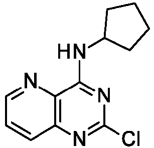

To a solution of 2,4-dichloropyrido[3,2-d]pyrimidine (125 mg, 0.625 mmol) in THF (5.0 mL) and water (3.0 mL), was added cyclopentanamine (62 µL, 0.625 mmol) followed by and CH₃COONa (0.0513 g, 0.625 mmol) and the reaction mixture was stirred at rt for 12 h. The mixture was diluted with EtOAc and the layers were separated. The organic layer was washed with water (3 x), then dried (Na₂SO₄), filtered and concentrated under reduced pressure. The material was purified by column chromatography on silica gel using a mixture of 20% EtOAc in hexane to afford title compound (130 mg, 84%) as a solid. ¹H NMR (500 MHz, CDCl₃) δ 8.64 (d, J = 3.8 Hz, 1H), 8.00 (d, J = 8.4 Hz, 1H), 7.63 (dd, J = 8.4, 4.1 Hz, 1H), 7.29 (bs, 1H), 4.68 - 4.55 (m, 1H), 2.21 2.16 (m, 2H), 1.90 - 1.77 (m, 2H), 1.76 - 1.66 (m, 2H), 1.67 - 1.54 (m, 2H). LCMS m/z: ES+ [M+H]+ = 249.1; t_{R} = 2.44 min.

### Example 18

### Synthesis of B-456

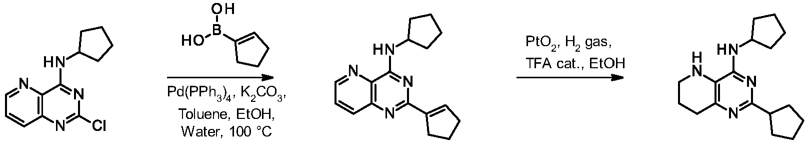

### Step 1: Synthesis of 2-(Cyclopenten-1-yl)-N-cyclopentyl-pyrido[3,2-d]pyrimidin-4-amine

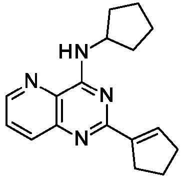

To a solution of 2-chloro-N-cyclopentyl-pyrido[3,2-d]pyrimidin-4-amine (90 mg, 0.362 mmol), 1-cyclopentylboronic acid (122 mg, 1.09 mmol), and potassium carbonate (150 mg, 1.09 mmol) in toluene (1.5 mL), ethanol (0.35 mL), and water (0.35 mL) was degassed for 10 min by bubbling argon. Pd(PPh₃)₄ (83 mg, 0.724 mmol) was then added, and the vial was sealed and heated at 100 °C for 8 h. The mixture was cooled to rt and the mixture was diluted with saturated aqueous. NaHCO₃ and EtOAc. The layers were separated, and the aqueous layer was extracted with EtOAc (2x). The combined organic layers were washed with brine, then dried (Na₂SO₄), filtered, and concentrated under reduced pressure. The material was purified by column chromatography on silica gel using a gradient of 0-70% EtOAc in hexane to afford title compound (35 mg, 35%) as a solid. ¹H NMR (500 MHz, CDCl₃) δ 8.57 (dd, J = 4.2, 1.4 Hz, 1H), 8.05 (dd, J = 8.5, 1.4 Hz, 1H), 7.57 (dd, J = 8.5, 4.2 Hz, 1H), 7.08 - 7.01 (m, 1H), 6.98 (d, J = 6.5 Hz, 1H), 4.68 - 4.55 (m, 1H), 2.91 (td, J = 7.7, 2.1 Hz, 2H), 2.60 (ddt, J = 10.0, 4.8, 2.4 Hz, 2H), 2.19 (dt, J = 13.0, 6.3 Hz, 2H), 2.11 - 2.02 (m, 2H), 1.87 - 1.75 (m, 2H), 1.76 - 1.57 (m, 4H). LCMS m/z: ES+ [M+H]+ = 281.2.; t_{R} = 1.91 min.

### Step 2: Synthesis of N,2-Dicyclopentyl-5,6,7,8-tetrahydropyrido[3,2-d]pyrimidin-4-amine

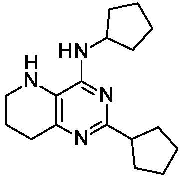

To a solution of 2-(cyclopenten-1-yl)-N-cyclopentyl-pyrido[3,2-d]pyrimidin-4-amine (30 mg, 0.107 mmol) in ethanol (2.0 mL) under argon was added PtO₂ ( 7.2 mg, 0.0321 mmol) followed by 3 drops of TFA and the reaction mixture was hydrogenated under hydrogen atmosphere at rt fro 2 h. The mixture was filtered on Celite, washed and the filtrate was concentrated under reduced pressure. The material was purified by column chromatography on silica gel using a gradient 0-30% MeOH in DCM to afford title compound (30 mg, 97%) as a solid. ¹H NMR (500 MHz, CD₃OD) δ 4.51 (p, J = 6.8 Hz, 1H), 3.37 - 3.25 (m, 2H), 3.11 (p, J = 7.8 Hz, 1H), 2.75 (t, J = 6.4 Hz, 2H), 2.17 - 1.99 (m, 4H), 1.99 - 1.81 (m, 6H), 1.78 (dd, J = 9.0, 5.7 Hz, 2H), 1.74 - 1.49 (m, 6H). LCMS m/z: ES+ [M+H]+ = 287.3; t_{R} = 3.45 min.

### Example 19

### Synthesis of B-349

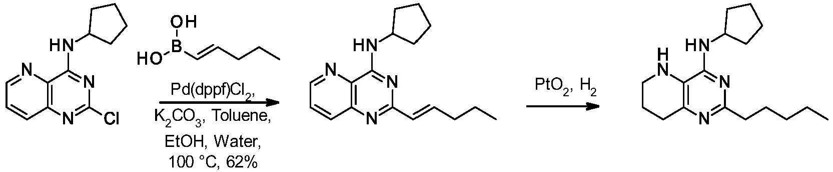

### Step 1: Synthesis of N-Cyclopentyl-2-[(E)-pent-1-enyl]pyrido[3,2-d]pyrimidin-4-amine

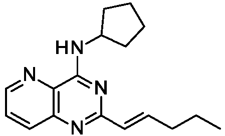

A solution of 2-chloro-N-cyclopentyl-pyrido[3,2-d]pyrimidin-4-amine (50 mg, 0.201 mmol), 1-pentenylboronic acid (30 mg, 0.261 mmol), and potassium carbonate (84 mg, 0.603 mmol) in toluene (1.5 mL), ethanol (0.35 mL), and water (0.35 mL) was degassed for 10 min by bubbling argon. Pd(dppf)Cl₂ (30 mg, 0.0402 mmol) and PPh₃ (21 mg, 0.0804 mmol) were added, and the vial was sealed and heated at 100 °C overnight. The mixture was cooled to rt and the diluted with saturated aqueous NaHCO₃. The aqueous layer was extracted with EtOAc (2 x 15.0 mL) and the combined organic layers were washed with brine, then dried (Na₂SO₄), filtered, and concentrated under reduced pressure. The material was purified by column chromatography on silica gel using a gradient of 0-70% EtOAc in hexane to afford title compound (35 mg, 62%) as a solid. LCMS m/z: ES+ [M+H]+ = 283.3; t_{R} = 2.00 min.

### Step 2: Synthesis of N-Cyclopentyl-2-pentyl-5,6,7,8-tetrahydropyrido[3,2-d]pyrimidin-4-amine

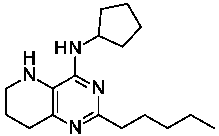

To a mixture of N-cyclopentyl-2-[(E)-pent-1-enyl]pyrido[3,2-d]pyrimidin-4-amine (30 mg, 0.105 mmol) and PtO₂ (7 mg, 0.0315 mmol) in ethanol (2.0 mL), was added TFA (15.6 µL, 0.0210 mmol) and the resulting mixture was hydrogenated under hydrogen atmosphere for 2 h at rt. The mixture was filtered on Celite, washed and the filtrate was concentrated under reduced pressure. The material was purified by reverse phase chromatography on C18 (5.5 g) using a gradient 10-100% MeCN and water (contains 0.1% formic acid) to afford title compound (30 mg, 99%) as a solid. ¹H NMR (500 MHz, CD₃OD) δ 4.55 (p, J = 7.0 Hz, 1H), 3.36 - 3.31 (m, 2H), 2.75 (t, J = 6.4 Hz, 2H), 2.69 (t, J = 7.5 Hz, 2H), 2.17 - 2.03 (m, 2H), 2.01 - 1.92 (m, 2H), 1.83 - 1.74 (m, 4H), 1.68 (dt, J = 8.4, 7.6 Hz, 2H), 1.62 - 1.53 (m, 2H), 1.41 - 1.31 (m, 4H), 0.91 (t, J = 6.8 Hz, 3H). LCMS m/z: ES+ [M+H]+ = 289.3; t_{R} = 3.89 mins (10 mins run).

### Example 20

### Synthesis of B-323

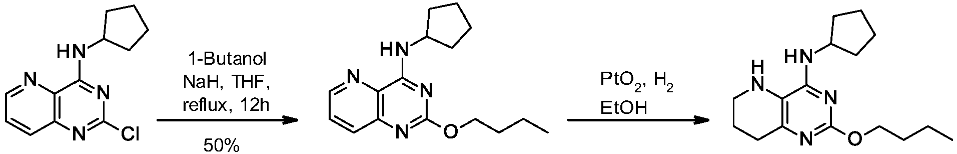

### Step 1: Synthesis of 2-Butoxy-N-cyclopentyl-pyrido[3,2-d]pyrimidin-4-amine

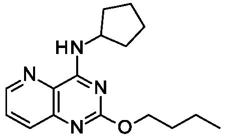

To a solution of 1-butanol (55 mg, 0.754 mmol) in anhydrous THF (10.0 mL) under argon at 0 °C, was added NaH (48 mg, 2.01 mmol) and the mixture was stirred for 10 min at rt. And then, a solution of 2-chloro-N-cyclopentyl-pyrido[3,2-d]pyrimidin-4-amine (125 mg, 0.502 mmol) in THF (2.0 mL) was added and the resulting mixture was stirred at 65 °C for 30 min. The mixture was cooled to rt and diluted with saturated aqueous NH₄Cl. The aqueous layer was extracted EtOAc (3 x 10.0 mL) and the combined organic layers were washed with brine, then dried (Na₂SO₄), filtered, and concentrated under reduced pressure. The material was purified by column chromatography on silica gel using a gradient of 0-30% methanol in DCM to afford title compound (73 mg, 50%) as a solid. LCMS m/z: ES+ [M+H]+ = 287.2.; t_{R} = 1.78 min.

### Step 2: Synthesis of 2-Butoxy-N-cyclopentyl-5,6,7,8-tetrahydropyrido[3,2-d]pyrimidin-4-amine

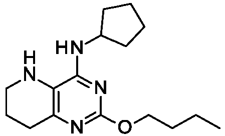

To a mixture of 2-butoxy-N-cyclopentyl-pyrido[3,2-d]pyrimidin-4-amine (50 mg, 0.175 mmol) and PtO₂ (3.97 mg, 0.0175 mmol) in anhydrous EtOH (10.0 mL) under argon atmosphere, was TFA (13 µL, 0.0175mmol) and the resulting mixture was hydrogenated under hydrogen atmosphere for 6 h at rt. The mixture was filtered on Celite, washed and the filtrate was concentrated under reduced pressure. The material was purified by column chromatography on silica gel using a gradient of 0-30% MeOH in DCM to afford title compound (15 mg, 30%) as a solid. ¹H NMR (500 MHz, CD₃OD) δ 4.50 - 4.45 (m, 1H), 4.42 (t, J = 6.5 Hz, 2H), 3.26 - 3.21 (m, 2H), 2.63 (t, J = 6.4 Hz, 2H), 2.13 - 2.04 (m, 2H), 1.90 (dd, J = 11.3, 5.9 Hz, 2H), 1.83 - 1.73 (m, 4H), 1.69 - 1.58 (m, 4H), 1.47 (dt, J = 13.2, 6.6 Hz, 2H), 0.97 (t, J = 7.4 Hz, 3H). LCMS m/z: ES+ [M+H]+ = 291.3; t_{R} = 3.59 min.

### Example 21

### Synthesis of B-433

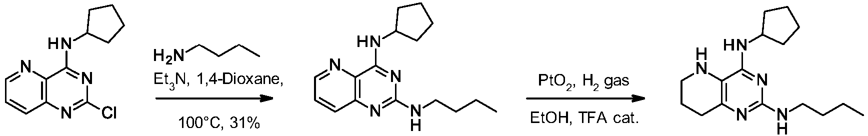

### Step 1: Synthesis of N2-butyl-N4-cyclopentyl-pyrido[3,2-d]pyrimidine-2,4-diamine

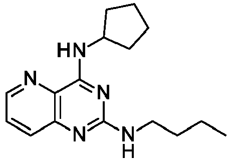

To a solution of 2-chloro-N-cyclopentyl-pyrido[3,2-d]pyrimidin-4-amine (100 mg, 0.402 mmol) in anhydrous 1,4-dioxane (8.0 mL), was added n-butylamine (52 µL, 0.523 mmol) followed by triethylamine (0.112 mL, 0.804 mmol) and the reaction mixture was stirred at reflux for 12 h. The mixture was cooled to rt, and then diluted with water and EtOAc. The layers were separated, and the aqueous layer was extracted with EtOAc (3 x 20.0 mL). The combined organic layers were washed with brine, then dried (Na₂SO₄), filtered and concentrated under reduced pressure. The material was purified by column chromatography on silica gel using a gradient 0-25% MeOH in DCM to afford title compound (42 mg, 37%) as a solid. ¹H NMR (500 MHz, CDCl₃) δ 8.35 - 8.17 (m, 1H), 7.65 (d, J = 7.1 Hz, 1H), 7.39 (dd, J = 8.5, 4.2 Hz, 1H), 6.90 (d, J = 6.0 Hz, 1H), 4.98 (s, 1H), 4.47 (dd, J = 13.6, 6.8 Hz, 1H), 3.48 (dd, J = 13.0, 6.9 Hz, 2H), 2.12 (dd, J = 12.1, 5.7 Hz, 2H), 1.84 - 1.73 (m, 2H), 1.74 - 1.50 (m, 7H), 1.52 -1.34 (m, 2H), 0.95 (t, J = 7.4 Hz, 3H). LCMS m/z: ES+ [M+H]+ = 286.3; t_{R} = 1.87 min.

### Step 2: Synthesis of N2-Butyl-N4-cyclopentyl-5,6,7,8-tetrahydropyrido[3,2-d]pyrimidine-2,4-diamine

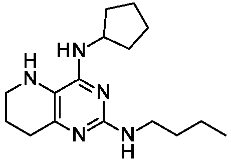

To a mixture of N-cyclopentyl-2-[(E)-pent-1-enyl]pyrido[3,2-d]pyrimidin-4-amine (10 mg, 0.0350 mmol) and PtO₂ (3 mg, 0.0105 mmol) in ethanol (5.0 mL) was added 3 drops of TFA and the resulting mixture was hydrogenated under hydrogen atmosphere for 2 h at rt. The mixture was filtered on Celite, washed and the filtrate was concentrated under reduced pressure. The material was purified by reverse phase chromatography on C18 (5.5 g) using a gradient 10-100% MeCN in water (contains 0.1% formic acid) to afford title compound (3 mg, 99%) as a solid. ¹H NMR (500 MHz, CDCl₃) δ 9.62 (s, 1H), 8.66 (s, 1H), 5.80 (d, J = 6.2 Hz, 1H), 4.42 - 4.29 (m, 1H), 3.36 (dd, J = 12.4, 6.5 Hz, 2H), 3.14 - 3.05 (m, 2H), 2.67 (t, J = 6.5 Hz, 2H), 2.09 (td, J = 12.4, 6.6 Hz, 2H), 1.87 - 1.79 (m, 2H), 1.78 - 1.55 (m, 6H), 1.49 (td, J = 13.1, 6.6 Hz, 2H), 1.43 - 1.33 (m, 2H), 0.92 (t, J = 7.3 Hz, 3H). LCMS m/z: ES+ [M+H]+ = 290.3.; t_{R} = 3.45 min.

### Example 22

### Synthesis of B-434

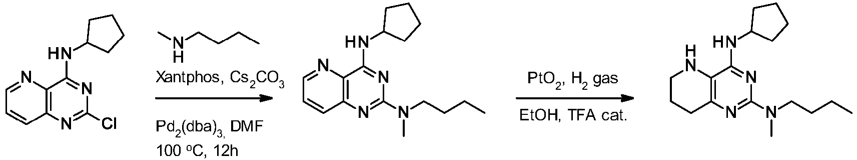

### Step 1: Synthesis of N2-Butyl-N4-cyclopentyl-N2-methyl-pyrido[3,2-d]pyrimidine-2,4-diamine

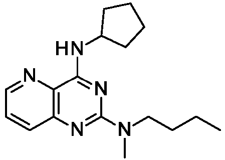

To a solution of 2-chloro-N-cyclopentyl-pyrido[3,2-d]pyrimidin-4-amine (150 mg, 0.603 mmol) in anhydrous DMF, was added N-methylbutylamine (52.6 mg, 0.603 mmol) followed by Cs₂CO₃ (393 mg, 1.21 mmol) and the mixture was degassed for 5 min by bubbling N₂. Xantphos (41.9 mg, 0.0724 mmol) was then added, followed by Pd₂dba₃ (69.4 mg, 0.121 mmol) and the resulting mixture was degassed for 5 min and then heated to 100 °C for 12 h. The mixture was diluted with water (10.0 mL) and the organic layer was extracted with EtOAc (2x). The combined organic layers were dried (Na₂SO₄), filtered and concentrated under reduced pressure. The material was purified by column chromatography on silica gel using a gradient 0-100% EtOAc in hexane to afford title compound (90 mg, 49.8 %) as a solid. LCMS m/z: ES+ [M+H]+ = 300.3, t_{R} = 1.90 min.

### Step 2: Synthesis of N2-Butyl-N4-cyclopentyl-N2-methyl-5,6,7,8-tetrahydropyrido[3,2-d]pyrimidine-2,4-diamine

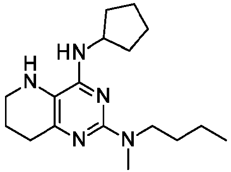

To a mixture of 2-butoxy-N-cyclopentyl-pyrido[3,2-d]pyrimidin-4-amine (50 mg, 0.167 mmol) and PtO₂ (3.80 mg, 0.0167 mmol) in anhydrous EtOH (10.0 mL) was added 3 drops of TFA and the resulting mixture was hydrogenated under hydrogen atmosphere for 6 h at rt. The mixture was filtered on Celite, washed and the filtrate was concentrated under reduced pressure. The material was purified by flash chromatography on silica gel using 0-100% EtOAc in hexane to afford title compound (15 mg, 29%) as a solid. ¹H NMR (500 MHz, CD₃OD) δ 4.42 (p, J = 6.7 Hz, 1H), 3.64 - 3.58 (m, 2H), 3.29 (s, 3H), 3.21 3.16 (m, 2H), 2.65 (t, J = 6.4 Hz, 2H), 2.10 - 2.01 (m, 2H), 1.93 - 1.86 (m, 2H), 1.77 (d, J = 6.2 Hz, 2H), 1.68 - 1.56 (m, 6H), 1.40 - 1.32 (m, 2H), 0.96 (t, J = 7.4 Hz, 3H). LCMS m/z: ES+ [M+H]+ = 304.3; t_{R} = 3.62 min.

### Example 23

### Synthesis of B-495

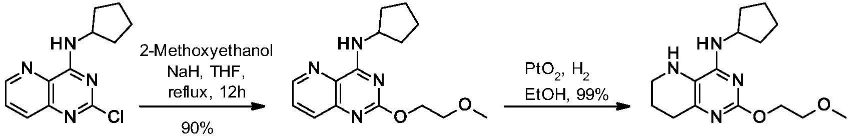

### Step 1: Synthesis of N-cyclopentyl-2-(2-methoxyethoxy)pyrido[3,2-d]pyrimidin-4-amine

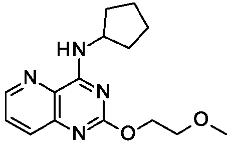

To a solution of 2-Methoxyethanol (0.0594 mL, 0.754 mmol) in anhydrous THF (10.0 mL) at 0 °C, was added NaH (60 % oil dispersion, 77 mg, 2.01 mmol) and the mixture was stirred for 10 min at rt. 2-chloro-N-cyclopentyl-pyrido[3,2-d]pyrimidin-4-amine (125 mg, 0.503 mmol) was then added and the resulting the mixture was stirred at 65 °C for 30 min. The mixture was cooled to rt and diluted with saturated aqueous NH₄Cl. The aqueous layer was extracted EtOAc and the combined organic layers were washed with brine, then dried (Na₂SO₄), filtered and concentrated under reduced pressure. The material was purified by column chromatography on silica gel using a gradient 0-30% methanol in DCM to afford title compound (130 mg, 90%) as a solid. LCMS m/z: ES+ [M+H]⁺ = 289.2.; t_{R} = 1.73 min.

### Step 2: Synthesis of N-cyclopentyl-2-(2-methoxyethoxy)-5,6,7,8-tetrahydropyrido[3,2-d]pyrimidin-4-amine

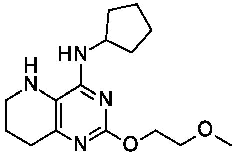

To a mixture of N-cyclopentyl-2-(2-methoxyethoxy)pyrido[3,2-d]pyrimidin-4-amine (30 mg, 0.104 mmol) and PtO₂ (7.1 mg, 0.0312 mmol) in ethanol (2 mL), was added TFA (1.55 µL, 0.0208 mmol) and the resulting mixture was hydrogenated under hydrogen atmosphere for 2 h at rt. The mixture was filtered on Celite, rinsed with EtOH and the filtrate was concentrated under reduced pressure. The material was purified by column chromatography on C18 (5.5 g) using a gradient 10-100% MeCN in water (contains 0.1% formic acid) to afford title compound (30 mg, 99%) as a solid. ¹H NMR (500 MHz, CDCl₃) δ 5.22 (bs, 1H), 4.48 - 4.32 (m, 3H), 3.73 (t, J = 5.1 Hz, 2H), 3.41 (s, 3H), 3.17 (bs, 2H), 2.66 (t, J = 5.9 Hz, 2H), 2.06 (dt, J = 12.4, 6.2 Hz, 2H), 1.92 - 1.82 (m, 2H), 1.78 = 1.67 (m, 2H), 1.66 - 1.56 (m, 2H), 1.51 - 1.39 (m, 2H). LCMS m/z: ES+ [M+H]⁺ = 293.2.; t_{R} = 2.72 min.

### Example 24

### Synthesis of B-710

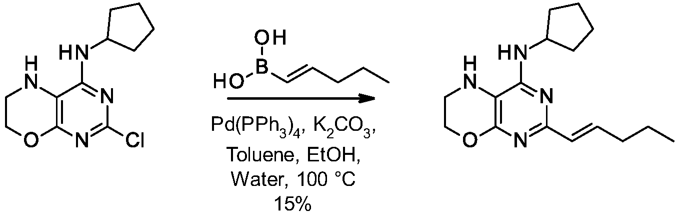

### Step 1: Synthesis of 1-[8-(tert-butylamino)-3-(trifluoromethyl)-1,2,3,4-tetrahydro-1,7-naphthyridin-6-yl]pentan-1-one

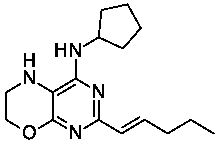

A mixture of 2-chloro-N-cyclopentyl-6,7-dihydro-5H-pyrimido[4,5-b][1,4]oxazin-4-amine (150 mg, 0.589 mmol), 1-pentenylboronic acid (67.1 mg, 0.589 mmol), and potassium carbonate (244 mg, 1.77 mmol) in toluene (1.5 mL), ethanol (0.7 mL), and water (0.7 mL) was degassed for 10 min by bubbling argon. Pd(PPh₃)₄ (136 mg, 0.118 mmol) was then added, the resulting mixture was heated at 100 °C for 12 h. The mixture was cooled to rt and diluted with saturated aqueous NaHCO₃ and EtOAc. The layers were separated, and the organic layer was dried (Na₂SO₄) filtered and concentrated under reduced pressure. The material was purified by column chromatography on silica gel using a gradient 0-100% EtOAc in hexane to afford title compound (25 mg, 15%) as a solid. ¹H NMR (500 MHz, CD₃OD) δ 6.85 - 6.77 (m, 1H), 6.13 (d, J = 15.4 Hz, 1H), 4.42 (p, J = 6.7 Hz, 1H), 4.25 (d, J = 3.5 Hz, 2H), 3.30 (d, J = 2.2 Hz, 2H), 2.18 (q, J = 7.1 Hz, 2H), 2.06 (dd, J = 12.2, 5.8 Hz, 2H), 1.77-1.73 (m, 2H), 1.65-1.61 (m, 2H), 1.50 (dt, J = 14.6, 7.5 Hz, 4H), 0.95 (t, J = 7.3 Hz, 3H). LCMS m/z: ES+ [M+H]+ = 289.2; QC t_{R} = 3.63 min.

### Example 25

### Synthesis of B-711

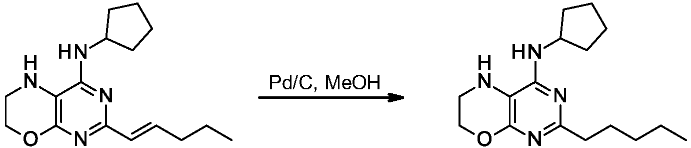

### Step 1: Synthesis of N-cyclopentyl-2-pentyl-5H,6H,7H-pyrimido[4,5-b][1,4]oxazin-4-amine

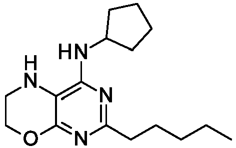

A mixture of N-cyclopentyl-2-[(E)-pent-1-enyl]-6,7-dihydro-5H-pyrimido[4,5-b][1,4]oxazin-4-amine (150 mg, 0.520 mmol) and Pd/C (20% wt, 55 mg, 0.520 mmol) in MeOH (10 mL) was hydrogenated under hydrogen atmosphere for 2 h at rt. The mixture was filtered on Celite, washed and the filtrate was concentrated under reduced pressure. The material was purified by column chromatography on silica gel using a gradient 0-100% EtOAc in hexane to afford title compound (155 mg, 99%) as a solid. ¹H NMR (500 MHz, CD₃OD) δ 4.53 (p, J = 6.9 Hz, 1H), 4.45 (dd, J = 13.8, 9.7 Hz, 2H), 3.45 (dt, J = 8.1, 4.1 Hz, 2H), 2.67 (t, J = 7.4 Hz, 2H), 2.13 - 2.02 (m, 2H), 1.84 - 1.72 (m, 4H), 1.66 (dd, J = 14.3, 10.1 Hz, 2H), 1.62 - 1.52 (m, 2H), 1.36 (d, J = 3.4 Hz, 4H), 0.91 (t, J = 6.5 Hz, 3H). LCMS m/z: ES+ [M+H]⁺ = 291.2; t_{R} = 1.94 min.

### Example 26

### Synthesis of B-763

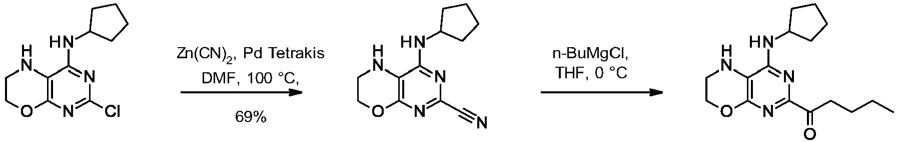

### Step 1: Synthesis of 4-(cyclopentylamino)-6,7-dihydro-5H-pyrimido[4,5-b][1,4]oxazine-2-carbonitrile

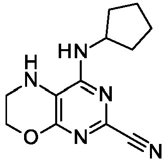

To a solution of 2-chloro-N-cyclopentyl-6,7-dihydro-5H-pyrimido[4,5-b][1,4]oxazin-4-amine (150 mg, 0.589 mmol) in DMF (10.0 mL), was added Zn(CN)₂ (0.138 g, 1.18 mmol) followed by Pd(PPh₃₎₄ (204 mg, 0.177 mmol) and the mixture was degassed by bubbling argon for 5 min and then heated at 100 °C for 12 h. The mixture was cooled to rt, saturated aqueous NH₄Cl was added, and the aqueous layer was extracted with EtOAc. The organic layer was washed with brine, then dried (Na₂SO₄), filtered, concentrated under reduced pressure. The material was purified by column chromatography on silica gel using a gradient 0-100% EtOAc in hexane to afford title compound (100 mg, 69%) as a solid. LCMS (ES+): m/z [M+H]⁺ 246.1; t_{R} = 2.23 min.

### Step 2: Synthesis of 1-[4-(cyclopentylamino)-5H,6H,7H-pyrimido[4,5-b][1,4]oxazin-2-yl]pentan-1-one

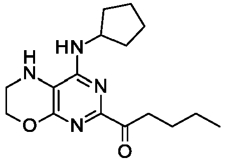

To a solution of 4-(cyclopentylamino)-6,7-dihydro-5H-pyrimido[4,5-b][1,4]oxazine-2-carbonitrile (40.0 mg, 0.163 mmol) in THF (1.5 mL), was added n-butylmagnesium chloride solution (2 M in THF, 0.16 mL, 0.326 mmol) at 0 °C and the reaction mixture was warmed to rt and stirred for 2 h. The mixture was diluted with saturated aqueous NH₄Cl and the aqueous layer was extracted with EtOAc (3 x 20.0 mL). The combined organic layers were washed with brine, then dried (Na₂SO₄), filtered and concentrated under reduced pressure. The material was purified by column chromatography on silica gel using a gradient 0-100% EtOAc in hexane to afford title compound (2.5 mg, 5%) as a solid. LCMS m/z: ES+ [M+H]⁺ = 305.2; t_{R} = 4.74 min.

### Example 27

### Synthesis of B-602

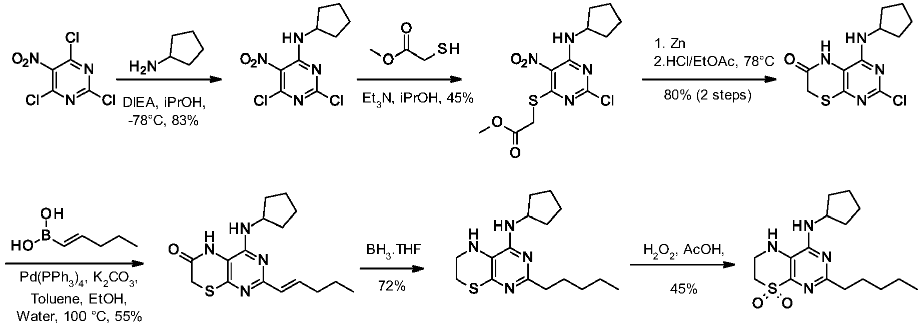

### Step 1: Synthesis of 2,6-dichloro-N-cyclopentyl-5-nitro-pyrimidin-4-amine

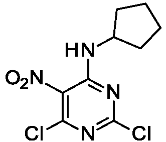

To a solution of 2,4,6-trichloro-5-nitro-pyrimidine (100 mg, 0.438 mmol) in 2-propanol (3 mL) at -78 °C, was added a solution of cyclopentanamine (43 µL, 0.438 mmol) in 2-propanol (1 mL) over 15 min and the resulting mixture was stirred at 30 min at -78 °C and then warmed to rt and stirred 1 h. DIPEA (0.15 mL, 0.876 mmol) was then added dropwise and the mixture was stirred for 2 h at rt. The volatiles were evaporated under reduced pressure and the material was purified by column chromatography on silica gel (12 g) using a gradient of 0-100% EtOAc in hexane to afford title compound (100 mg, 83%) as a solid. ¹H NMR (500 MHz, CDCl₃) δ 7.76 (s, 1H), 4.50 (dd, J = 13.9, 7.0 Hz, 1H), 2.12 (tt, J = 13.5, 6.7 Hz, 2H), 1.88 - 1.61 (m, 4H), 1.52 (td, J = 13.2, 6.6 Hz, 2H). LCMS m/z: ES+ [M+H]⁺ = 277.5.; t_{R} = 2.72 min.

### Step 2: Synthesis of methyl 2-[2-chloro-6-(cyclopentylamino)-5-nitro-pyrimidin-4-yl]sulfanylacetate

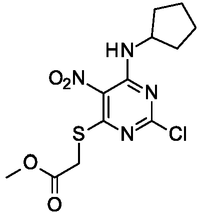

To a solution of 2,6-dichloro-N-cyclopentyl-5-nitro-pyrimidin-4-amine (500 mg, 1.80 mmol) in THF (15.0 mL) at 0 °C, was added methyl thioglycolate (0.192 g, 1.80 mmol) followed by DIPEA (0.309 mL, 1.80 mmol) and the reaction mixture was stirred at 0 °C for 1 h. The mixture was diluted with water (10 mL) and EtOAc (25 mL). The separated organic layer was dried (Na₂SO₄), filtered, concentrated under reduced pressure. The material was purified by column chromatography on silica gel using a gradient of 0-100% EtOAc in hexane to afford title compound (403 mg, 65%) as a solid. LCMS m/z: ES+ [M+H]⁺ = 347.1; t_{R} = 2.95 min.

### Step 3: Synthesis of methyl 2-[2-chloro-6-(cyclopentylamino)-5-nitro-pyrimidin-4-yl]sulfanylacetate

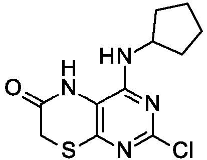

To a solution of methyl 2-[2-chloro-6-(cyclopentylamino)-5-nitro-pyrimidin-4-yl]sulfanylacetate (250 mg, 0.721 mmol) in a mixture THF (6 mL) 10% aqueous HCl (3.0 mL), was added zinc (141 mg, 2.16 mmol) and the resulting suspension was heated to 70 °C for 30 min. The mixture was diluted slowly with saturated aqueous NaHCO₃ and the aqueous layer was extracted with EtOAc (3 x 20 mL). The combined organic layers were dried (Na₂SO₄), filtered, and concentrated under reduced pressure. The material was purified by column chromatography on silica gel using a gradient of 0-100% EtOAc in hexane to afford title compound (100 mg, 49%) as a solid. LCMS (ES+): m/z [M+H]⁺ 285.1; t_{R} = 2.36 min.

### Step 4: Synthesis of 4-(cyclopentylamino)-2-[(1E)-pent-1-en-1-yl]-5H,6H,7H-pyrimido[4,5-b][1,4]thiazin-6-one (B-600)

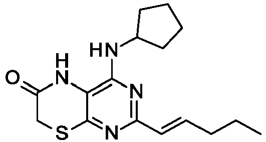

A mixture of 2-chloro-4-(cyclopentylamino)-5H-pyrimido[4,5-b][1,4]thiazin-6-one (250 mg, 0.79 mmol), 1-pentenylboronic acid (100 mg, 0.88 mmol), and potassium carbonate (364 mg, 2.63 mmol) in toluene (1.5 mL), ethanol (0.7 mL), and water (0.7 mL) was degassed for 10 min by bubbling argon. Pd(PPh₃)₄ (46 mg, 0.04 mmol) was added, and the mixture was heated at 100 °C for 12 h. The mixture was cooled rt and diluted saturated aqueous NaHCO₃ and EtOAc. The separated organic layer was washed with brine, then dried (Na₂SO₄), filtered, concentrated under reduced pressure. The material was purified by column chromatography on silica gel using a gradient 0-100% EtOAc in hexane to afford title compound (155 mg, 56%) as a solid. ¹H NMR (500 MHz,CD₃OD ) δ 7.02 - 6.92 (m, 1H), 6.22 (d, J = 15.4 Hz, 1H), 4.44 (p, J = 6.7 Hz, 1H), 3.53 (s, 2H), 2.21 (q, J = 7.2 Hz, 2H), 2.08 (dt, J = 12.3, 6.1 Hz, 2H), 1.82 - 1.71 (m, 2H), 1.66 (dd, J = 14.9, 7.9 Hz, 2H), 1.53 (tq, J = 14.6, 7.2 Hz, 4H), 0.96 (t, J = 7.4 Hz, 3H). LCMS m/z: ES+ [M+H]⁺ = 319.2; t_{R} = 4.82 min.

### Step 5: Synthesis of N-cyclopentyl-2-pentyl-5H,6H,7H-pyrimido[4,5-b][1,4]thiazin-4-amine (B-601)

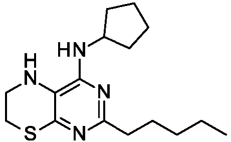

To a solution of 4-(cyclopentylamino)-2-[(E)-pent-1-enyl]-5H-pyrimido[4,5-b][1,4]thiazin-6-one (150 mg, 0.471 mmol) in dry tetrahydrofuran (10 mL), was added BH₃.THF (1 M in THF; 4.71 mL, 4.71 mmol) and the reaction mixture was stirred for 1 h at rt. The mixture was diluted with water and EtOAc, and the layers were separated. The organic layer was washed with brine, then dried (Na₂SO₄), filtered, concentrated under reduced pressure. The material was purified by column chromatography on silica gel using a gradient of 0-100% EtOAc in hexane to afford title compound (102 mg, 71%) as a solid. ¹H NMR (500 MHz, CD₃OD) δ 4.38 (p, J = 6.8 Hz, 1H), 3.52 - 3.47 (m, 2H), 3.10 - 3.05 (m, 2H), 2.51 (t, J = 7.5 Hz, 2H), 2.04 (dt, J = 14.1, 6.5 Hz, 2H), 1.74 (d, J = 6.5 Hz, 2H), 1.70 - 1.59 (m, 4H), 1.49 (td, J = 13.7, 7.1 Hz, 2H), 1.38 - 1.25 (m, 4H), 0.89 (t, J = 6.9 Hz, 3H). LCMS m/z: ES+ [M+H]⁺ = 307.2; t_{R} = 3.70 min.

### Step 6: Synthesis of N-cyclopentyl-8,8-dioxo-2-pentyl-6,7-dihydro-5H-pyrimido[4,5-b][1,4]thiazin-4-amine

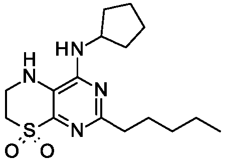

To a solution of N-cyclopentyl-2-[(E)-pent-1-enyl]-6,7-dihydro-5H-pyrimido[4,5-b][1,4]thiazin-4-amine (40 mg, 0.131 mol) in AcOH (3 mL), was added slowly H₂O₂ (31 µL, 0.393 mmol; 30% solution) and the reaction mixture was stirred at 60 °C for 1 h. The mixture was cooled to rt and diluted with saturated aqueous NaHCO₃. The aqueous layer was extracted EtOAc, and the combined organic layers were dried (Na₂SO₄), filtered, concentrated under reduced pressure. The material was purified by column chromatography on silica gel using a gradient of 0-100% EtOAc in hexane to afford title compound (19 mg, 43%) as a solid. ¹H NMR (500 MHz, CD₃OD) δ 4.43 (p, J = 6.8 Hz, 1H), 3.87 - 3.81 (m, 2H), 3.43 - 3.37 (m, 2H), 2.60 (t, J = 7.5 Hz, 2H), 2.12 - 2.04 (m, 2H), 1.75 (d, J = 7.3 Hz, 2H), 1.70 (dd, J = 14.5, 7.3 Hz, 2H), 1.66 (dd, J = 14.4, 7.5 Hz, 2H), 1.57 - 1.48 (m, 2H), 1.39 - 1.27 (m, 4H), 0.89 (t, J = 6.9 Hz, 3H). LCMS m/z: ES+ [M+H]⁺ = 339.2; t_{R} = 5.01 min.

### Example 28

### Synthesis of B-100

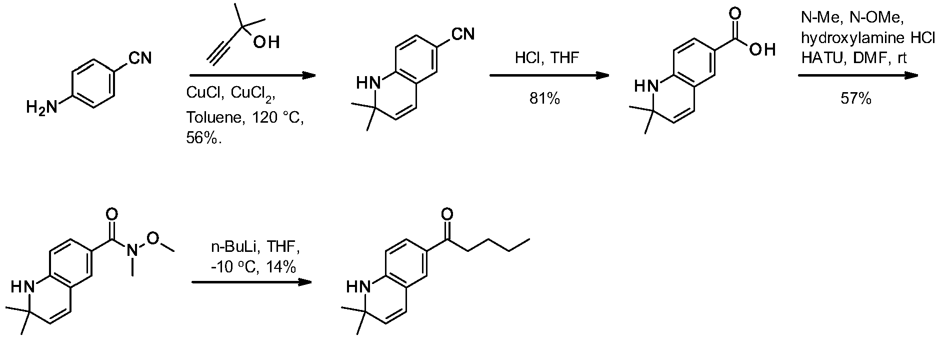

### Step 1: Synthesis of 2,2-dimethyl-1H-quinoline-6-carbonitrile

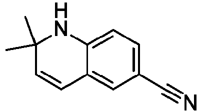

To a solution of 4-aminobenzonitrile (5.0 g, 42.3 mmol) and 2-methylbut-3-yn-2-ol (5.29 mL, 63.5 mmol) in anhydrous toluene (40 mL) was bubbled argon for 5 min, and then CuCl₂ (570 mg, 4.23 mmol) and CuCl (419 mg, 4.23 mmol) were added and the resulting mixture was stirred at 110 °C for 48 h. The mixture was cooled to rt and diluted with EtOAc and brine. The layers were separated, and the aqueous layer was extracted with EtOAc. The combined organic layers were washed with brine, then dried (Na₂SO₄), filtered, and concentrated under reduced pressure. The material was purified by column chromatography on silica gel (80 g) using a gradient of 0-100% EtOAc in hexane to afford title compound (4.65 g, 60%) as a solid. ¹H NMR (500 MHz, CDCl₃) δ 7.18 (dd, J = 8.3, 1.7 Hz, 1H), 7.08 (d, J = 1.5 Hz, 1H), 6.33 (d, J = 8.3 Hz, 1H), 6.19 (d, J = 9.9 Hz, 1H), 5.50 (d, J = 9.8 Hz, 1H), 4.11 (s, 1H), 1.34 (s, 6H). LCMS m/z: ES+ [M+H]⁺ = 185.1. t_{R} = 2.50 min.

### Step 2: Synthesis of 2,2-dimethyl-1H-quinoline-6-carboxylic acid

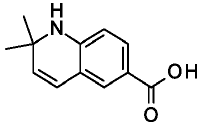

A mixture of 2,2-dimethyl-1H-quinoline-6-carbonitrile (2.06 g, 11.2 mmol) in 12 N HCl (25.0 mL) was was heated at 90 °C for 3 h. The mixture was concentrated under reduced pressure, diluted with water, and then cooled to 0 °C. The pH was adjusted to 3 by slow addition of saturated aqueous NaHCO₃. The aqueous layer was extracted with EtOAc, and the combined organic layers were washed with brine, then dried (Na₂SO₄), filtered and concentrated under reduced pressure to afford title compound (1.94 g, 86%) as a solid, which was used in the next step without further purification. LCMS m/z: ES+ [M+H]⁺ = 204.1; (B05) t_{R} = 2.20 min.

### Step 3: Synthesis of N-methoxy-N,2,2-trimethyl-1H-quinoline-6-carboxamide

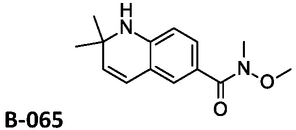

To a solution of 2,2-dimethyl-1H-quinoline-6-carboxylic acid (1.54 g, 7.58 mmol) in anhydrous DMF (30 mL), was added N,O-dimethylhydroxylamine hydrochloride (1.11 g, 11.4 mmol), followed by HATU (3.46 g, 9.09 mmol) and DIPEA (3.89 mL, 22.7 mmol) and the resulting mixture was stirred for 18h at rt. The mixture was diluted with EtOAc and brine. The layers were separated, and the aqueous layer was extracted with EtOAc (2x). The combined organic layers were dried (Na₂SO₄), filtered and concentrated under reduced pressure. The material was purified by column chromatography on silica gel (80 g) using a gradient of 0-100% EtOAc in hexane to afford title compound (1.9 g, 38%) as a solid. ¹H NMR (500 MHz, CDCl₃) δ 7.44 (dd, J = 8.3, 1.2 Hz, 1H), 7.36 (s, 1H), 6.34 (d, J = 8.3 Hz, 1H), 6.26 (d, J = 9.8 Hz, 1H), 5.46 (d, J = 9.8 Hz, 1H), 3.95 (s, 1H), 3.58 (s, 3H), 3.32 (s, 3H), 1.32 (s, 6H); LCMS m/z: ES+ [M+H]⁺ = 247.2; QC t_{R} = 4.28 min.

### Step 4: Synthesis of 1-(2,2-dimethyl-1,2-dihydroquinolin-6-yl)pentan-1-one

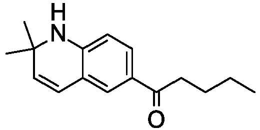

To a solution of n-BuLi (1.50 M in hexane, 1.89 mL, 2.84 mmol) in anhydrous THF (2 mL) at -10 ºC, was added a -10 ºC solution of N-methoxy-N,2,2-trimethyl-1H-quinoline-6-carboxamide (700 mg, 2.84 mmol) in anhydrous THF (7.à mL) and the resulting mixture was stirred 15 min at -10 ºC. The mixture was diluted with brine, and the aqueous layer was extracted with EtOAc (3 x 50 mL). The combined organic layers were washed with brine, then dried (Na₂SO₄), filtered and concentrated under reduced pressure. The material was purified by column chromatography on silica gel (12 g) using a gradient of 0-30% EtOAc in hexane to afford title compound (95 mg, 14%) as a solid. ¹H NMR (500 MHz, CDCl₃) δ 7.61 (dd, J = 8.5, 1.3 Hz, 1H), 7.50 (s, 1H), 6.34 (d, J = 8.4 Hz, 1H), 6.26 (d, J = 9.8 Hz, 1H), 5.46 (d, J = 9.9 Hz, 1H), 4.32 (s, 1H), 2.81 (t, J = 7.5 Hz, 2H), 1.70 - 1.61 (m, 2H), 1.40 (d, J = 7.4 Hz, 2H), 1.32 (s, 6H), 0.92 (t, J = 7.3 Hz, 3H). LCMS m/z: ES+ [M+H]⁺ = 244.2; QC t_{R} = 5.0 min.

### Example 29

### Synthesis of B-101

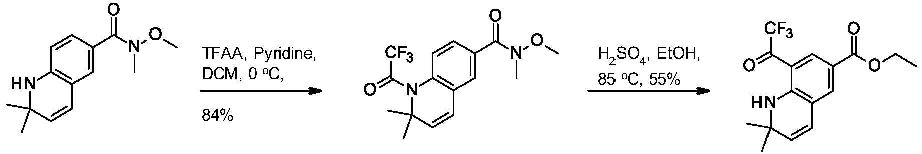

### Step 1: Synthesis of N-methoxy-N,2,2-trimethyl-8-(2,2,2-trifluoroacetyl)-1H-quinoline-6-carboxamide

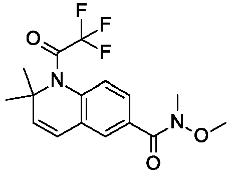

To a solution of N-methoxy-N,2,2-trimethyl-1H-quinoline-6-carboxamide (219 mg, 0.889 mmol) in mixture of DCM (4 mL) and pyridine (0.281 mL, 5.33 mmol), was added trifluoroacetic anhydride (0.162 mL, 1.16 mmol) and the resulting mixture was stirred for 4 h at rt. The mixture was diluted with DCM and the organic layer was washed subsequently with 1 M aqueous HCl, water, saturated aqueous NaHCO₃ and brine. The organic layer was dried (Na₂SO₄), filtered and concentrated under reduced pressure. The material was purified by column chromatography on slica gel (12 g) using a gradient of 0-50% EtOAc in hexane to afford title compound (255 mg, 84%) as a solid. LCMS m/z: ES+ [M+H]⁺ = 343.2; t_{R} = 2.73 min.

### Step 2: Synthesis of Ethyl 2,2-dimethyl-8-(2,2,2-trifluoroacetyl)-1,2-dihydroquinoline-6-carboxylate

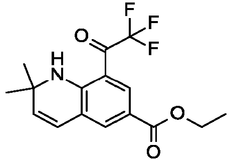

To a solution of N-methoxy-N,2,2-trimethyl-8-(2,2,2-trifluoroacetyl)-1H-quinoline-6-carboxamide (210 mg, 0.613 mmol) in absolute ethanol (5 mL) at rt, was added H₂SO₄ (12 µL, 0.123 mmol) and the reaction mixture was heated at 85 °C for 12 h. The mixture was cooled to rt and the volatiles were concentrated under reduced pressure. The material was purified by reverse phase chromatography on C18 (5.5 g) using a gradient of 10-100% MeCN in water (contains 0.1% formic acid) to afford title compound (110 mg, 55%) as a solid. ¹H NMR (500 MHz, CDCl₃) δ 9.09 (s, 1H), 8.09 (s, 1H), 7.64 (s, 1H), 6.47 (d, J = 10.1 Hz, 1H), 5.75 (d, J = 10.2 Hz, 1H), 4.23 (q, J = 7.0 Hz, 2H), 1.40 (s, 6H), 1.25 (t, J = 7.1 Hz, 3H). LCMS m/z: ES+ [M+H]⁺ = 328.1; QC t_{R} = 5.81 min.

### Example 30

### Synthesis of B-251

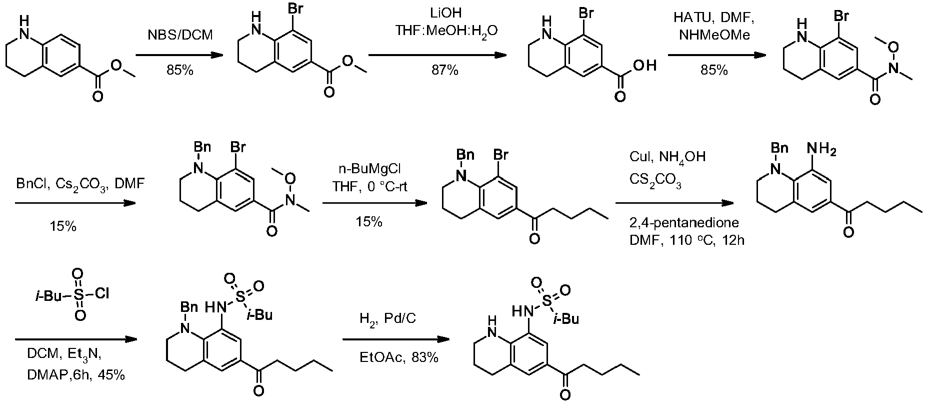

### Step 1: Synthesis of Methyl 8-bromo-1,2,3,4-tetrahydroquinoline-6-carboxylate

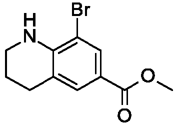

To a solution of methyl 1,2,3,4-tetrahydroquinoline-6-carboxylate (1.0 g, 4.82 mmol) in anhydrous DCM (27 mL) at rt, was added NBS (945 mg, 5.31 mmol) and the reaction mixture was stirred for 30 min. The mixture was diluted with saturated aqueous NaHCO₃ and the layers were separated. The aqueous layer was extracted with DCM. The combined organic layers were washed with brine, then dried (Na₂SO₄), filtered and concentrated under reduced pressure. The material was purified by column chromatography on silica gel using a mixture of 15% EtOAc in hexane to afford title compound (1.12 g, 86%) as a solid. ¹H NMR (500 MHz, CDCl₃) δ 7.94 (s, 1H), 7.58 (s, 1H), 5.30 (bs, 1H), 3.83(s, 3H), 3.45-3.43(m, 2H), 2.80-2.77 (m, 2H), 1.93-1.92 (m, 2H). LCMS m/z: ES+ [M+H]⁺ = 270.1; t_{R} = 2.60 min.

### Step 2: Synthesis of 8-bromo-1,2,3,4-tetrahydroquinoline-6-carboxylic acid

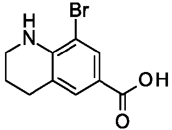

To a solution of methyl 1,2,3,4-tetrahydroquinoline-8-bromo-6-carboxylate (2.9 g, 10.1 mmol) in THF, MeOH and H₂O (3:1:1; 30 mL), was added LiOH (851 mg, 20.3 mmol) and the reaction mixture was stirred at 50 °C for 4 h. The volatiles were evaporated under reduced pressure and diluted with EtOAc. The pH was adjusted to ~2 with in 1 N aqueous HCl and the aqueous layer was extracted with EtOAc (3 × 15 mL). The combined organic layers were dried (Na₂SO₄), filtered and concentrated under reduced pressure to afford title compound as a solid, which was used in the next step without further purification. LC-MS m/z: ES+ [M+H]⁺ = 256.0; t_{R} = 2.20 min.

### Step 3: Synthesis of 8-bromo-N-methoxy-N-methyl-1,2,3,4-etrahydroquinoline-6-carboxamide

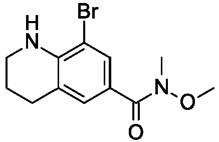

To a solution of 8-bromo-1,2,3,4-tetrahydroquinoline-6-carboxylic acid (900 mg, 3.51 mmol), N,O-dimethylhydroxylamine;hydrochloride (411 mg, 4.22 mmol) and HATU (1.66 g, 4.22 mmol) in anhydrous DMF (25 mL) was added DIPEA (1.84 mL, 10.5 mmol), and the reaction mixture was stirred overnight at rt. The mixture was diluted with saturated aqueous NaHCO₃ and the aqueous layer was extracted with EtOAc (3 x 20 mL). The combined organic layers were washed with brine, then dried (Na₂SO₄,), filtered and concentrated under reduced pressure. The material was purified by column chromatography on silica gel using a gradient of 0-100% EtOAc in hexane to afford title compound (895 mg, 85%) as a solid. LCMS m/z: ES+ [M+H]⁺ = 301.1; t_{R} = 2.32 mins

### Step 4: Synthesis of 1-benzyl-8-bromo-N-methoxy-N-methyl-3,4-dihydro-2H-quinoline-6-carboxamide

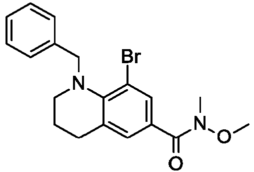

To a solution of 8-bromo-N-methoxy-N-methyl-1,2,3,4-tetrahydroquinoline-6-carboxamide (400 mg, 1.34 mmol) in DMF (10 mL), was added Cs₂CO₃ (871 mg, 2.67 mmol) followed by benzyl chloride (154 µL, 1.34 mmol) and the reaction mixture stirred at 90 °C for 12 h. The mixture was cooled to rt and diluted with H₂O (15 mL). The aqueous layer was extracted with EtOAc (2 x 15 mL) and the combined organic layers were dried (Na₂SO₄), filtered and concentrated under reduced pressure. The material was purified by column chromatography on silica gel using a mixture of 15% EtOAc in hexane to afford title compound (75 mg, 15%) as a solid. LCMS m/z: ES+ [M+H]⁺ = 389.1; t_{R} = 2.74 min.

### Step 5: Synthesis of 1-(1-benzyl-8-bromo-3,4-dihydro-2H-quinolin-6-yl)pentan-1-one

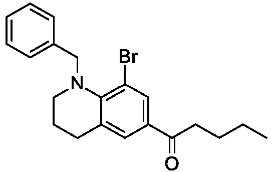

To a solution of 1-benzyl-8-bromo-N-methoxy-N-methyl-3,4-dihydro-2H-quinoline-6-carboxamide (700 mg, 1.80 mmol) in THF (20.0 mL) at 0 °C, was added n-BuMgCl (2 M in THF, 1.18 mL, 2.36 mmol) and the reaction mixture was warmed up to rt and then stirred for 6 h. The mixture was diluted with saturated aqueous NH₄Cl and the aqueous layer was extracted with EtOAc (3 x 20 mL). The combined organic layers were dried (Na₂SO₄), filtered and concentrated under reduced pressure. The material was purified by column chromatography on silica gel using a gradient of 0-20% EtOAc in hexane to afford title compound (600 mg, 73% yield) as a solid. LCMS m/z: ES+ [M+H]⁺ = 386.1, LCMS; t_{R} = 2.70 min.

### Step 6: Synthesis of 1-(8-amino-1-benzyl-1,2,3,4-tetrahydroquinolin-6-yl)pentan-1-one

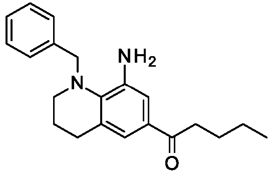

To a solution of 1-(1-benzyl-8-bromo-3,4-dihydro-2H-quinolin-6-yl)pentan-1-one (70 mg, 0.181 mmol) in ammonium hydroxide (1 mL) and DMF (1 mL), was added 2,4-pentanedione (5.4 mg, 0.054 mmol), followed by cesium carbonate (177 mg, 0.544 mmol), and Cul (8.60 mg, 0.045 mmol) and the reaction mixture was heated at 110 °C for 6 h. The mixture was cooled to rt, diluted with EtOAc (10 mL) was added. The organic layer was washed with water (10 mL) and brine (5 mL), then dried (Na₂SO₄), filtered, and concentrated under reduced pressure. The material was purified by purified by column chromatography on silica gel using a gradient of 0-100% EtOAc in hexane to afford title compound (7.0 mg, 12%) as a solid. LCMS m/z: ES+ [M+H]⁺ = 323.2; LCMS; t_{R} = 2.97 min.

### Step 7: Synthesis of N-(1-benzyl-6-pentanoyl-3,4-dihydro-2H-quinolin-8-yl)-2-methyl-propane-1-sulfonamide

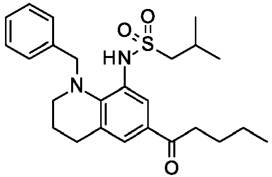

To a solution of 1-(8-amino-1-benzyl-3,4-dihydro-2H-quinolin-6-yl)pentan-1-one (30 mg, 0.0930 mmol) in DCM (3 mL) at 0 °C, was added DMAP (2.4 mg, 0.02 mmol) followed by triethylamine (14.2 µL, 0.102 mmol) and a solution of isobutanesulfonyl chloride (14.6 mg, 0.093 mmol) in DCM (0.5 mL), and the reaction mixture was stirred at rt for 12 h. The mixture was diluted with saturated aqueous NaHCO₃ and the aqueous layer was extracted with DCM. The combined organic layers were washed with brine, then dried (Na₂SO₄), filtered and concentrated under reduced pressure. The material was purified by column chromatography on silica gel using a mixture of 5% EtOAc in hexane to afford title compound (7 mg, 17%) as a solid. LCMS m/z: ES+ [M+H]⁺ = 443.2, t_{R} = 3.07 min.

### Step 8: Synthesis of 2-methyl-N-(6-pentanoyl-1,2,3,4-tetrahydroquinolin-8-yl)propane-1-sulfonamide

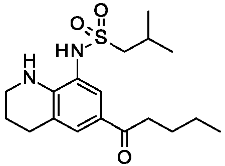

To a mixture of N-(1-benzyl-6-pentanoyl-3,4-dihydro-2H-quinolin-8-yl)-2-methylpropane-1-sulfonamide (10.0 mg, 0.0226 mmol) and 10% Pd/C (24 mg, 0.226 mmol) in anhydrous EtOAc (5 mL) was hydrogenated under hydrogen atmosphere for 6 h at rt. The mixture was filtered on Celite, rinsed with EtOAc and the filtrate was concentrated under reduced pressure. The material was purified by column chromatography on silica gel using a gradient of 0-50% EtOAc in hexane to afford title compound (4 mg, 53%) as a solid. ¹H NMR (500 MHz, CD₃OD) δ 7.60 (s, 1H), 7.54 (s, 1H), 3.42 - 3.37 (m, 2H), 2.99 (d, J = 6.4 Hz, 2H), 2.86 - 2.81 (m, 2H), 2.79 (t, J = 6.2 Hz, 2H), 2.25 (dt, J = 13.3, 6.7 Hz, 1H), 1.91 - 1.84 (m, 2H), 1.63 (dt, J = 15.2, 7.5 Hz, 2H), 1.39 (dt, J = 15.0, 7.4 Hz, 2H), 1.08 (d, J = 6.7 Hz, 6H), 0.93 (t, J = 7.3 Hz, 3H). LCMS m/z: ES+ [M+H]⁺ = 353.2, t_{R} = 5.58 min.

### Example 31

### Synthesis of B-059

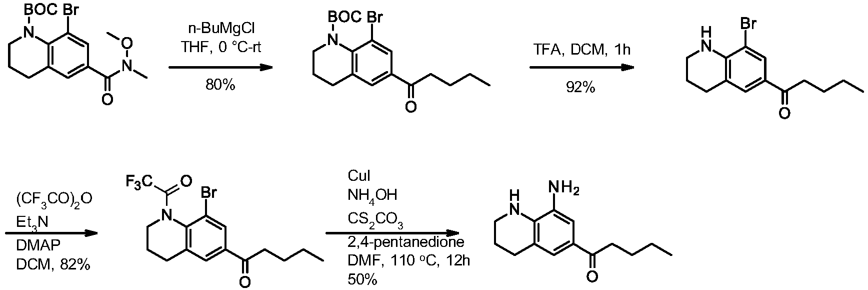

### Step 1: Synthesis of tert-butyl 8-bromo-6-[methoxy(methyl)carbamoyl]-3,4-dihydro-2H-quinoline-1-carboxylate

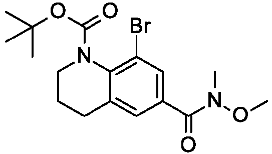

A solution of 8-bromo-N-methoxy-N-methyl-1,2,3,4-tetrahydroquinoline-6-carboxamide (900 mg, 3.01 mmol), di-tert-butyl dicarbonate (788 mg, 3.61 mmol) and DMAP (110 mg, 0.903 mmol) in THF (25 mL) was heated to 68 °C for 12 h. The reaction was cooled to rt, diluted with saturated aqueous NaHCO₃ (10. mL) and the aqueous layer was extracted with EtOAc (3 x 20 mL). The combined organic layers were dried (Na₂SO₄), filtered and concentrated to afford title compound (1.02 g, 85%) as a solid. LCMS m/z: ES+ [M-Boc]: 399.1; t_{R} = 2.72 min.

### Step 2: Synthesis of tert-butyl 8-bromo-6-pentanoyl-3,4-dihydro-2H-quinoline-1-carboxylate

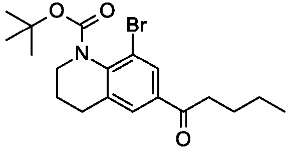

To a solution of tert-butyl 8-bromo-6-pentanoyl-3,4-dihydro-2H-quinoline-1-carboxylate (500 mg, 1.25 mmol) in THF (15 mL) was added n-BuMgCl (2 M, 0.95 mL, 1.87 mmol) at 0 °C, the reaction mixture was warmed to rt and stirred for 2 h. The mixture was diluted with saturated aqueous NH₄Cl and the aqueous layer was extracted with EtOAc (3 x 15 mL). The combined organic layers were washed with brine, then dried (Na₂SO₄), filtered and concentrated under reduced pressure. The material was purified by column chromatography on silica gel using a gradient 0-100% EtOAc in hexane to afford title compound (400 mg, 80%) as an oil. LCMS m/z: ES+ [M-Boc]: 296.1, t_{R} = 2.90 min.

### Step 3: Synthesis of 1-(8-bromo-1,2,3,4-tetrahydroquinolin-6-yl)pentan-1-one

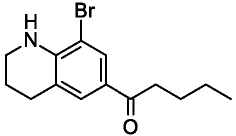

To a solution of tert-butyl 8-bromo-6-pentanoyl-3,4-dihydro-2H-quinoline-1-carboxylate (500 mg, 1.26 mmol) in DCM (10 mL), was added TFA (2.34 mL, 31.5 mmol) and the mixture was stirred at rt for 2 h. The volatiles were evaporated under reduced pressure, and the residue was dissolved in 2 mL of water and pH was adjusted to 7 with saturated aqueous NaHCO₃ at 0 °C. The aqueous layer was extracted with EtOAc (3 × 10 mL) and the combined organic layers were dried (Na₂SO₄), filtered and concentrated to afford title compound (345 mg, 92%) as an oil. LCMS m/z: ES+ [M+H]⁺ = 296.1; t_{R} = 2.86 min.

### Step 4: Synthesis of 1-[8-bromo-1-(2,2,2-trifluoroacetyl)-3,4-dihydro-2H-quinolin-6-yl]pentan-1-one

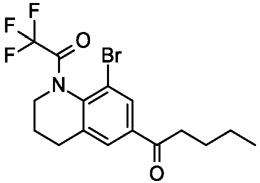

To a solution of 1-(8-bromo-1,2,3,4-tetrahydroquinolin-6-yl)pentan-1-one (500 mg, 1.69 mmol) in DCM (15 mL) at 0 °C, were successively added triethylamine (342 mg, 3.38 mmol), DMAP (412 mg, 0.338 mmol) and trifluoroacetic anhydride (0.307 mL, 2.19 mmol) and the reaction mixture was stirred at rt for 6 h. The mixture was poured into saturated aqueous NaHCO₃ and the layers were separated. The aqueous layer was extracted with DCM. The combined organic layers were dried (Na₂SO₄), filtered and concentrated under reduced pressure. The material was purified by column chromatography on silica gel using a gradient of 0-50% EtOAc in hexane to afford title compound (545 mg, 82%) as an oil. ¹H NMR (500 MHz, CD₃OD) δ 8.06 (s, 1H), 7.87 (s,1H), 4.30 (s, 1H), 3.49 - 3.36 (m, 1H), 3.00 (t, J = 7.3 Hz, 2H), 2.92 -2.73 (m, 2H), 2.24 (s, 1H), 2.00 (s, 1H), 1.70 - 1.58 (m, 2H), 1.46 - 1.31 (m, 2H),1.01 - 0.90 (m, 3H). LCMS m/z: ES+ [M+H]⁺ = 392.1, t_{R} = 2.93 min.

### Step 5: Synthesis of 1-(8-amino-1,2,3,4-tetrahydroquinolin-6-yl)pentan-1-one

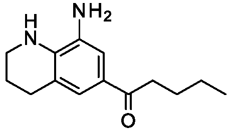

To a solution of 1-[8-bromo-1-(2,2,2-trifluoroacetyl)-3,4-dihydro-2H-quinolin-6-yl]pentan-1-one (150 mg, 0.382 mmol) in ammonium hydroxide (2 mL) and DMF (2 mL), was added pentane-2,4-dione (11.4 mg, 0.114 mmol) followed by Cs₂CO₃ (249 mg, 0.765 mmol) and Cul (18 mg, 0.096 mmol) and the reaction mixture was heated at 120 °C for 3 h. The mixture was cooled to rt and diluted with EtOAc (100 mL) and water (20 mL). The layers were separated, and the organic layer was washed with brine (2 x 20 mL), then dried (Na₂SO₄), filtered, and concentrated under reduced pressure. The material was purified by column chromatography on silica gel using a gradient 0-100% EtOAc in hexane to afford title compound (40 mg, 45%) as a solid. ¹H NMR (500 MHz, CD₃OD) δ 7.18 (d, J = 3.7 Hz, 2H), 3.42 - 3.34 (m, 2H), 2.87 - 2.78 (m, 2H), 2.75 (t, J = 6.2 Hz, 2H), 1.89 (dt, J = 11.9, 6.1 Hz, 2H), 1.64 - 1.57 (m, 2H), 1.41 -1.33 (m, 2H), 0.96 - 0.90 (m, 3H). LCMS m/z: ES+ [M+H]⁺ = 233.1; t_{R} = 3.82 min.

### Example 32

### Synthesis of B-060

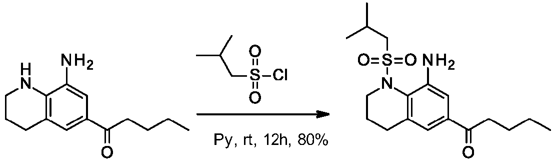

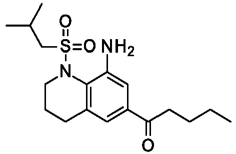

To a solution of 1-(8-amino-1,2,3,4-tetrahydroquinolin-6-yl)pentan-1-one (12 mg, 0.052 mmol) in dry pyridine (2 mL) at 0 °C, was added isobutanesulfonyl chloride (7.41 µL, 0.057 mmol) and the reaction mixture was stirred for 12 h at rt. The mixture was diluted with water (20 mL) and the aqueous layer was extracted with EtOAc (2 x 10 mL). The combined organic layers were washed with 0.5 M aqueous HCl (5 mL) and brine (10 mL), then dried (Na₂SO₄), filtered and concentrated under reduced pressure. The material was purified by column chromatography on silica gel using a mixture of 50% EtOAc in hexane to afford title compound (8 mg, 44%) as a solid. ¹H NMR (500 MHz, CD₃OD) δ 7.60 (s, 1H), 7.53 (s, 1H), 3.42 - 3.36 (m, 2H), 2.99 (d, J = 6.4 Hz, 2H), 2.84 (t, J = 7.5 Hz, 2H), 2.78 (t, J = 6.2 Hz, 2H), 2.27-2.23 (m, 1H), 1.88 (dt, J = 11.9, 6.1 Hz, 2H), 1.63 (dt, J = 15.1, 7.5 Hz, 2H), 1.37 (dt, J = 13.4, 6.7 Hz, 2H), 1.08 (d, J = 6.6 Hz, 6H), 0.93 (t, J = 7.3 Hz, 3H). LCMS m/z: ES+ [M+H]⁺ = 353.2 ; t_{R} = 5.23 min.

### Example 33

### Synthesis of B-035

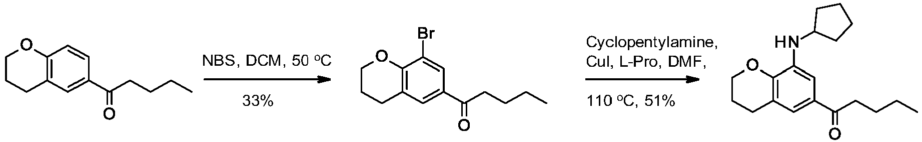

### Step 1: Synthesis of 1-(8-Bromochroman-6-yl)pentan-1-one

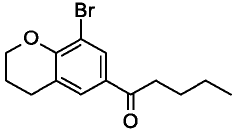

To a solution of valeryl chloride (562 µL, 559 mg, 4.64 mmol,) in anhydrous DCM (4 mL) at -10 °C, was added AlCl₃ (619 mg, 4.64 mmol) in portion and the mixture was stirred 15 min. The mixture was then added to a solution of 8-bromochromane (989 mg, 4.64 mmol) in anhydrous DCM (2.5 mL) and the resulting mixture was stirred for 1.5 h. The mixture was poured into a mixture of ice and 12 N HCl. The aqueous layer was extracted with DCM. The combined organic layers were washed with brine, then dried (MgSO₄), filtered, and concentrated under reduced pressure. The material was purified by column chromatography on silica gel (12 g) using a gradient of 0-45% EtOAc in hexane to afford title compound (801 mg, 58%) as a solid. ¹H NMR (500 MHz, CDCl₃) δ 7.94 (d, J = 1.8 Hz, 1H), 7.61 (s, 1H), 4.54 - 4.23 (m, 2H), 2.86 - 2.81 (m, 4H), 2.14 - 1.92 (m, 2H), 1.71 - 1.56 (m, 2H), 1.44 - 1.27 (m, 2H), 0.92 (t, J = 7.4 Hz, 3H). LCMS m/z: ES+ [M+H]⁺ = 299.1; t_{R} = 3.00 min.

### Step 2: Synthesis of 1-[8-(cyclopentylamino)-3,4-dihydro-2H-1-benzopyran-6-yl]pentan-1-one

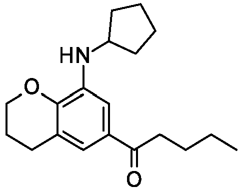

A mixture of 1-(8-bromochroman-6-yl)pentan-1-one (100 mg, 0.336 mmol), D-proline (39 mg, 0.336 mmol), cyclopentylamine (60.0 µL, 0.707 mmol), and K₂CO₃ (93 mg, 0.673 mmol) in anhydrous DMF (0.75 mL) was degassed by bubbling argon for 5 min. Cul (32 mg, 0.168 mmol) was then added and the resulting mixture was stirred overnight at 120 °C. The mixture was cooled to rt and diluted with brine and EtOAc. The layers were separated, and the aqueous layer was extracted with EtOAc. The combined organic layers were dried (Na₂SO₄), filtered and concentrated under reduced pressure. The material was purified by reverse phase chromatography on C18 (15 g) using a gradient 15-100% MeCN and water (contains 0.1% formic acid) to afford title compound (40 mg, 40%) as a solid. ¹H NMR (500 MHz, CDCl₃) δ 7.06 (s, 1H), 4.43 - 4.19 (m, 1H), 4.14 (s, 1H), 3.83 (p, J = 6.3 Hz, 1H), 2.96 - 2.84 (m, 1H), 2.78 (t, J = 6.4 Hz, 1H), 2.13 - 1.95 (m, 2H), 1.82 - 1.58 (m, 3H), 1.49 (qd, J = 7.2, 3.8 Hz, 1H), 1.38 (dt, J = 14.7, 7.4 Hz, 1H), 0.94 (t, J = 7.3 Hz, 1H). LCMS m/z: ES+ [M+H]⁺ = 302.3; QC t_{R} = 6.80 min.

### Example 34

### Synthesis of Q-980

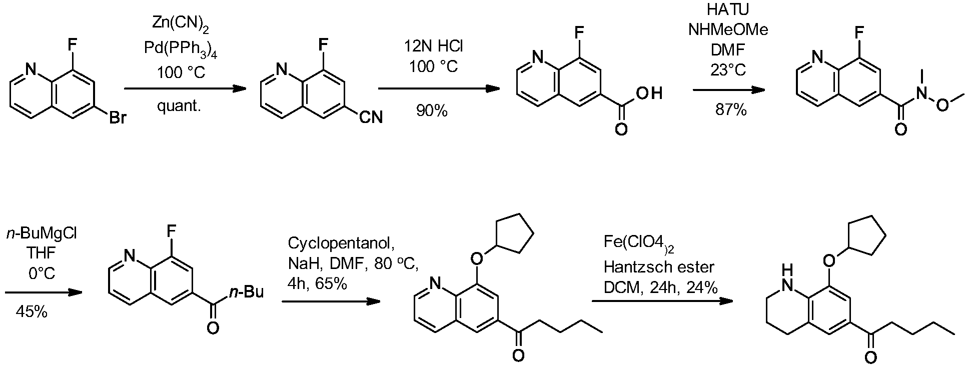

### Step 1: Synthesis of 8-fluoroquinoline-6-carbonitrile

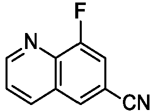

To a solution of 6-bromo-8-fluoro-quinoline (1.5 g, 6.63 mmol) in DMF (30 mL) was added, Zn(CN)₂ (1.55 g, 13.26 mmol) followed by Pd(PPh₃)₄ (383 mg, 0.331 mmol) and the mixture was degassed by bubbling argon for 5 min and then heated at 100 °C for 3 h. The mixture was cooled to rt and diluted with saturated aqueous NH₄Cl. The aqueous layer was extracted with EtOAc (3 x 30 mL) and the combined organic layers were washed with brine, then dried (Na₂SO₄), filtered, concentrated under reduced pressure. The material was purified by column chromatography on silica gel using a gradient 0-100% EtOAc in hexane to afford title compound (1.2 g, 100%) as a solid. LCMS (ES+): m/z [M+H]⁺ 173.1; t_{R} = 2.23 min.

### Step 2: Synthesis of 8-fluoroquinoline-6-carboxylic acid

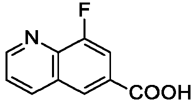

A solution of 8-fluroquniloine 6-carbonitrile (1.2 g, 6.93 mmol) in 12 N HCl (25.0 mL) was heated at 90 °C for 2 h. The mixture was cooled to rt and the volatiles were evaporated under reduced pressure. The residue was diluted with water, cooled to 0 °C and the pH was adjusted to 3 by addition of saturated aqueous sodium carbonate (NaHCO₃). The aqueous layer was extracted with EtOAc (3 x 30 mL) and the combined organic layers were washed with brine, then dried (Na₂SO₄), filtered, and concentrated under reduced pressure to afford title compound (1.0 g, 75%) as a solid, which was used in the next step without further purification. LCMS m/z: ES+ [M+H]⁺ = 192.02; t_{R} = 1.54 mins.

### Step 3: Synthesis of 8-fluoro-N-methoxy-N-methyl-quinoline-6-carboxamide

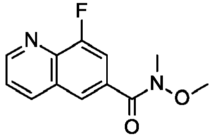

To a solution of 8-fluoroquinoline-6-carboxylic acid (700 mg, 3.66 mmol) in anhydrous dimethylformamide (25 mL), was added N,O-dimethylhydroxylamine hydrochloride (428 mg, 4.39 mmol) followed by HATU (1.66 g, 4.39 mmol) and DIPEA (1 mL, 5.49 mmol) and the reaction mixture was stirred overnight at rt. The mixture was diluted with saturated aqueous NaHCO₃ and the aqueous layer was extracted with EtOAc (3 x 20 mL). The combined organic layers were washed with brine, then dried (Na₂SO₄), filtered, and concentrated under reduced pressure. The material was purified by column chromatography on silica gel using a gradient of 0-100% EtOAc in hexane to afford title compound (750 mg, 87%) as a solid. LCMS m/z: ES+ [M+H]⁺ = 235.1; t_{R} = 2.31 min.

### Step 4: Synthesis of 1-(8-fluoro-6-quinolyl)pentan-1-one

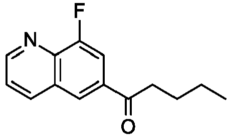

To a solution of 8-fluoro-N-methoxy-N-methyl-quinoline-6-carboxamide (750 mg, 3.20 mmol) in THF (20 mL) at 0 °C, was added n-BuMgCl (2 M in THF, 2.4 mL, 4.80 mmol) and the reaction mixture was warmed to rt and stirred for 2 h. The mixture was diluted with saturated aqueous NH₄Cl and the aqueous layer was extracted with EtOAc (3 x 15 mL). The combined organic layers were washed with brine, then dried (Na₂SO₄), filtered, and concentrated under reduced pressure. The material was purified by column chromatography on silica gel using a gradient of 0-100% EtOAc in hexane to afford title compound (565 mg, 65%) as a solid. LCMS m/z: ES+ [M+H]⁺ = 232.1, t_{R} =2.83 min.

### Step 5: Synthesis of 1-[8-(cyclopentoxy)-6-quinolyl]pentan-1-one

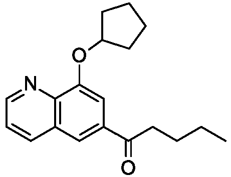

To a suspension of NaH (60% oil dispersion, 151 mg, 4.5 mmol) in anhydrous DMF (10 mL) was added a solution of cyclopentanol (0.294 mL, 3.0 mmol) in DMF (2.0 mL) at 0 °C and the mixture was stirred at rt for 15 min. (8-fluoro-6-quinolyl)pentan-1-one (231 mg, 1.0 mmol) was then added and the reaction mixture was heated to 80 °C for 4 h. The mixture was diluted with saturated aqueous NH₄Cl and the aqueous layer was extracted with EtOAc (3 x 15 mL). The combined organic layers were washed with brine, then dried (Na₂SO₄), filtered, and concentrated under reduced pressure. The material was purified by column chromatography on silica gel using a gradient of 0-100% EtOAc in hexane to afford title compound (195 mg, 65%) as a solid. LCMS m/z: ES+ [M+H]⁺ = 298.1; t_{R} = 2.15 min.

### Step 6: Synthesis of 1-[8-(cyclopentoxy)-1,2,3,4-tetrahydroquinolin-6-yl]pentan-1-one

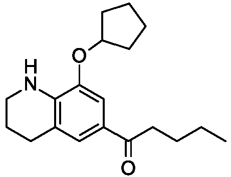

To a solution of 1-[8-(cyclopentoxy)-6-quinolyl]pentan-1-one (149 mg, 0.5 mmol) in DCM (8 mL) at rt, was added Fe(ClO₄)₂ (63 mg, 0.25 mmol) followed by Hantzsch ester (253 mg, 1.0 mmol) and the reaction mixture was stirred for 24 h at rt. The volatiles were evaporated under reduced pressure and the material was purified by column chromatography on silica gel using a gradient of 0-15% MeOH in DCM to afford title compound (35 mg, 24%) as a solid. ¹H NMR (500 MHz, MeOD); δ 7.25 (s, 1H), 7.05 (s, 1H), 4.14 - 4.07 (m, 1H), 4.02 - 3.90 (m, 2H), 3.82 (td, J = 8.1, 5.4 Hz, 1H), 3.70 (dd, J = 9.0, 3.2 Hz, 1H), 3.41 - 3.34 (m, 4H), 2.85 (t, J = 7.5 Hz, 2H), 2.74 (t, J = 6.2 Hz, 2H), 2.31 - 2.26 (m, 1H), 1.94 - 1.82 (m, 3H), 1.65 - 1.61 (m, 2H), 1.45 - 1.32 (m, 2H), 0.95 (t, 3H). LCMS m/z: ES+ [M+H]⁺ = 302. 2 ; t_{R} = 3.88 min.

### Example 35

### Synthesis of Q-950

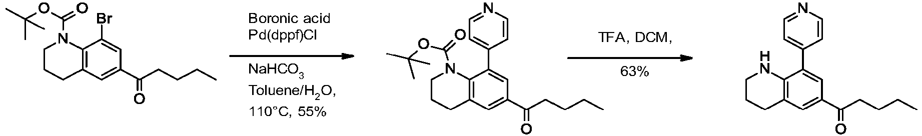

### Step 1: Synthesis of tert-butyl 6-pentanoyl-8-(4-pyridyl)-3,4-dihydro-2H-quinoline-1-carboxylate

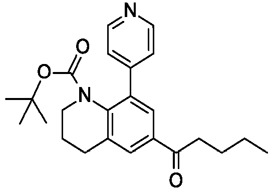

To a solution of tert-butyl 8-bromo-6-pentanoyl-3,4-dihydro-2H-quinoline-1-carboxylate (200 mg, 0.506 mmol), 4-pyridinylboronic acid (74 mg, 0.606 mmol) and NaHCO₃ (85 mg, 1.01 mmol) in toluene (6 mL) and water (1 mL) was degassed for 10 min by bubbling argon. Pd(dppf)Cl₂ (49 mg, 0.067 mmol) was then added, degassed for 5 min with N₂ and the resulting mixture was heated at 110 °C for 12 h. The mixture was cooled to rt, diluted with EtOAc and filtered on celite. The filtrate was concentrated under reduced pressure and the material was purified by column chromatography on silica using a gradient of 0-100% EOAc in hexane to afford title compound (110 mg, 55%) as a solid. LCMS m/z: ES+ [M+H]⁺ = 395.1 ; t_{R} = 2.53 min.

### Step 2: Synthesis of 1-[8-(4-pyridyl)-1,2,3,4-tetrahydroquinolin-6-yl]pentan-1-one

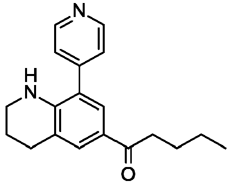

To a solution of tert-butyl 6-pentanoyl-8-(4-pyridyl)-3,4-dihydro-2H-quinoline-1-carboxylate (80 mg, 0.202 mmol) in DCM (0 mL) was added TFA (1.0 mL) and the reaction mixture was stirred at rt for 2 h. The volatiles were evaporated under reduced pressure and the residue was diluted with water (2 mL) and saturated aqueous NaHCO₃ (10 mL). The aqueous layer was extracted with EtOAc (3 x 10 mL) and the combined organic layers were dried (Na₂SO₄), filtered and concentrated under reduced pressure. The material was purified by column chromatography on silica gel using a gradient of 0-100% EtOAc in hexane to afford title compound (38 mg, 63%) as a solid. ¹H NMR (500 MHz, MeOD) δ 7.75 (d, J = 7.2 Hz, 2H), 7.67 (s, 1H), 7.54 (s, 1H), 7.32 (d, J = 7.4 Hz, 2H), 3.42-3.30 (m, 2H), 2.87 (dd, J = 12.2, 6.4 Hz, 4H), 1.93 (dt, J = 11.4, 6.4 Hz, 2H), 1.60 (dd, J = 12.1, 7.4 Hz, 2H), 1.41 - 1.30 (m, 2H), 0.95 (d, J = 7.3 Hz, 3H). LCMS m/z: ES+ [M+H]⁺ = 295.1, QC t_{R} = 3.74 min.

### Example 36

### Synthesis of B-006

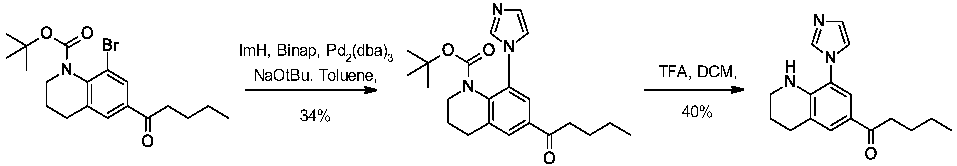

### Step 1: Synthesis of tert-butyl 8-imidazol-1-yl-6-pentanoyl-3,4-dihydro-2H-quinoline-1-carboxylate

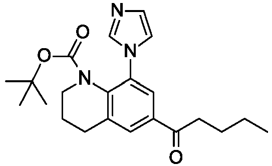

To a mixture of tert-butyl 8-bromo-6-pentanoyl-3,4-dihydro-2H-quinoline-1-carboxylate (400 mg, 1.01 mmol), imidazole (109 mg, 1.60 mmol), Pd₂dba₃ (122 mg, 0.134 mmol), BINAP (83 mg, 0.134 mmol), and sodium t-butoxide (193 mg, 2.01 mmol) in toluene (5 mL) was degassed for 10 min with nitrogen and the resulting mixture was heated at 100 °C for 12 h. The mixture was cool to rt, diluted with EtOAc and filtered on Celite. The filtrate was concentrated under reduced pressure and the material was purified by column chromatography on silica gel using a gradient of 0-100% EtOAc in hexane to afford title compound (133 mg, 34%) as a solid. LCMS m/z: ES+ [M+H]⁺ = 384. 2; t_{R} = 2.48 min.

### Step 2: Synthesis of 1-(8-imidazol-1-yl-1,2,3,4-tetrahydroquinolin-6-yl)pentan-1-one

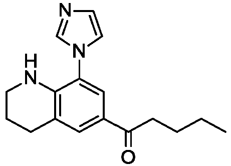

To a solution of tert-butyl 8-imidazol-1-yl-6-pentanoyl-3,4-dihydro-2H-quinoline-1-carboxylate (40 mg, 0.104 mmol) in DCM (3 mL) was added TFA (1.0 mL) and the reaction mixture was stirred at rt for 2 h. The volatiles were evaporated under reduced pressure and the residue was diluted in water (2.0 mL) and saturated aqueous NaHCO₃ (10 mL). The aqueous layer was extracted with EtOAc (3 x 10 mL) and the combined organic layers were dried (Na₂SO₄), filtered and concentrated under reduced pressure. The material was purified by column chromatography on silica gel using a gradient of 0-100% EtOAc in hexane to afford title compound (11.5 mg, 40%) as a solid. ¹H NMR (500 MHz, MeOD) δ 7.75 (s, 1H), 7.67 (s, 1H), 7.54 (s, 1H), 7.20 (d, J = 7.2 Hz, 2H), 3.31-3.29 (m, 2H), 2.85 (dd, J = 14.2, 6.9 Hz, 4H), 1.90 (dt, J = 11.8, 6.1 Hz, 2H), 1.61 (dd, J = 15.1, 7.5 Hz, 2H), 1.40 - 1.30 (m, 2H), 0.93 (d, J = 7.3 Hz, 3H). LCMS m/z: ES+ [M+H]⁺ = 284.1, QC t_{R} = 3.62 min.

### Example 37

### Synthesis of Q-979

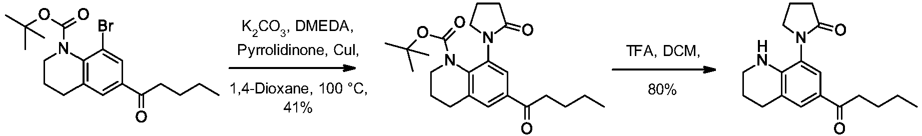

### Step 1: Synthesis of tert-butyl 8-(2-oxopyrrolidin-1-yl)-6-pentanoyl-3,4-dihydro-2H-quinoline-1-carboxylate

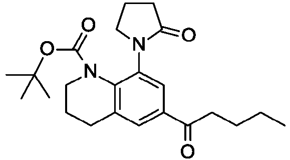

To a mixture of tert-butyl 8-bromo-6-pentanoyl-3,4-dihydro-2H-quinoline-1-carboxylate (200 mg, 0.506 mmol), 2-pyrrolidinone (43 mg, 0. 506 mmol), N,N'-dimethylethylenediamine (8.9 mg, 0.101 mmol), K₂CO₃ (139 mg, 1.01 mmol) and Cul (48 mg, 0.253 mmol) in dioxane (5 mL) was heated at 110 °C overnight. The mixture was cooled to rt, filtered on Celite and the filtrate was concentrated under reduced pressure. The material was purified by column chromatography on silica gel using a gradient of 0-100% EtOAc in hexane to afford title compound (81 mg, 40%) as a solid. LCMS m/z: ES+ [M+H]⁺ = 401.2 ; t_{R} = 2.44 min.

### Step 2: Synthesis of 1-(6-pentanoyl-1,2,3,4-tetrahydroquinolin-8-yl)pyrrolidin-2-one

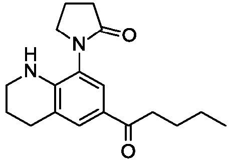

To a solution of tert-butyl 8-(2-oxopyrrolidin-1-yl)-6-pentanoyl-3,4-dihydro-2H-quinoline-1-carboxylate (80 mg, 0.199 mmol) in DCM (3 mL), was added TFA (1.0 mL) was stirred at rt for 2 h. The volatiles were evaporated under reduced pressure and the residue was diluted with water (2 mL) and saturated aqueous NaHCO₃ (10 mL). The aqueous layer was extracted with EtOAc (3 x 10 mL) and the combined organic layers were dried (Na₂SO₄), filtered and concentrated under reduced pressure. The material was purified by column chromatography on silica gel using a gradient of 0-100% EtOAc in hexane to afford title compound (48 mg, 80%) as a solid. ¹H NMR (500 MHz, MeOD); δ 7.23 (s, 1H), 7.06 (s, 1H), 4.02 - 3.92 (m, 2H), 3.70 (t, J = 9.0, 3.2 Hz, 2H), 3.41 3.34 (m, 4H), 2.85 (t, J = 7.5 Hz, 2H), 2.74 (t, J = 6.2 Hz, 2H), 1.94-1.61 (m, 4H), 1.45 - 1.32 (m, 2H), 0.95 (m, 3H). LCMS m/z: ES+ [M+H]+ = 302. 2 ; t_{R} = 3.88 min. LCMS m/z: ES+ [M+H]⁺ = 301.1, QC t_{R} = 3.58 min.

### Example 38

### Synthesis of B-273

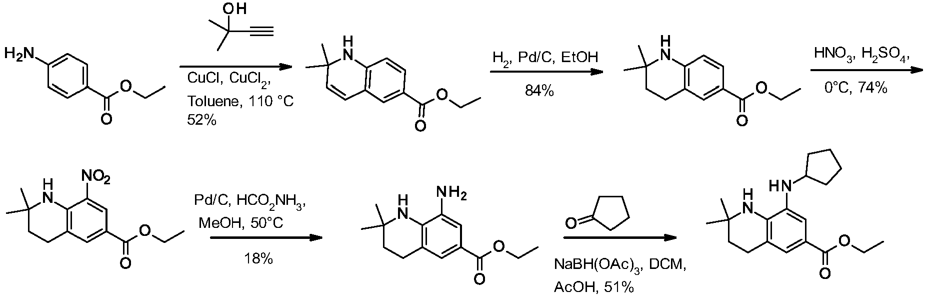

### Step 1: Synthesis of Ethyl 2,2-dimethyl-1H-quinoline-6-carboxylate

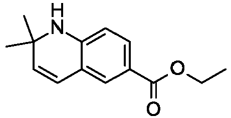

A solution of ethyl 4-aminobenzoate (1.00 g, 6.05 mmol) and 2-methylbut-3-yn-2-ol (0.76 mL, 9.08 mmol) in anhydrous toluene (10 mL) was sparged with bubbling argon for 5 min. CuCl₂ (81 mg, 0.605 mmol) was added followed by CuCl (60 mg, 0.605 mmol) and the resulting mixture was stirred at 110 °C for 48 h. The mixture was cooled to rt and diluted with EtOAc and brine. The layers were separated, and the aqueous layer was extracted with EtOAc (2 x 150 mL). The combined organic layers were washed with brine, then dried (Na₂SO₄), filtered and concentrated under reduced pressure. The material was purified by column chromatography on silica (12 g) using a gradient of 0-100% EtOAc in hexane to afford title compound (727 mg, 52%) as a solid. ¹H NMR (500 MHz, CDCl₃) δ 7.66 (dd, J = 8.3, 1.9 Hz, 1H), 7.56 (d, J = 1.6 Hz, 1H), 6.34 (d, J = 8.4 Hz, 1H), 6.27 (d, J = 9.8 Hz, 1H), 5.46 (d, J = 9.8 Hz, 1H), 4.29 (q, J = 7.1 Hz, 2H), 4.06 (s, 1H), 1.41 - 1.28 (m, 9H). LCMS m/z: ES+ [M+H]+ = 232.2; (B05) t_{R} = 2.60 min.

### Step 2: Synthesis of Ethyl 2,2-dimethyl-3,4-dihydro-1H-quinoline-6-carboxylate

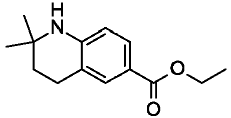

A mixture of ethyl 2,2-dimethyl-1H-quinoline-6-carboxylate (727 mg, 3.14 mmol) and Pd/C (10% on carbon, 335 mg, 3.14 mmol) in ethanol (10 mL) was hydrogenated under hydrogen atmosphere for 1 h. The mixture was filtered on Celite, rinsed with EtOH and the filtrate was concentrated under reduced pressure. The material was purified by column chromatography on silica gel (12 g) using a gradient of 0-100% EtOAc in hexane to afford title compound (615 mg, 84%) as a solid. ¹H NMR (500 MHz, CDCl₃) δ 7.70 (s, 1H), 7.65 (dd, J = 8.4, 1.8 Hz, 1H), 6.38 (d, J = 8.4 Hz, 1H), 4.29 (q, J = 7.2 Hz, 2H), 4.11 (s, 1H), 2.79 (t, J = 6.7 Hz, 2H), 1.70 (t, J = 6.7 Hz, 2H), 1.35 (t, J = 7.1 Hz, 3H), 1.22 (s, 6H). LCMS m/z: ES+ [M+H]⁺ = 234.2; t_{R} = 2.65 min.

### Step 3: Synthesis of Ethyl 2,2-dimethyl-8-nitro-3,4-dihydro-1H-quinoline-6-carboxylate

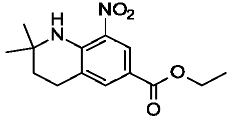

A solution of HNO₃ (0.0284 mL, 0.675 mmol) in H₂SO₄ (0.50 mL) was added dropwise to a solution of ethyl 2,2-dimethyl-3,4-dihydro-1H-quinoline-6-carboxylate (150 mg, 0.643 mmol) in H₂SO₄ (1.50 mL) at 0 °C and the reaction mixture was stirred for 30 min at 0 °C. The mixture was added slowly onto crushed ice and the resulting solid that formed was collected by filtration and dried under high vacuum to afford title compound (146 mg, 74%) as a solid which was used in the next step without purification. LCMS m/z: ES+ [M+H]⁺ = 279.2; t_{R} = 2.69 min.

### Step 4: Synthesis of ethyl 8-amino-2,2-dimethyl-3,4-dihydro-1H-quinoline-6-carboxylate.

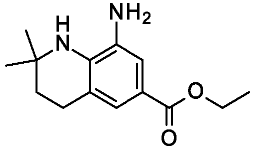

To a solution of crude ethyl 2,2-dimethyl-8-nitro-3,4-dihydro-1H-quinoline-6-carboxylate (131 mg, 0.471 mmol) in methanol (5 mL) was added ammonium formate (297 mg, 4.71 mmol) followed by Pd/C (10% on carbon, 50 mg, 0.471 mmol) and the reaction mixture was stirred at 50 °C overnight. The mixture was filtered on Celite, rinsed with methanol, and the filtrate was concentrated under reduced pressure. The material was purified by reverse phase chromatography on C18 (5.5 g) using a gradient 10-100% MeCN in water (contains 0.1% formic acid) to afford title compound (20 mg, 18%) as a solid. ¹H NMR (500 MHz, DMSO) δ 7.24 (s, 1H), 6.20 - 6.15 (m, 3H), 5.64 (s, 1H), 4.09 (q, J = 7.1 Hz, 2H), 2.52 - 2.48 (m, 2H), 1.50 (t, J = 6.6 Hz, 2H), 1.21 (t, J = 7.1 Hz, 3H), 1.09 (s, 6H). LCMS m/z: ES+ [M+H]⁺ = 249.2; QC t_{R} = 4.99 min.

### Step 5: Synthesis of Ethyl 8-cyclopentylamino-2,2-dimethyl-3,4-dihydro-1H-quinoline-6-carboxylate.

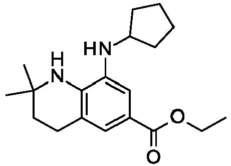

To a mixture of ethyl 8-amino-2,2-dimethyl-3,4-dihydro-1H-quinoline-6-carboxylate (10 mg, 0.04 mmol) and cyclopentanone (21 µL, 0.242 mmol) in anhydrous DCM (0.5 mL) was successively added sodium triacetoxyborohydride (51 mg, 0.242 mmol) and TFA (2.3 µL, 0.040 mmol) and the reaction mixture was stirred overnight at rt. The mixture was diluted with DCM (5 mL) and saturated aqueous NaHCO₃ (10 mL). The layers were separated, and the aqueous layer was extracted with DCM. The combined organic layers were washed with brine, then dried (Na₂SO₄), filtered, and concentrated under reduced pressure. The material was purified by reverse phase chromatography on C18 (5.5 g) using a gradient 10-100% MeCN in water (contains 0.1% formic acid) to afford title compound (65 mg, 51%) as a solid. ¹H NMR (500 MHz, CDCl₃) δ 7.62 (bs, 1H), 7.56 (s, 1H), 5.63 (s, 1H), 4.23 (q, J = 7.2 Hz, 2H), 4.08 (bs, 1H), 3.72 (s, 1H), 2.66 (t, J = 6.6 Hz, 2H), 2.06 - 1.90 (m, 2H), 1.74 (dd, J = 12.2, 8.1 Hz, 2H), 1.67 (t, J = 6.7 Hz, 2H), 1.57 (dt, J = 15.8, 6.4 Hz, 4H), 1.33 (t, J = 7.1 Hz, 3H), 1.21 (s, 6H). LCMS m/z: ES+ [M+H]+ = 317.3; QC t_{R} = 6.83 min.

### Example 39

### Synthesis of B-250

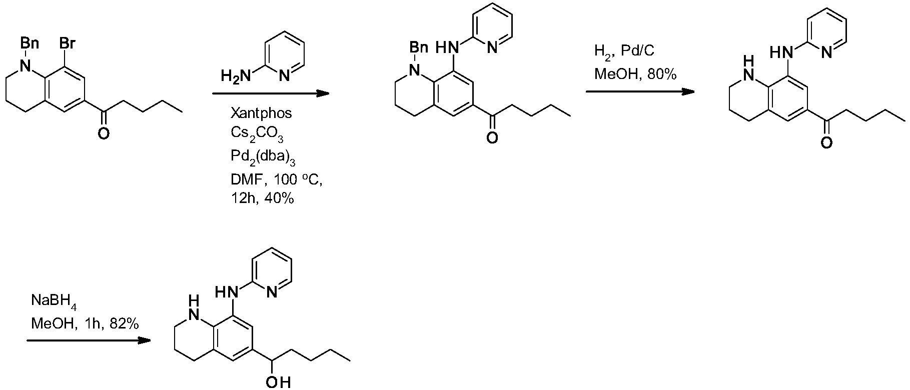

### Step 1: Synthesis of 1-[1-benzyl-8-(2-pyridylamino)-3,4-dihydro-2H-quinolin-6-yl]pentan-1-one

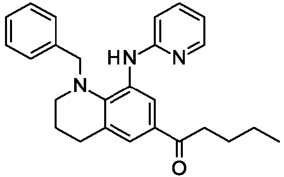

To a solution of 1-(1-benzyl-8-bromo-3,4-dihydro-2H-quinolin-6-yl)pentan-1-one (50 mg, 0.129 mmol) in anhydrous DMF (1.0 mL), was added 2-aminopyridine (12.2 mg, 0.130 mmol) and Cs₂CO₃ (84.3 mg, 0.259 mmol), and the mixture was degassed for 5 min by bubbling argon. Xantphos (9.0 mg, 0.0155 mmol) and Pd₂dba₃ (14.9 mg, 0.0259 mmol) were added and the mixture was degassed for another 5 min and then the reaction mixture was stirred at 100 °C for 12 h. The mixture was cooled to rt and diluted with water (1 mL) and EtOAc (10 mL). The separated organic layer was washed with brine, then dried (Na₂SO₄), filtered, and concentrated under reduced pressure. The material was purified by column chromatography on silica gel using a mixture of 5% MeOH in DCM to afford title compound (20 mg, 40%) as a solid. LCMS m/z: ES+ [M+H]⁺ = 400.3, t_{R} = 2.17 min.

### Step 2: Synthesis of 1-[8-(2-pyridylamino)-1,2,3,4-tetrahydroquinolin-6-yl]pentan-1-one

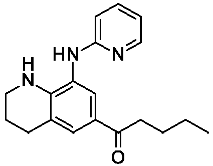

A mixture of 1-[1-benzyl-8-(2-pyridylamino)-3,4-dihydro-2H-quinolin-6-yl]pentan-1-one (17 mg, 0.043 mmol) and Pd/C (10% on carbon, 45 mg, 0.43 mmol) in EtOAc (5 mL) was hydrogenated under hydrogen atmosphere for 6 h at rt. The mixture was filtered on Celite, rinsed with EtOAc and the filtrate was concentrated under reduced pressure. The material was purified by column chromatography on silica gel using 0-50% EtOAc in hexane to afford title compound (9 mg, 70%) as a solid. ¹H NMR (500 MHz, CD₃OD) δ 7.95 (d, J = 4.3 Hz, 1H), 7.60 (d, J = 1.8 Hz, 1H), 7.52 (s, 1H), 7.51-7.46 (m, 1H), 6.69 - 6.65 (m, 1H), 6.49 (d, J = 8.5 Hz, 1H), 3.37 - 3.33 (m, 2H), 3.29 (dt, J = 2.9, 1.5 Hz, 2H), 2.85 - 2.79 (m, 4H), 1.90 (dt, J = 11.9, 6.1 Hz, 2H), 1.62 (dt, J = 20.8, 7.6 Hz, 2H), 1.41 - 1.32 (m, 2H), 0.92 (t, J = 7.4 Hz, 3H). LCMS m/z: ES+ [M+H]⁺ = 310.2, QC t_{R} = 3.29 min.

### Step 3: Synthesis of 1-[8-(2-pyridylamino)-1,2,3,4-tetrahydroquinolin-6-yl]pentan-1-ol

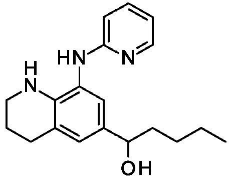

To a solution of 1-[8-(2-pyridylamino)-1,2,3,4-tetrahydroquinolin-6-yl]pentan-1-one (20 mg, 0.065 mmol) in MeOH (5 mL) at 0 °C, was added NaBH₄ (4.89 mg, 0.129 mmol) and the reaction mixture was stirred for 30 min at 0 °C, and then warmed to rt and stirred for 1 h. The mixture was diluted with water and the aqueous layer was extracted with EtOAc (2 x 10 mL). The combined organic layers were washed with brine (10 mL), then dried (Na₂SO₄), filtered and concentrated under reduced pressure. The material was purified by column chromatography on silica gel using a gradient of 1-5% MeOH in DCM to afford title compound (12 mg, 60%) as a solid. ¹H NMR (500 MHz, CD₃OD) δ 7.95 (dd, J = 5.1, 1.2 Hz, 1H), 7.45 (ddd, J = 8.6, 7.1, 1.8 Hz, 1H), 6.90 (d, J = 1.6 Hz, 1H), 6.80 (s, 1H), 6.64 (dd, J = 6.5, 5.4 Hz, 1H), 6.49 (d, J = 8.3 Hz, 1H), 4.38 (t, J = 6.8 Hz, 1H), 3.27 - 3.23 (m, 2H), 2.78 (t, J = 6.3 Hz, 2H), 1.94-1.85 (m, 2H), 1.77 - 1.67 (m, 1H), 1.67 - 1.56 (m, 1H), 1.35 - 1.27 (m, 3H), 1.18 (ddt, J = 10.8, 7.4, 5.2 Hz, 1H), 0.90 - 0.84 (m, 3H). LCMS m/z: ES+ [M+H]⁺ = 312.2, t_{R}: 3.08 min.

### Example 40

### Synthesis of B-308

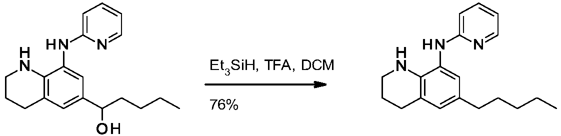

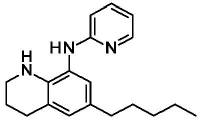

To a solution of 1-[8-(2-pyridylamino)-1,2,3,4-tetrahydroquinolin-6-yl]pentan-1-ol (16 mg, 0.051 mmol) in DCM (5 mL), was added (Et)₃SiH (0.017 mL, 0.103 mmol) followed by TFA (7.6 µL, 0.103 mmol) and the reaction mixture was stirred for 2 h at rt. The mixture was diluted with saturated aqueous NaHCO₃ and the aqueous layer was extracted with DCM (2 x 10 mL). The combined organic layers were washed with brine (10 mL), then dried (Na₂SO₄), filtered, and concentrated under reduced pressure. The material was purified by column chromatography on silica gel using a gradient of 1-5% MeOH in DCM to afford title compound (12 mg, 76%) as a solid. ¹H NMR (500 MHz, CD₃OD) δ 7.92 (dd, J = 5.0, 1.2 Hz, 1H), 7.51 (ddd, J = 8.6, 7.1, 1.7 Hz, 1H), 6.74 (d, J = 1.4 Hz, 1H), 6.67 (d, J = 7.9 Hz, 2H), 6.56 (d, J = 8.5 Hz, 1H), 3.25-3.21 (m, 2H), 2.76 (t, J = 6.4 Hz, 2H), 2.46 - 2.40 (m, 2H), 1.92 - 1.86 (m, 2H), 1.58-1.49 (m, 2H), 1.35 - 1.24 (m, 4H), 0.87 (t, J = 7.0 Hz, 3H). LCMS m/z: ES+ [M+H]⁺ = 296.3, QC t_{R}: 3.83 min.

### Example 41

### Synthesis of B-397

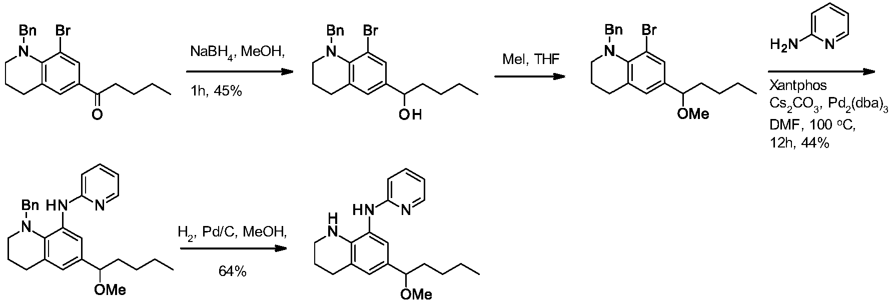

### Step 1: Synthesis of 1-(1-benzyl-8-bromo-3,4-dihydro-2H-quinolin-6-yl)pentan-1-ol

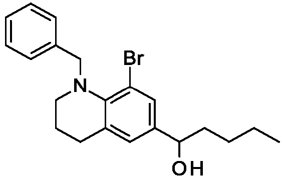

To a solution of 1-(1-benzyl-8-bromo-3,4-dihydro-2H-quinolin-6-yl)pentan-1-one (350 mg, 0.906 mmol) in methanol (5 mL) at 0 °C, was added NaBH₄ (68.6 mg, 1.81 mmol) and the reaction mixture was stirred for 30 min at 0 °C then 1 h at rt. The mixture was diluted with water and the aqueous layer was extracted with EtOAc (2 x 10 mL). The combined organic layers were washed with brine (10 mL), then dried (Na₂SO₄), filtered, and concentrated under reduced pressure. The material was purified by column chromatography on silica gel using a mixture of 30% EtOAc in hexane to afford title compound (300 mg, 86%) as a solid. LCMS m/z: ES+ [M+H]⁺ = 388.1, t_{R}: 3.01 min.

### Step 2: Synthesis of 1-benzyl-8-bromo-6-(1-methoxypentyl)-3,4-dihydro-2H-quinoline

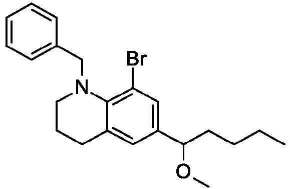

To a solution of 1-(1-benzyl-8-bromo-3,4-dihydro-2H-quinolin-6-yl) pentan-1-ol (500 mg, 1.29 mmol) in anhydrous THF (20 mL) at 0 °C, was added NaH (60% oil dispersion, 44 mg, 1.93 mmol) and the mixture was warmed to rt and stirred for 20 min. Mel (96 µL, 1.55 mmol) was then added at 0 °C and the reaction mixture warmed to rt and stirred for 12 h. The mixture was diluted with saturated aqueous NH₄Cl (10.0 mL) and the aqueous layer was extracted with EtOAc (3 x 20 mL). The combined organic layers were dried (Na₂SO₄), filtered and concentrated under reduced pressure. The material was purified by column chromatography on silica gel using a gradient 0-100% EtOAc in hexane to afford title compound (500 mg, 85%) as a solid. LCMS (ES+): m/z [M+H]⁺ 402.1, t_{R} = 2.46 min.

### Step 3: Synthesis of 1-benzyl-6-(1-methoxypentyl)-N-(2-pyridyl)-3,4-dihydro-2H-quinolin-8-amine

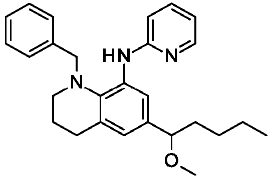

To a solution of 1-benzyl-8-bromo-6-(1-methoxypentyl)-3,4-dihydro-2H-quinoline (200 mg, 0.497 mmol) in anhydrous DMF (3 mL), was added 2-aminopyridine (46.9 mg, 0.498 mmol) followed by Cs₂CO₃ (324 mg, 0.994 mmol), and then the mixture was degassed for 5 min by bubbling argon. Xantphos (35 mg, 0.06 mmol) and Pd₂dba₃ (57 mg, 0.01 mmol) were added and the mixture was degassed for another 5 min and then the reaction mixture was stirred at 100 °C for 12 h. The mixture was cooled to rt then diluted with water (10 mL) and EtOAc (50 mL). The separated organic layer was washed with brine, then dried (Na₂SO₄) filtered, and concentrated under reduced pressure. The material was purified by column chromatography on silica gel using a gradient 0-100% EtOAc in hexane to afford title compound (90 mg, 44%) as a solid. LCMS m/z: ES+ [M+H]⁺ = 416.3, t_{R} = 2.25 min.

### Step 4: Synthesis of 6-(1-methoxypentyl)-N-(2-pyridyl)-1,2,3,4-tetrahydroquinolin-8-amine

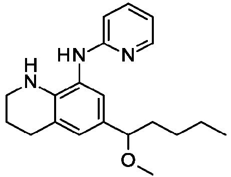

A mixture of 1-benzyl-6-(1-methoxypentyl)-N-(2-pyridyl)-3,4-dihydro-2H-quinolin-8-amine (50.0 mg, 0.120 mmol) and Pd/C (10% on carbon, 2.0 mg, 0.012 mmol) in EtOAc (5 mL) was hydrogenated under hydrogen atmosphere for 6 h at rt. The mixture was filtered on Celite, washed and the filtrate was concentrated under reduced pressure. The material was purified by column chromatography on silica gel using a gradient of 0-50% EtOAc in hexane to afford title compound (25 mg, 64%) as a solid. ¹H NMR (500 MHz, ) δ 8.02 - 7.94 (m, 1H), 7.53 - 7.42 (m, 1H), 6.86 (d, *J* = 1.4 Hz, 1H), 6.77 (s, 1H), 6.67 (dd, *J* = 6.5, 5.7 Hz, 1H), 6.49 (d, *J* = 8.5 Hz, 1H), 3.94 (t, *J =* 6.9 Hz, 1H), 3.30 - 3.26 (m, 2H), 3.16 (s, 3H), 2.80 (t, *J* = 6.3 Hz, 2H), 1.99 - 1.85 (m, 2H), 1.77 (tdd, *J* = 12.1, 6.9, 4.9 Hz, 1H), 1.65 - 1.52 (m, 1H), 1.39 - 1.24 (m, 3H), 1.19 (tt, *J* = 11.4, 4.2 Hz, 1H), 0.87 (t, *J* = 7.1 Hz, 3H).LCMS m/z: ES+ [M+H]⁺ = 326.3, QC t_{R} = 3.56 min.

### Example 42

### Synthesis of B-148

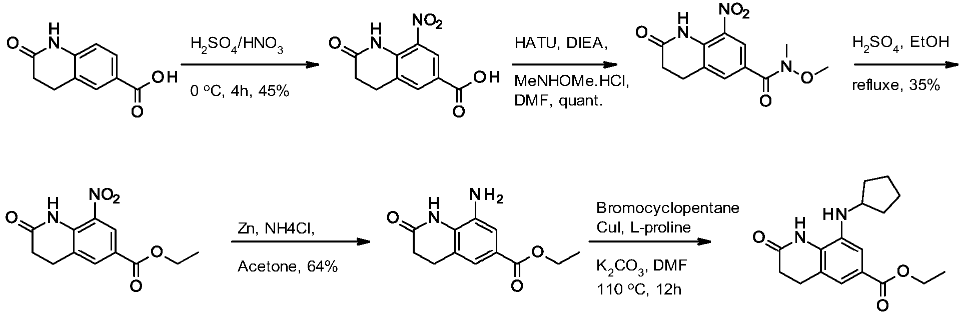

### Step 1: Synthesis of 8-nitro-2-oxo-3,4-dihydro-1H-quinoline-6-carboxylic acid

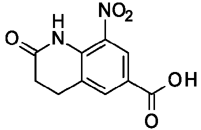

A mixture of HNO₃ (3.80 g, 60.3 mmol) and concentrated H₂SO₄ (9 mL) was added dropwise to a solution of 3-methyl-4-(propanoylamino)benzoic acid (2.50 g, 12.1 mmol) in H₂SO₄ (3 mL) at 0 °C and the mixture was stirred for 3 h at rt. The mixture was poured into ice-water and the resulting precipitate was collected by filtration and washed with water. The material was recrystallized from MeOH to afford title compound (810 mg, 29%) as a solid. ¹H NMR (500 MHz, CD₃OD) δ 8.68 (d, J = 1.5 Hz, 1H), 8.16 (s, 1H), 3.23 - 3.08 (m, 2H), 2.79 - 2.58 (m, 2H). LCMS m/z: ES+ [M+H]+ = 237.1, QC t_{R} = 3.37 min.

### Step 2: Synthesis of 8-nitro-2-oxo-3,4-dihydro-1H-quinoline-6-carboxylic acid

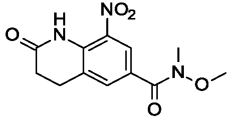

To a solution of 8-nitro-2-oxo-3,4-dihydro-1H-quinoline-6-carboxylic acid (500 mg, 2.12 mmol) in DMF (15 mL), were successively added N,O-dimethylhydroxylamine,HCl (227 mg, 2.33 mmol), HATU (966 mg, 2.54 mmol) and DIPEA (410 mg, 3.18 mmol) and the reaction mixture was stirred for 8 h. The mixture was diluted with water and the aqueous layer was extracted with EtOAc. The combined organic layers were washed with 0.1 N aqueous HCl, and brine, then dried (Na₂SO₄), filtered, and concentrated under reduced. The material was purified by column chromatography silica gel using a gradient 0-40% EtOAc in hexane to afford title compound (810 mg, 99%) as a solid. LCMS m/z: ES+ [M+H]⁺ = 280.1, LCMS; t_{R} = 1.91 min.

### Step 3: Synthesis of methyl 8-nitro-2-oxo-3,4-dihydro-1H-quinoline-6-carboxylate

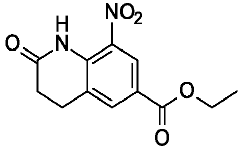

Sulfuric acid (193 mg, 1.97 mmol) was added to a solution of N-methoxy-N-methyl-8-nitro-2-oxo-3,4-dihydro-1H-quinoline-6-carboxamide (550 mg, 1.97 mmol) in absolute ethanol (15 ml) at room temperature. After refluxing for 3 h, the reaction mixture was concentrated in vacuo and purified by silica-gel column chromatography using a gradient 0-100% EtOAc in hexane to afford title compound (200 mg, 35%) as a solid. LC-MS m/z: ES+ [M+H]⁺:265.1, LCMS; t_{R} = 2.30 min.

### Step 4: Synthesis of ethyl 8-amino-2-oxo-3,4-dihydro-1H-quinoline-6-carboxylate

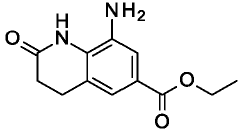

To a solution of ethyl 8-nitro-2-oxo-3,4-dihydro-1H-quinoline-6-carboxylate (400 mg, 1.51 mmol) in acetone (5 mL) at rt, was added saturated aqueous NH₄Cl (5.0 mL) followed by zinc (297 mg, 4.54 mmol), and the resulting mixture was stirred vigorously for 30 min. The mixture was diluted with EtOAc (25 mL) and then filtered on Celite. The organic layer was washed with saturated aqueous NaHCO₃ (10 mL) and brine (15 mL), then dried (Na₂SO₄), filtered, concentrated under reduced pressure to afford title compound (250 mg, 64%) as a solid, which was used in the next step without further purification. LCMS m/z: ES+ [M+H]⁺ = 235.1, LCMS; t_{R} = 1.94 min.

### Step 5: Synthesis of ethyl 8-(cyclopentylamino)-2-oxo-1,2,3,4-tetrahydroquinoline-6-carboxylate

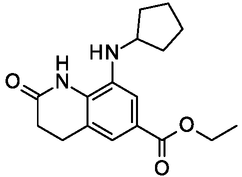

To a mixture of ethyl 8-amino-2-oxo-3,4-dihydro-1H-quinoline-6-carboxylate (50 mg, 0.213 mmol) and cyclopentanone (18 mg, 0.213 mmol) in DCM (5 mL) at rt, was added NaBH(OAc)₃ (90 mg, 0.427 mmol) and the reaction mixture was stirred for 16 h at rt. The mixture was diluted with saturated aqueous NaHCO₃ (10 mL) and the mixture was gently stirred for 5 min. The layers were separated, and the aqueous layer was extracted with DCM (3 x 10 mL). The combined organic layers were dried (Na₂SO₄), filtered and concentrated under reduced pressure. The material was purified by column chromatography on silica gel using a gradient of 0-60% EtOAc in hexane to afford title compound (15 mg, 22%) as a solid. ¹H NMR (500 MHz, CDCl₃) δ 9.21 (s, 1H), 7.33 (d, J = 6.2 Hz, 2H), 4.39 - 4.32 (m, 2H), 3.01 - 2.94 (m, 2H), 2.66-2.59 (m, 2H), 2.03 (dt, J = 13.5, 6.6 Hz, 2H), 1.81 - 1.73 (m, 2H), 1.69 - 1.55 (m, 6H), 1.43 - 1.35 (m, 3H); LCMS m/z: ES+ [M+H]⁺ = 303.2, t_{R} = 4.91 min.

### Example 43

### Synthesis of B-099

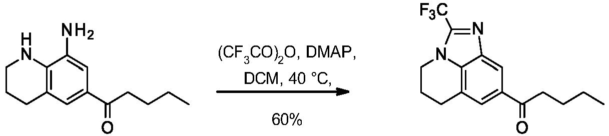

### Step 1: 1-[2-(trifluoromethyl)-1,3-diazatricyclo[6.3.1.04,12]dodeca-2,4(12),5,7-tetraen-6-yl]pentan-1-one

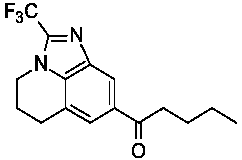

To a solution of 1-(8-amino-1,2,3,4-tetrahydroquinolin-6-yl)pentan-1-one (25.0 mg, 0.108 mmol) in DCM (5.0 mL) at rt, was added triethylamine (0.2 mL, 0.143 mmol) followed by DMAP (2.00 mg, 0.0164 mmol) and trifluoroacetic anhydride (24.9 mg, 0.118 mmol) and the reaction mixture was stirred at rt for 4 h and then stirred at 40 °C for 1 h. The mixture was poured onto saturated aqueous NaHCO₃ and the layers were separated. The aqueous layer was extracted with EtOAc (2x), and the combined organic layers were dried (Na₂SO₄), filtered and concentrated under reduced pressure. The material was purified by column chromatography on silica using a gradient of 0-50% EtOAc in hexane to afford title compound (20 mg, 60%) as a solid. ¹H NMR (500 MHz, CD₃OD) δ 8.26 (s, 1H), 7.85 (s, 1H), 4.46 - 4.41 (m, 2H), 3.12 - 3.05 (m, 4H), 2.37 - 2.27 (m, 2H), 1.74 - 1.66 (m, 2H), 1.41 (dt, J = 14.7, 7.4 Hz, 2H), 0.96 (t, J = 7.4 Hz, 3H); LCMS m/z: ES+ [M+H]+ = 311.2, t_{R} = 2.60 min.

### Example 44

### Synthesis of B-248

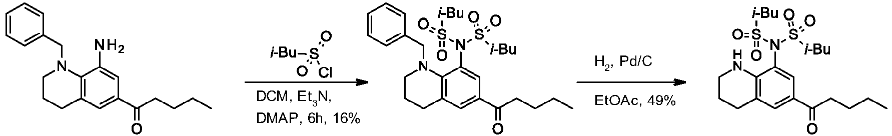

### Step 1: Synthesis of N-(1-benzyl-6-pentanoyl-3,4-dihydro-2H-quinolin-8-yl)-N-isobutylsulfonyl-2-methyl-propane-1-sulfonamide

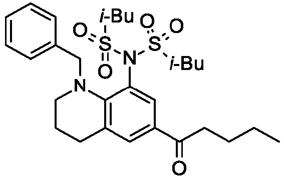

To a solution of 1-(8-amino-1-benzyl-3,4-dihydro-2H-quinolin-6-yl)pentan-1-one (25 mg, 0.078 mmol) in DCM (3 mL) at 0 °C, were successively added DMAP (2.0 mg, 0.016 mmol), triethylamine (6.2 µL, 0.085 mmol) then a solution of isobutanesulfonyl chloride (24 mg, 0.16 mmol) in DCM (0.5 mL) and the reaction mixture was stirred at rt for 12 h. The mixture was diluted with saturated aqueous NaHCO₃ and the aqueous layer was extracted with DCM. The combined organic layers were washed with brine, then dried (Na₂SO₄), filtered and concentrated under reduced pressure. The material was purified by column chromatography on silica gel using a mixture of 5% EtOAc in hexane to afford title compound (7 mg, 16%) as an oil. LCMS m/z: ES+

[M+H]⁺ = 443.2, t_{R} = 3.07 min.

### Step 2: Synthesis of N-isobutylsulfonyl-2-methyl-N-(6-pentanoyl-1,2,3,4-tetrahydroquinolin-8-yl)propane-1-sulfonamide

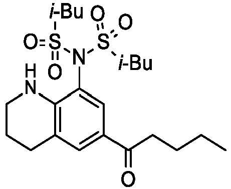

A mixture of N-(1-benzyl-6-pentanoyl-3,4-dihydro-2H-quinolin-8-yl)-N-isobutylsulfonyl-2-methyl-propane-1-sulfonamide (17 mg, 0.030 mmol) and Pd/C (10% on carbon, 32 mg, 0.302 mmol) in anhydrous MeOH (5 mL), was hydrogenated under hydrogen atmosphere for 6 h at rt. The mixture was filtered on Celite, rinsed with MeOH and the filtrate was concentrated under reduced pressure. The material was purified by column chromatography on silica gel using a gradient of 0-50% EtOAc in hexane to afford title compound (7 mg, 49%) as a solid. ¹H NMR (500 MHz, CDCl₃+CD₃OD) δ 7.24 (s, 1H), 7.21 (s, 1H), 3.22 (dd, J = 13.6, 6.8 Hz, 2H), 3.11 - 3.00 (m, 4H), 2.45 (dt, J = 13.2, 6.8 Hz, 4H), 2.00 (dp, J = 13.4, 6.7 Hz, 2H), 1.59 - 1.50 (m, 2H), 1.32 - 1.22 (m, 2H), 1.04 - 0.95 (m, 2H), 0.73 (dd, J = 6.6, 4.3 Hz, 12H), 0.55 (t, J = 7.3 Hz, 3H). LCMS m/z: ES+ [M+H]⁺ = 473.2, QC t_{R}: 6.29 min.

### Example 45

### Synthesis of B-388

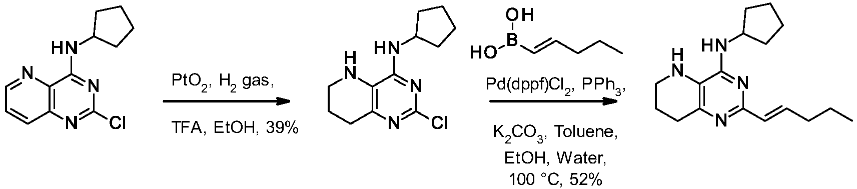

### Step 1: Synthesis of 2-chloro-N-cyclopentyl-5,6,7,8-tetrahydropyrido[3,2-d]pyrimidin-4-amine

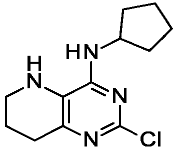

To a solution of 2-chloro-N-cyclopentyl-pyrido[3,2-d]pyrimidin-4-amine (20 mg, 0.080 mmol) in anhydrous ethanol (10 mL), was added PtO₂ (1.83 mg, 0.008 mmol) followed by TFA (0.6 µL, 0.008 mmol) and the resulting mixture was hydrogenated under hydrogen atmosphere for 6 h. The mixture was filtered on Celite, washed and the filtrate was concentrated under reduced pressure. The material was purified by column chromatography on silica gel using a gradient of 0-50% EtOAc in hexane to afford title compound (8 mg, 39%) as a solid. 1H NMR (500 MHz, CD₃OD) δ 4.42 (q, J = 6.9 Hz, 1H), 2.69 (t, J = 6.4 Hz, 2H), 2.07 (td, J = 12.1, 6.5 Hz, 2H), 1.97 - 1.89 (m, 2H), 1.83 - 1.72 (m, 2H), 1.67 (ddd, J = 10.8, 10.1, 6.0 Hz, 2H), 1.54 (td, J = 13.6, 6.9 Hz, 2H). LCMS m/z: ES+ [M+H]⁺ = 253.1; QC t_{R} = 3.67 min.

### Step 2: Synthesis of N-cyclopentyl-2-[(E)-pent-1-enyl]-5,6,7,8-tetrahydropyrido [3,2-d]pyrimidin-4-amine

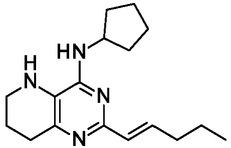

A mixture composed of 2-chloro-N-cyclopentyl-5,6,7,8-tetrahydropyrido[3,2-d]pyrimidin-4-amine (60 mg, 0.237 mmol), 1-pentenylboronic acid (35 mg, 0.309 mmol), and K₂CO₃ (98 mg, 0.71 mmol) in toluene (1.5 mL), ethanol (0.4 mL), and water (0.4 mL) was degassed for 10 min by bubbling argon. Pd(dppf)2Cl₂ (35 mg, 0.048 mmol) and triphenylphosphine (25 mg, 0.095 mmol) were then added, the resulting mixture was heated at 100 °C overnight. The mixture was cooled to rt and diluted with saturated aqueous NaHCO₃ and EtOAc. The layers were separated, and the aqueous layer was extracted with EtOAc. The combined organic layers were washed with brine, then dried (Na₂SO₄), filtered, and concentrated under reduced pressure. The material was purified by column chromatography on silica gel (4 g) using a gradient of 0-70% EtOAc in hexane to afford title compound (35 mg, 52%) as a solid. ¹H NMR (500 MHz, CDCl₃) δ 8.72 (s, 1H), 7.04 - 6.85 (m, 1H), 6.38 (d, J = 15.1 Hz, 1H), 4.54 - 4.38 (m, 2H), 3.22 - 3.12 (m, 2H), 2.73 - 2.62 (m, 2H), 2.22 (dd, J = 14.2, 7.0 Hz, 2H), 2.14 - 2.01 (m, 2H), 1.90 - 1.79 (m, 2H), 1.78 - 1.69 (m, 2H), 1.67 - 1.58 (m, 2H), 1.57 - 1.46 (m, 4H), 0.94 (t, J = 7.3 Hz, 3H). LCMS m/z: ES+ [M+H]⁺ = 287.2; t_{R} = 2.05 min.

### Example 46

### Synthesis of Q-879

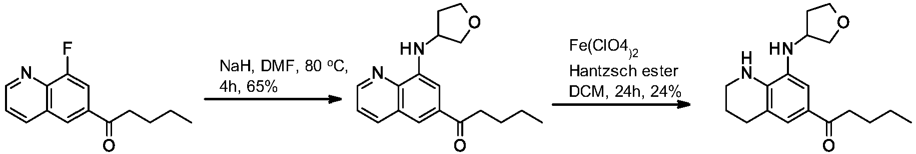

### Step 1: Synthesis of 1-[8-(tetrahydrofuran-3-ylamino)-6-quinolyl]pentan-1-one

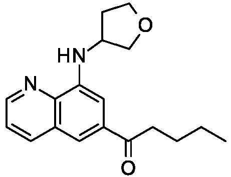

To a solution of 1-(8-fluoro-6-quinolyl)pentan-1-one (92 mg, 399 µmol) and tetrahydrofuran-3-amine (343 µL, 3.99 mmol) in dry DMSO (1 mL) at rt, was added DIPEA (139 µL, 797 µmol) and the reaction mixture was stirred at 150 °C for 40 h. The mixture was cooled to rt and diluted with water (25 mL) and DCM (10 mL). The layers were separated, and the aqueous layer was extracted with DCM (3 x 10 mL). The combined organic layers were washed with brine (30 mL), then dried (Na₂SO₄), filtered and concentrated under reduced pressure. The material was purified by column chromatography on silica gel (12 g cartridge) using a gradient of 0-30% EtOAc and hexane and was further purified by reversed chromatography on C18 (12g) using 50-100% MeCN and water (contains 0.1% formic acid) to afford title compound (65 mg, 55%) as an oil. ¹H NMR (500 MHz, CDCl₃) δ 8.80 (dd, J = 4.2, 1.7 Hz, 1H), 8.18 (dd, J = 8.3, 1.7 Hz, 1H), 7.70 (d, J = 1.7 Hz, 1H), 7.45 (dd, J = 8.2, 4.2 Hz, 1H), 7.20 (d, J = 1.7 Hz, 1H), 6.34 (d, J = 6.9 Hz, 1H), 4.41 - 4.33 (m, 1H), 4.14 (dd, J = 9.2, 5.6 Hz, 1H), 4.10 - 4.00 (m, 1H), 3.94 (td, J = 8.4, 5.2 Hz, 1H), 3.88 (dd, J = 9.2, 3.3 Hz, 1H), 3.13 - 3.03 (m, 2H), 2.48 - 2.32 (m, 1H), 2.13 - 2.00 (m, 1H), 1.78 (dt, J = 15.0, 7.5 Hz, 2H), 1.50 - 1.40 (m, 2H), 0.98 (t, J = 7.3 Hz, 3H). LCMS m/z: ES+ [M+H]⁺ = 299.92; (A05) t_{R} = 1.89 min.

### Step 2: Synthesis of 1-[8-(tetrahydrofuran-3-ylamino)-1,2,3,4-tetrahydroquinolin-6-yl]pentan-1-one

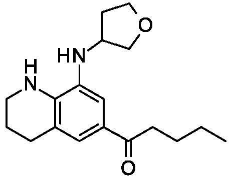

To a solution of 1-[8-(cyclopentylamino)-6-quinolyl]pentan-1-one (65 mg, 218 µmol) and Hantzsch ester (276 mg, 1.09 mmol) in CHCl₃ (2 mL), was added Fe(ClO₄)₂ (11.1 mg, 44 µmol) at rt, and the reaction mixture was stirred at rt for 60 h. The mixture was concentrated under reduced pressure and the material was purified by column chromatography on silica gel (12 g) using a gradient 0-60% of EtOAc in hexane and was further purified by preparative HPLC (BEH 5µm C18 30x100 mm; using 42-62% MeCN and 10 mM ammonium formate pH 3.8) to afford title compound (12.0 mg, 18%) as a solid. ¹H NMR (500 MHz, CD₃OD) δ 7.23 (s, 1H), 7.04 (d, J = 1.6 Hz, 1H), 4.15 - 4.06 (m, 1H), 4.03 - 3.93 (m, 2H), 3.84 (td, J = 8.3, 5.4 Hz, 1H), 3.71 (dd, J = 9.0, 3.2 Hz, 1H), 3.43 - 3.36 (m, 2H), 2.87 (t, J = 7.5 Hz, 2H), 2.78 (t, J = 6.2 Hz, 2H), 2.34-2.26 (m, 1H), 1.96 - 1.86 (m, 3H), 1.69 - 1.61 (m, 2H), 1.46 - 1.35 (m, 2H), 0.95 (t, 3H). LCMS m/z: ES+ [M+H]+ = 302.70; (A05) tR = 1.73 m. LCMS m/z: ES+ [M+H]⁺ = 302.62; (B05) t_{R} = 1.88 min.

### Example 47

### Synthesis of Q-912

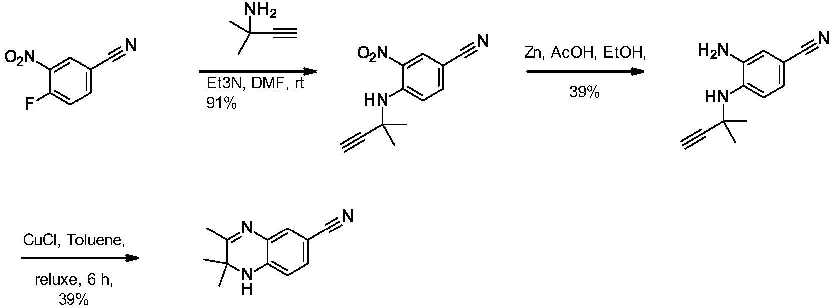

### Step 1: Synthesis of 2,2,3-trimethyl-1H-quinoxaline-6-carbonitrile

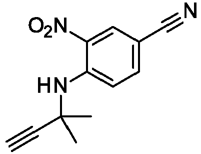

To a solution of 4-fluoro-3-nitro-benzonitrile (10.0 g, 60.2 mmol) and 2-methylbut-3-yn-2-amine (6.3 mL, 60.2 mmol) in DMF (60 mL), was added Et₃N (9.2 mL, 66.2 mmol) and the reaction was stirred at rt for 2 h. The volatiles were evaporated under reduced pressure and the residue was diluted with DCM. Water was added (20 mL) and the aqueous layer was extracted with DCM (3 x 60 mL). The combined organic layers were dried (MgSO₄), filtered and concentrated under reduced pressure. The resulting solid was triturated with Et₂O and filtered to afford title compound (12.5 g, 91%) as solid, which was used in the nest step without further purification. 1H NMR (500 MHz, DMSO) δ 8.57 (d, J = 2.0 Hz, 1H), 8.28 (s, 1H), 7.95 (dd, J = 9.1, 2.0 Hz, 1H), 7.64 (d, J = 9.1 Hz, 1H), 3.65 (s, 1H), 1.70 (s, 6H).

### Step 2: Synthesis of 2,2,3-trimethyl-1H-quinoxaline-6-carbonitrile

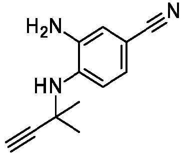

To a suspension of 4-(1,1-dimethylprop-2-ynylamino)-3-nitro-benzonitrile (5.00 g, 21.8 mmol) in EtOH (220.0 mL), were added AcOH (6.2 mL, 0.109 mmol) and Zn (7.13 g, 0.109 mmol) and the resulting mixture was stirred at rt for 4 h. The mixture was then filtered on Celite, washed and the filtrate was concentrated under reduced pressure. The residue was diluted with water (60 mL) and the aqueous layer was extracted with DCM (4 x 100 mL). The combined organic layers were dried (MgSO₄), filtered and concentrated under reduced pressure. The material was purified by column chromatography on silica gel using a gradient of 0-50% EtOAc in hexane to afford title compound (1.70 g, 39%) as a solid. ¹H NMR (500 MHz, CDCl₃) δ 7.29 (d, J = 8.4 Hz, 1H), 7.16 (dd, J = 8.4, 1.9 Hz, 1H), 6.97 (d, J = 1.9 Hz, 1H), 4.01 (s, 1H), 3.34 (s, 2H), 2.42 (s, 1H), 1.66 (s, 6H). LCMS m/z: ES+ [M+H]⁺ = 200.06; (B05) t_{R} = 1.68 min.

### Step 3: Synthesis of 2,2,3-trimethyl-1H-quinoxaline-6-carbonitrile

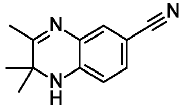

To a solution of 3-amino-4-(1,1-dimethylprop-2-ynylamino)benzonitrile (345 mg, 1.73 mmol) in toluene (3.5 mL), was added CuCl (86 mg, 0.87 mmol) and the reaction mixture was degassed with nitrogen for 5 min and then refluxed for 6 h. The mixture was cooled at rt and diluted with water (3.5 mL). The layers were separated, and the aqueous layer was extracted with EtOAc (3 x 10 mL). The combined organic layers were dried (MgSO₄), filtered and concentrated under reduced pressure. The material was purified by column chromatography on silica gel (24 g) using a gradient of 0-100% EtOAc in hexane to afford title compound (135 mg, 39%) as a solid. ¹H NMR (500 MHz, CDCl₃) δ 7.44 (d, J = 1.7 Hz, 1H), 7.24 (dd, J = 8.2, 1.9 Hz, 1H), 6.49 (d, J = 8.2 Hz, 1H), 4.04 (s, 1H), 2.17 (s, 3H), 1.37 (s, 6H). LCMS m/z: ES+ [M+H]⁺ = 200.05; (B05) t_{R} = 1.54 min.

### Example 48

### Synthesis of S-101

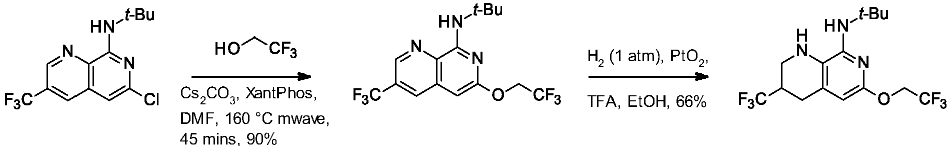

### Step 1: Synthesis of N-tert-butyl-6-(2,2,2-trifluoroethoxy)-3-(trifluoromethyl)-1,7-naphthyridin-8-amine

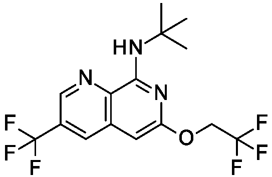

To a solution of N-tert-butyl-6-chloro-3-(trifluoromethyl)-1,7-naphthyridin-8-amine (100 mg, 0.296 mmol) in N,N-Dimethylformamide (1.27 mL) was successively added cesium carbonate (290 mg, 0.889 mmol) and BrettPhos (32 mg, 0.059 mmol). The resulting mixture was degassed by bubbling argon for 5 mins under stirring then 2,2,2-Trifluoroethanol (0.043 mL, 0.59 mmol) and Pd₂(dba)₃ (14 mg, 0.015 mmol) were added. The vial was sealed then stirred for 1 h at 160 °C in the microwave oven. The mixture was diluted with sat. aq. NaHCO₃ and extracted with EtOAc (3 x 5 mL). The combined organic layers were washed with brine, dried over Na₂SO₄, filtered, then concentrated. The residue obtained was purified by silica-gel column chromatography (0-100% DCM in hexanes) to afford the title compound (35 mg, 33 %) as an oil. ¹H NMR (500 MHz, CDCl₃) δ 8.62 (d, J = 1.9 Hz, 1H), 8.05 (s, 1H), 7.10 (s, 1H), 6.27 (s, 1H), 4.80 (q, J = 8.6 Hz, 2H), 1.59 (s, 9H). LC-MS m/z: ES+ [M+H]⁺ = 368.2, LCMS; t_{R} = 3.06 min.

### Step 2: Synthesis of N-tert-butyl-6-(2,2,2-trifluoroethoxy)-3-(trifluoromethyl)-1,2,3,4-tetrahydro-1,7-naphthyridin-8-amine

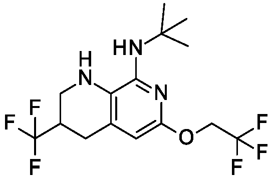

To a solution of N-tert-butyl-6-(2,2,2-trifluoroethoxy)-3-(trifluoromethyl)-1,7-naphthyridin-8-amine (33 mg, 0.09 mmol) in EtOH (1.65 mL) under argon at rt was added TFA (6 µL, 0.09 mmol) followed by PtO₂ (13 mg, 0.108 mmol). The mixture was hydrogenated under hydrogen atmosphere for 10 h. The mixture was degassed with nitrogen, then filtered on celite, rinsed with EtOH and the filtrate was concentrated under reduced pressure. The residue was purified by reversed phase gel column chromatography C18 (5.5 g) using a gradient of 10-100% acetonitrile in water (contains 0.1% formic acid) to afford the title compound (22 mg, 66%) as a solid. ¹H NMR (500 MHz, CDCl₃) δ 5.89 (s, 1H), 4.71 - 4.57 (m, 2H), 3.55 (d, J = 11.9 Hz, 1H), 3.06 (dd, J = 13.0, 10.4 Hz, 1H), 2.93 - 2.72 (m, 2H), 2.59 - 2.39 (m, 1H), 1.46 (s, 9H). LC-MS m/z: ES+ [M+H]⁺ = 372.1, LCMS; t_{R} = 3.16 min.

### Example 49

### Additional Syntheses

Structures of the following synthesized compounds are shown in Table 1 above.

### Synthesis of N²-(3- methyltetrahydrofuran-3-yl)-6-(3-pyridyl)-N³-tetrahydrofuran-3-yl-pyridine-2,3-diamine (L-42)

A solution of N²-(3-methyltetrahydrofuran-3-yl)-6-(3-pyridyl)pyridine-2,3-diamine (0.220 g, 0.81 mmol) in methanol (3 mL) was successively treated with tetrahydrofuran-3-one (0.14 g, 1.6 mmol, 2 eq) and then glacial acetic acid (93 uL, 1.6 mmol, 2eq). After 20 min, the reaction mixture was treated with sodium cyanoborohydride (77 mg, 1.2 mmol, 1.5 eq). After stirring overnight, LC/MS analysis showed clean conversion to the desired product. The reaction mixture was dried and purified by flash chromatography (4g silica, 0-10% methanol/methylene chloride) to afford N²-(3-methyltetrahydrofuran-3-yl)-6-(3-pyridyl)-N³-tetrahydrofuran-3-yl-pyridine-2,3-diamine (0.26 g, 0.277 g theor, 93%) as a brown viscous oil.

### Synthesis of N²-(3- methyltetrahydrofuran-3-yl)-6-(4-pyridyl)-N³-tetrahydrofuran-3-yl-pyridine-2,3-diamine (L-45)

A solution of N²-(3-methyltetrahydrofuran-3-yl)-6-(4-pyridyl)pyridine-2,3-diamine (0.155 g, 0.57 mmol) in methanol (3 mL) was successively treated with tetrahydrofuran-3-one (0.10 g, 1.15 mmol, 2 eq) and then acetic acid (66 uL, 1.15 mmol, 2 eq). After 10 min, the reaction mixture was then treated with sodium cyanoborohydride (55 mg, 0.86 mmol, 1.5 eq). After stirring overnight, LC/MS analysis showed clean conversion to the desired product. The reaction mixture was adsorbed onto silica (4g) and then purified by flash chromatography (12 g silica, 0-10% methanol/methylene chloride) to afford N²-(3-methyltetrahydrofuran-3-yl)-6-(4-pyridyl)-N³-tetrahydrofuran-3-yl- pyridine-2,3-diamine (0.115 g, 0.195 g theor, 58%) as a reddish-brown solid.

### Synthesis of N-(3- methyltetrahydrofuran-3-yl)-2-(2-pyridyl)-5,6,7,8-tetrahydropyrido[3,2-d]pyrimidin-4- amine (L-46)

A solution of N-(3-methyltetrahydrofuran-3-yl)-2-(2-pyridyl)pyrido[3,2-d]pyrimidin-4-amine (0.15 g, 0.49 mmol) in ethanol (2 mL) was treated with TFA (36 uL, 0.49 mmol, 1 eq) and then degassed with nitrogen by bubbling through the solution. The reaction mixture was then treated with Pt (IV) oxide (23 mg, 98 umol, 0.2 eq) and the solution was bubbled with hydrogen gas via balloon for 10 min. The needle was removed from the solution and the reaction mixture was stirred overnight under a balloon pressure of hydrogen gas. LC/MS analysis showed partial complete consumption of the starting material. The reaction mixture was filtered through Celite and the solvent was removed *in vacuo.* The residue was purified by flash chromatography (12 g silica, 0-10% methanol/methylene chloride) to afford N-(3-methyltetrahydrofuran-3-yl)-2-(2-pyridyl)-5,6,7,8-tetrahydropyrido[3,2-d]pyrimidin-4-amine (0.15 g, 0.152 g theor, 99%) as a reddish-brown solid.

### Synthesis of 2-(4-fluorophenyl)-N-(3-methyltetrahydrofuran- 3-yl)-5,6,7,8-tetrahydropyrido[3,2- d]pyrimidin-4-amine (M-14)

In a 40-mL vial, 2-(4-fluorophenyl)-N-(3-methyltetrahydrofuran-3-yl)pyrido[3,2-d]pyrimidin-4-amine (M-13, presumed to contain 0.245 g desired material) was stirred in ethanol (5 mL). To this was added 0.056 mL TFA. The solution was stirred and degassed by bubbling N₂ gas through the mixture. After 10 min, PtO₂ (0.0343 g, 0.2 eq) was added. The reaction mixture was again purged with nitrogen. A balloon of hydrogen was then added, and the reaction stirred at room temperature No reaction seen after 1 hr by LCMS. Minimal reaction after 4.5 hours. LCMS shows complete reaction after weekend. Reaction mix filtered and loaded onto silica for purification. Initial purification in hexanes/EtOAc left most of desired product stuck on column. Re-ran purification in DCM/methanol to elute desired product. Fractions 12-14 were dried down separately from fractions 15-17. Fractions 12-14: orange solid 0.0979 g; fractions 15-17: yellow glassy solid 0.1568 g. ¹H-NMR (400 MHz, DMSO-d6): δ 8.15 (m, 2H), 7.41 (m, 2H), 4.08 (d, 1H), 3.85 (m, 3H), 3.30 (m, 2H), 2.83 (m, 2H), 2.50 (m, 1H), 2.09 (m, 1H), 1.89 (m, 2H), 1.61 (s, 3H).

### Synthesis of 6-(4-fluorophenyl)-N²-(3-methyltetrahydrofuran-3-yl)-N³-tetrahydrofuran-3-yl-pyridine-2,3-diamine (M-23)

A vial was charged with 6-(4-fluorophenyl)-N2-(3-methyltetrahydrofuran-3-yl)pyridine-2,3-diamine (N-01, 0.06 g, 0.209 mmol) and methanol (2 mL). A stir bar, tetrahydrofuran-3-one (2 eq, 0.036 g, 0.418 mmol) and acetic acid (2 eq, 0.024 mL, 0.418 mmol) were added. After 20 min, sodium cyanoborohydride (1.5 eq., 0.0197 g, 0.313 mmol) was added. The reaction was stirred at room temperature overnight. LCMS at this time suggests predominant peak is desired product, with minor impurity peaks present. The reaction mixture was loaded directly onto a plug of silica, dried, and purified by column chromatography (0-100% Hex/EtOAc). Two dominant peaks, each containing desired product with trace impurity. Dried fractions 22-25 (42 mg) and 26-28 (32 mg) for total 74 mg. ¹H-NMR (400 MHz, DMSO-d6): δ 7.90 (m, 2H), 7.19 (m, 2H), 7.04 (d, 1H), 6.63 (d, 1H), 5.80 (m, 1H (NH)), 5.24 (m, 1H (NH)), 4.00 (m, 2H), 3.90 (m, 2H), 3.82 (m, 3H), 3.72 (m, 1H), 3.61 (m, 1H), 2.42 (m, 1H), 2.22 (m, 1H), 2.02 (m, 1H), 1.82 (m, 1H), 1.58 (s, 3H).

### Synthesis of 6-(4-fluorophenyl)-N²-(3-methyltetrahydrofuran-3-yl)-N³-tetrahydropyran-4-yl-pyridine-2,3-diamine (N-53)

A 40 mL vial was charged with 6-(4-fluorophenyl)-N²-(3-methyltetrahydrofuran-3-yl)pyridine-2,3-diamine (300 mg, 1.04 mmol) and a stir bar, tetrahydropyran-4-one (1.25 eq, 131 mg, 1.60 mmol), TFA (2.5 eq, 0.194 mL, 2.61 mmol), and isopropyl acetate(3 mL, 0.3 M) were added. To this was added sodium triacetoxyborohydride (2.5 eq, 553 mg, 2.61 mmol). The reaction was then allowed to stir at room temperature. After 20 minutes, the reaction mixture was made basic with the careful addition of sat. NaHCO₃ and then partitioned between 25 mL of water and 25 mL of EtOAc. The water layer was extracted twice with 15 mL EtOAc, dried over Na₂SO₄, filtered and concentrated under reduced pressure. The organic layer was concentrated to provide a grey solid that was recrystallized from MeOH to provide 60 mg of white solid. The remaining MeOH was concentrated. The residue was purified on silica gel (24 g, 0-100% EtOAc/hexanes) to provide a total of 220 mg of 6-(4-fluorophenyl)-N²-(3- methyl-tetrahydrofuran-3-yl)-N3-tetrahydropyran-4-yl-pyridine-2,3-diamine (388 mg theo., 58%) as a white solid. LCMS: 372.1 M+H+. ¹H NMR: δ 7.90 (t, 2H), 7.08 (m, 3H), 6.90 (t, 1H), 4.36 (bs, 1H), 4.14 (d, 1H), 3.98 (m, 5H), 3.52 (m, 2H), 3.45(bs, 1H), 2.88 (bs, 1H), 2.13 (m, 1H), 2.03 (m, 2H), 1.72 (s, 3H), 1.56 (m, 2H).

### Synthesis of N²-(3,3-difluoro-1-methyl-cyclobutyl)-6-(4-fluorophenyl)-N³-sec-butyl-pyridine-2,3-diamine (P-52)

N²-(3,3-difluoro-1-methyl-cyclobutyl)-6-(4-fluorophenyl)pyridine-2,3-diamine (163 mg) was dissolved in 10 ml of isopropyl acetate. 48 mg butan-2-one was added followed by 82 uL of TFA. The mixture was stirred at RT for 10-15 min and then sodium triacetoxyborohydride (147 mg) was added in 2 portions. The mixture was stirred at RT for 2 hrs. LC-MS indicated the reaction is complete. The reaction mixture was diluted with EtOAc and washed with water. The EtOAc was evaporated and the residue was run through a 24 g silica column with a gradient of DCM in hexane. LC-MS showed clean product, but the material is a dark blue tar. The material was dissolved in a small amount of dioxane and 0.5 ml of 4N HCl in dioxane was added and no precipitate percieved. The mixture was evaporated down to give a gray solid. NMR and LC-MS indicate the desired product in good purity.

### Synthesis of 4-[5-(cyclobutylamino)-6-[(3-methyltetrahydrofuran-3-yl)amino]-2-pyridyl]-N,N-dimethyl-benzamide (P-53)

100 mg N-03 was dissolved in 10 ml of isopropylacetate. 25 mg of cyclobutanone was added and the mixture was stirred at RT for 10 to 15 min. 44 uL of TFA was added and stirred was continued for an additional 10 to 15 min. 81 mgs of sodium triacetoxyborohydride was added in 2 portions. The reaction mixture was stirred at RT for 1.5 hrs. LC-MS indicated the reaction was complete. The reaction was diluted with EtOAc and washed with water. The EtOAc layer was evaporated down and run through a 12 g silica column. The product was eluted with a gradient of EtOAc in hexane to give 69 mg (60%) pale yellow solid.

### Synthesis of N³-cyclobutyl-6-(4-fluorophenyl)-N²-(3-methyltetrahydrofuran-3-yl)pyridine-2,3-diamine (P-54)

100 mg of N-01 was dissolved in 10 ml of isopropylacetate.

31 uL of cycolbutanone was added and the mixture was stirred at RT for 10 to 15 min. 52 uL of TFA was added and stirred was continued for an additional 10 to 15 min. 96 mgs of sodium triacetoxyborohydride was added in 2 portions and the mixture was stirred at RT for 1.5 hrs. LC-MS indicated the reaction was complete. The reaction mixture was diluted with EtOAc and washed with water. The EtOAc was evaporated down and the residue was run through a 24 g silica column. The product was eluted with a gradient of EtOAc in hexane to give 70 mg (59%) white solid.

### Synthesis of N-(3-methyltetrahydrofuran-3-yl)-2-(4-pyridyl)-5,6,7,8-tetrahydropyrido[3,2-d] pyrimidin-4-amine (P-71)

2-chloro-N-(3-methyltetrahydrofuran-3-yl)-5,6,7,8-tetrahydropyrido[3,2-d]pyrimidin-4-amine (120 mg) and 4-pyridylboronic acid (82 mg) were dissolved in a mixture of 10 ml of dioxane and 2 ml of water. The mixture was de-aerated by bubbling nitrogen through the solution for 15 min. 142 mg sodium carbonate was added, followed by 33 mg of Pd(dppf)Cl₂ - DCM. The mixture was heated in a microwave for 1 h at 100° C. LC-MS indicated the reaction was about 50% complete. The reaction was worked up by evaporating the solvent. The residue was run through a 24 g silica column the product was eluted with a MeOH in DCM gradient to give 32 mg final product.

### Synthesis of N-(3-methyltetrahydrofuran-3-yl)-2-(3-pyridyl)-5,6,7,8-tetrahydropyrido[3,2-d] pyrimidin-4-amine (P-72)

2-chloro-N-(3-methyltetrahydrofuran-3-yl)-5,6,7,8-tetrahydropyrido[3,2-d]pyrimidin-4-amine (120 mg) and 3-pyridylboronic acid (89 mg) were dissolved in a mixture of 10 ml of dioxane and 2 ml of water. The mixture was de-aerated by bubbling nitrogen through the solution for 15 min. 154 mg sodium carbonate was added, followed by 40 mgs of Pd(dppf)Cl₂-DCM. The mixture was heated in a microwave for 1 h at 100° C. LC-MS indicated the reaction to be about 50% complete. After heating for an additional 30 min, LC-MS showed the reaction to be about 60% complete. The reaction mixture was worked up by evaporating the solvents and running the residue through a 24 g silica column. The product was eluted with a MeOH/DCM gradient to give 37 mg final product.

### Example 50

### MTT assay in N27 Rat Dopaminergic Neural Cells

N27 rat dopaminergic neural cells (Millipore, SCC048) were cultured in RPMI-1640 media (Wisent, 350-000) supplemented with 1X GlutaMAX (Gibco, 35050-061), 10% FBS (embryonic stem cell qualified; Wisent, 920-040), and 1X Penicillin-Streptomycin (Wisent, 450-201). Cells were grown in humidified tissue culture incubator at 37°C with 5% CO₂, for up to 10 passages. To evaluate the ability of test compounds to inhibit RSL3-induced cell death, N27 cells were seeded in clear 96-well tissue culture plates (Sarstedt, 83.3924) at a density of 15,000 cells/well. On the next day, the medium was changed to fresh complete RPMI-1640. Eight-point dilution series of test compounds were added to the cells. Then, ferroptosis was induced by addition of 0.5 µM RSL3 (vehicle-treated cells served as control). Ferrostatin-1 (Fer-1) was included in each assay as control inhibitor of ferroptosis. Plates were incubated for 24 hours at 37°C in a tissue culture incubator, then cell viability was determined by MTT assay. Briefly, Thiazolyl Blue Tetrazolium Bromide [3-(4,5-dimethylthiazol-2-yl)-2,5-diphenyltetrazolium bromide or "MTT"] (Sigma) stock solution (5 mg/mL in PBS, sterilized by filtration) was diluted 1:1 with complete growth medium, and added on the cells to a final concentration of 0.5 mg/mL. Cells were incubated with MTT for 4 hours at 37°C, protected from light, in a humidified, 5% CO₂ atmosphere. Medium was then completely removed from the wells by gentle aspiration and converted MTT formazan crystals were dissolved in 150 µL of DMSO. Absorbance of converted dye was measured at 595 nm with background subtraction at 690 nm using the BioTek Cytation5 Microplate Reader. Blank reading (absorbance from wells containing media and vehicle only, no cells) was removed from all sample wells. Viability was calculated as a percentage of control cells (vehicle-treated cells were set as 100% viability). Dose-response curves were drawn and IC₅₀ values were calculated using GraphPad Prism software, version 7.

## Claims

1. A compound of formula II: 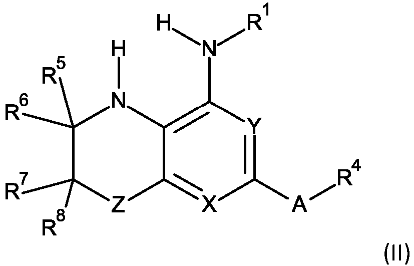 wherein
R¹ is selected from the group consisting of H, substituted or unsubstituted C₁-C₁₀ linear or branched alkyl, substituted or unsubstituted C₂-C₁₀ linear or branched alkenyl, substituted or unsubstituted C₂-C₁₀ linear or branched alkynyl, substituted or unsubstituted C₆-C₁₀ aryl, substituted or unsubstituted C₃-C₁₀ cycloalkyl, substituted or unsubstituted C₃-C₁₀ heterocycloalkyl, substituted or unsubstituted C₅-C₁₀ heteroaryl, substituted or unsubstituted C₆-C₁₀ arylalkyl, substituted or unsubstituted C₅-C₁₀ heteroarylalkyl, substituted or unsubstituted C₁-C₁₀ linear or branched alkylamino and substituted or unsubstituted C₁-C₁₀ linear or branched dialkylamino, or R¹ and its attached N together form a substituted or unsubstituted C₃-C₆ heterocycloalkyl or heteroaryl ring (replacing the H attached to the N);
A is selected from the group consisting of a bond, substituted or unsubstituted C₆-C₁₀ aryl, substituted or unsubstituted C₅-C₁₀ heteroaryl, substituted or unsubstituted C₂-C₁₀ linear or branched alkenyl, substituted or unsubstituted C₂-C₁₀ linear or branched alkynyl, C=O, C=S, -CH₂-, -CH(OH)-, -NH-, -N(CH₃)-, -O-, -S-, and SO₂; and
R⁴ is selected from the group consisting of substituted or unsubstituted C₁-C₁₀ linear or branched alkyl, substituted or unsubstituted C₁-C₁₀ linear or branched alkylamino, substituted or unsubstituted C₁-C₁₀ linear or branched dialkylamino, substituted or unsubstituted C₃-C₁₀ cycloalkyl or heterocycloalkyl, substituted or unsubstituted C₆-C₁₀ aryl, substituted or unsubstituted C₅-C₁₀ heteroaryl, -CN and halo;
R⁵, R⁶, R⁷, R⁸, R⁹ and R¹⁰ are independently selected from the group consisting of H, substituted or unsubstituted C₁-C₁₀ linear or branched alkyl, substituted or unsubstituted C₂-C₁₀ linear or branched alkenyl, substituted or unsubstituted C₂-C₁₀ linear or branched alkynyl, substituted or unsubstituted C₆-C₁₀ aryl, substituted or unsubstituted C₃-C₁₀ cycloalkyl, substituted or unsubstituted C₃-C₁₀ heterocycloalkyl, substituted or unsubstituted C₅-C₁₀ heteroaryl, substituted or unsubstituted C₆-C₁₀ arylalkyl, substituted or unsubstituted C₁-C₁₀ linear or branched alkylamino, and substituted or unsubstituted C₁-C₁₀ linear or branched dialkylamino, or R⁷ and R⁸ together are =O;
X and Y are independently selected from the group consisting of -CH- and -N-; and Z is selected from the group consisting of C=O, -CR⁹R¹⁰-, -NR⁹-, -O-, -S-, -S(O)- and - SO₂-;
and wherein a substituted group has 1, 2 or 3 -H atoms substituted by 1, 2 or 3 substituents selected from halo, trifluoromethyl, trifluoromethoxy, methoxy, -COOH, -CHO, -NH₂, -NO₂, -OH, -SH, -SMe, -NHCH₃, -N(CH₃)₂, -CN, and lower alkyl.

2. The compound of claim 1 wherein X and Y are-CH-, and Z is -CH₂-.

3. The compound of claim 1 wherein X and Y are -CH- and Z is O.

4. The compound of claim 1 selected from the group consisting of the compounds listed in Table 1.

5. The compound of claim 1 selected from the group consisting of

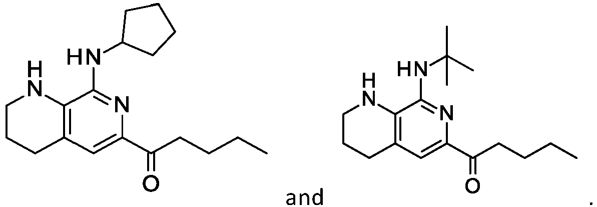

6. A pharmaceutical composition comprising a therapeutically effective amount of a compound of claim 1 and a pharmaceutically acceptable excipient.

7. A compound of formula II: 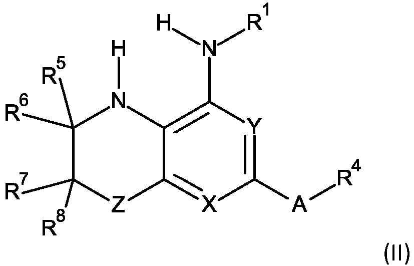 wherein
R¹ is selected from the group consisting of H, substituted or unsubstituted C₁-C₁₀ linear or branched alkyl, substituted or unsubstituted C₂-C₁₀ linear or branched alkenyl, substituted or unsubstituted C₂-C₁₀ linear or branched alkynyl, substituted or unsubstituted C₆-C₁₀ aryl, substituted or unsubstituted C₃-C₁₀ cycloalkyl, substituted or unsubstituted C₃-C₁₀ heterocycloalkyl, substituted or unsubstituted C₅-C₁₀ heteroaryl, substituted or unsubstituted C₆-C₁₀ arylalkyl, substituted or unsubstituted C₅-C₁₀ heteroarylalkyl, substituted or unsubstituted C₁-C₁₀ linear or branched alkylamino and substituted or unsubstituted C₁-C₁₀ linear or branched dialkylamino, or R¹ and its attached N together form a substituted or unsubstituted C₃-C₆ heterocycloalkyl or heteroaryl ring (replacing the H attached to the N);
A is selected from the group consisting of a bond, substituted or unsubstituted C₆-C₁₀ aryl, substituted or unsubstituted C₅-C₁₀ heteroaryl, substituted or unsubstituted C₂-C₁₀ linear or branched alkenyl, substituted or unsubstituted C₂-C₁₀ linear or branched alkynyl, C=O, C=S, -CH₂-, -CH(OH)-, -NH-, -N(CH₃)-, -O-, -S-, and SO₂; and
R⁴ is selected from the group consisting of substituted or unsubstituted C₁-C₁₀ linear or branched alkyl, substituted or unsubstituted C₁-C₁₀ linear or branched alkylamino, substituted or unsubstituted C₁-C₁₀ linear or branched dialkylamino, substituted or unsubstituted C₃-C₁₀ cycloalkyl or heterocycloalkyl, substituted or unsubstituted C₆-C₁₀ aryl, substituted or unsubstituted C₅-C₁₀ heteroaryl, -CN and halo;
R⁵, R⁶, R⁷, R⁸, R⁹ and R¹⁰ are independently selected from the group consisting of H, substituted or unsubstituted C₁-C₁₀ linear or branched alkyl, substituted or unsubstituted C₂-C₁₀ linear or branched alkenyl, substituted or unsubstituted C₂-C₁₀ linear or branched alkynyl, substituted or unsubstituted C₆-C₁₀ aryl, substituted or unsubstituted C₃-C₁₀ cycloalkyl, substituted or unsubstituted C₃-C₁₀ heterocycloalkyl, substituted or unsubstituted C₅-C₁₀ heteroaryl, substituted or unsubstituted C₆-C₁₀ arylalkyl, substituted or unsubstituted C₁-C₁₀ linear or branched alkylamino, and substituted or unsubstituted C₁-C₁₀ linear or branched dialkylamino, or R⁷ and R⁸ together are =O,;
X and Y are independently selected from the group consisting of -CH- and -N-; and Z is selected from the group consisting of C=O, -CR⁹R¹⁰-, -NR⁹-, -O-, -S-, -S(O)- and-SO₂-;
and wherein a substituted group has 1, 2 or 3 -H atoms substituted by 1, 2 or 3 substituents selected from halo, trifluoromethyl, trifluoromethoxy, methoxy, -COOH, -CHO, -NH₂, -NO₂, -OH, -SH, -SMe, -NHCH₃, -N(CH₃)₂, -CN, and lower alkyl for use in a method of treating a ferroptosis-related disease in a patient in need thereof comprising administering to the patient a therapeutically effective amount of the compound of formula II.

8. The compound for use of claim 7 wherein the ferroptosis-related disease is selected from the group consisting of lipid peroxidation-related degenerative diseases, excitotoxic diseases, neurodegenerative diseases, non-apoptotic regulated cell-death diseases, wasting- or necrosis-related diseases, intoxication-related diseases, and infectious diseases.

## Patentansprüche

1. Verbindung der Formel II: 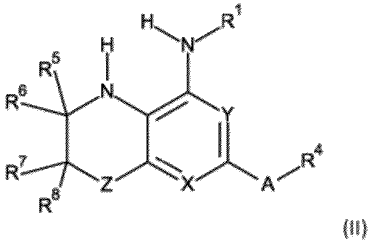 wobei
R¹ aus der Gruppe bestehend aus H, substituiertem oder unsubstituiertem linearem oder verzweigtem C₁-C₁₀-Alkyl, substituiertem oder unsubstituiertem linearem oder verzweigtem C₂-C₁₀-Alkenyl, substituiertem oder unsubstituiertem linearem oder verzweigtem C₂-C₁₀-Alkinyl, substituiertem oder unsubstituiertem C₆-C₁₀-Aryl, substituiertem oder unsubstituiertem C₃-C₁₀-Cycloalkyl, substituiertem oder unsubstituiertem C₃-C₁₀-Heterocycloalkyl, substituiertem oder unsubstituiertem C₅-C₁₀-Heteroaryl, substituiertem oder unsubstituiertem C₆-C₁₀-Arylalkyl, substituiertem oder unsubstituiertem C₅-C₁₀-Heteroarylalkyl, substituiertem oder unsubstituiertem linearem oder verzweigtem C₁-C₁₀-Alkylamino und substituiertem oder unsubstituiertem linearem oder verzweigtem C₁-C₁₀-Dialkylamino ausgewählt ist, oder R¹ und sein gebundenes N zusammen ein substituiertes oder unsubstituiertes C₃-C₆-Heterocycloalkyl oder einen Heteroarylring (ersetzt das an das N gebundene H) bilden;
A aus der Gruppe bestehend aus einer Bindung, substituiertem oder unsubstituiertem C₆-C₁₀-Aryl, substituiertem oder unsubstituiertem C₅-C₁₀-Heteroaryl, substituiertem oder unsubstituiertem linearem oder verzweigtem C₂-C₁₀-Alkenyl, substituiertem oder unsubstituiertem linearem oder verzweigtem C₂-C₁₀-Alkinyl, C=O, C=S, -CH₂-, -CH(OH)-, -NH-, -N(CH₃)-, -O-, -S- und SO₂ ausgewählt ist; und
R⁴ aus der Gruppe bestehend aus substituiertem oder unsubstituiertem linearem oder verzweigtem C₁-C₁₀-Alkyl, substituiertem oder unsubstituiertem linearem oder verzweigtem C₁-C₁₀-Alkylamino, substituiertem oder unsubstituiertem linearem oder verzweigtem C₁-C₁₀-Dialkylamino, substituiertem oder unsubstituiertem C₃-C₁₀-Cycloalkyl oder -Heterocycloalkyl, substituiertem oder unsubstituiertem C₆-C₁₀-Aryl, substituiertem oder unsubstituiertem C₅-C₁₀-Heteroaryl, -CN und Halogen ausgewählt ist;
R⁵, R⁶, R⁷, R⁸, R⁹ und R¹⁰ unabhängig aus der Gruppe bestehend aus H, substituiertem oder unsubstituiertem linearem oder verzweigtem C₁-C₁₀-Alkyl, substituiertem oder unsubstituiertem linearem oder verzweigtem C₂-C₁₀-Alkenyl, substituiertem oder unsubstituiertem linearem oder verzweigtem C₂-C₁₀-Alkinyl, substituiertem oder unsubstituiertem C₆-C₁₀-Aryl, substituiertem oder unsubstituiertem C₃-C₁₀-Cycloalkyl, substituiertem oder unsubstituiertem C₃-C₁₀-Heterocycloalkyl, substituiertem oder unsubstituiertem C₅-C₁₀-Heteroaryl, substituiertem oder unsubstituiertem C₆-C₁₀-Arylalkyl, substituiertem oder unsubstituiertem linearem oder verzweigtem C₁-C₁₀-Alkylamino und substituiertem oder unsubstituiertem linearem oder verzweigtem C₁-C₁₀-Dialkylamino, oder R⁷ ausgewählt sind, und R⁸ zusammen =O sind;
X und Y unabhängig aus der Gruppe bestehend aus -CH- und -N- ausgewählt sind; und Z aus der Gruppe bestehend aus C=O, -CR⁹R¹⁰-, -NR⁹-, -O-, -S-, -S(O)- und -SO₂-ausgewählt ist;
und wobei eine substituierte Gruppe 1, 2 oder 3 -H-Atome aufweist, die durch 1, 2 oder 3 Substituenten substituiert sind, die aus Halogen, Trifluormethyl, Trifluormethoxy, Methoxy, -COOH, -CHO, -NH₂, -NO₂, -OH, -SH, -SMe, -NHCH₃, -N(CH₃)₂,-CN und Niederalkyl ausgewählt sind.

2. Verbindung nach Anspruch 1, wobei X und Y -CH- sind und Z -CH₂- ist.

3. Verbindung nach Anspruch 1, wobei X und Y -CH- sind, und Z O ist.

4. Verbindung nach Anspruch 1, die aus der Gruppe ausgewählt ist, die aus den in Tabelle 1 aufgelisteten Verbindungen besteht.

5. Verbindung nach Anspruch 1, ausgewählt aus der Gruppe bestehend aus

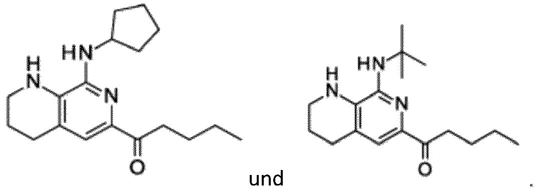

6. Pharmazeutische Zusammensetzung, die eine therapeutisch wirksame Menge einer Verbindung nach Anspruch 1 und einen pharmazeutisch verträglichen Hilfsstoff umfasst.

7. Verbindung der Formel II: 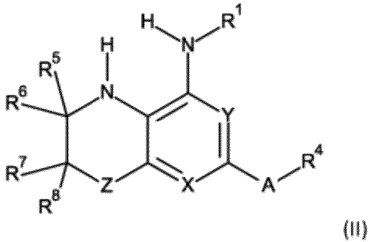 wobei
R¹ aus der Gruppe bestehend aus H, substituiertem oder unsubstituiertem linearem oder verzweigtem C₁-C₁₀-Alkyl, substituiertem oder unsubstituiertem linearem oder verzweigtem C₂-C₁₀-Alkenyl, substituiertem oder unsubstituiertem linearem oder verzweigtem C₂-C₁₀-Alkinyl, substituiertem oder unsubstituiertem C₆-C₁₀-Aryl, substituiertem oder unsubstituiertem C₃-C₁₀-Cycloalkyl, substituiertem oder unsubstituiertem C₃-C₁₀-Heterocycloalkyl, substituiertem oder unsubstituiertem C₅-C₁₀-Heteroaryl, substituiertem oder unsubstituiertem C₆-C₁₀-Arylalkyl, substituiertem oder unsubstituiertem C₅-C₁₀-Heteroarylalkyl, substituiertem oder unsubstituiertem linearem oder verzweigtem C₁-C₁₀-Alkylamino und substituiertem oder unsubstituiertem linearem oder verzweigtem C₁-C₁₀-Dialkylamino ausgewählt ist, oder R¹ und sein gebundenes N zusammen ein substituiertes oder unsubstituiertes C₃-C₆-Heterocycloalkyl oder einen Heteroarylring (ersetzt das an das N gebundene H) bilden;
A aus der Gruppe bestehend aus einer Bindung, substituiertem oder unsubstituiertem C₆-C₁₀-Aryl, substituiertem oder unsubstituiertem C₅-C₁₀-Heteroaryl, substituiertem oder unsubstituiertem linearem oder verzweigtem C₂-C₁₀-Alkenyl, substituiertem oder unsubstituiertem linearem oder verzweigtem C₂-C₁₀-Alkinyl, C=O, C=S, -CH₂-, -CH(OH)-, -NH-, -N(CH₃)-, -O-, -S- und SO₂ ausgewählt ist; und
R⁴ aus der Gruppe bestehend aus substituiertem oder unsubstituiertem linearem oder verzweigtem C₁-C₁₀-Alkyl, substituiertem oder unsubstituiertem linearem oder verzweigtem C₁-C₁₀-Alkylamino, substituiertem oder unsubstituiertem linearem oder verzweigtem C₁-C₁₀-Dialkylamino, substituiertem oder unsubstituiertem C₃-C₁₀-Cycloalkyl oder -Heterocycloalkyl, substituiertem oder unsubstituiertem C₆-C₁₀-Aryl, substituiertem oder unsubstituiertem C₅-C₁₀-Heteroaryl, -CN und Halogen ausgewählt ist;
R⁵, R⁶, R⁷, R⁸, R⁹ und R¹⁰ unabhängig aus der Gruppe bestehend aus H, substituiertem oder unsubstituiertem linearem oder verzweigtem C₁-C₁₀-Alkyl, substituiertem oder unsubstituiertem linearem oder verzweigtem C₂-C₁₀-Alkenyl, substituiertem oder unsubstituiertem linearem oder verzweigtem C₂-C₁₀-Alkinyl, substituiertem oder unsubstituiertem C₆-C₁₀-Aryl, substituiertem oder unsubstituiertem C₃-C₁₀-Cycloalkyl, substituiertem oder unsubstituiertem C₃-C₁₀-Heterocycloalkyl, substituiertem oder unsubstituiertem C₅-C₁₀-Heteroaryl, substituiertem oder unsubstituiertem C₆-C₁₀-Arylalkyl, substituiertem oder unsubstituiertem linearem oder verzweigtem C₁-C₁₀-Alkylamino und substituiertem oder unsubstituiertem linearem oder verzweigtem C₁-C₁₀-Dialkylamino, oder R⁷ ausgewählt sind, und R⁸ zusammen =O sind;
X und Y unabhängig aus der Gruppe bestehend aus -CH- und -N- ausgewählt sind; und Z aus der Gruppe bestehend aus C=O, -CR⁹R¹⁰-, -NR⁹-, -O-, -S-, -S(O)- und -SO₂-ausgewählt ist;
und wobei eine substituierte Gruppe 1, 2 oder 3 -H-Atome aufweist, die durch 1, 2 oder 3 Substituenten substituiert sind, die aus Halogen, Trifluormethyl, Trifluormethoxy, Methoxy, -COOH, -CHO, -NH₂, -NO₂, -OH, -SH, -SMe, -NHCH₃, -N(CH₃)₂,-CN und Niederalkyl ausgewählt sind, zur Verwendung in einem Verfahren zur Behandlung einer mit Ferroptose verbundenen Erkrankung bei einem Patienten, der diese benötigt, umfassend Verabreichung einer therapeutisch wirksamen Menge der Verbindung der Formel II an den Patienten.

8. Verbindung zur Verwendung nach Anspruch 7, wobei die mit Ferroptose verbundene Erkrankung aus der Gruppe bestehend aus mit Lipidperoxidation verbundenen degenerativen Erkrankungen, exzitotoxischen Erkrankungen, neurodegenerativen Erkrankungen, nicht-apoptotisch regulierten Zelltoderkrankungen, mit Auszehrung oder Nekrose verbundenen Erkrankungen, mit Intoxikation verbundenen Erkrankungen und Infektionskrankheiten ausgewählt ist.

## Revendications

1. Composé de formule II : 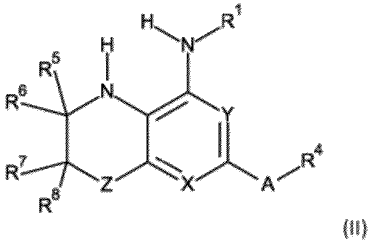 dans lequel
R¹ est choisi dans le groupe consistant en H, un alkyle linéaire ou ramifié en C₁-C₁₀ substitué ou non substitué, un alcényle linéaire ou ramifié en C₂-C₁₀ substitué ou non substitué, un alcynyle linéaire ou ramifié en C₂-C₁₀ substitué ou non substitué, un aryle en C₆-C₁₀ substitué ou non substitué, un cycloalkyle en C₃-C₁₀ substitué ou non substitué, un hétérocycloalkyle en C₃-C₁₀ substitué ou non substitué, un hétéroaryle en C₅-C₁₀ substitué ou non substitué, un arylalkyle en C₆-C₁₀ substitué ou non substitué, un hétéroarylalkyle en C₅-C₁₀ substitué ou non substitué, un alkylamino linéaire ou ramifié en C₁-C₁₀ substitué ou non substitué et un dialkylamino linéaire ou ramifié en C₁-C₁₀ substitué ou non substitué, ou R¹ et son N attaché forment ensemble un cycle hétérocycloalkyle ou hétéroaryle en C₃-C₆ substitué ou non substitué (remplaçant le H attaché au N) ;
A est choisi dans le groupe consistant en une liaison, un aryle en C₆-C₁₀ substitué ou non substitué, un hétéroaryle en C₅-C₁₀ substitué ou non substitué, un alcényle linéaire ou ramifié en C₂-C₁₀ substitué ou non substitué, un alcynyle linéaire ou ramifié en C₂-C₁₀ substitué ou non substitué, C=O, C=S, -CH₂-, -CH(OH)-, -NH-, -N(CH₃)-,-O-, -S- et SO₂ ; et
R⁴ est choisi dans le groupe consistant en un alkyle linéaire ou ramifié en C₁-C₁₀ substitué ou non substitué, un alkylamino linéaire ou ramifié en C₁-C₁₀ substitué ou non substitué, un dialkylamino linéaire ou ramifié en C₁-C₁₀ substitué ou non substitué, un cycloalkyle ou hétérocycloalkyle en C₃-C₁₀ substitué ou non substitué, un aryle en C₆-C₁₀ substitué ou non substitué, un hétéroaryle en C₅-C₁₀ substitué ou non substitué, -CN et halo ;
R⁵, R⁶, R⁷, R⁸, R⁹ et R¹⁰ sont indépendamment choisis dans le groupe consistant en H, un alkyle linéaire ou ramifié en C₁-C₁₀ substitué ou non substitué, un alcényle linéaire ou ramifié en C₂-C₁₀ substitué ou non substitué, un alcynyle linéaire ou ramifié en C₂-C₁₀ substitué ou non substitué, un aryle en C₆-C₁₀ substitué ou non substitué, un cycloalkyle en C₃-C₁₀ substitué ou non substitué, un hétérocycloalkyle en C₃-C₁₀ substitué ou non substitué, un hétéroaryle en C₅-C₁₀ substitué ou non substitué, un arylalkyle en C₆-C₁₀ substitué ou non substitué, un alkylamino linéaire ou ramifié en C₁-C₁₀ substitué ou non substitué, et un dialkylamino linéaire ou ramifié en C₁-C₁₀ substitué ou non substitué, ou R⁷ et R⁸ ensemble sont =O ;
X et Y sont indépendamment choisis dans le groupe consistant en -CH- et - N- ; et Z est choisi dans le groupe consistant en C=O, -CR⁹R¹⁰-, -NR⁹-, -O-, -S-, -S(O)- et - SO₂- ;
et dans lequel un groupe substitué présente 1, 2 ou 3 atomes -H substitués par 1, 2 ou 3 substituants choisis parmi halo, trifluorométhyle, trifluorométhoxy, méthoxy, -COOH, -CHO, -NH₂, -NO₂, -OH, -SH, -SMe, -NHCH₃, -N(CH₃)₂, -CN et alkyle inférieur.

2. Composé selon la revendication 1 dans lequel X et Y sont -CH-, et Z est -CH₂-.

3. Composé selon la revendication 1 dans lequel X et Y sont -CH-, et Z est O.

4. Composé selon la revendication 1 choisi dans le groupe consistant en les composés énumérés dans le Tableau 1.

5. Composé selon la revendication 1 choisi dans le groupe consistant en

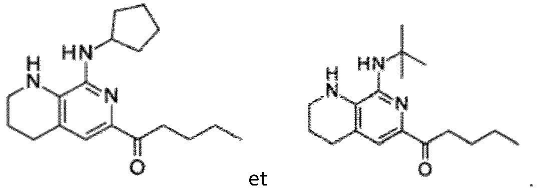

6. Composition pharmaceutique comprenant une quantité thérapeutiquement efficace d'un composé selon la revendication 1 et un excipient pharmaceutiquement acceptable.

7. Composé de formule II : 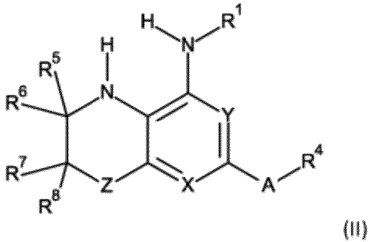 dans lequel
R¹ est choisi dans le groupe consistant en H, un alkyle linéaire ou ramifié en C₁-C₁₀ substitué ou non substitué, un alcényle linéaire ou ramifié en C₂-C₁₀ substitué ou non substitué, un alcynyle linéaire ou ramifié en C₂-C₁₀ substitué ou non substitué, un aryle en C₆-C₁₀ substitué ou non substitué, un cycloalkyle en C₃-C₁₀ substitué ou non substitué, un hétérocycloalkyle en C₃-C₁₀ substitué ou non substitué, un hétéroaryle en C₅-C₁₀ substitué ou non substitué, un arylalkyle en C₆-C₁₀ substitué ou non substitué, un hétéroarylalkyle en C₅-C₁₀ substitué ou non substitué, un alkylamino linéaire ou ramifié en C₁-C₁₀ substitué ou non substitué et un dialkylamino linéaire ou ramifié en C₁-C₁₀ substitué ou non substitué, ou R¹ et son N attaché forment ensemble un cycle hétérocycloalkyle ou hétéroaryle en C₃-C₆ substitué ou non substitué (remplaçant le H attaché au N) ;
A est choisi dans le groupe consistant en une liaison, un aryle en C₆-C₁₀ substitué ou non substitué, un hétéroaryle en C₅-C₁₀ substitué ou non substitué, un alcényle linéaire ou ramifié en C₂-C₁₀ substitué ou non substitué, un alcynyle linéaire ou ramifié en C₂-C₁₀ substitué ou non substitué, C=O, C=S, -CH₂-, -CH(OH)-, -NH-, -N(CH₃)-,-O-, -S- et SO₂ ; et
R⁴ est choisi dans le groupe consistant en un alkyle linéaire ou ramifié en C₁-C₁₀ substitué ou non substitué, un alkylamino linéaire ou ramifié en C₁-C₁₀ substitué ou non substitué, un dialkylamino linéaire ou ramifié en C₁-C₁₀ substitué ou non substitué, un cycloalkyle ou hétérocycloalkyle en C₃-C₁₀ substitué ou non substitué, un aryle en C₆-C₁₀ substitué ou non substitué, un hétéroaryle en C₅-C₁₀ substitué ou non substitué, -CN et halo ;
R⁵, R⁶, R⁷, R⁸, R⁹ et R¹⁰ sont indépendamment choisis dans le groupe consistant en H, un alkyle linéaire ou ramifié en C₁-C₁₀ substitué ou non substitué, un alcényle linéaire ou ramifié en C₂-C₁₀ substitué ou non substitué, un alcynyle linéaire ou ramifié en C₂-C₁₀ substitué ou non substitué, un aryle en C₆-C₁₀ substitué ou non substitué, un cycloalkyle en C₃-C₁₀ substitué ou non substitué, un hétérocycloalkyle en C₃-C₁₀ substitué ou non substitué, un hétéroaryle en C₅-C₁₀ substitué ou non substitué, un arylalkyle en C₆-C₁₀ substitué ou non substitué, un alkylamino linéaire ou ramifié en C₁-C₁₀ substitué ou non substitué, et un dialkylamino linéaire ou ramifié en C₁-C₁₀ substitué ou non substitué, ou R⁷ et R⁸ ensemble sont =O ;
X et Y sont indépendamment choisis dans le groupe consistant en -CH- et-N- ; et Z est choisi dans le groupe consistant en C=O, -CR⁹R¹⁰-, -NR⁹-, -O-, -S-, -S(O)- et-SO₂- ;
et dans lequel un groupe substitué présente 1, 2 ou 3 atomes -H substitués par 1, 2 ou 3 substituants choisis parmi halo, trifluorométhyle, trifluorométhoxy, méthoxy, -COOH, -CHO, -NH₂, -NO₂, -OH, -SH, -SMe, -NHCH₃, -N(CH₃)₂, -CN, et alkyle inférieur pour une utilisation dans un procédé de traitement d'une maladie liée à la ferroptose chez un patient en ayant besoin, comprenant l'administration au patient d'une quantité thérapeutiquement efficace du composé de formule II.

8. Composé pour utilisation selon la revendication 7, dans lequel la maladie liée à la ferroptose est choisie dans le groupe consistant en maladies dégénératives liées à la peroxydation lipidique, les maladies excitotoxiques, les maladies neurodégénératives, les maladies de mort cellulaire régulée non apoptotique, les maladies liées au dépérissement ou à la nécrose, les maladies liées à l'intoxication et les maladies infectieuses.
